# EUROPEAN PATENT SPECIFICATION

(11) **EP 2 163 629 B1**
(45) Date of publication and mention of the grant of the patent: **08.03.2017**
(21) Application number: 09179840.5
(22) Date of filing: 21.09.2000
(51) Int. Cl.: C12N 15/52, C12N 15/53, C12N 15/54, C12N 15/60, C12N 15/75, C12N 9/00, C12N 9/02, C12N 9/04, C12N 9/10, C12N 9/12, C12N 9/88, C12P 7/42, C12P 13/02, C12P 13/06, C12P 17/04

(54) **Methods and microorganisms for production of panto-compounds**
Verfahren und Mikroorganismen zur Produktion von Panto-Verbindungen
Procédés et micro-organismes de production de composés panto

(30) Priority: 21.09.1999 US 400494; 07.06.2000 US 210072 P; 28.07.2000 US 221836 P; 24.08.2000 US 227860 P
(43) Date of publication of application: 17.03.2010
(62) Divisional of application: 00966799.9
(73) Proprietor: BASF SE, 67056 Ludwigshafen am Rhein (DE)
(72) Inventor: Hermann, Theron, Kinnelon, NJ 07405 (US); Patterson, Thomas A., North Attleboro, MA 02760 (US); Pero, Janice, Lexington, MA 02420 (US); Yocum, Rogers R., Lexington, MA 02420 (US)

(56) References cited:
- EP-A- 0 224 294
- EP-A- 0 590 857
- WO-A-98/18954
- SAHM H ET AL.: "D-Pantothenate synthesis in Corynebacterium glutamicum and use of panBC and genes encoding L-valine synthesis for D-pantothenate overproduction" APPLIED AND ENVIRONMENTAL MICROBIOLOGY, vol. 65, no. 5, May 1999 (1999-05), pages 1973-1979, XP002169517
- SOROKIN A ET AL.: "Sequence analysis of the Bacillus subtilis chromosome region between the serA and kdg loci cloned in a yeast artificial chromosome" MICROBIOLOGY, vol. 142, no. 8, 1996, pages 2005-2016, XP000910121 ISSN: 1350-0872
- DATABASE EM_PRO EMBL; ID BSYPIA, AC L47709 23 January 1996 (1996-01-23), HENNER D ET AL.: "Bacillus subtilis (clone YAC15-6B) ypiABF genes, qcrABC genes, ypjABCDEFGHI genes, birA gene, panBCD genes, dinG gene, ypmB gene, aspB gene, asnS gene, dnaD gene, nth gene and ypoC gene, complete cds." XP002171539
- BAIGORI M ET AL.: "Isolation and characterization of Bacillus subtilis mutants blocked in the syntehsis of pantothenic acid" JOURNAL OF BACTERIOLOGY, vol. 173, no. 13, July 1991 (1991-07), pages 4240-4242, XP001002216
- MIYAKE K ET AL: "Enzymological properties of pantothenate synthetase EC-6.3.2.1 from Escherichia coli B", JOURNAL OF NUTRITIONAL SCIENCE AND VITAMINOLOGY, UNIVERSITY OF TOKYO PRESS, TOKYO, JP, vol. 24, no. 3, 1 January 1978 (1978-01-01), pages 243-253, XP002092394, ISSN: 0301-4800

## Description

### Background

Pantothenate, also known as pantothenic acid or vitamin B5, is a member of the B complex of vitamins and is a nutritional requirement for mammals, including livestock and humans (e.g., from food sources, as a water soluble vitamin supplement or as a feed additive). In cells, pantothenate is used primarily for the biosynthesis of coenzyme A (CoA) and acyl carrier protein (ACP). These coenzymes function in the metabolism of acyl moieties which form thioesters with the sulfhydryl group of the 4'-phosphopantetheine portion of these molecules. These coenzymes are essential in all cells, participating in over 100 different intermediary reactions in cellular metabolism.

The conventional means of synthesizing pantothenate (in particular, the bioactive D isomer) is *via* chemical synthesis from bulk chemicals, a process which is hampered by excessive substrate cost as well as the requirement for optical resolution of racemic intermediates (e.g., resolution of DL-pantolactone to obtain D-pantolactone for chemical condensation with β-alanine). Accordingly, researchers have recently looked to bacterial or microbial systems that produce enzymes useful in pantothenate biosynthesis processes (as bacteria are themselves capable of synthesizing pantothenate). In particular, bioconversion processes have been evaluated as a means of favoring production of the D isomer of pantothenic acid, e.g., using microorganisms which selectively hydrolyze a DL-pantothenic acid ester to D-pantothenic acid; microorganisms which selectively decompose L-pantolactone resulting in D-pantolactone alone; and microorganisms which selectively hydrolyze DL-pantolactone to D-pantoic acid.

There is still, however, significant need for improved pantothenate production processes, in particular, for processes requiring reduced quantities of substrates and/or less expensive substrates. To this end, methods of direct microbial synthesis have recently been examined as a means of improving D-pantothenate production. In microbes, pantothenate biosynthetis is a multistep pathway resulting in condensation of pantoate (derived from α-ketoisovalerate) and β-alanine to form D-pantothenate. The isoleucine-valine (*ilv*) pathway biosynthetic enzymes, acetohydroxyacid synthetase (the *ilvBN* or *alsS* gene product), acetohydroxyacid isomeroreductase (the *ilvC* gene product) and dihydroxyacid dehydratase (the *ilvD* gene product) catalyze the conversion of pyruvate to α-ketoisovalerate. The reactions are further catalyzed by the pantothenate (*pan*) pathway biosynthetic enzymes ketopantoate hydroxymethyltransferase (the *panB* gene product), ketopantoate reductase (the *panE* gene product), aspartate-α-decarboxylase (the *panD* gene product) and pantothenate synthetase (the *panC* gene product).

The genes encoding the enzymes involved in the biosynthesis of pantothenic acid in *Salmonella typhimurium* and *Escherichia coli* have recently been identified and characterized (Frodyma and Downs (1998) J. Biol. Chem. 273:5572-5576 and Jackowski (1996) pp. 687-694, In Neidhardt et al (ed.) Escherichia coli and Salmonella: Cellular and Molecular Biology, 2nd ed. Am. Soc. Microbiol. Wash, D.C). In *E. coli,* for example, the biosynthesis of pantothenic acid consists of four key steps. The first reaction is catalyzed by the *panB* gene product, ketopantoate hydroxymethyltransferase, and uses the L-valine intermediate α-ketoisovalerate to generate ketopantoate, which is subsequently reduced to pantoate by the *panE* gene product, ketopantoate reductase. The *panD* gene product, aspartate-α-decarboxylase, generates β-alanine from aspartate. The *panC* gene product, pantothenate synthetase, subsequently ligates β-alanine with pantoate to yield D-pantothenate.

The authors Dusch *et al.* described the identification of the *Corynebacterium glutamicum panD* gene and reported that expression of the C. *glutamicum panD* gene in *E. coli* yielded a strain producing pantothenate with a specific productivity of 140 ng of pantothenate per mg (dry weight) per hour. (Dusch et al. (1999) Appl. Environ. Microbial 65:1530-1539).

The authors Sahm and Eggeling have further identified the *Corynebacterium glutamicum panB* and *pan* C genes and have described a genetically engineered strain of C. *glutamicum* which overexpresses the *panBC* genes (Sahm and Eggeling (1999) Appl. Environ. Microbiol. 65:1973-1979). The engineered strain produces pantothenate, however, it was necessary to overexpress the genes responsible for a-ketoisovalerate production in the host organism in order that pantothenic acid production could be detected. Moreover, without the addition of β-alanine, no substantial amounts of pantothenate accumulated with the strain constructed.

The production of D-pantoic-acid and D-pantothenic acid comprising culturing *E. coli* which overexpress *E.coli panB, panC and panD* genes is disclosed in EP 0590857 A. Neither this application nor Sahm and Eggeling disclose further
combination of overexpressed enzymes for the production of panto-compounds, especially without supplementation of beta-alanine.

Likewise, a method of producing D-pantothenic acid has been described that takes advantage of a sodium salicylate resistant mutant strain of *E. coli* which produces D-pantothenic acid when cultured in the presence of β-alanine (U.S. Patent No. 5,518,906). Generation of *E. coli* strains resistant to α-ketoisovaleric acid and/or α-ketobutyric acid, and/or α-aminobutyric acid, and/or β-hydroxyaspartic acid and/or O-methyl-threonine, in addition to salicylic acid, further increased pantothenic acid production. Moreover, transformation of a plasmid DNA carrying the *panB*, *panC* and *panD* genes into the salicylic acid resistant mutant strain resulted in increased pantothenate production, however, up to 20 g/L β-alanine or more was fed in the examples given. The *panB-panC-panD* genes are clustered on the *E. coli* chromosome.

Finally, a method of producing D-pantothenic acid has been described which utilizes a salicylic acid-resistant, α-ketoisovalerate-resistant, α-ketobutyrate-resistant, β-hydroxyaspartate-resistant, o-methylthreonine-resistent *E. coli* strain transformed with pantothenate biosynthesis gene-containing DNA fragments and/or branched amino acid biosynthesis gene-containing DNA fragments and cultured in the presence of β-alanine (U.S. Patent No. 5,932,457).

Pantothenate production in bacteria results from the condensation of pantoate and β-alanine and involves the pantothenate biosynthetic enzymes ketopantoate hydroxymethyltransferase (the *panB* gene product), ketopantoate reductase (the *panE* gene product), aspartate-α-decarboxylase (the *panD* gene product) and pantothenate synthetase (the *panC* gene product). Although pantothenate is biologically active as a vitamin, it is further metabolized in all cells to Coenzyme A (CoA) which participates as an acyl group carrier in the tricarboxylic acid (TCA) cycle, fatty acid metabolism and numerous other reactions of intermediary metabolism. The initial (and possibly rate-controlling) step in the conversion of pantothenate to Coenzyme A (CoA) is phosphorylation of pantothenate by pantothenate kinase. A pantothenate kinase activity was first identified in *Salmonella typhimurium* by screening for temperature-sensitive mutants which synthesized CoA at permissive temperatures but excreted pantothenate at non-permissive temperatures. The mutations were mapped in the *Salmonella* chromosome and the genetic locus was designated *coaA.* The gene encodes the enzyme that catalyzes the first step in the biosynthesis of coenzyme A from pantothenate (Dunn and Snell (1979) J. Bacteriol 140:805-808). *Escherichia coli* temperature sensitive mutants have also been isolated and characterized (Vallari and Rock (1987) J. Bacteriol 169:5795-5800). These mutants (named *coaA15(Ts)*) are defective in the conversion of pantothenate to CoA and further exhibit a temperature-sensitive growth phenotype, indicating that pantothenate kinase activity is essential for growth. Moreover, it was noted that CoA inhibited pantothenate kinase activity to the same degree in the mutant as compared to the wild-type enzyme.

Feedback resistant *E. coli* mutants (named *coaA16(Fr)*) have also been isolated that posses a pantothenate kinase activity that is refractory to feedback inhibition by CoA (Vallari and Jackowski (1988) J. Bacteriol. 170:3961-3966). The mutation responsible for the reversion is, suprisingly, not genetically linked to the *coaA* gene by transduction. Additional data described therein support the view that the total cellular CoA content is controlled by both modulation of biosynthesis at the pantothenate kinase step and possibly by degradation of CoA to 4'-phosphopantetheine.

The wild-type *E. coli coaA* gene was cloned by functional complementation of *E. coli* temperature-sensitive mutants. The sequence of the wild-type gene was determined (Song and Jackowski (1992) J. Bacteriol. 174:6411-6417 and Flamm et al. (1988) Gene (Amst.) 74:555-558). Strains containing multiple copies of the *coaA* gene possessed 76-fold higher specific activity of pantothenate kinase, however, there was only a 2.7-fold increase in the steady state level of CoA (Song and Jackowski, *supra*)*.* It has further been reported that the prokaryotic enzyme (encoded by *coaA* in *E.coli* and a variety of other microorganisms) is feedback inhibited by CoA both *in vivo* and *in vitro* with CoA being about five times more potent than acetyl-CoA in inhibiting the enzyme (*Song and Jackowski, supra* and Vallari *et al., supra*). Moreover, it has been reported that the *panB* gene product in *E. coli* is inhibited by CoA (Powers and Snell (1976) J. Biol. Chem. 251:3786-3793). These data further support the view that feedback inhibition of pantothenate kinase activity is a critical factor controlling intracellular CoA concentration.

Using standard search and alignment tools, *coaA* homologues have been identified in *Hemophilus influenzae, Mycobacterium tuberculosis, Vibrio cholerae, Streptococcus pyogenes* and *Bacillus subtilis.* By contrast, proteins with significant similarity could not be identified in eukaryotic cells including *Saccharomyces cerevisiae* or in mammalian expressed sequence tag (EST) databases. Using a genetic selection strategy, a cDNA encoding pantothenate kinase activity has recently been identified from *Aspergillus nidulans* (Calder et al. (1999) J. Biol. Chem. 274:2014-2020). The eukaryotic pantothenate kinase gene (*panK*) has distinct primary structure and unique regulatory properties that clearly distinguish it from its prokaryotic counterpart. A mammalian pantothenate kinase gene (*mpanK1α*) has also been isolated which encodes a protein having homology to the *A. nidulans* PanK protein and to the predicted gene product of GenBank™ Accession Number 927798 identified in the S. *cerevisiae* genome (Rock et al. (2000) J. Biol. Chem. 275:1377-1383).

### Summary

The present disclosure is based, at least in part, on the discovery of key enzyme-encoding genes of the pantothenate biosynthetic pathway in *Bacillus subtilis.* In particular, the present inventors have identified the *panE* gene of *B. subtilis.* Overexpression or deregulation of the *panE* gene in *B. subtilis* results in enhanced production of the *panE* gene product, ketopantoate reductase, further resulting in increased production of pantothenate. Likewise, mutations in this gene reduce pantothenate production in *B. subtilis* >90%. The present inventors have further identified the presumptive *panBCD* operon in *B. subtilis,* overexpression or deregulation of which results in increased pantothenate production. The present inventors have further demonstrated that overexpression or deregulation of the *panD* gene in *B. subtilis* (resulting in enhanced production of the *panD* gene product, aspartate-α-decarboxylase) further results in increased production of pantothenate, in particular, in combination with deregulation of genes encoding key enzymes of the isoleucine-valine (*ilv*) biosynthetic pathway.

The present invention is directed to a method of producing a panto-compound comprising culturing a manipulated microorganism which overexpresses (i) at least Bacillus pantothenate synthetase; and further (ii) ketopantoate hydroxymethyltransferase, and aspartate-alpha-decarboxylase at a level greater than that expressed prior to manipulation of said microorganism or in a comparable microorganism which has not been manipulated, under conditions such that the panto-compound is produced, wherein the method is without supplementation of beta-alanine.
In one embodiment the microorganism further overexpresses ketopantoate reductase.

Accordingly, the present disclosure features methods of producing pantothenate, as well as other compounds of the pantothenate biosynthetic pathway (e.g., ketopantoate, pantoate and β-alanine), termed "panto-compounds" herein, using microorganisms in which the pantothenate biosynthetic pathway and/or isoleucine-valine biosynthetic pathway has been manipulated such that pantothenate or other desired panto-compounds are produced. In one embodiment, the disclosure features a method of producing a panto-compound (e.g., pantothenate or pantoate) that involves culturing a microorganism which overexpresses the *panE* gene product, ketopantoate reductase, also referred to herein as a ketopantoate reductase-overexpressing or "KPAR-O" microorganism, under conditions such that the panto-compound (*e.g*., pantothenate or pantoate) is produced. In another embodiment, the present disclosure features a method of producing panto-compounds (*e.g*., pantothenate or pantoate) which includes culturing a microorganism which overexpresses at least one pantothenate biosynthetic enzyme (*e.g*., at least one of the *panB, panC* or *panD* gene products), preferably in a KPAR-O microorganism, under conditions such that the panto-compound (*e.g*., pantothenate or pantoate) is produced.

Yet another aspect of the disclosure features methods of producing panto-compounds which are independent of the need to feed precursors (*e.g*., β-alanine or aspartate and/or α-ketoisovalerate or valine). In one embodiment, the disclosure features a method of producing pantothenate in a manner independent of precursor feed that includes culturing an aspartate-α-decarboxylase-overexpressing (AαD-O) microorganism having a deregulated isoleucine-valine (*ilv*) pathway under conditions such that pantothenate is produced. In another embodiment, the disclosure features a method of producing pantothenate in a manner independent of precursor feed that includes culturing an AαD-O microorganism having a deregulated pantothenate (*pan*) pathway and a deregulated isoleucine-valine (*ilv*) pathway, under conditions such that pantothenate is produced. In another embodiment, the disclosure features a method of producing pantothenate in a manner independent of aspartate or β-alanine feed that includes culturing an AαD-O microorganism under conditions such that pantothenate is produced. In another embodiment, the disclosure features a method of producing pantothenate in a manner independent of valine or α-ketoisovalerate feed that includes culturing a microorganism having a deregulated isoleucine-valine (*ilv*) biosynthetic pathway under conditions such that pantothenate is produced. In yet another embodiment, the disclosure features a high yield production method for producing pantothenate that includes culturing a manipulated microorganism under conditions such that pantothenate is produced at a significantly high yield *(e.g.,* at a level greater than 10 g/L, 20 g/L, 30 g/L or 40g/L).

The methods of the present disclosure further feature microorganisms that overexpresses acetohydroxyacid synthetase or acetohydroxyacid isomeroreductase (*e.g*., microorganisms transformed with a vector that includes an *ilvBNC* nucleic acid sequence), microorganisms that overexpresses dihydroxyacid dehydratase (*e.g*., microorganisms transformed with a vector that includes an *ilvD* nucleic acid sequence), microorganisms that overexpresses aspartate-α-decarboxylase (*e.g*., microorganisms transformed with a vector that includes a *panD* nucleic acid sequence), microorganisms having a deregulated isoleucine-valine (*ilv*) biosynthetic pathway and microorganisms having a deregulated pantothenate biosynthetic pathway (*e.g*., microorganisms that overexpress any of ketopantoate hydroxymethyltransferase, ketopantoate reductase, pantothenate synthetase and aspartate-α-decarboxylase, for example, microorganisms transformed with a vector comprising a *panBCD* nucleic acid sequence or a vector comprising a *panE1* nucleic acid sequence). In one embodiment, the recombinant microorganism is Gram positive (*e.g*., microorganisms belonging to the genus *Bacillus, Cornyebacterium, Lactobacillus, Lactococci* or *Streptomyces*). In another embodiment, the recombinant microorganism is Gram negative. Particularly preferred is a *Bacillus* recombinant microorganism (*e.g*., *Bacillus licheniformis, Bacillus amyloliquefaciens, Bacillus subtilis, Bacillus pumilus, Bacillus halodurans,* and the like). Recombinant vectors that contain the genes encoding *Bacillus* pantothenate and/or isoleucine-valine biosynthetic enzymes (*e.g*., *B. subtilis* pantothenate and/or isoleucine-valine biosynthetic enzymes) are also described.

Also featured are methods of producing β-alanine that include culturing an aspartate-α-decarboxylase-overexpressing (AαD-O) microorganism under conditions such that β-alanine is produced and methods of producing β-alanine that involve contacting a composition comprising aspartate with an isolated *Bacillus* aspartate-α-decarboxylase enzyme under conditions such that β-alanine is produced.

The production methods of the present disclosure further can include recovering the panto-compound (*e.g*., pantothenate or pantoate).

The present disclosure further features recombinant microorganisms (*e.g*., AαD-O microorganisms, microorganisms having a deregulated isoleucine-valine (*ilv*) pathway, microorganisms overexpressing at least one of ketopantoate hydroxymethyltransferase (the *panB* gene product), pantothenate synthetase (the *panC* gene product), aspartate-α-decarboxylase (the *panD* gene product), ketopantoate reductase (the *panE1* gene product) and microorganisms having a deregulated *panBCD* operon. Also featured are *panB, panC, panD, panE, ilvB, ilvN, alsS, ilvC,* and/or *ilvD* nucleic acid molecules, as well as vectors including such nucleic acid molecules and gene products encoded by such nucleic acid molecules.

The methodology of the present disclosure further includes, for example in addition to overexpressing at least one pantothenate biosynthetic enzyme, deleting or mutating a second pantothenate biosynthetic enzyme, said second pantothenate biosynthetic enzyme preferably being downstream of the desired product in the pantothenate biosynthetic pathway. For example, mutating *panC,* in addition to overexpressing the *panE* gene product, results in even further enhanced or increased production of pantoate. Accordingly, in one embodiment, the disclosure features a method of producing pantoate which includes culturing a microorganism which overexpresses the *panE* gene product and which has a deletion in the *panC* gene. In another embodiment, the disclosure features a method of producing pantoate which includes culturing a microorganism which overexpresses the *panE* gene product and/or *panB* gene product and which has a deletion in the *panC* gene. Other exemplary embodiments include a method of producing ketopantoate which includes culturing a microorganism which overexpresses the *panB* gene product and which has a deletion in the *panE* gene and a method of producing β-alanine which includes culturing a microorganism which overexpresses the *panD* gene product and which has a deletion in the *panC* gene. Also included are methods of producing panto-compounds which include overexpressing at least one valine biosynthetic enzyme in a microorganism which has at least one pantothenate biosynthetic enzyme deleted.

The present disclosure is also based at least in part, on the identification and characterization of a previously unidentified microbial pantothenate kinase gene, *coaX. CoaX* was first identified in *Bacillus subtilis* and corresponds to an open reading frame in a portion of the chromosomal DNA that includes the 5' end of the *ftsH* gene, and all of the *yacB, yacC, yacD, cysK* and *pabB* genes. The present inventors have demonstrated that the *yacB* open reading frame encodes a novel pantothenate kinase activity, the gene being unrelated by homology to any previously known pantothenate kinase gene. The gene has been renamed *coaX,* as it encodes the enzyme which catalyzes the first step in the pathway from pantothenate to CoaA.

Accordingly, the present disclosure features new and improved methods of producing pantothenate and other key compounds of the pantothenate biosynthetic pathway (e.g., panto-compounds) utilizing microorganisms having modified pantothenate kinase activity. In particular, the present disclosure features recombinant microorganisms that contain the *coaX gene* or that contain a mutant *coaX gene,* having reduced pantothenate kinase activity. In one embodiment, the disclosure features such recombinant microorganisms further having a deregulated pantothenate biosynthetic pathway. In another embodiment, the disclosure features such recombinant microorganisms further having a deregulated isoleucine-valine (*ilv*) pathway. In a preferred embodiment, the microorganisms belong to the genus *Bacillus* (*e.g*., *B. subtilis*)*.*

The present disclosure also features recombinant microorganisms (e.g., microorganisms belonging to the genus *Bacillus,* for example, *B. subtilis*) that contain the *coaA* gene or that contain a mutant *coaA* gene, optionally including a *coaX* gene or mutant thereof, having reduced pantothenate kinase activity. In one embodiment, the disclosure features such recombinant microorganisms further having a deregulated pantothenate biosynthetic pathway or having a deregulated isoleucine-valine (*ilv*) pathway.

Also featured are vectors that contain isolated *coaX* or *coaA* genes as well as mutant *coaX* and/or *coaA* genes. Isolated nucleic acid molecules that contain isolated *coaX* genes or mutant *coaX* genes are featured in addition to isolated CoaX proteins and mutant CoaX proteins.

The nucleic acids, vectors and recombinant microorganisms described above are particularly useful in the methodologies of the present disclosure. In particular, the disclosure features methods of enhancing panto-compound production (e.g., ketopantoate, pantoate and or pantothenate production) that include culturing a recombinant microorganism having a mutant *coaX* gene under conditions such that panto-compound production is enhanced. In one embodiment, the recombinant microorganism further includes a mutant *coaA* gene. In another embodiment, the recombinant microorganism further includes a mutant *avtA* and/or mutant *ilvE* gene and/or mutant *ansB* gene and/or mutant *alsD* gene. Also featured are methods for identifying pantothenate modulators utilizing the recombinant microorganisms and purified CoaX proteins of the present disclosure.

Other features and advantages of the disclosure will be apparent from the following detailed description and claims.

### Brief Description of the Drawings

*Figure 1* is a schematic representation of the pantothenate biosynthetic pathway.
*Figure* 2 is a schematic representation of the plasmid pAN240, containing sequences ligated upstream of the *P₂₆panBCD* cassette, equivalent to the integrated version in strain PA221.
*Figure 3A* is a schematic representation of the plasmid pAN004, containing the *panBCD* operon expressed from *P₂₆* and RBS 1.
*Figure 3B* is a schematic representation of the plasmid pAN006, containing the *panBCD* operon expressed from *P₂₆* and RBS2.
*Figure 4* is a schematic representation of the plasmid pAN236, containing an integratable and amplifiable *P₂₆*-RBS2-*panE1* expression cassette.
*Figure 5* is a schematic representation of the construction of plasmid pAN423.
*Figure 6* is a schematic representation of the construction of plasmids pAN426 and pAN427.
*Figure 7* is a schematic representation of the construction of plasmids pAN428 and pAN429.
*Figure 8* is a schematic representation of the construction of plasmid pAN431.
*Figure 9* is a schematic representation of the construction of plasmid pAN441.
*Figure 10* is a schematic representation of the construction of plasmid pAN440.
*Figure 11* is a schematic representation of the plasmid pAN251 designed to integrate a single copy of a *P₂₆₋panE1* cassette at the *panE1* locus by double crossover.
*Figure 12* is a schematic representation of the plasmid pAN267 designed to integrate a single copy of a *P₂₆-ilvBNC* cassette at the *amyE* locus.
*Figure 13* is a schematic representation of the plasmid pAN257, a clone of *Bacillus subtilis ilvD* in a low copy vector.
*Figure 14* is a schematic representation of the plasmid pAN263, designed to integrate a single copy of a *P₂₆-ilvD* cassette at the *ilvD* locus.
*Figure 15* is a schematic representation of the plasmid pAN261, designed to disrupt the *Bacillus subtilis ilvD* gene with the *cat* gene.
*Figure 16* is a schematic representation of the Coenzyme A biosynthetic pathway in *E. coli.*
*Figure 17* is a schematic representation of the structure of pAN296, a plasmid designed to delete most of the *B. subtilis coaA* gene and substitute a chloramphenicol resistance gene.
*Figure 18* is a schematic representation of the structure of the *Bacillus subtilis* genome in the region of the *coaA* gene. The scale is in base pairs and the significant open reading frames are shown by open arrows.
*Figure 19* is a schematic representation of the plasmid pAN281, a plasmid for expressing *Bacillus subtilis coaA* after integration at the *bpr* locus.

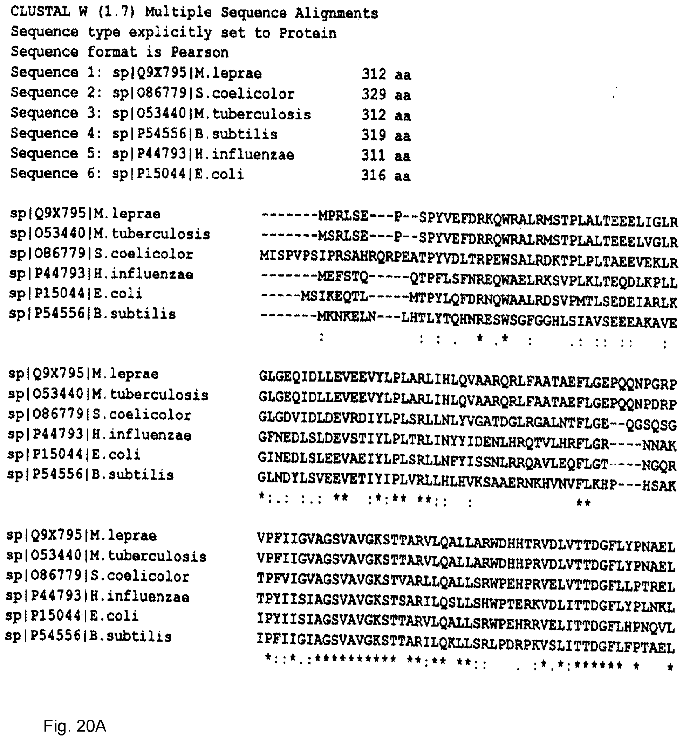

*Figure 20A-B* depicts a multiple sequence alignment (MSA) of the amino acid sequences encoded by six known or predicted microbial *coaA* genes. SEQ ID NOs:4-6 and 1-3 correspond to the amino acid sequences of *Mycobacterium leprae* (SwissProt™ Accession No. Q9X795), *Mycobacterium tuberculosis* (SwissProt™ Accession No. 053440), *Streptomyces coelicolor* (SwissProt™ Accession No. 086799), *Haemophilus influenzae* (SwissProt™ Accession No. P44793), *Escherichia coli* SwissProt™ Accession No. P15044) and *Bacillus subtilis* (SwissProt™ Accession No. P54556), respectively. The alignment was generated using ClustalW MSA software at the GenomeNet CLUSTALW Server at the Institute for Chemical Research, Kyoto University. The following parameters were used: Pairwise Alignment, K-tuple (word) size = 1, Window size = 5, Gap Penalty = 3, Number of Top Diagonals = 5, Scoring Method = Percent; Multiple Alignment, Gap Open Penalty = 10, Gap Extension Penalty = 0.0, Weight Transition = No, Hydrophilic residues = Gly, Pro, Ser, Asn, Asp, Gln, Glu, Arg and Lys, Hydrophobic Gaps = Yes; and Scoring Matrix = BLOSUM.
*Figure 21* is a schematic representation of the structure of the *Bacillus subtilis* genome in the region of the *coaX* (*yacB*) gene. The scale is in base pairs, the significant open reading frames are shown by open arrows and certain predicted restriction fragments are indicated by thick bars.
*Figure 22* is a schematic representation of the structure of pAN341 and pAN342, two independent PCR-derived clones of *B. subtilis yacB* (remaned herein as *coaX*).

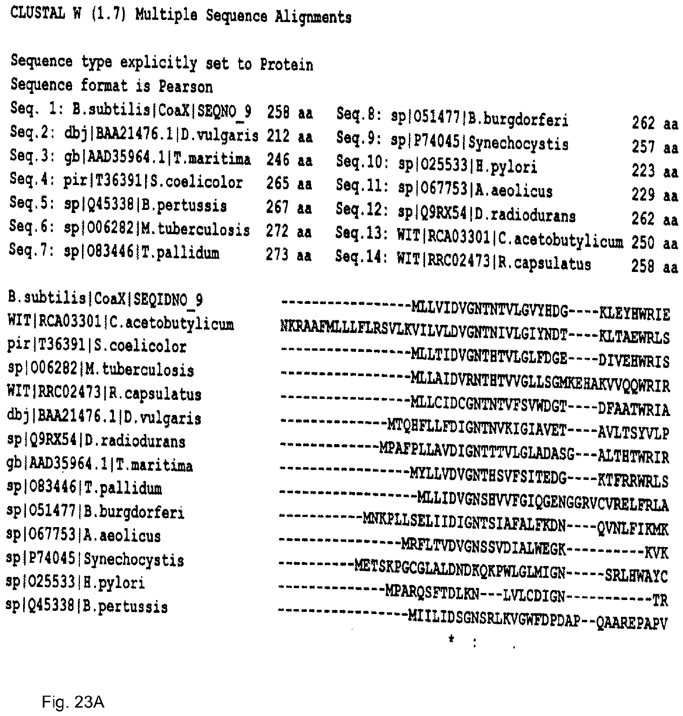

*Figure 23A-D* depicts a multiple sequence alignment (MSA) of the amino acid sequences encoded by fourteen known or predicted microbial *coaX* genes. SEQ ID NOs:9, 74, 7-8, 75, 11, 10 and 12-18 correspond to the amino acid sequences of *Bacillus subtilis* (SwissProt™ Accession No. P37564), *Clostridium acetobulyticum* (WIT™ Accession No. RCA03301, Argonne National Laboratories), *Streptomyces coelicolor* (PIR™ Accession No. T36391), *Mycobacterium tuberculosis* (SwissProt™ Accession No. 006282), *Rhodobacter capsulatus* (WIT™ Accession No. RRC02473), *Desulfovibrio vulgaris* (DBJ™ Accession No. BAA21476.1), *Deinococcus radiodurans* (SwissProt™ Accession No. Q9RX54), *Thermotoga maritima* (GenBank™ Accession No. AAD35964.1), *Treponema pallidum* (SwissProt™ Accession No. 083446), *Borrelia burgdorferi* (SwissProt™ Accession No.O51477), *Aquifex aeolicus* (SwissProt™ Accession No. 067753), *Synechocystis sp.* (SwissProt™ Accession No. P74045), *Helicobacter pylori* (SwissProt™ Accession No. 025533), and *Bordetella pertussis* (SwissProt™ Accession No. Q45338), respectively. The alignment was generated using ClustalW MSA software at the GenomeNet CLUSTALW Server at the Institute for Chemical Research, Kyoto University. The following parameters were used: Pairwise Alignment, K-tuple (word) size = 1, Window size = 5, Gap Penalty = 3, Number of Top Diagonals = 5, Scoring Method = Percent; Multiple Alignment, Gap Open Penalty = 10, Gap Extension Penalty = 0.0, Weight Transition = No, Hydrophilic residues = Gly, Pro, Ser, Asn, Asp, Gln, Glu, Arg and Lys, Hydrophobic Gaps = Yes; and Scoring Matrix = BLOSUM.

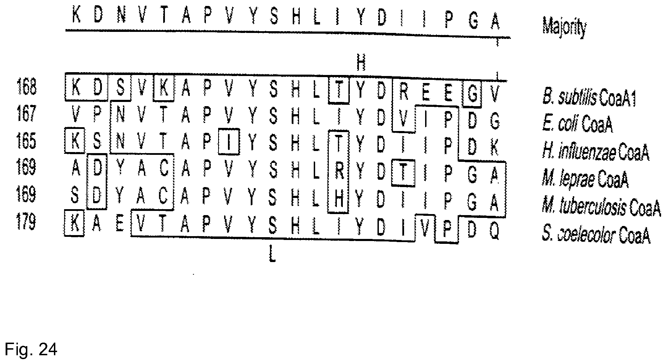

*Figure 24* depicts a multiple sequence alignment of a portion of the protein sequences of the *coaA* gene products from the following microorganisms: *Bacillus subtilis, Escherichia coli, Haemophilus influenzae, Mycobacterium leprae, Mycobacterium tuberculosis,* and *Streptomyces coelicolor.* The residues that are mutated in *E. coli coaA15*(*Ts*) and *B. subtilis coaA282A* are indicated below and above the alignment, respectively. The portions correspond to amino acid residues 168-187 of SEQ ID NO:3, 167-186 of SEQ ID NO:2, 165-184 of SEQ ID NO:1, 169-188 of SEQ ID NO:4, 169-188 of SEQ ID NO:5 and 179-198 of SEQ ID NO:6, respectively.
*Figure 25* is a schematic representation of the structure of pAN294, a plasmid for integrating mutagenized *B. subtilis coaA* at its native locus.
*Figure 26* is a schematic representation of the structure of pAN336, a plasmid designed to delete *B. subtilis coaX* from its chromosomal locus and replace it with a kanamycin resistence gene.

### Detailed Description

The present disclosure features new and improved methods of producing pantothenate and other key compounds of the pantothenate biosynthetic pathway (referred to herein as "panto-compounds", for example, pantothenate, ketopantoate, pantoate and β-alanine) using microorganisms in which the pantothenate biosynthetic pathway has been manipulated such that pantothenate or other desired panto-compounds are produced.

The new and improved methodologies of the present disclosure include methods of producing panto-compounds (*e.g*., pantothenate) in microorganisms having at least one enzyme of the pantothenate biosynthetic pathway manipulated such that pantothenate or other desired panto-compounds are produced (*e.g*., produced at an increased level). For example, the disclosure features methods of producing panto-compounds (*e.g*., pantothenate) in microorganisms having at least one of ketopantoate hydroxymethyltransferase, ketopantoate reductase, pantothenate synthetase or aspartate-α-decarboxylase manipulated such that pantothenate or other desired panto-compounds are produced. The methodologies of the present disclosure also include methods of producing panto-compounds (*e.g*., pantothenate) in microorganisms having at least one valine-isoleucine biosynthetic enzyme, described herein, manipulated such that pantothenate or other desired panto-compounds are produced. For example, the disclosure features methods of producing panto-compounds (*e.g*., pantothenate) in microorganisms having at least one of acetohydroxyacid synthetase, acetohydroxyacid isomeroreductase or dihydroxyacid dehydratase manipulated such that pantothenate or other desired panto-compounds are produced.

The disclosure also features methods of producing panto-compounds that involve culturing a ketopantoate reductase-overexpressing (KPAR-O) microorganism under conditions such that the panto-compound is produced. The disclosure also features methods of producing pantothenate in a manner independent of precursor feed that involve culturing an aspartate-α-decarboxylase-overexpressing (AαD-O) microorganism under conditions such that pantothenate is produced. Also featured are β-alanine independent high yield pantothenate production methods as well as methods of producing β-alanine. The present disclosure also features methods for enhancing production of panto-compounds that involve culturing pantothenate kinase mutants. In particular, the present disclosure features new and improved methods of producing pantothenate and other key compounds of the pantothenate biosynthetic pathway (*e.g*., panto-compounds) utilizing microorganisms having modified pantothenate kinase activity, for example, microorganisms that include the *coaX gene* or that include a mutant *coaX gene*, having reduced pantothenate kinase activity.

In order that the present disclosure may be more readily understood, certain terms are first defined herein.

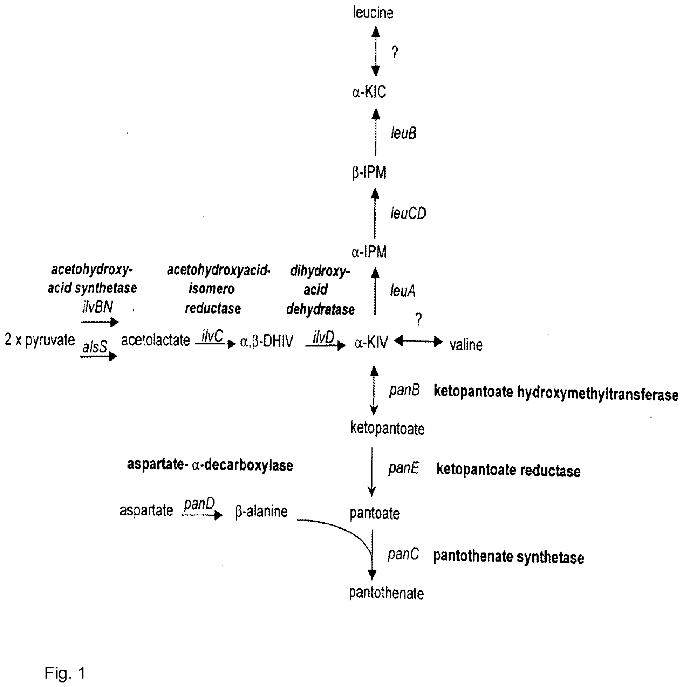

The term "pantothenate biosynthetic pathway" includes the biosynthetic pathway involving pantothenate biosynthetic enzymes (*e.g*., polypeptides encoded by biosynthetic enzyme-encoding genes), compounds (*e.g*., precursors, substrates, intermediates or products), cofactors and the like utilized in the formation or synthesis of pantothenate. The term "pantothenate biosynthetic pathway" includes the biosynthetic pathway leading to the synthesis of pantothenate in a microorganisms (*e.g*., *in vivo*) as well as the biosynthetic pathway leading to the synthesis of pantothenate *in vitro.* Figure 1 includes a schematic representation of the pantothenate biosynthetic pathway. Pantothenate biosynthetic enzymes are depicted in bold and their corresponding genes indicated in italics.

The term "pantothenate biosynthetic enzyme" includes any enzyme utilized in the formation of a compound (*e.g*., intermediate or product) of the pantothenate biosynthetic pathway. According to Figure 1, synthesis of pantoate from α-ketoisovalerate (α-KIV) proceeds *via* the intermediate, ketopantoate. Formation of ketopantoate is catalyzed by the pantothenate biosynthetic enzyme ketopantoate hydroxymethyltransferase (the *panB* gene product). Formation of pantoate is catalyzed by the pantothenate biosynthetic enzyme ketopantoate reductase (the *panE* gene product). Synthesis of β-alanine from aspartate is catalyzed by the pantothenate biosynthetic enzyme aspartate-α-decarboxylase (the *panD* gene product). Formation of pantothenate from pantoate and β-alanine (*e.g*., condensation) is catalyzed by the pantothenate biosynthetic enzyme pantothenate synthetase (the *panC* gene product).

The term "isoleucine-valine biosynthetic pathway" includes the biosynthetic pathway involving isoleucine-valine biosynthetic enzymes (*e.g*., polypeptides encoded by biosynthetic enzyme-encoding genes), compounds (*e.g*., precursors, substrates, intermediates or products), cofactors and the like utilized in the formation or synthesis of conversion of pyruvate to valine or isoleucine. The term "isoleucine-valine biosynthetic pathway" includes the biosynthetic pathway leading to the synthesis of valine or isoleucine in a microorganisms (*e.g*., *in vivo*) as well as the biosynthetic pathway leading to the synthesis of valine or isoleucine *in vitro.* Figure 1 includes a schematic representation of the isoleucine-valine biosynthetic pathway. Isoleucine-valine biosynthetic enzymes are depicted in bold italics and their corresponding genes indicated in italics

The term "isoleucine-valine biosynthetic enzyme" includes any enzyme utilized in the formation of a compound (*e.g*., intermediate or product) of the isoleucine-valine biosynthetic pathway. According to Figure 1, synthesis of valine from pyruvate proceeds *via* the intermediates, acetolactate, α,β-dihydroxyisovalerate (α,β-DHIV) and α-ketoisovalerate (α-KIV). Formation of acetolactate from pyruvate is catalyzed by the isoleucine-valine biosynthetic enzyme acetohydroxyacid synthetase (the *ilvBN* gene product, or alternatively, the *alsS* gene product). Formation of α,β-DHIV from acetolactate is catalyzed by the isoleucine-valine biosynthetic enzyme acetohydroxyacidisomero reductase (the *ilvC* gene product). Synthesis of α-KIV from α,β-DHIV is catalyzed by the isoleucine-valine biosynthetic enzyme dihydroxyacid dehydratase (the *ilvD* gene product). Moreover, valine and isoleucine can be interconverted by branched chain amino acid transaminases.

As used herein, each of ketopantoate, pantoate, β-alanine and pantothenate are "panto-compounds". The term "panto-compound" includes a compound (*e.g*., a substrate, intermediate or product) in the pantothenate biosynthetic pathway which is downstream from a particular pantothenate biosynthetic enzyme. In one example, a panto-compound is downstream of the pantothenate biosynthetic enzyme ketopantoate hydroxymethyltransferase (the *panB* gene product) and can include ketopantoate, pantoate and/or pantothenate. In another example, a panto-compound is downstream of the pantothenate biosynthetic enzyme ketopantoate reductase (the *panE* gene product) and can include pantoate and/or pantothenate. In yet another example, a panto-compound is downstream of the pantothenate biosynthetic enzyme pantothenate synthetase (the *panC* gene product) and can include pantothenate. In yet another example, a panto-compound is downstream of the pantothenate biosynthetic enzyme aspartate-α-decarboxylase (the *panD* gene product) and can include β-alanine and/or pantothenate.

Preferred panto-compounds include pantothenate and pantoate. The term "pantothenate" includes the free acid form of pantothenate, also referred to as "pantothenic acid" as well as any salt thereof (*e.g*., derived by replacing the acidic hydrogen of pantothenate or pantothenic acid with a cation, for example, calcium, sodium, potassium, ammonium), also referred to as a "pantothenate salt". The term "panto-compound" also includes alcohol derivatives of pantothenate. Preferred pantothenate salts are calcium pantothenate or sodium pantothenate. A preferred alcohol derivative is pantothenol. Pantothenate salts and/or alcohols of the present disclosure include salts and/or alcohols prepared *via* conventional methods from the free acids described herein. In another embodiment, calcium pantothenate is synthesized directly by a microorganism of the present disclosure. A pantothenate salt of the present disclosure can likewise be converted to a free acid form of pantothenate or pantothenic acid by conventional methodology.

The term "pantoate" includes the free acid form of pantoate, also referred to as "pantoic acid" as well as any salt thereof (*e.g*., derived by replacing the acidic hydrogen of pantoate or pantoic acid with a cation, for example, calcium, sodium, potassium, ammonium), also referred to as a "pantoate salt". Preferred pantoate salts are calcium pantoate or sodium pantoate. Pantoate salts of the present disclosure include salts prepared *via* conventional methods from the free acids described herein. A pantoate salt of the present disclosure can likewise be converted to a free acid form of pantoate or pantoic acid by conventional methodology. Moreover, a free acid form of pantoate or pantoic acid can be converted to pantolactone by conventional methodology.

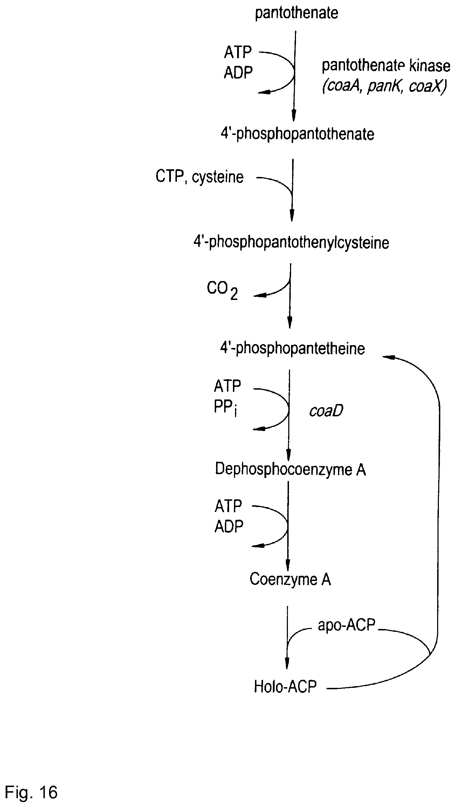

The term "CoA biosynthetic pathway" includes the biosynthetic pathway involving CoA biosynthetic enzymes (*e.g*., polypeptides encoded by biosynthetic enzyme-encoding genes), compounds (*e.g*., precursors, substrates, intermediates or products), cofactors and the like utilized in the formation or synthesis of CoA from pantothenate. A schematic representation of the CoA biosynthetic pathway in *E. coli* is set forth as Figure 16. (The pathway depicted is also presumed to be that utilized by other microorganisms.) The term "CoA biosynthetic pathway" includes the biosynthetic pathway leading to the synthesis of CoA in microorganisms (*e.g*., *in vivo*) as well as the biosynthetic pathway leading to the synthesis of *CoA in vitro.* The term "Coenzyme A or CoA biosynthetic enzyme" includes any enzyme utilized in the formation of a compound (e.g., intermediate or product) of the CoA biosynthetic pathway, for example, the *coaA, panK* or *coaX* gene product which catalyzes the phosphorylation of pantothenate to form 4'-phosphopantothenate, or the *coaD* gene product which catalyzes the conversion of 4'-phosphopantetheine to dephosphocoenzyme A.

### I. Recombinant Microorganisms and Methods for Culturing Microorganisms Such That Panto-Compounds are Produced

The methodologies of the present disclosure feature microorganisms, *e.g*., recombinant microorganisms, preferably including vectors or genes (*e.g*., wild-type and/or mutated genes) as described herein and/or cultured in a manner which results in the production of a desired product (*e.g.* a panto-compound or panto-compounds). The term "recombinant" microorganism includes a microorganism (*e.g*., bacteria, yeast cell, fungal cell, etc.) which has been genetically altered, modified or engineered (*e.g*., genetically engineered) such that it exhibits an altered, modified or different genotype and/or phenotype (*e.g*., when the genetic modification affects coding nucleic acid sequences of the microorganism) as compared to the naturally-occurring microorganism from which it was derived. Preferably, a "recombinant" microorganism of the present disclosure has been genetically engineered such that it overexpresses at least one bacterial gene or gene product (*e.g*., a pantothenate or isoleucine-valine biosynthetic enzyme encoding-gene) as described herein, preferably a biosynthetic enzyme encoding-gene included within a recombinant vector as described herein and/or a biosynthetic enzyme expressed from a recombinant vector. The ordinary skilled will appreciate that a microorganism expressing or overexpressing a gene product produces or overproduces the gene product as a result of expression or overexpression of nucleic acid sequences and/or genes encoding the gene product.

The term "manipulated microorganism" includes a microorganism that has been engineered (*e.g*., genetically engineered) or modified such that the microorganism has at least one enzyme of the pantothenate biosynthetic pathway and/or at least one enzyme of the isoleucine-valine biosynthetic pathway modified such that pantothenate or other desired panto-compounds are produced. Modification or engineering of such microorganisms can be according to any methodology described herein including, but not limited to, deregulation of a biosynthetic pathway and/or overexpression of at least one biosynthetic enzyme. A "manipulated" enzyme (*e.g*., a "manipulated" biosynthetic enzyme) includes an enzyme, the expression or production of which has been altered or modified such that at least one upstream or downstream precursor, substrate or product of the enzyme is altered or modified, for example, as compared to a corresponding wild-type or naturally occurring enzyme.

The term "overexpressed" or "overexpression" includes expression of a gene product (*e.g*., a pantothenate biosynthetic enzyme or isoleucine-valine biosynthetic enzyme) at a level greater than that expressed prior to manipulation of the microorganism or in a comparable microorganism which has not been manipulated. In one embodiment, the microorganism can be genetically manipulated (*e.g*., genetically engineered) to overexpress a level of gene product greater than that expressed prior to manipulation of the microorganism or in a comparable microorganism which has not been manipulated. Genetic manipulation can include, but is not limited to, altering or modifying regulatory sequences or sites associated with expression of a particular gene (*e.g*., by adding strong promoters, inducible promoters or multiple promoters or by removing regulatory sequences such that expression is constitutive), modifying the chromosomal location of a particular gene, altering nucleic acid sequences adjacent to a particular gene such as a ribosome binding site or transcription terminator, increasing the copy number of a particular gene, modifying proteins (*e.g*., regulatory proteins, suppressors, enhancers, transcriptional activators and the like) involved in transcription of a particular gene and/or translation of a particular gene product, or any other conventional means of deregulating expression of a particular gene routine in the art (including but not limited to use of antisense nucleic acid molecules, for example, to block expression of repressor proteins).

In another embodiment, the microorganism can be physically or environmentally manipulated to overexpress a level of gene product greater than that expressed prior to manipulation of the microorganism or in a comparable microorganism which has not been manipulated. For example, a microorganism can be treated with or cultured in the presence of an agent known or suspected to increase transcription of a particular gene and/or translation of a particular gene product such that transcription and/or translation are enhanced or increased. Alternatively, a microorganism can be cultured at a temperature selected to increase transcription of a particular gene and/or translation of a particular gene product such that transcription and/or translation are enhanced or increased.

The term "deregulated" or "deregulation" includes the alteration or modification of at least one gene in a microorganism that encodes an enzyme in a biosynthetic pathway, such that the level or activity of the biosynthetic enzyme in the microorganism is altered or modified. Preferably, at least one gene that encodes an enzyme in a biosynthetic pathway is altered or modified such that the gene product is enhanced or increased. The phrase "deregulated pathway" can also include a biosynthetic pathway in which more than one gene that encodes an enzyme in a biosynthetic pathway is altered or modified such that the level or activity of more than one biosynthetic enzyme is altered or modified. The ability to "deregulate" a pathway (*e.g*., to simultaneously deregulate more than one gene in a given biosynthetic pathway) in a microorganism arises from the particular phenomenon of microorganisms in which more than one enzyme (*e.g*., two or three biosynthetic enzymes) are encoded by genes occurring adjacent to one another on a contiguous piece of genetic material termed an "operon".

The term "operon" includes a coordinated unit of gene expression that contains a promoter and possibly a regulatory element associated with one or more, preferably at least two, structural genes (*e.g*., genes encoding enzymes, for example, biosynthetic enzymes). Expression of the structural genes can be coordinately regulated, for example, by regulatory proteins binding to the regulatory element or by anti-termination of transcription. The structural genes can be transcribed to give a single mRNA that encodes all of the structural proteins. Due to the coordinated regulation of genes included in an operon, alteration or modification of the single promoter and/or regulatory element can result in alteration or modification of each gene product encoded by the operon. Alteration or modification of the regulatory element can include, but is not limited to removing the endogenous promoter and/or regulatory element(s), adding strong promoters, inducible promoters or multiple promoters or removing regulatory sequences such that expression of the gene products is modified, modifying the chromosomal location of the operon, altering nucleic acid sequences adjacent to the operon or within the operon such as a ribosome binding site, increasing the copy number of the operon, modifying proteins (*e.g*., regulatory proteins, suppressors, enhancers, transcriptional activators and the like) involved in transcription of the operon and/or translation of the gene products of the operon, or any other conventional means of deregulating expression of genes routine in the art (including but not limited to use of antisense nucleic acid molecules, for example, to block expression of repressor proteins). Deregulation can also involve altering the coding region of one or more genes to yield, for example, an enzyme that is feedback resistant or has a higher or lower specific activity.

A particularly preferred "recombinant" microorganism of the present disclosure has been genetically engineered to overexpress a bacterially-derived gene or gene product. The term "bacterially-derived" or "derived-from", for example bacteria, includes a gene which is naturally found in bacteria or a gene product (*e.g*., ketopantoate hydroxymethyltransferase, ketopantoate reductase, pantothenate synthetase, aspartate-α-decarboxylate, acetohydroxyacid synthetase, acetohydroxyacid isomeroreductase or dihydroxyacid dehydratase) which is encoded by a bacterial gene (*e.g*., encoded by *panB, panE, panC, panD, ilvB, ilvN, alsS, ilvC,* or *ilvD*).

The methodologies of the present disclosure feature recombinant microorganisms which overexpress at least one of ketopantoate hydroxymethyltransferase, ketopantoate reductase, pantothenate synthetase or aspartate-α-decarboxylase. A particularly preferred recombinant microorganism of the present disclosure has been genetically engineered to overexpress a *Bacillus* (*e.g*., *Bacillus licheniformis, Bacillus amyloliquefaciens, Bacillus halodurans, Bacillus subtilis,* and *Bacillus pumilus,* etc.) biosynthetic enzyme (*e.g*., has been engineered to overexpress at least one of *B. subtilis* ketopantoate reductase (the *panE* gene product) (*e.g*., ketopantoate reductase having the amino acid sequence of SEQ ID NO:30 or encoded by the nucleic acid sequence of SEQ ID NO:29), *B. subtilis* ketopantoate hydroxymethyltransferase (the *panB* gene product) (*e.g*., ketopantoate hydroxymethyltransferase having the amino acid sequence of SEQ ID NO:24 or encoded by a nucleic acid molecule having the nucleotide sequence of SEQ ID NO:23), *B. subtilis* pantothenate synthetase (the *panC* gene product) *(e.g.,* pantothenate synthetase having the amino acid sequence of SEQ ID NO:26 or encoded by a nucleic acid molecule having the nucleotide sequence of SEQ ID NO:25) and/or *B. subtilis* aspartate-α-decarboxylase (the *panD* gene product) (*e.g*., aspartate-α-decarboxylase having the amino acid sequence of SEQ ID NO:28 or encoded by a nucleic acid molecule having the nucleotide sequence of SEQ ID NO:27).

In an exemplary embodiment, the disclosure features a microorganism (*e.g*., a KPAR-O microorganism) that has been transformed with a vector comprising a *panE* nucleic acid sequence (*e.g*., a *panE* nucleic acid seqeunce as set forth in SEQ ID NO:29). In another embodiment, the disclosure features a microorganism that has been transformed with a vector comprising a *panB* nucleic acid sequence (*e.g*., a *panB* nucleic acid sequence as set forth in SEQ ID NO:23), a vector comprising a *panC* nucleic acid sequence (*e.g*., a *panC* nucleic acid sequence as set forth in SEQ ID NO:25) or a vector comprising a *panD* nucleic acid sequence (*e.g*., a *panD* nucleic acid sequence as set forth in SEQ ID NO:27). In yet another embodiment, the disclosure features a microorganism having a deregulated *panBCD* operon (*e.g*., SEQ ID NO:59).

Other preferred "recombinant" microorganisms of the present disclosure have a deregulated isoleucine-valine (*ilv*) pathway. The phrase "microorganism having a deregulated isoleucine-valine (*ilv*) pathway" includes a microorganism having an alteration or modification in at least one gene encoding an enzyme of the isoleucine-valine (*ilv*) pathway or having an alteration or modification in an operon including more than one gene encoding an enzyme of the isoleucine-valine (*ilv*) pathway. A preferred "microorganism having a deregulated isoleucine-valine (*ilv*) pathway" has been genetically engineered to overexpress a *Bacillus* (*e.g*., *B. subtilis*) *ilv* biosynthetic enzyme (*e.g*., has been engineered to overexpress at least one of acetohydroxyacid synthetase (the *ilvBN* gene products or the *alsS* gene product) (*e.g*., acetohydroxyacid synthetase having subunits having the amino acid sequences of SEQ ID NO:32 and SEQ ID NO:34 or encoded by nucleic acid molecules having the nucleotide sequence of SEQ ID NO:31 and SEQ ID NO:33 or the nucleotide sequence of SEQ ID NO:58 from nucleotides 1-2246 or acetohydroxyacid synthetase encoded by a nucleic acid molecule having the nucleotide sequence of SEQ ID NO:86), acetohydroxyacid isomeroreductase (the *ilvC* gene product) (*e.g*., acetohydroxyacid isomeroreductase having the amino acid sequence of SEQ ID NO:36 or encoded by a nucleic acid molecule having the nucleotide sequence of SEQ ID NO:35), dihydroxyacid dehydratase (the *ilvD* gene product) (*e.g*., dihydroxyacid dehydratase having the amino acid sequence of SEQ ID NO:38 or encoded by a nucleic acid molecule having the nucleotide sequence of SEQ ID NO:37), and/or has been transformed with a vector comprising an *ilvBNC* nucleic acid sequence (SEQ ID NO:58, coding regions from nucleotides 1-1725, 1722-2246 and 2263-3291) and/or an *ilvD* nucleic acid sequence (SEQ ID NO:37).

In another preferred embodiment, a recombinant microorganism is designed or engineered such that a mutant CoaA and/or CoaX biosynthetic enzyme is expressed and at least one pantothenate biosynthetic enzyme and/or at least one isoleucine-valine biosynthetic enzyme is overexpressed or deregulated.

In another preferred embodiment, a microorganism of the present disclosure overexpresses or is mutated for a gene or biosynthetic enzyme (*e.g*., a CoA biosynthetic enzyme, pantothenate biosynthetic enzyme or isoleucine-valine biosynthetic enzyme) which is bacterially-derived. The term "bacterially-derived" or "derived-from", for example bacteria, includes a gene product (*e.g*., ketopantoate hydroxymethyltransferase, ketopantoate reductase, pantothenate synthetase, aspartate-α-decarboxylate, acetohydroxyacid synthetase, acetohydroxyacid isomeroreductase, dihydroxyacid dehydratase or pantothenate kinase) which is encoded by a bacterial gene (*e.g., panB, panE, panC, panD, ilvBN* (*or also, ilvC, ilvD,* or encoded by *coaA* or *coaX*)*.*

Still other preferred recombinant microorganisms of the present disclosure are mutant microorganisms. As used herein, the term "mutant microorganism" includes a recombinant microorganism that has been genetically engineered to express a mutated gene or protein that is normally or naturally expressed by the microorganism. Preferably, a mutant microorganism expresses a mutated gene or protein such that the microorganism exhibits an altered, modified or different phenotype (*e.g*., has been engineered to express a mutated CoaA biosynthetic enzyme, for example, pantothenate kinase). In one embodiment, a mutant microorganism is designed or engineered such that it includes a mutant *coaX* gene, as defined herein. In another embodiment, a recombinant microorganism is designed or engineered such that it includes a mutant *coaA* gene, as defined herein. In another embodiment, a mutant microorganism is designed or engineered such that a *coaX* gene has been deleted (*i.e*., the protein encoded by the *coaX* gene is not produced). In another embodiment, a mutant microorganism is designed or engineered such that a *coaA* gene has been deleted (*i.e*., the protein encoded by the *coaA* gene is not produced). Preferably, a mutant microorganism has a mutant *coaX* gene or a mutant *coaA* gene, or has been engineered to have a *coaX* gene and/or *coaA* deleted, such that that the mutant microorganism encodes a "reduced pantothenate kinase activity". In the context of a whole microorganism, a "reduced pantothenate kinase activity" can be determined by measuring or assaying for a decrease in an intermediate or product of the CoA biosynthetic pathway, for example, measuring or assaying for 4'-phosphopantothenate, 4'-phosphopantothenylcysteine, 4'-phosphopantetheine, dephosphocoenzyme A, Coenzyme A, apo-acyl carrier protein (apo-ACP) or holo-acyl carrier protein (ACP) in the microorganism (*e.g*., in a lysate isolated or derived from the microorganism) or in the medium in which the microorganism is cultured (see *e.g*., Figure 16). Alternatively, a "reduced pantothenate kinase activity" can be determined by measuring or assaying for decreased growth of the microorganism. Alternatively, a "reduced pantothenate kinase activity" can be determined by measuring or assaying for an increase in a panto-compound (*e.g*., pantothenate) in the microorganism or surrounding media, as panto-compounds lie upstream of the CoA biosynthetic pathway, the first step of which is catalyzed by pantothenate kinase. The disclosure also features recombinant microorganisms that, in addition to having reduced pantothenate kinase activity (*e.g*., expressing mutant *coaA* and/or mutant *coaX* genes) have a deregulated pantothenate biosynthesis pathway and/or a deregulated isoleucine-valine (*ilv*) biosynthetic pathway.

In one embodiment, a recombinant microorganism of the present disclosure is a Gram positive organism (*e.g*., a microorganism which retains basic dye, for example, crystal violet, due to the presence of a Gram-positive wall surrounding the microorganism). In a preferred embodiment, the recombinant microorganism is a microorganism belonging to a genus selected from the group consisting of *Bacillus, Cornyebacterium, Lactobacillus, Lactococci* and *Streptomyces.* In a more preferred embodiment, the recombinant microorganism is of the genus *Bacillus.* In another preferred embodiment, the recombinant microorganism is selected from the group consisting of *Bacillus subtilis, Bacillus lentimorbus, Bacillus lentus, Bacillus firmus, Bacillus pantothenticus, Bacillus amyloliquefaciens, Bacillus cereus, Bacillus circulans, Bacillus coagulans, Bacillus licheniformis, Bacillus megaterium, Bacillus pumilus, Bacillus thuringiensis,* and other Group 1 *Bacillus* species, for example, as characterized by 16S rRNA type (Priest (1993) in Bacillus subtilis and Other Gram-Positive Bacteria eds. Sonenshein et al., ASM, Washington, D.C., p. 6). In another preferred embodiment, the recombinant microorganism is *Bacillus brevis* or *Bacillus stearothermophilus.* In another preferred embodiment, the recombinant microorganism is selected from the group consisting of *Bacillus licheniformis, Bacillus amyloliquefaciens, Bacillus halodurans, Bacillus subtilis,* and *Bacillus pumilus.*

In another embodiment, the recombinant microorganism is a Gram negative (excludes basic dye) organism. In a preferred embodiment, the recombinant microorganism is a microorganism belonging to a genus selected from the group consisting of *Salmonella, Escherichia, Klebsiella, Serratia,* and *Proteus.* In a more preferred embodiment, the recombinant microorganism is of the genus *Escherichia.* In an even more preferred embodiment, the recombinant microorganism is *Escherichia coli.* In another embodiment, the recombinant microorganism is *Saccharomyces* (*e.g*., *S. cerevisiae*)*.*

An important aspect of the present disclosure involves culturing the recombinant microorganisms described herein, such that a desired compound (*e.g*., a desired panto-compound) is produced. The term "culturing" includes maintaining and/or growing a living microorganism of the present disclosure (*e.g*., maintaining and/or growing a culture or strain). In one embodiment, a microorganism of the disclosure is cultured in liquid media. In another embodiment, a microorganism of the disclosure is cultured in solid media or semi-solid media. In a preferred embodiment, a microorganism of the disclosure is cultured in media (*e.g*., a sterile, liquid media) comprising nutrients essential or beneficial to the maintenance and/or growth of the microorganism (*e.g*., carbon sources or carbon substrate, for example complex carbohydrates such as bean or grain meal, starches, sugars, sugar alcohols, hydrocarbons, oils, fats, fatty acids, organic acids and alcohols; nitrogen sources, for example, vegetable proteins, peptones, peptides and amino acids derived from grains, beans and tubers, proteins, peptides and amino acids derived form animal sources such as meat, milk and animal byproducts such as peptones, meat extracts and casein hydrolysates; inorganic nitrogen sources such as urea, ammonium sulfate, ammonium chloride, ammonium nitrate and ammonium phosphate; phosphorus sources, for example, phosphoric acid, sodium and potassium salts thereof; trace elements, for example, magnesium, iron, manganese, calcium, copper, zinc, boron, molybdenum, and/or cobalt salts; as well as growth factors such as amino acids, vitamins, growth promoters and the like).

Preferably, microorganisms of the present disclosure are cultured under controlled pH. The term "controlled pH" includes any pH which results in production of the desired product (*e.g*., a panto-compound). In one embodiment, microorganisms are cultured at a pH of about 7. In another embodiment, microorganisms are cultured at a pH of between 6.0 and 8.5. The desired pH may be maintained by any number of methods known to those skilled in the art.

Also preferably, microorganisms of the present disclosure are cultured under controlled aeration. The term "controlled aeration" includes sufficient aeration (*e.g*., oxygen) to result in production of the desired product (*e.g*., panto-compound). In one embodiment, aeration is controlled by regulating oxygen levels in the culture, for example, by regulating the amount of oxygen dissolved in culture media. Preferably, aeration of the culture is controlled by agitating the culture. Agitation may be provided by a propeller or similar mechanical agitation equipment, by revolving or shaking the growth vessel (*e.g*., fermentor) or by various pumping equipment. Aeration may be further controlled by the passage of sterile air or oxygen through the medium (e.g., through the fermentation mixture). Also preferably, microorganisms of the present disclosure are cultured without excess foaming (*e.g*., *via* addition of antifoaming agents).

Moreover, microorganisms of the present disclosure can be cultured under controlled temperatures. The term "controlled temperature" includes any temperature which results in production of the desired product (*e.g*., a panto-compound). In one embodiment, controlled temperatures include temperatures between 15°C and 95°C. In another embodiment, controlled temperatures include temperatures between 15°C and 70°C. Preferred temperatures are between 20°C and 55°C, more preferably between 30°C and 45°C or between 30°C and 50°C.

Microorganisms can be cultured (e.g., maintained and/or grown) in liquid media and preferably are cultured, either continuously or intermittently, by conventional culturing methods such as standing culture, test tube culture, shaking culture (*e.g*., rotary shaking culture, shake flask culture, etc.), aeration spinner culture, or fermentation. In a preferred embodiment, the microorganisms are cultured in shake flasks. In a more preferred embodiment, the microorganisms are cultured in a fermentor (*e.g*., a fermentation process). Fermentation processes of the present disclosure include, but are not limited to, batch, fed-batch and continuous methods of fermentation. The phrase "batch process" or "batch fermentation" refers to a closed system in which the composition of media, nutrients, supplemental additives and the like is set at the beginning of the fermentation and not subject to alteration during the fermentation, however, attempts may be made to control such factors as pH and oxygen concentration to prevent excess media acidification and/or microorganism death. The phrase "fed-batch process" or "fed-batch" fermentation refers to a batch fermentation with the exception that one or more substrates or supplements are added (*e.g*., added in increments or continuously) as the fermentation progresses. The phrase "continuous process" or "continuous fermentation" refers to a system in which a defined fermentation media is added continuously to a fermentor and an equal amount of used or "conditioned" media is simultaneously removed, preferably for recovery of the desired product (*e.g*., panto-compound). A variety of such processes have been developed and are well-known in the art.

The phrase "culturing under conditions such that a desired compound (*e.g*., a panto-compound, for example, pantothenate) is produced" includes maintaining and/or growing microorganisms under conditions (*e.g*., temperature, pressure, pH, duration, etc.) appropriate or sufficient to obtain production of the desired compound or to obtain desired yields of the particular compound being produced. For example, culturing is continued for a time sufficient to produce the desired amount of a panto-compound (*e.g*., pantothenate, pantoate or β-alanine). Preferably, culturing is continued for a time sufficient to substantially reach maximal production of the panto-compound. In one embodiment, culturing is continued for about 12 to 24 hours. In another embodiment, culturing is continued for about 24 to 36 hours, 36 to 48 hours, 48 to 72 hours, 72 to 96 hours, 96 to 120 hours, 120 to 144 hours, or greater than 144 hours. In another embodiment, culturing is continued for a time sufficient to reach production yields of panto-compound, for example, cells are cultured such that at least about 15 to 20 g/L of panto-compound are produced, at least about 20 to 25 g/L panto-compound are produced, at least about 25 to 30 g/L panto-compound are produced, at least about 30 to 35 g/L panto-compound are produced, at least about 35 to 40 g/L panto-compound are produced (*e.g*., at least about 37 g/L panto-compound) or at least about 40 to 50 g/L panto compound are produced. In yet another embodiment, microorganisms are cultured under conditions such that a preferred yield of panto-compound, for example, a yield within a range set forth above, is produced in about 24 hours, in about 36 hours, in about 48 hours, in about 72 hours, or in about 96 hours.

The methodology of the present disclosure can further include a step of recovering a desired compound (*e.g*., a panto-compound). The term "recovering" a desired compound (*e.g*., a panto-compound) includes extracting, harvesting, isolating or purifying the compound from culture media. Recovering the compound can be performed according to any conventional isolation or purification methodology known in the art including, but not limited to, treatment with a conventional resin (*e.g*., anion or cation exchange resin, non-ionic adsorption resin, etc.), treatment with a conventional adsorbent (*e.g*., activated charcoal, silicic acid, silica gel, cellulose, alumina, etc.), alteration of pH, solvent extraction (*e.g*., with a conventional solvent such as an alcohol, ethyl acetate, hexane and the like), dialysis, filtration, concentration, crystallization, recrystallization, pH adjustment, lyophilization and the like. For example, a compound (*e.g*., a panto-compound) can be recovered from culture media by first removing the microorganisms from the culture. Media is then passed through or over a cation exchange resin to remove unwanted cations and then through or over an anion exchange resin to remove unwanted inorganic anions and organic acids having stronger acidities than the panto-compound of interest (*e.g*., pantothenate). The resulting panto-compound (*e.g*., pantothenate) can subsequently be converted to a pantothenate salt (*e.g*., calcium pantothenate) as described herein.

Preferably, a desired compound of the present disclosure is "extracted", "isolated" or "purified" such that the resulting preparation is substantially free of other components (*e.g*., free of media components and/or fermentation byproducts). The language "substantially free of other components" includes preparations of desired compound in which the compound is separated (*e.g*., purified or partially purified) from media components or fermentation byproducts of the culture from which it is produced. In one embodiment, the preparation has greater than about 80% (by dry weight) of the desired compound (*e.g*., less than about 20% of other media components or fermentation byproducts), more preferably greater than about 90% of the desired compound (*e.g*., less than about 10% of other media components or fermentation byproducts), still more preferably greater than about 95% of the desired compound (*e.g*., less than about 5% of other media components or fermentation byproducts), and most preferably greater than about 98-99% desired compound (*e.g*., less than about 1-2% other media components or fermentation byproducts). When the desired compound is a panto-compound that has been derivatized to a salt (*e.g.* a pantothenate salt or pantoate salt), the panto-compound is preferably further free (*e.g*., substantially free) of chemical contaminants associated with the formation of the salt. When the desired compound is a panto-compound that has been derivatized to an alcohol, the panto-compound is preferably further free (*e.g*., substantially free) of chemical contaminants associated with the formation of the alcohol.

In an alternative embodiment, the desired panto-compound is not purified from the microorganism, for example, when the microorganism is biologically non-hazardous (*e.g*., safe). For example, the entire culture (or culture supernatant) can be used as a source of product (*e.g*., crude product). In one embodiment, the culture (or culture supernatant) supernatant is used without modification. In another embodiment, the culture (or culture supernatant) is concentrated. In yet another embodiment, the culture (or culture supernatant) is dried or lyophilized.

### II. Panto-Compound Production Methodologies Featuring Ketopantoate Reductase-Overexpressing Microorganism

One aspect of the disclosure features methods of producing a panto-compounds that involve culturing a ketopantoate reductase-overexpressing (KPAR-O) microorganism under conditions such that the panto-compound is produced. The term "ketopantoate reductase-overexpressing (KPAR-O) microorganism" includes a microorganism which has been manipulated such that ketopantoate reductase is overexpressed (*e.g*., a *B. subtilis* ketopantoate reductase protein having the amino acid sequence of SEQ ID NO:30) and/or has been transformed with a vector comprising a *panE1* nucleic acid sequence (*e.g*., a *B. subtilis panE1* nucleic acid sequence as set forth in SEQ ID NO:29). In one embodiment, the panto-compound is pantothenate. In another embodiment, the panto-compound is pantoate. In another embodiment, the ketopantoate reductase is bacterial-derived. In another embodiemnt, the ketopantoate reductase is derived from *Bacillus* (*e.g*., is derived from *Bacillus subtilis*)*.* In yet another embodiment, the KPAR-O microorganism is Gram positive. In yet another embodiment, the KPAR-O microorganism is a microorganism belonging to a genus selected from the group consisting of *Bacillus, Cornyebacterium, Lactobacillus, Lactococci* and *Streptomyces.* In a preferred embodiemnt, the KPAR-O microorganism is of the genus *Bacillus.* In a more preferred embodiment, the KPAR-O microorganism is selected from the group consisting of *Bacillus licheniformis, Bacillus amyloliquefaciens, Bacillus halodurans, Bacillus subtilis* and *Bacillus pumilus.* In a particularly preferred embodiemnt, the KPAR-O microorganism is *Bacillus subtilis.*

In still other embodiments, the KPAR-O microorganism further overexpresses at least one pantothenate biosynthetic enzyme in addition to ketopantoate reductase. In an exemplary embodiment, the KPAR-O microorganism further overexpresses at least one of ketopantoate hydroxymethyltransferase, pantothenate synthetase and aspartate-α-decarboxylase. Also featured are methods of producing panto-compounds, for example, methods that involve culturing a KPAR-O microorganism, which further include the step of recovering the panto-compound.

### III. Methods of Producing Panto-Compounds Independent of Precursor Feed Requirements

Depending on the biosynthetic enzyme or combination of biosynthetic enzymes manipulated, it may be desirable or necessary to provide (*e.g*., feed) microorganisms of the present disclosure at least one pantothenate biosynthetic precursor such that pantothenate or other desired panto-compounds are produced. The term "pantothenate biosynthetic precursor" or "precursor" includes an agent or compound which, when provided to, brought into contact with, or included in the culture medium of a microorganism, serves to enhance or increase pantothenate biosynthesis. In one embodiment, the pantothenate biosynthetic precursor or precursor is aspartate. In another embodiment, the pantothenate biosynthetic precursor or precursor is β-alanine. The amount of aspartate or β-alanine added is preferably an amount that results in a concentration in the culture medium sufficient to enhance productivity of the microorganism (*e.g*., a concentration sufficient to enhance production of a panto-compound, for example, β-alanine, ketopantoate, pantoate or pantothenate). Pantothenate biosynthetic precursors of the present disclosure can be added in the form of a concentrated solution or suspension (*e.g*., in a suitable solvent such as water or buffer) or in the form of a solid (*e.g*., in the form of a powder). Moreover, pantothenate biosynthetic precursors of the present disclosure can be added as a single aliquot, continuously or intermittently over a given period of time.

In yet another embodiment, the pantothenate biosynthetic precursor is valine, see *e.g*., Example III. In yet another embodiment, the pantothenate biosynthetic precursor is α-ketoisovalerate. Preferably, valine or α-ketoisovalerate is added in an amount that results in a concentration in the medium sufficient for production of the desired product (*e.g*., panto-compound) to occur. Pantothenate biosynthetic precursors are also referred to herein as "supplemental pantothenate biosynthetic substrates".

Providing pantothenate biosynthetic precursors in the pantothenate biosynthetic methodologies of the present disclosure, can be associated with high costs, for example, when the methodologies are used to produce high yields of panto-compounds. Accordingly, preferred methodologies of the present disclosure feature microorganisms having at least one biosynthetic enzyme or combination of biosynthetic enzymes (*e.g*., at least one pantothenate biosynthetic enzyme and/or valine-isoleucine biosynthetic enzyme) manipulated such that pantothenate or other desired panto-compounds are produced in a manner independent of precursor feed. The phrase "a manner independent of precursor feed", for example, when referring to a method for producing a desired compound (*e.g*., a panto-compound), includes an approach to or a mode of producing the desired compound that does not depend or rely on precursors being provided (*e.g*., fed) to the microorganism being utilized to produce the desired compound. For example, microorganisms featured in the methodologies of the present disclosure can be used to produce panto-compounds in a manner requiring no feeding of the precursors aspartate, β-alanine, valine and/or α-KIV.

Alternative preferred methodologies of the present disclosure feature microorganisms having at least one biosynthetic enzyme or combination of biosynthetic enzymes manipulated such that pantothenate or other desired panto-compounds are produced in a manner substantially independent of precursor feed. The phrase "a manner substantially independent of precursor feed" includes an approach to or a method of producing the desired compound that depends or relies to a lesser extent on precursors being provided (*e.g*., fed) to the microorganism being utilized. For example, microorganisms featured in the methodologies of the present disclosure can be used to produce panto-compounds in a manner requiring feeding of substantially reduced amounts of the precursors aspartate, β-alanine, valine and/or α-KIV In one embodiment, the disclosure features methods of producing panto-compounds (*e.g*., pantothenate) in a manner that requires feeding of less than 5%-10% of the amount of precursor required by a control microorganism (*e.g*., a microorganism that is dependent, for example is wholly dependent, on precursor feed to efficiently produce the desired compound). In another embodiment, the disclosure features methods of producing panto-compounds in a manner that requires feeding of less than 15-20% of the amount of precursor required by a control microorganism. In another embodiment, the disclosure features methods of producing panto-compounds in a manner that requires feeding of less than 25-30%, 35-40%, 45-50% or 55-60% of the amount of precursor required by a control microorganism. As described in Examples I-III herein, particular microorganisms featured in the methodologies of the present disclosure require, for example, 5 g/L of aspartate, β-alanine, valine or α-KIV (*e.g*., in test tube or in shake flask cultures). Accordingly, in a preferred embodiment, the present disclosure features methods of producing panto-compounds (*e.g*., pantothenate) in a manner requiring feeding of less than 0.25 g/L, 0.5 g/L, 0.75 g/L, 1 g/L, 1.25 g/L, 1.5 g/L, 1.75 g/L, 2 g/L, 2.25 g/L, 2.5 g/L, 2.75 g/L or 3 g/L.

Preferred methods of producing desired compounds (*e.g*., panto-compounds) in a manner independent of precursor feed or alternatively, in a manner substantially independent of precursor feed, involve culturing microorganisms which have been manipulated (*e.g*., designed or engineered, for example, genetically engineered) such that expression of at least one pantothenate biosynthetic enzyme, and/or at least one isoleucine-valine biosynthetic enzyme, is modified. For example, in one embodiment, a microorganism is manipulated (*e.g*., designed or engineered) such that the production of at least one pantothenate biosynthetic enzyme, and/or at least one isoleucine/valine biosynthetic enzyme is deregulated. In a preferred embodiment, a microorganism is manipulated (*e.g*., designed or engineered) such that it has a deregulated biosynthetic pathway, for example, a deregulated pantothenate biosynthesis pathway and/or a deregulated isoleucine-valine biosynthetic pathway, as defined herein. In another preferred embodiment, a microorganism is manipulated (*e.g*., designed or engineered) such that at least one pantothenate biosynthetic enzyme, and/or at least one isoleucine-valine biosynthetic enzyme is overexpressed.

Preferred methods of producing desired compounds (*e.g*., panto-compounds) in a manner independent of precursor feed or alternatively, in a manner substantially independent of precursor feed, are as follows. In one embodiment, the disclosure features a method of producing pantothenate in a manner independent of precursor feed comprising culturing an aspartate-α-decarboxylase-overexpressing (AαD-O) microorganism having a deregulated isoleucine-valine (*ilv*) pathway under conditions such that pantothenate is produced. In another embodiment, the disclosure features a method of producing pantothenate in a manner independent of precursor feed comprising culturing an aspartate-α-decarboxylase-overexpressing (AαD-O) microorganism having a deregulated pantothenate (*pan*) pathway and a deregulated isoleucine-valine (*ilv*) pathway, under conditions such that pantothenate is produced. In another embodiment, the disclosure features a method of producing pantothenate in a manner independent of aspartate or β-alanine feed comprising culturing an aspartate-α-decarboxylase-overexpressing (AαD-O) microorganism under conditions such that pantothenate is produced. In yet another embodiment, the disclosure features a method of producing pantothenate in a manner independent of valine or α-ketoisovalerate feed comprising culturing a microorganism having a deregulated isoleucine-valine (*ilv*) biosynthetic pathway under conditions such that pantothenate is produced.

The term "aspartate-α-decarboxylase-overexpressing (AαD-O) microorganism" includes a microorganism which has been manipulated such that aspartate-α-decarboxylase is overexpressed. A preferred "aspartate-α-decarboxylase-overexpressing (AαD-O) microorganism" has been transformed with a vector comprising a *B. subtilis panD* nucleic acid sequence (*e.g*., a *panD* nucleic acid sequence that encodes an aspartate-α-decarboxylase protein having the amino acid sequence of SEQ ID NO:28, for example, a *panD* nucleic acid sequence as set forth in SEQ ID NO:27).

The phrase "microorganism having a deregulated isoleucine-valine (*ilv*) pathway" includes a microorganism having an alteration or modification in at least one gene encoding an enzyme of the isoleucine-valine (*ilv*) pathway or having an alteration or modification in an operon including more than one gene encoding an enzyme of the isoleucine-valine (*ilv*) pathway. A preferred "microorganism having a deregulated isoleucine-valine (*ilv*) pathway" overexpresses acetohydroxyacid synthetase (*e.g*., acetohydroxyacid synthetase having subunits having the amino acid sequences of SEQ ID NO:32 and SEQ ID NO:34 or acetohydroxyacid synthetase having the amino acid sequence of SEQ ID NO:87), acetohydroxyacid isomeroreductase (having the amino acid sequence of SEQ ID NO:36), or dihydroxyacid dehydratase (having the amino acid sequence of SEQ ID NO:38) and/or has been transformed with a vector comprising *ilvB, ilvN, ilvC, ilvBN, ilvBNC* or *alsS* nucleic acid sequences (SEQ ID NO:31, SEQ ID NO:33, SEQ ID NO:35, nucleotides 1-2246 of SEQ ID NO:58, SEQ ID NO:58 having coding regions from nucleotides 1-1725, 1722-2246 and 2263-3291, or SEQ ID NO:86, respectively) and/or an *ilvD* nucleic acid sequence (SEQ ID NO:37).

### IV. High Yield Production Methodologies

A particularly preferred embodiment of the present disclosure is a high yield production method for producing pantothenate comprising culturing a manipulated microorganism under conditions such that pantothenate is produced at a significantly high yield. The phrase "high yield production method", for example, a high yield production method for producing a desired compound (*e.g*., for producing a panto-compound) includes a method that results in production of the desired compound at a level which is elevated or above what is usual for comparable production methods. Preferably, a high yield production method results in production of the desired compound at a significantly high yield. The phrase "significantly high yield" includes a level of production or yield which is sufficiently elevated or above what is usual for comparable production methods, for example, which is elevated to a level sufficient for commercial production of the desired product (*e.g*., production of the product at a commercially feasible cost). In one embodiment, the disclosure features a high yield production method of producing pantothenate that includes culturing a manipulated microorganism under conditions such that pantothenate is produced at a level greater than 2 g/L. In another embodiment, the disclosure features a high yield production method of producing pantothenate that includes culturing a manipulated microorganism under conditions such that pantothenate is produced at a level greater than 10 g/L. In another embodiment, the disclosure features a high yield production method of producing pantothenate that includes culturing a manipulated microorganism under conditions such that pantothenate is produced at a level greater than 20 g/L. In yet another embodiment, the disclosure features a high yield production method of producing pantothenate that includes culturing a manipulated microorganism under conditions such that pantothenate is produced at a level greater than 30 g/L. In yet another embodiment, the disclosure features a high yield production method of producing pantothenate that includes culturing a manipulated microorganism under conditions such that pantothenate is produced at a level greater than 40 g/L.

The disclosure further features a high yield production method for producing a desired compound (*e.g*., for producing a panto-compound) that involves culturing a manipulated microorganism under conditions such that a sufficiently elevated level of compound is produced within a commercially desireable period of time. In an exemplary embodiment, the disclosure features a high yield production method of producing pantothenate that includes culturing a manipulated microorganism under conditions such that pantothenate is produced at a level greater than 15-20 g/L in 36 hours. In another embodiment, the disclosure features a high yield production method of producing pantothenate that includes culturing a manipulated microorganism under conditions such that pantothenate is produced at a level greater than 25-30 g/L in 48 hours. In another embodiment, the disclosure features a high yield production method of producing pantothenate that includes culturing a manipulated microorganism under conditions such that pantothenate is produced at a level greater than 35-40 g/L in 72 hours, for example, greater that 37 g/L in 72 hours. In another embodiment, the disclosure features a high yield production method of producing pantothenate that includes culturing a manipulated microorganism under conditions such that pantothenate is produced at a level greater than 30-40 g/L in 60 hours, for example, greater that 30, 35 or 40 g/L in 60 hours. Values and ranges included and/or intermediate within the ranges set forth herein are also intended to be within the scope of the present disclosure. For example, pantothenate production at levels of at least 31, 32, 33, 34, 35, 36, 37, 38 and 39 g/L in 60 hours are intended to be included within the range of 30-40 g/L in 60 hours. In another example, ranges of 30-35 g/L or 35-40 g/L are intended to be included within the range of 30-40 g/L in 60 hours. Moreover, the skilled artisan will appreciate that culturing a manipulated microorganism to achieve a production level of, for example, "30-40 g/L in 60 hours" includes culturing the microorganism for additional time periods (*e.g*., time periods longer than 60 hours), optionally resulting in even higher yields of pantothenate being produced.

### V Panto-Compound Production Methodologies Featuring Pantothenate Kinase Mutant Microorganisms

The present disclosure relates to methods of producing pantothenate using microorganisms engineered to produce high yields of pantothenate as well as other panto-compounds. Cells overproducing pantothenate result in high intracellular pantothenate levels that could overcome the feedback inhibition of pantothenate kinase by CoA, leading to overproduction of CoA. Besides consuming pantothenate, increased synthesis of CoA may cause increased feedback inhibition of the PanB, PanD, PanE or PanC reaction, thereby limiting pantothenate production. Accordingly, a reduction in pantothenate kinase activity may lead to a decrease in CoA levels with resulting increases in PanB, PanD, PanE or PanC activity and pantothenate production.

Thus, certain methodologies of the present disclosure are based, at least in part, on the identification and characterization of the *B. subtilis coaA* gene and the demonstration that the gene is neither essential for *B. subtilis* growth (*i.e*., deletion of the *coaA* gene from the chromosome of *B. subtilis* is not a lethal event) nor for pantothenate kinase activity in *B. subtilis.* A second pantothenate kinase-encoding gene has been identified and characterized in *B. subtilis,* and is termed "*coaX*". This gene complements an *E. coli* mutant that contains a temperature sensitive pantothenate kinase and is not related by homology to any previously known pantothenate kinase gene.

In one aspect, the methodologies of the disclosure feature recombinant microorganisms that include the *coaX* gene or that include a mutant *coaX* gene, having reduced pantothenate kinase activity. In one embodiment, the methodologies feature such recombinant microorganisms further having a deregulated pantothenate biosynthetic pathway. In another embodiment, the methodologies feature such recombinant microorganisms further having a deregulated isoleucine-valine (*ilv*) pathway. In a preferred embodiment, the microorganisms belong to the genus *Bacillus* (*e.g*., *B. subtilis*)*.*

The methodologies of the disclosure also feature recombinant microorganisms (*e.g*., microorganisms belong to the genus *Bacillus,* for example, *B. subtilis*) that include the *coaA* gene or that include a mutant *coaA* gene, optionally including a *coaX* gene or mutant thereof, having reduced pantothenate kinase activity. In one embodiment, the methodologies feature such recombinant microorganisms further having a deregulated pantothenate biosynthetic pathway or having a deregulated isoleucine-valine (*ilv*) pathway. Also featured are vectors that include isolated *coaX* or *coaA* genes as well as mutant *coaX* and/or *coaA* genes. Isolated nucleic acid molecules that include isolated *coaX* genes or mutant *coaX* genes are features in addition to isolated CoaX proteins and mutant CoaX proteins.

The above-described nucleic acid molecules (*e.g*., genes), proteins, vectors, and recombinant microorganisms (*e.g*., mutant microorganisms), are particularly suited for use in methods of producing panto-compounds and/or methods of enhancing panto-compound production. In one embodiment, the disclosure features a method for producing a panto-compound (*e.g*., pantothenate) that includes culturing a pantothenate kinase mutant (*e.g*., a recombinant microorganism that misexpresses, *e.g*., is mutated for, pantothenate kinase, as defined herein) under conditions such that panto-compound is produced. In another embodiment, the disclosure features a method for enhancing production of a panto-compound (*e.g*., pantothenate) that includes culturing a pantothenate kinase mutant (*e.g*., a recombinant microorganism that misexpresses, *e.g*., is mutated for, pantothenate kinase, as defined herein) under conditions such that production of the panto-compound is produced. As used herein, the term "enhancing" (for example, in the context of the phrase "enhancing production") includes increasing the level or rate of production of panto-compound (*e.g*., pantothenate) as compared to the level or rate of production in a non-mutant microorganism (*e.g*., a microorganism having a normal pantothenate kinase gene(s) and/or having normal pantothenate production rates and/or levels.

Preferably, the level of panto-compound produced in methodologies featuring the pantothenate kinase mutants of the present disclosure is increased by at least 5% as compared to the level produced by a non-mutant (*e.g*., a recombinant microorganism expressing non-mutated pantothenate kinase). Even more preferably, the level of panto-compound is increased 10% as compared to methodologies featuring non-mutants. Even more preferably, panto-compound levels (*e.g*., pantothenate levels) are increased 20%, 30%, 40%, 50%, 60%, 70%, 80%, 90%, are increased 2-fold, 5-fold, 10-fold, 50-fold, 100-fold or more as compared to methodologies featuring non-mutants.

### VI. Additional Mutations Resulting in Enhanced Panto-Compound Production

The methodologies of the present disclosuredisclosure further can include, for example in addition to overexpressing or deregulating a pantothenate biosynthetic enzyme and/or an isoleucine-valine biosynthetic enzyme, or in addition to mutating a pantothenate-kinase encoding gene, deleting or mutating an enzyme that catalyzes the conversion of key pantothenate biosynthesis substrates or precursors to unwanted or undesirable products. For example, mutating the *ilvE* gene (Kuramitsu et al. (1985) J. Biochem. 97:993-999) or a homologue thereof (SEQ ID NO:62 or SEQ ID NO:64), thereby limiting the conversion of α-ketoisovalerate to valine, in addition to mutating a pantothenate kinase encoding enzyme, is predicted to result in even further enhanced or increased production of panto-compound. Alternatively, mutating the *ansB* gene (Sun and Seflow (1991) J. Bacteriol. 173:3831-3845) or a homologue thereof (SEQ ID NO:66), thereby limiting the degradation of aspartate, in addition to mutating a pantothenate kinase encoding enzyme, is predicted to result in even further enhanced or increased production of panto-compound. Alternatively, mutating the *alsD* gene (Renna et al. (1993) J. Bacteriol. 175:3863-3875) or a homologue thereof (SEQ ID NO:68), thereby limiting the conversion of acetolactate to acetoin, in addition to mutating a pantothenate kinase encoding enzyme, is predicted to result in even further enhanced or increased production of panto-compound. Alternatively, mutating the *avtA* gene encoding alanine-valine transaminase or a homologue thereof, thereby limiting the conversion of α-ketoisovalerate to valine, in addition to mutating a pantothenate kinase encoding enzyme, is predicted to result in even further enhanced or increased production of panto-compound. Mutating the *avtA* gene can include mutating, for example, an *avtA* gene having the nucleotide sequence of SEQ ID NO:70 (*e.g*., the *E. coli avtA* gene), or a structural homolog thereof (*e.g*., a homologue encoding a protein having 30-40%, 40-50%, 50-60%, 60-70%, 70-80%, 80-90%, 90-95% or more identity with the amino acid sequence of SEQ ID NO:71) or a functional homologue (*e.g*., a gene encoding a structurally unrelated protein having alanine-valine transmainase activity. Such mutations can be accomplished using the methodologies as exemplified in the Examples (e.g., Examples XIII, XV, XVI and XVII).

Accordingly, in one embodiment, the disclosure features a method of producing a panto-compound which includes culturing a microorganism having a mutant pantothenate kinase-encoding gene and which further has a deletion or mutation in an *avtA, ilvE, ansB,* and/or *alsD* gene, or homologue thereof. In another embodiment, the disclosure features a method of producing a panto-compound which includes culturing a microorganism having a mutant pantothenate-kinase encoding gene and a deregulated pantothenate biosynthetic pathway enzyme and which further has a deletion or mutation in an *avtA, ilvE, ansB,* and/or *alsD* gene, or homologue thereof. In another embodiment, the disclosure features a method of producing a panto-compound which includes culturing a microorganism having a mutant pantothenate-kinase encoding gene and a deregulated isoleucine-valine biosynthetic pathway enzyme and which further has a deletion or mutation in an *avtA, ilvE, ansB,* and/or *alsD* gene, or homologue thereof.

Mutating the *alsD* gene can be particularly useful when accomplished in conjunction with overexpression or deregulation of the *alsS* gene, for example, to prevent carbon (e.g., acetolactate) from being drawn away from the precursor pool utilized for α-KIV production. Accordingly, to maximize the contribution of the *als* locus to panto-compound production, it is desirable to disrupt the *alsD* gene in addition to overexpressing the *alsS* gene. To disrupt the *alsD* gene, appropriate fragments of the *als* operon, flanking the *alsD* gene, are amplified by PCR and cloned to provide homology for creating the disruptions. A drug resistance gene, such as the *cat* gene, is cloned between the flanking DNA fragments in place of the *alsD* gene, and the linearized DNA is transformed into a pantothenate production strain such as PA824, selecting for drug-resistance. To overexpress *alsS,* the *alsS* coding sequence (*e.g*., an *alsS* coding sequence that has been engineered by PCR for expression) is cloned into an expression vector. Vectors which express *alsS* (or alternatively, vectors which express *alsS* plus *ilvC*) are the introduced into panto-compound production strains (*e.g*., the pantothenate producing strain PA824).

The methodologies of the present disclosure further can include, for example in addition to overexpressing or deregulating a pantothenate biosynthetic enzyme and/or an isoleucine-valine biosynthetic enzyme, or in addition to mutating a pantothenate-kinase encoding gene, deleting or mutating an enzyme that catalyzes the conversion of desired panto-compounds to unwanted or undesireable downstream products.

### VII Isolated Nucleic Acid Molecules and Genes

Another aspect of the present disclosure features isolated nucleic acid molecules that encode *Bacillus* proteins (*e.g*., *B. subtilis* proteins), for example, *Bacillus* pantothenate biosynthetic enzymes (*e.g*., *B. subtilis* pantothenate biosynthetic enzymes) or *Bacillus* valine-isoleucine biosynthetic enzymes (*e.g*., *B. subtilis* valine-isoleucine biosynthetic enzymes). Also featured are isolated *coaX* and/or *coaA* nucleic acid molecules (*e.g*., isolated *coaX* and/or *coaA* genes) as well as isolated nucleic acid molecules that include such *coaX* and/or *coaA* nucleic acid molecules or genes.

The term "nucleic acid molecule" includes DNA molecules (*e.g*., linear, circular, cDNA or chromosomal DNA) and RNA molecules (*e.g*., tRNA, rRNA, mRNA) and analogs of the DNA or RNA generated using nucleotide analogs. The nucleic acid molecule can be single-stranded or double-stranded, but preferably is double-stranded DNA. The term "isolated" nucleic acid molecule includes a nucleic acid molecule which is free of sequences which naturally flank the nucleic acid molecule (*i.e*., sequences located at the 5' and 3' ends of the nucleic acid molecule) in the chromosomal DNA of the organism from which the nucleic acid is derived. In various embodiments, an isolated nucleic acid molecule can contain less than about 10 kb, 5 kb, 4kb, 3kb, 2kb, 1 kb, 0.5 kb, 0.1 kb, 50 bp, 25 bp or 10 bp of nucleotide sequences which naturally flank the nucleic acid molecule in chromosomal DNA of the microorganism from which the nucleic acid molecule is derived. Moreover, an "isolated" nucleic acid molecule, such as a cDNA molecule, can be substantially free of other cellular materials when produced by recombinant techniques, or substantially free of chemical precursors or other chemicals when chemically synthesized.

The term "gene", as used herein, includes a nucleic acid molecule (*e.g*., a DNA molecule or segment thereof), for example, a protein or RNA-encoding nucleic acid molecule, that in an organism, is separated from another gene or other genes, by intergenic DNA (*i.e*., intervening or spacer DNA which naturally flanks the gene and/or separates genes in the chromosomal DNA of the organism). A gene may direct synthesis of an enzyme or other protein molecule (*e.g*., may comprise coding sequences, for example, a contiguous open reading frame (ORF) which encodes a protein) or may itself be functional in the organism. A gene in an organism, may be clustered in an operon, as defined herein, said operon being separated from other genes and/or operons by the intergenic DNA. Individual genes contained within an operon may overlap without intergenic DNA between said individual genes. An "isolated gene", as used herein, includes a gene which is essentially free of sequences which naturally flank the gene in the chromosomal DNA of the organism from which the gene is derived (*i.e*., is free of adjacent coding sequences which encode a second or distinct protein or RNA molecule, adjacent structural sequences or the like) and optionally includes 5' and 3' regulatory sequences, for example promoter sequences and/or terminator sequences. In one embodiment, an isolated gene includes predominantly coding sequences for a protein (*e.g*., sequences which encode *Bacillus* proteins). In another embodiment, an isolated gene includes coding sequences for a protein (*e.g*., for a *Bacillus* protein) and adjacent 5' and/or 3' regulatory sequences from the chromosomal DNA of the organism from which the gene is derived (*e.g*., adjacent 5' and/or 3' *Bacillus* regulatory sequences). Preferably, an isolated gene contains less than about 10 kb, 5 kb, 2 kb, 1 kb, 0.5 kb, 0.2 kb, 0.1 kb, 50 bp, 25 bp or 10 bp of nucleotide sequences which naturally flank the gene in the chromosomal DNA of the organism from which the gene is derived.

In one aspect, the present disclosure features isolated *panB* nucleic acid sequences or genes, isolated *panC* nucleic acid sequences or genes, isolated *panD* nucleic acid sequences or genes, isolated *panE* nucleic acid sequences or genes, isolated *ilvB, ilvN, ilvBN* nucleic acid sequences or genes, isolated *alsS* nucleic acid sequences or genes, isolated *ilvC* nucleic acid sequences or genes and/or isolated *ilvD* nucleic acid sequences or genes.

In a preferred embodiment, the nucleic acid or gene is derived from *Bacillus* (*e.g*., is *Bacillus-derived*). The term "derived from *Bacillus*" or "*Bacillus-*derived" includes a nucleic acid or gene which is naturally found in microorganisms of the genus *Bacillus.* Preferably, the nucleic acid or gene is derived from a microorganism selected from the group consisting of *Bacillus subtilis, Bacillus lentimorbus, Bacillus lentus, Bacillus firms, Bacillus pantothenticus, Bacillus amyloliquefaciens, Bacillus cereus, Bacillus circulars, Bacillus coagulans, Bacillus licheniformis, Bacillus megaterium, Bacillus pumilus, Bacillus thuringiensis,* and other Group 1 *Bacillus* species, for example, as characterized by 16S rRNA type (Priest, *supra*). In another preferred embodiment, the nucleic acid or gene is derived from *Bacillus brevis* or *Bacillus stearothermophilus.* In another preferred embodiment, the nucleic acid molecules and/or genes of the present disclosure are derived from a microorganism selected from the group consisting of *Bacillus licheniformis, Bacillus amyloliquefaciens, Bacillus halodurans, Bacillus subtilis,* and *Bacillus pumilus.* In a particularly preferred embodiment, the nucleic acid or gene is derived from *Bacillus subtilis* (*e.g*., is *Bacillus subtilis-derived*). The term "derived from *Bacillus subtilis*" or "*Bacillus subtilis-derived*" includes a nucleic acid or gene which is naturally found in *Bacillus subtilis.* In yet another preferred embodiment, the nucleic acid or gene is a *Bacillus* gene homologue *(e.g.,* is derived from a species distinct from *Bacillus* but having significant homology to a *Bacillus* gene of the present disclosure, for example, a *Bacillus pan* gene or *Bacillus ilv* gene).

Included within the scope of the present disclosure are bacterial-derived nucleic acid molecules or genes and/or *Bacillus-derived* nucleic acid molecules or genes (*e.g*., B. *subtilis-derived* nucleic acid molecules or genes), for example, the genes identified by the present inventors, for example, *Bacillus* or *B. subtilis coaX* genes, *coaA* genes, *pan* genes and/or *ilv* genes. Further included within the scope of the present disclosure are bacterial-derived nucleic acid molecules or genes and/or *Bacillus-*derived nucleic acid molecules or genes (*e.g*., *B. subtilis-*derived nucleic acid molecules or genes) (*e.g*., *B. subtilis* nucleic acid molecules or genes) which differ from naturally-occurring bacterial and/or *Bacillus* nucleic acid molecules or genes (*e.g*., *B. subtilis* nucleic acid molecules or genes), for example, nucleic acid molecules or genes which have nucleic acids that are substituted, inserted or deleted, but which encode proteins substantially similar to the naturally-occurring gene products of the present disclosure. In one embodiment, an isolated nucleic acid molecule comprises at least one of the nucleotide sequences set forth as SEQ ID NO:23, SEQ ID NO:25, SEQ ID NO:27, SEQ ID NO:29, SEQ ID NO 31, SEQ ID NO:33, SEQ ID NO:86, SEQ ID NO:35 or SEQ ID NO:37. In another preferred embodiment, an isolated nucleic acid molecule comprises at least two, three or four of the nucleotide sequences set forth as SEQ ID NO:23, SEQ ID NO:25, SEQ ID NO:27, SEQ ID NO:29, SEQ ID NO 31, SEQ ID NO:33, SEQ ID NO:88, SEQ ID NO:35 or SEQ ID NO:37. For example, a preferred isolated nucleic acid molecule of the present disclosure can include the nucleotide sequences of SEQ ID NO:23, SEQ ID NO:25 and SEQ ID NO:27, preferably linked such that the proteins encoded by the nucleotide sequences of SEQ ID NO:23, SEQ ID NO:25 and SEQ ID NO:27 are each produced when the isolated nucleic acid molecule is expressed in a microorganism (*e.g*., SEQ ID NO:59). In another example, a preferred isolated nucleic acid molecule of the present disclosure can include the nucleotide sequences of SEQ ID NO:31 and SEQ ID NO:33, preferably linked such that the proteins encoded by the nucleotide sequences of SEQ ID NO:31 and SEQ ID NO:33 are each produced when the isolated nucleic acid molecule is expressed in a microorganism (*e.g*., nucleotides 1-2246 of SEQ ID NO:58). In another example, a preferred isolated nucleic acid molecule of the present disclosure can include the nucleotide sequence of SEQ ID NO:86. In another example, a preferred isolated nucleic acid molecule of the present disclosure can include the nucleotide sequences of SEQ ID NO:31, SEQ ID NO:33 and SEQ ID NO:35, preferably linked such that the proteins encoded by the nucleotide sequences of SEQ ID NO:31, SEQ ID NO:33 and SEQ ID NO:35 are each produced when the isolated nucleic acid molecule is expressed in a microorganism (*e.g*., SEQ ID NO:58).

In another embodiment, an isolated nucleic acid molecule of the present disclosure comprises a nucleotide sequence which is at least about 60-65%, preferably at least about 70-75%, more preferable at least about 80-85%, and even more preferably at least about 90-95% or more identical to a nucleotide sequence set forth as SEQ ID NO:23, SEQ ID NO:25, SEQ ID NO:27, SEQ ID NO:29, SEQ ID NO 31, SEQ ID NO:33, SEQ ID NO:88, SEQ ID NO:35 or SEQ ID NO:37. In another embodiment, an isolated nucleic acid molecule hybridizes under stringent conditions to a nucleic acid molecule having a nucleotide sequence set forth as SEQ ID NO:23, SEQ ID NO:25, SEQ ID NO:27, SEQ ID NO:29, SEQ ID NO 31, SEQ ID NO:33, SEQ ID NO:88, SEQ ID NO:35 or SEQ ID NO:37. Such stringent conditions are known to those skilled in the art and can be found in Current Protocols in Molecular Biology, John Wiley & Sons, N.Y. (1989), 6.3.1-6.3.6. A preferred, non-limiting example of stringent (*e.g*. high stringency) hybridization conditions are hybridization in 6X sodium chloride/sodium citrate (SSC) at about 45°C, followed by one or more washes in 0.2 X SSC, 0.1% SDS at 50-65°C. Preferably, an isolated nucleic acid molecule of the disclosure that hybridizes under stringent conditions to the sequence of SEQ ID NO:23, SEQ ID NO:25, SEQ ID NO:27, SEQ ID NO:29, SEQ ID NO 31, SEQ ID NO:33, SEQ ID NO:88, SEQ ID NO:35 or SEQ ID NO:37 corresponds to a naturally-occurring nucleic acid molecule. As used herein, a "naturally-occurring" nucleic acid molecule refers to an RNA or DNA molecule having a nucleotide sequence that occurs in nature.

A nucleic acid molecule of the present disclosure (*e.g*., a nucleic acid molecule having the nucleotide sequence of SEQ ID NO:23, SEQ ID NO:25, SEQ ID NO:27, SEQ ID NO:29, SEQ ID NO 31, SEQ ID NO:33, SEQ ID NO:88, SEQ ID NO:35 or SEQ ID NO:37 can be isolated using standard molecular biology techniques and the sequence information provided herein. For example, nucleic acid molecules can be isolated using standard hybridization and cloning techniques (e.g., as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989) or can be isolated by the polymerase chain reaction using synthetic oligonucleotide primers designed based upon the sequence of SEQ ID NO:23, SEQ ID NO:25, SEQ ID NO:27, SEQ ID NO:29, SEQ ID NO 31, SEQ ID NO:33, SEQ ID NO:88, SEQ ID NO:35 or SEQ ID NO:37. A nucleic acid of the disclosure can be amplified using cDNA, mRNA or alternatively, genomic DNA, as a template and appropriate oligonucleotide primers according to standard PCR amplification techniques. In another preferred embodiment, an isolated nucleic acid molecule of the disclosure comprises a nucleic acid molecule which is a complement of the nucleotide sequence shown in SEQ ID NO:23, SEQ ID NO:25, SEQ ID NO:27, SEQ ID NO:29, SEQ ID NO:33, SEQ ID NO 31, SEQ ID NO:33, SEQ ID NO:88, SEQ ID NO:35.

Additional *panC* nucleic acid sequences include those that comprise the nucleotide sequence of SEQ ID NO:25, encode a homologue of the polypeptide having the amino acid sequence set forth in SEQ ID NO:26 (*e.g*., encode a polypeptide having at least 30%, 40%, 50%, 60%, 70%, 80%, 90%, 95% or more identity to the polypeptide having the amino acid sequence as set forth in SEQ ID NO:26 and a substantially identical activity as said polypeptide), hybridize under stringent conditions to all or a portion of a nucleic acid molecule having the nucleotide sequence of SEQ ID NO:25 or to all or a portion of a nucleic acid molecule that encodes a polypeptide having the amino acid sequence of SEQ ID NO:26, or are complementary to a *panC* nucleotide sequence as set forth herein.

Aditional *panD* nucleic acid sequences include those that comprise the nucleotide sequence of SEQ ID NO:27, encode a homologue of the polypeptide having the amino acid sequence set forth in SEQ ID NO:28 (*e.g*., encode a polypeptide having at least 30%, 40%, 50%, 60%, 70%, 80%, 90%, 95% or more identity to the polypeptide having the amino acid sequence as set forth in SEQ ID NO:28 and a substantially identical activity as said polypeptide), hybridize under stringent conditions to all or a portion of a nucleic acid molecule having the nucleotide sequence of SEQ ID NO:27 or to all or a portion of a nucleic acid molecule that encodes a polypeptide having the amino acid sequence of SEQ ID NO:28, or are complementary to a *panD* nucleotide sequence as set forth herein.

Additional *panE* nucleic acid sequences include those that comprise the nucleotide sequence of SEQ ID NO:29, encode a homologue of the polypeptide having the amino acid sequence set forth in SEQ ID NO:30 (*e.g*., encode a polypeptide having at least 30%, 40%, 50%, 60%, 70%, 80%, 90%, 95% or more identity to the polypeptide having the amino acid sequence as set forth in SEQ ID NO:30 and a substantially identical activity as said polypeptide), hybridize under stringent conditions to all or a portion of a nucleic acid molecule having the nucleotide sequence of SEQ ID NO:29 or to all or a portion of a nucleic acid molecule that encodes a polypeptide having the amino acid sequence of SEQ ID NO:30, or are complementary to a *panE* nucleotide sequence as set forth herein.

Additional *ilvB* nucleic acid sequences are those that comprise the nucleotide sequence of SEQ ID NO:31, encode a homologue of the polypeptide having the amino acid sequence set forth in SEQ ID NO:32 (*e.g*., encode a polypeptide having at least 30%, 40%, 50%, 60%, 70%, 80%, 90%, 95% or more identity to the polypeptide having the amino acid sequence as set forth in SEQ ID NO:32 and a substantially identical activity as said polypeptide), hybridize under stringent conditions to all or a portion of a nucleic acid molecule having the nucleotide sequence of SEQ ID NO:31 or to all or a portion of a nucleic acid molecule that encodes a polypeptide having the amino acid sequence of SEQ ID NO:32, or are complementary to an *ilvB* nucleotide sequence as set forth herein.

Additional *ilvN* nucleic acid sequences are those that comprise the nucleotide sequence of SEQ ID NO:33, encode a homologue of the polypeptide having the amino acid sequence set forth in SEQ ID NO:34 (*e.g*., encode a polypeptide having at least 30%, 40%, 50%, 60%, 70%, 80%, 90%, 95% or more identity to the polypeptide having the amino acid sequence as set forth in SEQ ID NO:34 and a substantially identical activity as said polypeptide), hybridize under stringent conditions to all or a portion of a nucleic acid molecule having the nucleotide sequence of SEQ ID NO:33 or to all or a portion of a nucleic acid molecule that encodes a polypeptide having the amino acid sequence of SEQ ID NO:34, or are complementary to an *ilvN* nucleotide sequence as set forth herein.

Additional *ilvC* nucleic acid sequences include those that comprise the nucleotide sequence of SEQ ID NO:35, encode a homologue of the polypeptide having the amino acid sequence set forth in SEQ ID NO:36 *(e.g.,* encode a polypeptide having at least 30%, 40%, 50%, 60%, 70%, 80%, 90%, 95% or more identity to the polypeptide having the amino acid sequence as set forth in SEQ ID NO:36 and a substantially identical activity as said polypeptide), hybridize under stringent conditions to all or a portion of a nucleic acid molecule having the nucleotide sequence of SEQ ID NO:35 or to all or a portion of a nucleic acid molecule that encodes a polypeptide having the amino acid sequence of SEQ ID NO:36, or are complementary to an *ilvC* nucleotide sequence as set forth herein.

Additional *ilvD* nucleic acid sequences include those that comprise the nucleotide sequence of SEQ ID NO:37, encode a homologue of the polypeptide having the amino acid sequence set forth in SEQ ID NO:38 (*e.g*., encode a polypeptide having at least 30%, 40%, 50%, 60%, 70%, 80%, 90%, 95% or more identity to the polypeptide having the amino acid sequence as set forth in SEQ ID NO:38 and a substantially identical activity as said polypeptide), hybridize under stringent conditions to all or a portion of a nucleic acid molecule having the nucleotide sequence of SEQ ID NO:37 or to all or a portion of a nucleic acid molecule that encodes a polypeptide having the amino acid sequence of SEQ ID NO:38, or are complementary to an *ilvD* nucleotide sequence as set forth herein.

Additional *alsS* nucleic acid sequences include those that comprise the nucleotide sequence of SEQ ID NO:86, encode a homologue of the polypeptide having the amino acid sequence set forth in SEQ ID NO:87 (*e.g*., encode a polypeptide having at least 30%, 40%, 50%, 60%, 70%, 80%, 90%, 95% or more identity to the polypeptide having the amino acid sequence as set forth in SEQ ID NO:87 and a substantially identical activity as said polypeptide), hybridize under stringent conditions to all or a portion of a nucleic acid molecule having the nucleotide sequence of SEQ ID NO:86 or to all or a portion of a nucleic acid molecule that encodes a polypeptide having the amino acid sequence of SEQ ID NO:87, or are complementary to an *alsS* nucleotide sequence as set forth herein.

In another embodiment, an isolated nucleic acid molecule is or includes a *coaX* gene*,* or portion or fragment thereof. In one embodiment, an isolated *coaX* nucleic acid molecule or gene comprises the nucleotide sequence as set forth in SEQ ID NO: 19 (*e.g*., comprises the *B. subtilis coaX* nucleotide sequence). In another embodiment, an isolated *coaX* nucleic acid molecule or gene comprises a nucleotide sequence that encodes the amino acid sequence as set forth in SEQ ID NO:9 (*e.g*., encodes the *B. subtilis* CoaX amino acid sequence). In yet another embodiment, an isolated *coaX* nucleic acid molecule or gene encodes a homologue of the CoaX protein having the amino acid sequence of SEQ ID NO:9. As used herein, the term "homologue" includes a protein or polypeptide sharing at least about 30-35%, preferably at least about 35-40%, more preferably at least about 40-50%, and even more preferably at least about 60%, 70%, 80%, 90% or more identity with the amino acid sequence of a wild-type protein or polypeptide described herein and having a substantially equivalent functional or biological activity as said wild-type protein or polypeptide. For example, a CoaX homologue shares at least about 30-35%, preferably at least about 35-40%, more preferably at least about 40-50%, and even more preferably at least about 60%, 70%, 80%, 90% or more identity with the protein having the amino acid sequence set forth as SEQ ID NO:9 and has a substantially equivalent functional or biological activity (*i.e*., is a functional equivalent) of the protein having the amino acid sequence set forth as SEQ ID NO:9 (*e.g*., has a substantially equivalent pantothenate kinase activity). In a preferred embodiment, an isolated *coaX* nucleic acid molecule or gene comprises a nucleotide sequence that encodes a polypeptide as set forth in any one of SEQ ID NO:7, SEQ ID NO:8, SEQ ID NO:10, SEQ ID NO:11, SEQ ID NO:12, SEQ ID NO:13, SEQ ID NO:14, SEQ ID NO:15, SEQ ID NO:16, SEQ ID NO:17, SEQ ID NO:18, SEQ ID NO:74 or SEQ ID NO:75. In another embodiment, an isolated *coaX* nucleic acid molecule hybridizes to all or a portion of a nucleic acid molecule having the nucleotide sequence set forth in SEQ ID NO: 19 or hybridizes to all or a portion of a nucleic acid molecule having a nucleotide sequence that encodes a polypeptide having the amino acid sequence of any of SEQ ID NOs:7-18, 74 or 75. Such hybridization conditions are known to those skilled in the art and can be found in Current Protocols in Molecular Biology, Ausubel et al., eds., John Wiley & Sons, Inc. (1995), sections 2, 4 and 6. Additional stringent conditions can be found in Molecular Cloning: A Laboratory Manual, Sambrook et al., Cold Spring Harbor Press, Cold Spring Harbor, NY (1989), chapters 7, 9 and 11. A preferred, non-limiting example of stringent hybridization conditions includes hybridization in 4X sodium chloride/sodium citrate (SSC), at about 65-70° C (or hybridization in 4X SSC plus 50% formamide at about 42-50° C) followed by one or more washes in 1X SSC, at about 65-70° C. A preferred, non-limiting example of highly stringent hybridization conditions includes hybridization in 1X SSC, at about 65-70° C (or hybridization in 1X SSC plus 50% formamide at about 42-50° C) followed by one or more washes in 0.3X SSC, at about 65-70° C. A preferred, non-limiting example of reduced stringency hybridization conditions includes hybridization in 4X SSC, at about 50-60° C (or alternatively hybridization in 6X SSC plus 50% formamide at about 40-45° C) followed by one or more washes in 2X SSC, at about 50-60° C. Ranges intermediate to the above-recited values, e.g., at 65-70° C or at 42-50° C are also intended to be encompassed by the present disclosure. SSPE (1X SSPE is 0.15 M NaCl, 10mM NaH₂PO₄, and 1.25 mM EDTA, pH 7.4) can be substituted for SSC (1X SSC is 0.15 M NaCl and 15 mM sodium citrate) in the hybridization and wash buffers; washes are performed for 15 minutes each after hybridization is complete. The hybridization temperature for hybrids anticipated to be less than 50 base pairs in length should be 5-10°C less than the melting temperature (Tₘ) of the hybrid, where Tₘ is determined according to the following equations. For hybrids less than 18 base pairs in length, Tₘ(°C) = 2(# of A + T bases) + 4(# of G + C bases). For hybrids between 18 and 49 base pairs in length, Tₘ(°C) = 81.5 + 16.6(log₁₀[Na⁺]) + 0.41(%G+C) - (600/N), where N is the number of bases in the hybrid, and [Na⁺] is the concentration of sodium ions in the hybridization buffer ([Na⁺] for 1X SSC = 0.165 M). It will also be recognized by the skilled practitioner that additional reagents may be added to hybridization and/or wash buffers to decrease nonspecific hybridization of nucleic acid molecules to membranes, for example, nitrocellulose or nylon membranes, including but not limited to blocking agents (*e.g*., BSA or salmon or herring sperm carrier DNA), detergents (*e.g*., SDS), chelating agents (*e.g*., EDTA), Ficoll, PVP and the like. When using nylon membranes, in particular, an additional preferred, non-limiting example of stringent hybridization conditions is hybridization in 0.25-0.5M NaH₂PO₄, 7% SDS at about 65°C, followed by one or more washes at 0.02M NaH₂PO₄, 1% SDS at 65°C, see *e.g*., Church and Gilbert (1984) Proc. Natl. Acad. Sci. USA 81:1991-1995, (or, alternatively, 0.2X SSC, 1% SDS). In another preferred embodiment, an isolated nucleic acid molecule comprises a nucleotide sequence that is complementary to a *coaX* nucleotide sequence as set forth herein (*e.g*., is the full complement of the nucleotide sequence set forth as SEQ ID NO:19).

In another preferred embodiment, an isolated nucleic acid molecule is or includes a *coaA* gene, for example, a *Bacillus* (*e.g*., *B. subtilis*) *coaA* gene, or portion or fragment thereof. Exemplary isolated *coaA* nucleic acid molecules and/or genes include (1) an isolated *coaA* nucleic acid molecule or gene comprising the nucleotide sequence as set forth in any one of SEQ ID NOs:20-22; (2) an isolated *coaA* nucleic acid molecule or gene comprising a nucleotide sequence that encodes the amino acid sequence as set forth in SEQ ID NO:3; (3) an isolated *coaA* nucleic acid molecule or gene comprising a nucleotide sequence which encodes a CoaA homologue (*e.g*., a polypeptide having an amino acid sequence at least about 30-35%, preferably at least about 35-40%, more preferably at least about 40-50%, and even more preferably at least about 60%, 70%, 80%, 90% or more identical to the amino acid sequence set forth as SEQ ID NO:3 and having a substantially equivalent enzymatic activity; (4) an isolated *coaA* nucleic acid molecule or gene comprising a nucleotide sequence that encodes a polypeptide as set forth in any one of SEQ ID NO:1, SEQ ID NO:2, SEQ ID NO:4, SEQ ID NO:5 or SEQ ID NO:6; (5) an isolated nucleic acid molecule that hybridizes under stringent conditions to all or a portion of a nucleic acid molecule having the nucleotide sequence set forth in SEQ ID NO:20, SEQ ID NO:21 or SEQ ID NO:22 or hybridizes to all or a portion of a nucleic acid molecule having a nucleotide sequence that encodes a polypeptide having the amino acid sequence of SEQ ID NO:3; and (6) an isolated nucleic acid molecule comprising a nucleotide sequence that is complementary to a *coaA* nucleotide sequence as set forth herein *(e.g.,* is the full complement of the nucleotide sequence set forth in SEQ ID N0:20, SEQ ID NO:21 or SEQ ID NO:22).

A nucleic acid molecule of the present disclosure (*e.g*., a *coaX* nucleic acid molecule or gene or a *coaA* nucleic acid molecule or gene), can be isolated using standard molecular biology techniques and the sequence information provided herein. For example, nucleic acid molecules can be isolated using standard hybridization and cloning techniques (*e.g*., as described in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989) or can be isolated by the polymerase chain reaction using synthetic oligonucleotide primers designed based upon the *coaX* or *coaA* nucleotide sequences set forth herein, or flanking sequences thereof. A nucleic acid of the disclosure (*e.g*., a *coaX* nucleic acid molecule or gene or a *coaA* nucleic acid molecule or gene), can be amplified using cDNA, mRNA or alternatively, chromosomal DNA, as a template and appropriate oligonucleotide primers according to standard PCR amplification techniques.

Yet another embodiment of the present disclosure features mutant *coaX* and *coaA* nucleic acid molecules or genes. The phrase "mutant nucleic acid molecule" or "mutant gene" as used herein, includes a nucleic acid molecule or gene having a nucleotide sequence which includes at least one alteration (*e.g*., substitution, insertion, deletion) such that the polypeptide or protein that may be encoded by said mutant exhibits an activity that differs from the polypeptide or protein encoded by the wild-type nucleic acid molecule or gene. Preferably, a mutant nucleic acid molecule or mutant gene (*e.g*., a mutant *coaA* or *coaX* gene) encodes a polypeptide or protein having a reduced activity (*e.g*., having a reduced pantothenate kinase activity) as compared to the polypeptide or protein encoded by the wild-type nucleic acid molecule or gene, for example, when assayed under similar conditions (*e.g*., assayed in microorganisms cultured at the same temperature). A mutant gene also can encode no polypeptide or have a reduced level of production of the wild-type polypeptide.

As used herein, a "reduced activity" or "reduced enzymatic activity" is one that is at least 5% less than that of the polypeptide or protein encoded by the wild-type nucleic acid molecule or gene, preferably at least 5-10% less, more preferably at least 10-25% less and even more preferably at least 25-50%, 50-75% or 75-100% less than that of the polypeptide or protein encoded by the wild-type nucleic acid molecule or gene. Ranges intermediate to the above-recited values, *e.g*., 75-85%, 85-90%, 90-95%, are also intended to be encompassed by the present disclosure. As used herein, a "reduced activity" or "reduced enzymatic activity" also includes an activity that has been deleted or "knocked out" (*e.g*., approximately 100% less activity than that of the polypeptide or protein encoded by the wild-type nucleic acid molecule or gene). Activity can be determined according to any well accepted assay for measuring activity of a particular protein of interest. Activity can be measured or assayed directly, for example, measuring an activity of a protein isolated or purified from a cell. Alternatively, an activity can be measured or assayed within a cell or in an extracellular medium. For example, assaying for a mutant *coaA* gene or a mutant *coaX* gene (*i.e*., said mutant encoding a reduced pantothenate kinase activity) can be accomplished by expressing the mutated gene in a microorganism, for example, a mutant microorganism which expresses pantothenate kinase in a temperature-sensitive manner, assaying the mutant gene for the ability to complement a temperature sensitive (Ts) mutant for pantothenate kinase activity. A *coaX* mutant gene or *coaA* mutant gene that encodes a "reduced pantothenate kinase activity" is one that complements the Ts mutant less effectively than, for example, a corresponding wild-type *coaX* gene or *coaA* gene.

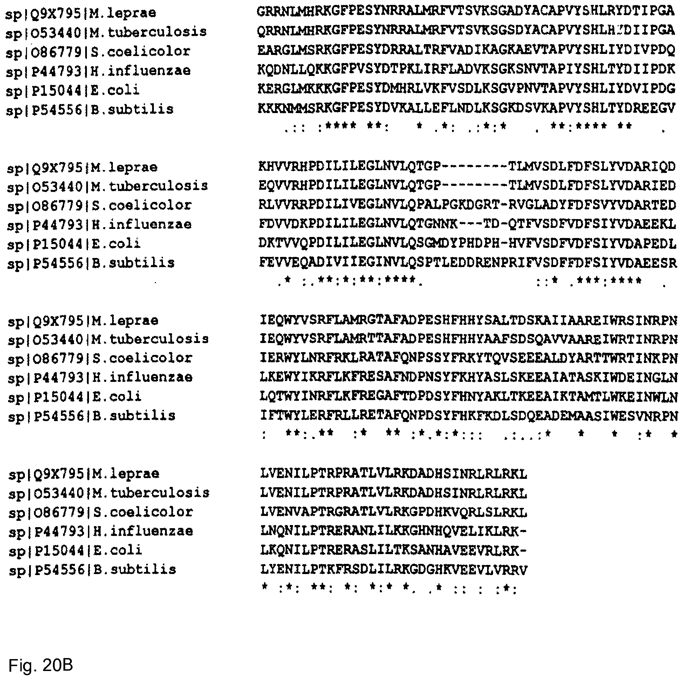

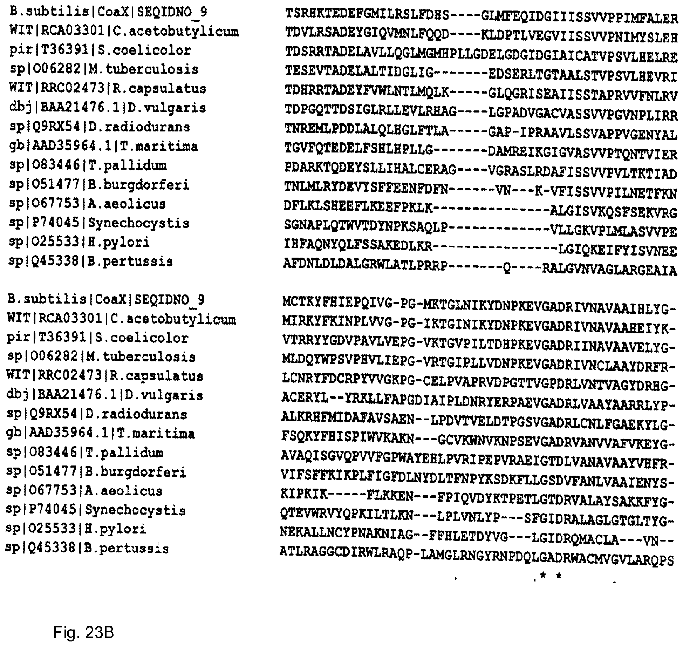

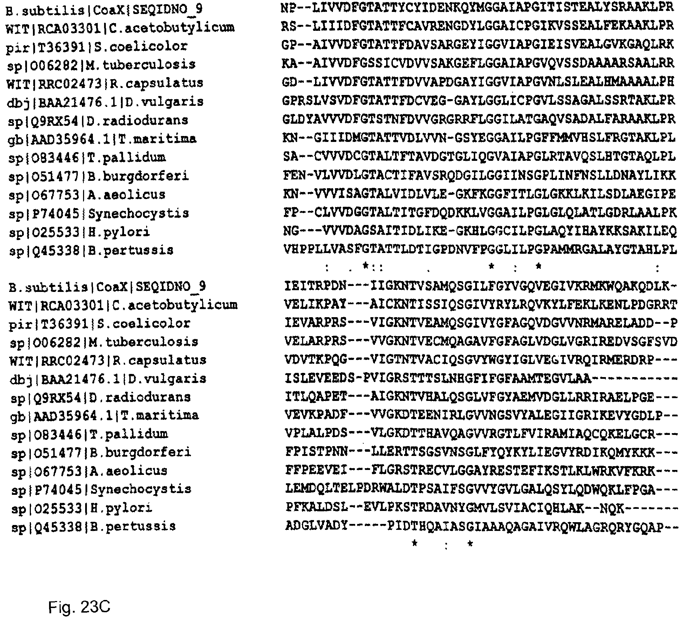

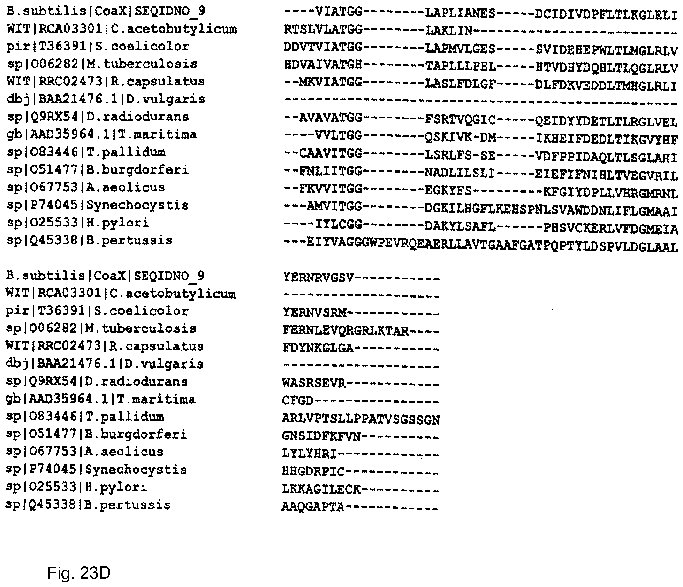

It will be appreciated by the skilled artisan that even a single substitution in a nucleic acid or gene sequence (*e.g*., a base substitution that encodes an amino acid change in the corresponding amino acid sequence) can dramatically affect the activity of an encoded polypeptide or protein as compared to the corresponding wild-type polypeptide or protein. A mutant nucleic acid or mutant gene (*e.g*., encoding a mutant polypeptide or protein), as defined herein, is readily distinguishable from a nucleic acid or gene encoding a protein homologue, as described above, in that a mutant nucleic acid or mutant gene encodes a protein or polypeptide having an altered activity, optionally observable as a different or distinct phenotype in a microorganism expressing said mutant gene or nucleic acid or producing said mutant protein or polypeptide (*i.e*., a mutant microorganism) as compared to a corresponding microorganism expressing the wild-type gene or nucleic acid or producing said mutant protein or polypeptide. By contrast, a protein homologue has an identical or substantially similar activity, optionally phenotypically indiscernable when produced in a microorganism, as compared to a corresponding microorganism expressing the wild-type gene or nucleic acid. Accordingly it is not, for example, the degree of sequence identity between nucleic acid molecules, genes, protein or polypeptides that serves to distinguish between homologues and mutants, rather it is the activity of the encoded protein or polypeptide that distinguishes between homologues and mutants: homologues having, for example, low (*e.g*., 30-50% sequence identity) sequence identity yet having substantially equivalent functional activities, and mutants, for example sharing 99% sequence identity yet having dramatically different or altered functional activities. Exemplary homologues are set forth in Figure 20 (*i.e*., CoaA homologues) and in Figure 23 (*i.e*., CoaX homologues). Exemplary mutants are described in Examples XV and XVIII herein.

### VIII. Recombinant Nucleic Acid Molecules and Vectors

The present disclosure further features recombinant nucleic acid molecules (*e.g*., recombinant DNA molecules) that include nucleic acid molecules and/or genes described herein (*e.g*., isolated nucleic acid molecules and/or genes), preferably *Bacillus* genes, more preferably *Bacillus subtilis* genes, even more preferably *Bacillus subtilis* pantothenate kinase genes (*e.g*., *coaX* genes or *coaA* genes), pantothenate biosynthetic genes (*e.g*., genes encoding pantothenate biosynthetic enzymes, for example, *panB* genes encoding ketopantoate hydroxymethyltransferase, *panE* genes encoding ketopantoate reductase, *panC* genes encoding pantothenate synthetase, and/or *panD* genes encoding aspartate-α-decarboxylase) and/or isoleucine-valine (*ilv*) biosynthetic genes (*e.g*., *ilvBN* or *alsS* genes encoding acetohydroxyacid synthetase, *ilvC* genes encoding acetohydroxyacid isomeroreductase and/or *ilvD* genes encoding dihydroxyacid dehydratase).

The present disclosure further features vectors (*e.g*., recombinant vectors) that include nucleic acid molecules (*e.g*., isolated or recombinant nucleic acid molecules and/or genes) described herein. In particular, recombinant vectors are featured that include nucleic acid sequences that encode bacterial gene products as described herein, preferably *Bacillus* gene products, more preferably *Bacillus subtilis* gene products, even more preferably *Bacillus subtilis* pantothenate biosynthetic gene products (*e.g*., pantothenate biosynthetic enzymes, for example, ketopantoate hydroxymethyltransferase, ketopantoate reductase, pantothenate synthetase, and/or aspartate-α-decarboxylase) and/or isoleucine-valine biosynthetic gene products (*e.g*., acetohydroxyacid synthetase, acetohydroxyacid isomeroreductase and/or dihydroxyacid dehydratase).

The term "recombinant nucleic acid molecule" includes a nucleic acid molecule (*e.g*., a DNA molecule) that has been altered, modified or engineered such that it differs in nucleotide sequence from the native or natural nucleic acid molecule from which the recombinant nucleic acid molecule was derived (*e.g*., by addition, deletion or substitution of one or more nucleotides). Preferably, a recombinant nucleic acid molecule (*e.g*., a recombinant DNA molecule) includes an isolated nucleic acid molecule or gene of the present disclosure (*e.g*., an isolated *coaX, coaA, pan or ilv* gene) operably linked to regulatory sequences.

The term "recombinant vector" includes a vector (*e.g*., plasmid, phage, phasmid, virus, cosmid or other purified nucleic acid vector) that has been altered, modified or engineered such that it contains greater, fewer or different nucleic acid sequences than those included in the native or natural nucleic acid molecule from which the recombinant vector was derived. Preferably, the recombinant vector includes a *coaX, coaA, pan or ilv* gene or recombinant nucleic acid molecule including such *coaX, coaA, pan or ilv* gene, operably linked to regulatory sequences, for example, promoter sequences, terminator sequences and/or artificial ribosome binding sites (RBSs), as defined herein.

The phrase "operably linked to regulatory sequence(s)" means that the nucleotide sequence of the nucleic acid molecule or gene of interest is linked to the regulatory sequence(s) in a manner which allows for expression (*e.g*., enhanced, increased, constitutive, basal, attenuated, decreased or repressed expression) of the nucleotide sequence, preferably expression of a gene product encoded by the nucleotide sequence (*e.g*., when the recombinant nucleic acid molecule is included in a recombinant vector, as defined herein, and is introduced into a microorganism).

The term "regulatory sequence" includes nucleic acid sequences which affect (*e.g*., modulate or regulate) expression of other nucleic acid sequences. In one embodiment, a regulatory sequence is included in a recombinant nucleic acid molecule or recombinant vector in a similar or identical position and/or orientation relative to a particular gene of interest as is observed for the regulatory sequence and gene of interest as it appears in nature, *e.g*., in a native position and/or orientation. For example, a gene of interest can be included in a recombinant nucleic acid molecule or recombinant vector operably linked to a regulatory sequence which accompanies or is adjacent to the gene of interest in the natural organism (*e.g*., operably linked to "native" regulatory sequences, for example, to the "native" promoter). Alternatively, a gene of interest can be included in a recombinant nucleic acid molecule or recombinant vector operably linked to a regulatory sequence which accompanies or is adjacent to another (*e.g*., a different) gene in the natural organism. Alternatively, a gene of interest can be included in a recombinant nucleic acid molecule or recombinant vector operably linked to a regulatory sequence from another organism. For example, regulatory sequences from other microbes (*e.g*., other bacterial regulatory sequences, bacteriophage regulatory sequences and the like) can be operably linked to a particular gene of interest.

In one embodiment, a regulatory sequence is a non-native or non-naturally-occurring sequence (*e.g*., a sequence which has been modified, mutated, substituted, derivatized, deleted including sequences which are chemically synthesized). Preferred regulatory sequences include promoters, enhancers, termination signals, anti-termination signals and other expression control elements (*e.g*., sequences to which repressors or inducers bind and/or binding sites for transcriptional and/or translational regulatory proteins, for example, in the transcribed mRNA). Such regulatory sequences are described, for example, in Sambrook, J., Fritsh, E. F., and Maniatis, T. Molecular Cloning: A Laboratory Manual. 2nd, ed., Cold Spring Harbor Laboratory, Cold Spring Harbor Laboratory Press, Cold Spring Harbor, NY, 1989. Regulatory sequences include those which direct constitutive expression of a nucleotide sequence in a microorganism (*e.g*., constitutive promoters and strong constitutive promoters), those which direct inducible expression of a nucleotide sequence in a microorganism (*e.g*., inducible promoters, for example, xylose inducible promoters) and those which attenuate or repress expression of a nucleotide sequence in a microorganism (*e.g*., attenuation signals or repressor sequences). It is also within the scope of the present disclosure to regulate expression of a gene of interest by removing or deleting regulatory sequences. For example, sequences involved in the negative regulation of transcription can be removed such that expression of a gene of interest is enhanced.

In one embodiment, a recombinant nucleic acid molecule or recombinant vector of the present disclosure includes a nucleic acid sequence or gene that encodes at least one bacterial gene product (*e.g*., a pantothenate biosynthetic enzyme, an isoleucine-valine biosynthetic enzyme, or a CoaA biosynthetic enzyme, for example CoaA or CoaX) operably linked to a promoter or promoter sequence. Preferred promoters of the present disclosure include *Bacillus* promoters and/or bacteriophage promoters (*e.g*., bacteriophage which infect *Bacillus*). In one embodiment, a promoter is a *Bacillus* promoter, preferably a strong *Bacillus* promoter (*e.g*., a promoter associated with a biochemical housekeeping gene in *Bacillus* or a promoter associated with a glycolytic pathway gene in *Bacillus*). In another embodiment, a promoter is a bacteriophage promoter. In a preferred embodiment, the promoter is from the bacteriophage SP01. In a particularly preferred embodiment, a promoter is selected from the group consisting of *P₁₅, P₂₆* or *P_{veg}*, for example, the promoters set forth in SEQ ID NO:39, SEQ ID NO:40 or SEQ ID NO:41. Additional preferred promoters include *tef* (the translational elongation factor (TEF) promoter) and *pyc* (the pyruvate carboxylase (PYC) promoter), which promote high level expression in *Bacillus* (*e.g*., *Bacillus subtilis*)*.* Additional preferred promoters, for example, for use in Gram positive microorganisms include, but are not limited to, the *amyE* promoter or phage SP02 promoters. Additional preferred promoters, for example, for use in Gram negative microorganisms include, but are not limited to *tac, trp, tet, trp-tet, lpp, lac, lpp-lac, lacIq, T7, T5, T3, gal, trc, ara, SP6, λ-P_{R}* or *λ-P_{L}.*

In another embodiment, a recombinant nucleic acid molecule or recombinant vector of the present disclosure includes a terminator sequence or terminator sequences (e.g., transcription terminator sequences). The term "terminator sequences" includes regulatory sequences which serve to terminate transcription of a gene. Terminator sequences (or tandem transcription terminators) can further serve to stabilize mRNA (e.g., by adding structure to mRNA), for example, against nucleases.

In yet another embodiment, a recombinant nucleic acid molecule or recombinant vector of the present disclosure includes sequences which allow for detection of the vector containing said sequences (*i.e*., detectable and/or selectable markers), for example, sequences that overcome auxotrophic mutations, for example, *ursa3* or *ilvE*, fluorescent markers, and/or colorimetric markers (*e.g*., *lacZ*/β-galactosidase), and/or antibiotic resistance genes (*e.g*., *amp* or *tet*)*.*

In yet another embodiment, a recombinant nucleic acid molecule or recombinant vector of the present disclosure includes an artificial ribosome binding site (RBS). The term "artificial ribosome binding site (RBS)" includes a site within an mRNA molecule (*e.g*., coded within DNA) to which a ribosome binds (*e.g*., to initiate translation) which differs from a native RBS (*e.g*., a RBS found in a naturally-occurring gene) by at least one nucleotide. Preferred artificial RBSs include about 5-6, 7-8, 9-10, 11-12, 13-14, 15-16, 17-18, 19-20, 21-22, 23-24, 25-26, 27-28, 29-30 or more nucleotides of which about 1-2, 3-4, 5-6, 7-8, 9-10, 11-12, 13-15 or more differ from the native RBS (*e.g*., the native RBS of a gene of interest). Preferably, nucleotides which differ are substituted such that they are identical to one or more nucleotides of an ideal RBS (*e.g*., SEQ ID NO:44, SEQ ID NO:45, SEQ ID NO:46, SEQ ID NO:47 or SEQ ID NO:48), when optimally aligned for comparisons. Artificial RBSs can be used to replace the naturally-occurring or native RBS associated with a particular gene. Artificial RBSs preferably increase translation of a particular gene. Preferred artificial RBSs (*e.g*., RBSs for increasing the translation of *panB*, for example, of *B. subtilis panB*) are depicted in Table IA (*e.g*., SEQ ID NO:49 and SEQ ID NO:50).

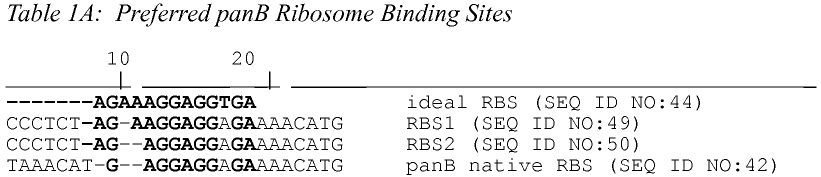

Additional preferred artificial RBSs (*e.g.,* RBSs for increasing the translation of *panD*, for example, of *B. subtilis panD*) are depicted in Table 1B (*e.g.,* SEQ ID NO:51, SEQ ID NO:52, SEQ ID NO:53 and SEQ ID NO:54).

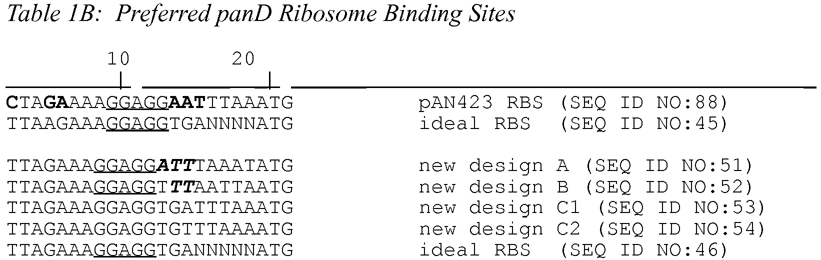

Additional preferred artificial RBSs (*e.g.,* RBSs for increasing the translation of *panD,* for example, of *B. subtilis panD*) are depicted in Table 1C (*e.g.,* SEQ ID NO:55, SEQ ID NO:56 and SEQ ID NO:57). The predicted amino acid sequence at the C-terminus of the PanC protein is shown. The start codon for PanD translation is underlined.

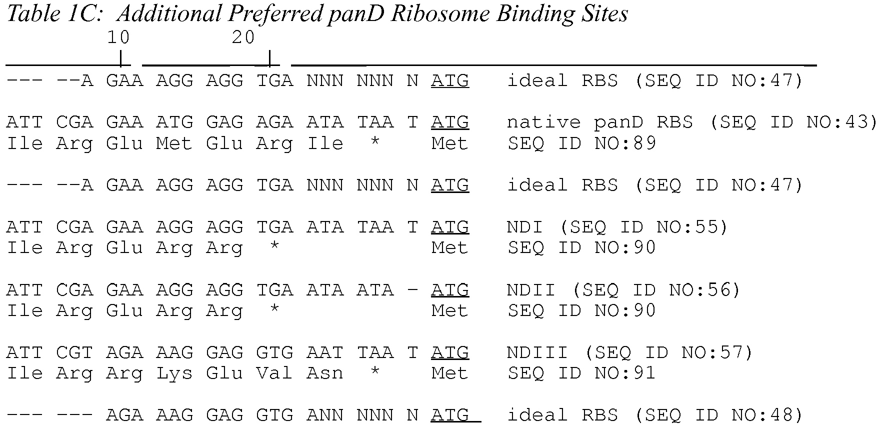

Accordingly, in one embodiment, a vector of the present disclosure includes an artificial RBS as set forth in SEQ ID NO:49 or SEQ ID NO:50. In another embodiment, a vector of the present disclosure includes an artificial RBS as set forth in SEQ ID NO:51, SEQ ID NO:52, SEQ ID NO:53 or SEQ ID NO:54. In yet another embodiment, a vector of the present disclosure includes an artificial RBS as set forth in SEQ ID NO:55, SEQ ID NO:56 or SEQ ID NO:57.

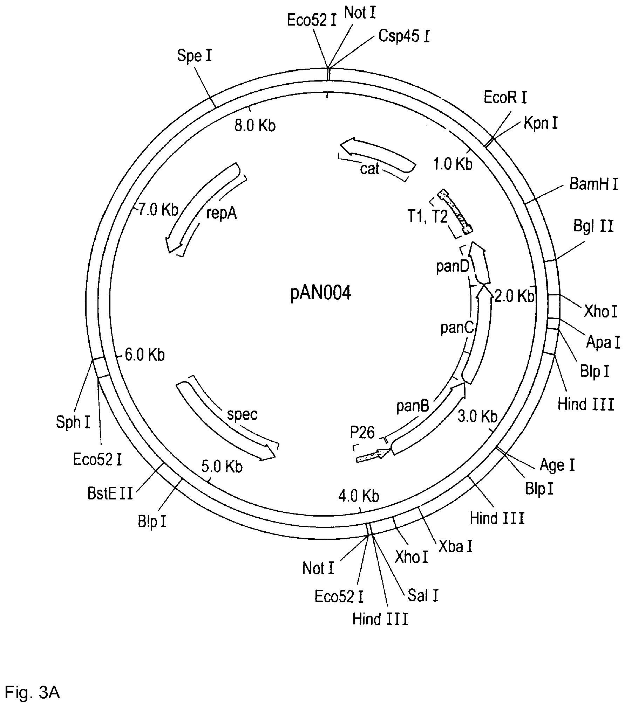

In another embodiment, a recombinant vector of the present disclosure includes sequences that enhance replication in bacteria (*e.g.,* replication-enhancing sequences). In one embodiment, replication-enhancing sequences are derived from *E. coli.* In another embodiment, replication-enhancing sequences are derived from pBR322 (*e.g.,* sequences included within the pBR322 derived portion of any of the pAN vectors as set forth in the Figures , *i.e*., the Not I-Not I sequences from about 5.0 kB to 9.0 kB of the vector depicted in Figure 3A).

In yet another embodiment, a recombinant vector of the present disclosure includes antibiotic resistance genes. The term "antibiotic resistance genes" includes sequences which promote or confer resistance to antibiotics on the host organism (*e.g., Bacillus*)*.* In one embodiment, the antibiotic resistance genes are selected from the group consisting of *cat* (chloramphenicol resistance) genes, *tet* (tetracycline resistance) genes, *erm* (erythromycin resistance) genes, *neo* (neomycin resistance) genes and *spec* (spectinomycin resistance) genes. Recombinant vectors of the present disclosure can further include homologous recombination sequences (*e.g.,* sequences designed to allow recombination of the gene of interest into the chromosome of the host organism). For example, *amyE* sequences can be used as homology targets for recombination into the host chromosome.

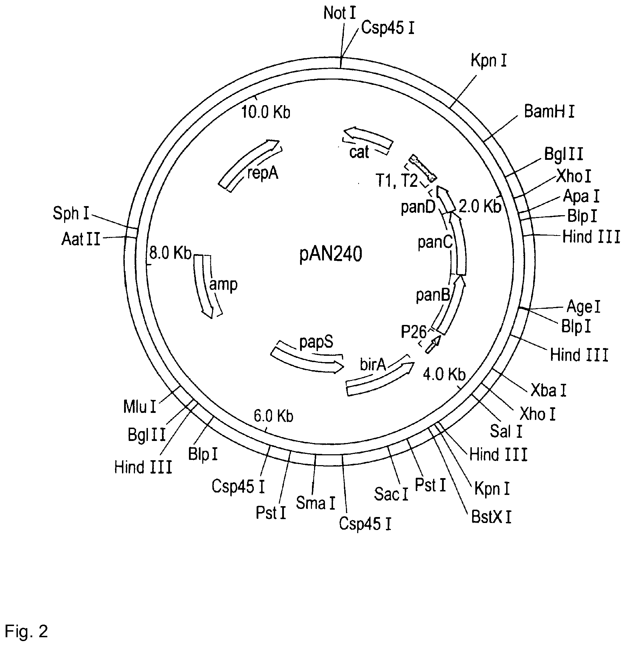

Preferred vectors of the present disclosure include, but are not limited to, vectors set forth in Figures 2-15, 17, 19, 22, 25 and 26. It will further be appreciated by one of skill in the art that the design of a vector can be tailored depending on such factors as the choice of microorganism to be genetically engineered, the level of expression of gene product desired and the like.

### IX. Isolated Proteins

Another aspect of the present disclosure features isolated proteins (*e.g.,* isolated pantothenate biosynthetic enzymes and/or valine-isoleucine biosynthetic enzymes and/or isolated CoA biosynthetic enzymes, for example isolated CoaA or CoaX). In one embodiment, proteins (*e.g.,* isolated pantothenate biosynthetic enzymes and/or valine-isoleucine biosynthetic enzymes and/or isolated CoaA biosynthetic enzymes, for example isolated CoaA or CoaX) are produced by recombinant DNA techniques and can be isolated from microorganisms of the present disclosure by an appropriate purification scheme using standard protein purification techniques. In another embodiment, proteins are synthesized chemically using standard peptide synthesis techniques.

An "isolated" or "purified" protein (*e.g.,* an isolated or purified biosynthetic enzyme) is substantially free of cellular material or other contaminating proteins from the microorganism from which the protein is derived, or substantially free from chemical precursors or other chemicals when chemically synthesized. In one embodiment, an isolated or purified protein has less than about 30% (by dry weight) of contaminating protein or chemicals, more preferably less than about 20% of contaminating protein or chemicals, still more preferably less than about 10% of contaminating protein or chemicals, and most preferably less than about 5% contaminating protein or chemicals.

In a preferred embodiment, the protein or gene product is derived from *Bacillus* (*e.g.,* is *Bacillus*-derived). The term "derived from *Bacillus*" or *"Bacillus-*derived" includes a protein or gene product which is encoded by a *Bacillus* gene. Preferably, the gene product is derived from a microorganism selected from the group consisting of *Bacillus subtilis, Bacillus lentimorbus*, *Bacillus lentus, Bacillus firmus*, *Bacillus pantothenticus, Bacillus amyloliquefaciens*, *Bacillus cereus, Bacillus circulans*, *Bacillus coagulans, Bacillus licheniformis*, *Bacillus megaterium*, *Bacillus pumilus*, *Bacillus thuringiensis*, and other Group 1 *Bacillus* species, for example, as characterized by 16S rRNA type (Priest, *supra*). In another preferred embodiment, the protein or gene product is derived from *Bacillus brevis* or *Bacillus stearothermophilus.* In another preferred embodiment, the protein or gene product is derived from a microorganism selected from the group consisting of *Bacillus licheniformis*, *Bacillus amyloliquefaciens*, *Bacillus halodurans*, *Bacillus subtilis*, and *Bacillus pumilus.* In a particularly preferred embodiment, the protein or gene product is derived from *Bacillus subtilis* (*e.g.,* is *Bacillus subtilis*-derived). The term "derived from *Bacillus subtilis*" or "*Bacillus subtilis*-derived" includes a protein or gene product which is encoded by a *Bacillus subtilis* gene. In yet another preferred embodiment, the protein or gene product is encoded by a *Bacillus* gene homologue (*e.g.,* a gene derived from a species distinct from *Bacillus* but having significant homology to a *Bacillus* gene of the present disclosure, for example, a *Bacillus pan* gene or *Bacillus ilv* gene).

Included within the scope of the present disclosure are bacterial-derived proteins or gene products and/or *Bacillus*-derived proteins or gene products (*e.g.,* B. *subtilis*-derived gene products) that are encoded by naturally-occurring bacterial and/or *Bacillus* genes (*e.g., B. subtilis* genes), for example, the genes identified by the present inventors, for example, *Bacillus* or *B. subtilis coaX* genes, *coaA* genes, *pan* genes and/or *ilv* genes. Further included within the scope of the present disclosure are bacterial-derived proteins or gene products and/or *Bacillus*-derived proteins or gene products (*e.g., B. subtilis*-derived gene products) that are encoded bacterial and/or *Bacillus* genes (*e.g., B. subtilis* genes) which differ from naturally-occurring bacterial and/or *Bacillus* genes (*e.g., B. subtilis* genes), for example, genes which have nucleic acids that are mutated, inserted or deleted, but which encode proteins substantially similar to the naturally-occurring gene products of the present disclosure. For example, it is well understood that one of skill in the art can mutate (*e.g.,* substitute) nucleic acids which, due to the degeneracy of the genetic code, encode for an identical amino acid as that encoded by the naturally-occurring gene. Moreover, it is well understood that one of skill in the art can mutate (*e.g.,* substitute) nucleic acids which encode for conservative amino acid substitutions. It is further well understood that one of skill in the art can substitute, add or delete amino acids to a certain degree without substantially affecting the function of a gene product as compared with a naturally-occurring gene product, each instance of which is intended to be included within the scope of the present disclosure.

In a preferred embodiment, an isolated protein of the present disclosure *(e.g.,* an isolated pantothenate biosynthetic enzyme and/or an isolated isoleucine-valine biosynthetic enzyme and/or an isolated CoaA biosynthetic enzymes, for example isolated CoaA or CoaX) has an amino acid sequence shown in SEQ ID NO:3, SEQ ID NO:9, SEQ ID NO:24, SEQ ID NO:26, SEQ ID NO:28, SEQ ID NO:30, SEQ ID NO:32, SEQ ID NO:34, SEQ ID NO:36, SEQ ID NO:38 or SEQ ID NO:87. In other embodiments, an isolated protein of the present disclosure is a homologue of the at least one of the proteins set forth as SEQ ID NO:3, SEQ ID NO:9, SEQ ID NO:24, SEQ ID NO:26, SEQ ID NO:28, SEQ ID NO:30, SEQ ID NO:32, SEQ ID NO:34, SEQ ID NO:36, SEQ ID NO:38 or SEQ ID NO:87 (*e.g*., comprises an amino acid sequence at least about 30-40% identical, preferably about 40-50% identical, more preferably about 50-60% identical, and even more preferably about 60-70%, 70-80%, 80-90%, 90-95% or more identical to the amino acid sequence of SEQ ID NO:3, SEQ ID NO:9, SEQ ID NO:24, SEQ ID NO:26, SEQ ID NO:28, SEQ ID NO:30, SEQ ID NO:32, SEQ ID NO:34, SEQ ID NO:36, SEQ ID NO:38 or SEQ ID NO:87, and has an activity that is substantially similar to that of the protein encoded by the amino acid sequence of SEQ ID NO:3, SEQ ID NO:9, SEQ ID NO:24, SEQ ID NO:26, SEQ ID NO:28, SEQ ID NO:30, SEQ ID NO:32, SEQ ID NO:34, SEQ ID NO:36, SEQ ID NO:38 or SEQ ID NO:87, respectively.

To determine the percent homology of two amino acid sequences or of two nucleic acids, the sequences are aligned for optimal comparison purposes (*e.g.,* gaps can be introduced in the sequence of a first amino acid or nucleic acid sequence for optimal alignment with a second amino or nucleic acid sequence). When a position in the first sequence is occupied by the same amino acid residue or nucleotide as the corresponding position in the second sequence, then the molecules are identical at that position. The percent identity between the two sequences is a function of the number of identical positions shared by the sequences (*i.e.,* % identity = # of identical positions/total # of positions x 100), preferably taking into account the number of gaps and size of said gaps necessary to produce an optimal alignment.

The comparison of sequences and determination of percent homology between two sequences can be accomplished using a mathematical algorithm. A preferred, non-limiting example of a mathematical algorithm utilized for the comparison of sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the NBLAST and XBLAST programs (version 2.0) of Altschul et al. (1990) J. Mol. Biol. 215:403-10. BLAST nucleotide searches can be performed with the NBLAST program, score = 100, wordlength = 12 to obtain nucleotide sequences homologous to nucleic acid molecules of the disclosure. BLAST protein searches can be performed with the XBLAST program, score = 50, wordlength = 3 to obtain amino acid sequences homologous to protein molecules of the disclosure. To obtain gapped alignments for comparison purposes, Gapped BLAST can be utilized as described in Altschul et al. (1997) Nucleic Acids Research 25(17):3389-3402. When utilizing BLAST and Gapped BLAST programs, the default parameters of the respective programs (e.g., XBLAST and NBLAST) can be used. See http://www.ncbi.nlm.nih.gov. Another preferred, non-limiting example of a mathematical algorithm utilized for the comparison of sequences is the algorithm of Myers and Miller (1988) Comput Appl Biosci. 4:11-17. Such an algorithm is incorporated into the ALIGN program available, for example, at the GENESTREAM network server, IGH Montpellier, FRANCE (http://vega.igh.cnrs.fr) or at the ISREC server (http://www.ch.embnet.org). When utilizing the ALIGN program for comparing amino acid sequences, a PAM120 weight residue table, a gap length penalty of 12, and a gap penalty of 4 can be used.

In another preferred embodiment, the percent homology between two amino acid sequences can be determined using the GAP program in the GCG software package (available at http://www.gcg.com), using either a Blossom 62 matrix or a PAM250 matrix, and a gap weight of 12, 10, 8, 6, or 4 and a length weight of 2, 3, or 4. In yet another preferred embodiment, the percent homology between two nucleic acid sequences can be accomplished using the GAP program in the GCG software package (available at http://www.gcg.com), using a gap weight of 50 and a length weight of 3.

### X. Biotransformations and Bioconversions

Another aspect of the present disclosure includes biotransformation processes which feature recombinant microorganisms (*e.g.,* mutant microorganisms) and/or isolated CoA, pantothenate or isoleucine-valine biosynthetic enzymes described herein. The term "biotransformation process", also referred to herein as "bioconversion processes", includes biological processes which result in the production (*e.g.,* transformation or conversion) of any compound (*e.g.,* intermediate or product) which is upstream of a CoA, pantothenate or isoleucine-valine biosynthetic enzyme to a compound (*e.g.,* substrate, intermediate or product) which is downstream of said CoA, pantothenate or isoleucine-valine biosynthetic enzyme.

In one embodiment, the disclosure features a biotransformation process for the production of a panto-compound comprising contacting a microorganism which overexpresses at least one pantothenate biosynthetic enzyme with at least one appropriate substrate or precursor under conditions such that said panto-compound is produced and recovering said panto-compound. In a preferred embodiment, the disclosure features a biotransformation process for the production of pantoate comprising contacting a microorganism which overexpresses ketopantoate reductase (the *panE* gene product) with an appropriate substrate (*e.g.,* ketopantoate) under conditions such that pantoate is produced and recovering said pantoate. In another preferred embodiment, the disclosure features a biotransformation process for the production of pantothenate comprising contacting a microorganism which overexpresses ketopantoate reductase and pantothenate synthetase with appropriate substrates (*e.g.*, ketopantoate and β-alanine) under conditions such that pantothenate is produced and recovering said pantothenate. In yet another preferred embodiment, the disclosure features a biotransformation process for the production of pantothenate comprising contacting a microorganism which overexpresses ketopantoate hydroxymethyltransferase, ketopantoate reductase and pantothenate synthetase with appropriate substrates (*e.g*., α-ketoisovalerate and β-alanine) under conditions such that pantothenate is produced and recovering said pantothenate. Preferred recombinant microorganisms for carrying out the above-described biotransformations include pantothenate kinase mutants. Conditions under which pantoate or pantothenate are produced can include any conditions which result in the desired production of pantoate or pantothenate, respectively.

In yet another embodiment, the present disclosure includes a method of producing β-alanine that includes culturing a microorganism which overexpresses aspartate-α-decarboxylase under conditions such that β-alanine is produced. Preferably, the aspartate-α-decarboxylase-overexpressing microorganism has a mutation in a nucleic acid sequence encoding a pantothenate biosynthetic enzyme selected from the group consisting of ketopantoate hydroxymethyltransferase, ketopantoate reductase and pantothenate synthetase.

The disclosure further features a method of producing β-alanine that includes contacting a composition comprising aspartate with an isolated *Bacillus* aspartate-α-decarboxylase enzyme under conditions such that β-alanine is produced (*e.g.,* an *in vitro* synthesis method).

The microorganism(s) and/or enzymes used in the biotransformation reactions are in a form allowing them to perform their intended function (*e.g.,* producing a desired compound). The microorganisms can be whole cells, or can be only those portions of the cells necessary to obtain the desired end result. The microorganisms can be suspended (*e.g.,* in an appropriate solution such as buffered solutions or media), rinsed (*e.g.,* rinsed free of media from culturing the microorganism), acetone-dried, immobilized (*e.g.,* with polyacrylamide gel or k-carrageenan or on synthetic supports, for example, beads, matrices and the like), fixed, cross-linked or permeablized (*e.g.,* have permeablized membranes and/or walls such that compounds, for example, substrates, intermediates or products can more easily pass through said membrane or wall).

Purified or unpurified CoA biosynthetic enzyme(s) (*e.g.,* CoaA and/or CoaX), pantothenate biosynthetic enzyme(s) and/or valine-isoleucine biosynthetic enzyme(s) can also be used in biotransformation reactions. The enzyme can be in a form that allows it to perform its intended function *(*e.g., obtaining the desired compound). For example, the enzyme can be in free form or immobilized. Purified or unpurified CoA biosynthetic enzyme(s), pantothenate biosynthetic enzyme(s) and/or valine-isoleucine biosynthetic enzyme(s) can be contacted in one or more *in vitro* reactions with appropriate substrate(s) such that the desired product is produced.

With respect to at least the above-described methodologies (*e.g.,* the production methodologies of the present disclosure), at least one aspect of the disclosure features the folowing: embodiments is which the methods do not use microorganisms of the genus *Corynebacterium* and/or microorganisms of the genus *Escherichia*; embodiments in which the methods do not use microorganisms selected from the group consiting of *Escherichia coli* and *Corynebacterium glutamicum*; embodiments in which the methods do not use gram negative microorganisms; embodiments in which the microorganisms utilized do not include, express or produce nucleic acid molecules, genes or proteins (*e.g*., biosynthetic emzymes) derived from microorganisms of the genus *Corynebacterium* and/or microorganisms of the genus *Escherichia*; embodiments in which the microorganisms to not include, express or produce nucleic acid molecules, genes or proteins (*e.g.*, biosynthetic emzymes) derived from microorganisms selected from the group consisting of *Escherichia coli* and *Corynebacterium glutamicum.*

### XI. Screening Assays

Because CoA is an essential factor in bacteria, proteins (*e.g.,* enzymes) involved in the biosynthesis of CoA provide valuable tools in the search for novel antibiotics. In particular, the CoaX protein is a valuable target for identifying bacteriocidal compounds because it bears no resemblance in primary sequence to mammalian pantothenate kinase enzymes. Accordingly, the present disclosure also provides a method (also referred to herein as a "screening assay") for identifying modulators, *i.e.,* candidate or test compounds or agents (*e.g.*, peptides, peptidomimetics, small molecules or other drugs) which bind to CoaX, or have a stimulatory or inhibitory effect on, for example, *coaX* expression or CoaX activity.

In one embodiment, the disclosure provides assays for screening candidate or test compounds which are capable of binding to CoaX proteins or a biologically active portion thereof. In another embodiment, the disclosure provides assays for screening candidate or test compounds which modulate the activity of CoaX proteins or biologically active portions thereof. The test compounds of the present disclosure can be obtained using any of the numerous approaches in combinatorial library methods known in the art, including: biological libraries; spatially addressable parallel solid phase or solution phase libraries; synthetic library methods requiring deconvolution; the 'one-bead one-compound' library method; and synthetic library methods using affinity chromatography selection. The biological library approach is limited to peptide libraries, while the other four approaches are applicable to peptide, non-peptide oligomer or small molecule libraries of compounds (Lam, K.S. (1997) Anticancer Drug Des. 12:145).

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233. Libraries of compounds may be presented in solution *(e.g.,* Houghten (1992) Biotechniques 13:412-421), or on beads (Lam (1991) Nature 354:82-84), chips (Fodor (1993) Nature 364:555-556), bacteria (Ladner USP 5,223,409), spores (Ladner USP'409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad. Sci. 87:6378-6382); (Felici (1991) J. Mol. Biol. 222:301-310); (Ladner *supra*.).

In one embodiment, an assay is a microorganism-based assay in which a recombinant microorganism which expresses a CoaX protein or biologically active portion thereof is contacted with a test compound and the ability of the test compound to modulate CoaX activity is determined. Determining the ability of the test compound to modulate CoaX activity can be accomplished by monitoring, for example, intracellular phosphopanthoate or CoA concentrations or secreted pantothenate concentrations (as compounds that inhibit CoaX will result in a buildup of pantothenate in the test microorganism). CoaX substrate can be labeled with a radioisotope or enzymatic label such that modulation of CoaX activity can be determined by detecting a conversion of labeled substrate to intermediate or product. For example, CoaX substrates can be labeled with ³²P, ¹⁴C, or ³H, either directly or indirectly, and the radioisotope detected by direct counting of radioemmission or by scintillation counting. Determining the ability of a compound to modulate CoaX activity can alternatively be determined by detecting the induction of a reporter gene (comprising a CoA-responsive regulatory element operatively linked to a nucleic acid encoding a detectable marker, e.g., luciferase), or detecting a CoA-regulated cellular response.

In yet another embodiment, a screening assay of the present disclosure is a cell-free assay in which a CoaX protein or biologically active portion thereof is contacted with a test compound *in vitro* and the ability of the test compound to bind to or modulate the activity of the CoaX protein or biologically active portion thereof is determined. In a preferred embodiment, the assay includes contacting the CoaX protein or biologically active portion thereof with known substrates to form an assay mixture, contacting the assay mixture with a test compound, and determining the ability of the test compound to modulate enzymatic activity of the CoaX on its substrates.

Screening assays can be accomplished in any vessel suitable for containing the microorganisms, proteins, and/or reactants. Examples of such vessels include microtiter plates, test tubes, and micro-centrifuge tubes. In more than one embodiment of the above assay methods of the present disclosure, it may be desirable to immobilize either CoaX protein or a recombinant microorganism expressing CoaX protein to facilitate separation of products and/or substrates, as well as to accommodate automation of the assay. For example, glutathione-S-transferase/CoaX fusion proteins can be adsorbed onto glutathione sepharose beads (Sigma Chemical, St. Louis, MO) or glutathione derivatized microtiter plates. Other techniques for immobilizing proteins on matrices (*e.g.,* biotin-conjugation and streptavidin immobilization or antibody conjugation) can also be used in the screening assays of the disclosure.

In another embodiment, modulators of CoaX expression are identified in a method wherein a cell is contacted with a candidate compound and the expression of *coaX* mRNA or CoaX polypeptide in the cell is determined. The level of expression in the presence of the candidate compound is compared to the level of expression in the absence of the candidate compound (or to a suitable control, for example, an appropriate buffer control or standard). The candidate compound can then be identified as a modulator of *coaX* mRNA or CoaX polypeptide expression based on this comparison.

This disclosure further pertains to novel agents identified by the above-described screening assays. Accordingly, it is within the scope of this disclosure to further use an agent identified as described herein in an appropriate animal model. For example, an CoaX modulating agent identified as described herein (*e.g.,* an anti-bactericidal compound) can be used in an infectious animal model to determine the efficacy, toxicity, or side effects of treatment with such an agent.

This disclosure is further illustrated by the following examples which should not be construed as limiting.

### EXAMPLES

### General Methodology:

***Strains.** Bacillus subtilis* strains of the present disclosure are generally derived from either of two strains. The first is variously named "168", "1A1", or "RL-1". The genotype is *trpC2.* This strain was derived from the wild type "Marburg" strain by mutagenesis and has been the basis of much of the molecular biology work done on *B. subtilis.* The second strain is PY79, a prototrophic derivative of 168 that was made Trp⁺ by transduction from the wild type strain W23.

***Media.*** Standard minimal medium for *B. subtilis* is comprised of 1 x Spizizen salts and 0.5% glucose. Standard solid "rich medium" is Tryptone Blood Agar Broth (Difco), and standard liquid "rich medium" is VY, a mixture of veal infusion broth and yeast extract. For testing production of pantothenate in liquid test tube cultures, an enriched form of VY, called "Special VY" or "SVY" is used. For batch fermentations, SVYG and PFMG are used. The compositions of these media are given below.
*VY, a rich liquid medium:* 25 g Difco Veal Infusion Broth, 5 g Difco Yeast Extract, 1L water (autoclave).
*TBAB, a rich solid medium:* 33 g Difco Tryptone Blood Agar Broth, 1L water (autoclave).
*MIN, a minimal medium:* 100 ml 10 x Spizizen salts; 10 ml 50% glucose; 2 ml 10% arginine HCl*; 10 ml 0.8% tryptophan**; water to 1 liter. (*In some cases, arginine is omitted or replace by sodium glutamate at 0.04% final concentration. In general, *B. subtilis* grows faster in minimal medium when certain amino acids, such as arginine, glutamine, glutamate, or proline, are added as an auxiliary nitrogen source.) (**For strains that are tryptophan auxotrophs, tryptophan is routinely added to most minimal media.)
*10 x Spizizen Salts:* 174 g K₂HPO₄·3H₂O; 20 g (NH₄)₂SO₄; 60 g KH₂PO₄; 10 g Na₃Citrate·2H₂O; 2 g MgSO₄·7H₂O; water to 993 mls; then add 3.5 ml FeCl₃ solution and 3.5 ml Trace Elements solution.
*FeCl₃Solution:* 4 g FeCl₃·6H₂O; 197 g Na₃Citrate·2H₂O; water to 1 liter (filter sterilize)
*Trace Elements Solution:* 0.15 g Na₂MoO₄·2H₂O; 2.5 g H₃BO₃; 0.7 g CoCl₂·6H₂O; 0.25 g CuSO₄·5H₂O; 1.6 g MnCl₂·4H₂O; 0.3 g ZnSO₄·7H₂O; water to 1 liter (filter sterilize).
*SVY, Special VY, a supplemented* rich medium for testing pantothenate production in test tube cultures:* 25 g Difco Veal Infusion Broth; 5 g Difco yeast extract; 5 g sodium glutamate; 2.7 g ammonium sulfate; 740 ml water (autoclave); add 200 ml 1 M potassium phosphate, pH 7.0; 60 ml 50% glucose. (*For testing pantothenate production in liquid SVY test tube cultures, Na α-ketoisovalerate and/or β -alanine can be added to a concentration of 5 g/L from filter-sterilized stocks.)
*PFMG, a yeast extract based medium used in fermentors:* 20 g Amberex 1003™ yeast extract; 5 g sodium glutamate, 2 g ammonium sulfate; 5 g tryptophan; 10 g KH₂PO4; 20 g K₂HPO₄·3H₂O; 1 g MgCl₂·6H₂O; 0.1 g CaCl₂·2H₂O; 1 g sodium citrate; 0.01 g FeSO₄·7H₂O; 1 ml trace elements solution; 20 g glucose; add water to 1 L. Glucose or other sugars are fed as needed. Feed solutions can contain minerals, defined or food grade nutrients.
*PF, a chemically defined pantothenate free medium for testing pantothenate auxotrophy:* 100 ml 10 x Spizizen Salts; 100 ml 1 x Difco Pantothenate Assay Medium; 10 ml 50% glucose; water to 1 liter.

For pantothenate auxotrophs, 1 mM Na pantothenate is added to both minimal and rich media, since there is generally not enough pantothenate in rich media to support *B. subtilis pan* mutants. Amino acids are at 100 mg per liter, when used. Selection for antibiotic resistance is done with 5 mg/L chloramphenicol, 100 mg/L spectinomycin HCl, 15 mg/L tetracycline HCl, or 1 mg/L erythromycin plus 25 mg/L lincomycin.

***Pantothenate Assays: Biological assay.*** The indicator organism, *Lactobacillus plantarum,* requires pantothenate for growth, and responds to low concentrations (µ g/L). Thus, using serial dilutions, a wide range of concentrations can be assayed. Commercially available medium (*e.g.,* Pantothenate Assay Medium (PAM), Difco), can be used. However, Difco PAM supplemented with pantothenate does not support growth to the same level as obtainable using a fresh-mixed version of Pantothenate Assay Medium (FM-PAM), made up of the individual components as specified by Difco, which is accordingly, routinely used instead of the commercial product.

Before assaying *B. subtilis* culture supernatants, the *B. subtilis* cells must be either removed or killed. *B. subtilis* culture supernatants give approximately the same pantothenate titer when the supernatants are autoclaved as when they are sterile filtered. Accordingly, routine procedures involve autoclaving samples for 5 minutes prior to the biological assay.

***Pantothenate Assays : HPLC assay.*** Pantothenic acid production is measured by HPLC with a detector wavelength of 197 nm and a reference at 450 nm. The procedure is a modification of one recommended by Hewlett-Packard for water soluble vitamins. Samples of culture broth are diluted into an equal volume of 60% acetronitrile (ACN), centrifuged and filtered. Typically a further 10-fold dilution before analysis brings the final dilution to 20-fold. Higher concentrations of product are diluted further. Compounds are separated on a C18 Phenomenex 5µ Aqua 250 x 4.6 mm column with 5% acetronitrile (ACN) in 50 mM Na phosphate buffer at pH 2.5. An ACN gradient from 5% to 95% washes the column between every sample. The area of the pantothenate peak is proportional to the concentration between 5 to 1000 mg/L. Other panto-compounds are also separated and quantitated by this method.

***Amino Acid Analysis: HPLC assay.*** Amino acids present in the fermentation medium and throughout the fermentation are measured by HPLC with a detector wavelength of 338 nm and a reference at 390 nm. The procedure is a modification of one recommended by Hewlett-Packard for amino acid analysis. Samples of culture broth are prepared identically as for the panto-compound analysis. Compounds are separated on a C18 Hypersil 5µ ODS 200 x 2.1 mm column. Solvent A is 20 mM Na acetate buffer at pH 7.2. Solvent B contains 40% ACN and 40% methanol. A gradient from 100% Solvent A to 100% Solvent B separates amino acids and washes the column between every sample.

***Batch Fermentations.*** Pantothenate producing strains are grown in stirred tank fermentors, for example, in CF3000 Chemap 14 liter vessels with 10 liter working volumes. Computer control and data collection is by commercial software, for example, B. Braun Biotech MFCS software. Fermentations can be batch processes but are preferably sugar-limited, fed batch processes. Some media components (e.g. of SVYG and PMFG) are added to the fermentor and sterilized in place. Portions of the media are sterilized separately and added to the fermentors aseptically. This procedure is well known to those familiar with the art. Additional nitrogen sources in feeds are sterilized separately and added to the carbon source after cooling.

The initial sugar in the medium is consumed in approximately 6 hours. Afterwards, glucose or other sugars are fed with the possible addition of minerals, and defined or food grade nutrients. Alternatively, feeds are scheduled based on a consensus profile of nutritional requirements from samples taken from earlier fermentations.

After inoculation, agitation is set at a relatively low speed, *e.g.* 200 rpm. When the dissolved oxygen (pO2) falls to 30%, computer control automatically adjusts the agitation to maintain a dissolved oxygen concentration between 25 and 30% pO2.

### EXAMPLE I: Enhanced Production of a Panto-Compound Using Bacteria Overexpressing panBCD Gene Products.

This Example describes the cloning of the *B. subtilis panBCD* operon and the generation of microorganisms overexpressing the *panBCD* gene products.

To clone the *B. subtilis panBCD* operon, a plasmid library of *B. subtilis* GP275 (a derivative of 168) genomic DNA was transformed in *E. coli* BM4062 (*birA*^{ts}), and temperature resistant clones were selected at 42° C. By comparison of restriction maps to the genome sequence, one particular clone was deduced to contain the *B. subtilis birA* gene and the adjacent *panBCD* genes. This plasmid was named pAN201.

To overexpress the *panBCD* operon and produce pantothenate, the native promoter of the *panBCD* operon was replaced by either of two strong, constitutive promoters derived from the *B. subtilis* bacteriophage SP01. These two promoters are named *P₂₆* and *P₁₅*. In addition, either of two artificial ribosome binding sites (RBSs) were used to replace the native *panB* RBS. These two artificial RBSs (set forth as SEQ ID NO:49 and SEQ ID NO:50) were predicted to increase translation of *panBCD*; their sequences are shown in Table 1A. Three such engineered *panBCD* expression cassettes were built into circular plasmids capable of replicating in *E. coli.* Other features of the plasmids include a strong rho-independent transcription terminator from the *E.* coli ribosomal RNA transcription unit, called T₁T₂, a Gram-positive chloramphenicol resistance gene *(cat),* derived from pC194, and a pair of *Not*I restriction sites at the junctions between the *E. coli* replicon and the segment intended for integration into *B. subtilis.* Three plasmids of this series, pAN004, pAN005, and pAN006 were constructed. pAN004 contains the *P₂₆* promoter, RBS1, and a low copy *E. coli* replicon. pAN005 contains the *P₁₅* promoter, which in our experience is not as strong as *P₂₆*, RBS1, and the low copy replicon. pAN006 contains the *P₂₆* promoter, RBS2, and a medium copy replicon.

The three *panBCD* expression cassettes contained in the above-mentioned three plasmids were all ligated to a DNA fragment consisting of sequences that naturally occur immediately upstream from the native *panB* gene and integrated in single copy by homologous recombination into the *panBCD* locus of *B. subtilis* strains RL-1 and PY79, replacing the wild-type operon. This was accomplished in two steps. First a deletion-substitution that replaced about two thirds of the *panB* coding region with a Gram-positive spectinomycin resistance gene (*spec*) was integrated at *panB* to yield Spec⁻, pantothenate auxotrophs. These intermediate strains were than transformed with the *panBCD* expression cassettes of pAN004, pAN005, and pAN006 after ligating them to a DNA fragment containing chromosomal sequences just upstream of *panB.* Selection of the incoming cassette was for pantothenate prototrophy. The resulting strains were named PA221, PA222 and PA223 (from RL-1), and PA235, PA232 and PA233 (from PY79), respectively. An example of a plasmid that contains the joined upstream sequence that is in the integrated strain in PA221 is pAN240 (see Figure 2). The nucleotide sequence of pAN240 is set forth as SEQ ID NO:76.

Polymerase chain reaction using appropriate primers was used to verify the correct chromosomal structures of these engineered strains. When extracts of strain PA221 were examined by SDS-PAGE, two proteins were found to be overexpressed. One protein had an apparent molecular weight of 29,000 and the other protein appeared to be 39,000 daltons. The 29,000 dalton bands is presumably PanB (predicted molecular weight of 29,761). The larger protein band presumably represents PanC (predicted size 31,960 daltons).

The ability of these strains to produce pantothenate in test tube cultures was assesed as follows. Each strain was grown in SVY medium supplemented with 5 g/L α-ketoisovalerate (α-KIV) and 5 g/L β -alanine, to ensure that these precursors were not limiting. Culture supernatants were autoclaved and assayed using the bioassay. Relative to the parent strains, RL-1 and PY79, the engineered strains produced about 8- to 30-fold more pantothenate, attaining 1 g/L pantothenate in some cases.

**Table 2. Production of pantothenate by engineered B. subtilis strains in liquid test tube cultures grown in SVY medium with 5 g/L α-KIV and 5 g/L β-alanine.**

| Expt. | Strain | Promoter | RBS at *pan*B | [pantothenate] mg/L |
|---|---|---|---|---|
| 1 | RL-1 | Native | Native | 30 |
| | PA221 | P*₂₆* | RBS1 | 990 |
| | | | | 790 |
| | PA222 | P*₁₅* | RBS1 | 250 |
| | | | | 250 |
| | PA223 | P*₂₆* | RBS2 | 790 |
| | | | | 790 |
| 2 | PY79 | Native | Native | 40 |
| | PA235 | P*₂₆* | RBS1 | 930 |
| | | | | 860 |
| | PA221 | P*₂₆* | RBS1 | 1100 |
| | | | | 1030 |

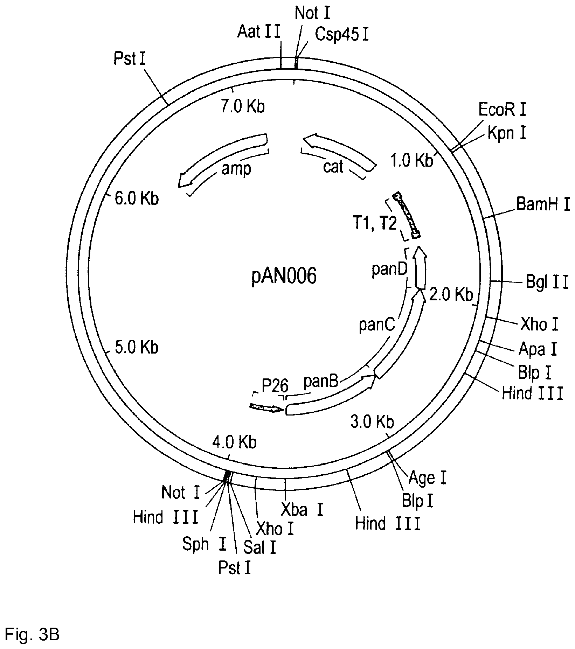

The *P₂₆* promoter was about 3- to 4-fold more effective than the *P₁₅* promoter, while RBS1 and RBS2 were roughly equivalent. Plasmids such as pAN004, pAN005, pAN006 can also be recombined as circles into the *B. subtilis* wild type *panBCD* locus by Campbell-type (single crossover) integration, selecting for chloramphenicol resistance at 5 mg/L. Strains obtained in this fashion produce about the same amount of pantothenate as strains PA221, PA222, and PA223, respectively. pAN004 containing the *P₂₆* promoter, RBS1 and a low copy *E. coli* replicon, is depicted schematically in Figure 3A. The nucleotide sequence of plasmid pAN004 is set forth as SEQ ID NO:93. pAN006 containing the *P₂₆* promoter, RBS2 and a medium copy *E. coli* replicon, is depicted schematically in Figure 3B. The nucleotide sequence of plasmid pAN006 is set forth as SEQ ID NO:94. The nucleotide sequence of *panBCD* is set forth as SEQ ID NO:59 and the predicted amino acid sequences of PanB, PanC and PanD are set forth as SEQ ID NO:24, SEQ ID NO:26 and SEQ ID NO:28, respectively. Methods for manipulating *Bacilli* are described, for example, in Harwood, C.R. and Cutting, S.M. (editors), Molecular Biological Methods for Bacillus (1990) John Wiley & Sons, Ltd., Chichester, England.

### EXAMPLE II: Enhanced Production of a Panto-Compound Using Bacteria Overexpressing the panE1 Gene Product - Ketopantoate Reductase.

This Example describes the cloning of the *B. subtilis panE1* gene and the generation of microorganisms overexpressing the *panE1* gene product.

Pan⁻ *B. subtilis* strains (*e.g., B. subtilis* mutants blocked in the synthesis of pantothenic acid) had previously been isolated, one of which was reported to be affected in ketopantoate reductase activity (Baigori et al. (1991) J. Bacteriol. 173:4240-4242). However, the mutations in these strains were incorrectly mapped to the *purE-tre* interval of the *B. subtilis* genetic map which does not contain the *panE* or *panBCD* genes. Furthermore as shown below, a *panE* mutant does not have a Pan⁻ phenotype as the *ilvC* gene product can substitute for the *panE* gene product in *B. subtilis* as in other bacterial strains such as *E. coli.* More recently, the *S. typhimuruim panE* gene has been located and determined to be allelic to *apbA*, a gene required for anaerobic purine biosynthesis (Frodyma et al. (1998) J. Biol. Chem. 273:5572-5576). *E. coli* carries a highly homologous gene at the same map location. Identification of the *panE* genes in *E. coli* and *S. typhimurium* was complicated by the fact that the *ilvC* gene product, acetohydroxy acid isomeroreductase, is also capable of carrying out the ketopantoate reductase reaction. As a result, pantothenate auxotrophy is not obtained unless both *panE* and *ilvC* are mutated.

To identify the *B. subtilis panE1* gene, the *B. subtilis* genome was searched using the protein sequence of *E. coli* or *S. typhimurium* ApbA (PanE), and two open reading frames were identified having homology to ApbA, named *ylbQ* and *ykpB.* These genes were renamed *panE1* and *panE2,* due to their proposed function in pantothenate biosynthesis. Both *panE1* and *panE2* were cloned as PCR products generated from RL-1 genomic DNA as a template. Both genes were disrupted by either a spectinomycin resistance gene (*spec*) or a chloramphenicol resistance gene (*cat*). The interrupted genes were each integrated by double crossover into PY79 to give PA240 (*ΔpanEl::spec*) and PA241 (Δ*panE2*::*cat*). Neither of these strains were pantothenate auxotrophs when tested on pantothenate-free (PF) plates, although PA240 containing *ΔpanEl::spec* grew slightly more slowly on TBAB without added pantothenate than with a 1 mM pantothenate supplement. By comparison, a Δ*panB::spec* strain does not produce single colonies on TBAB, presumably because *B. subtilis* has no active uptake system for pantothenate.

It was hypothesized that the *B. subtilis* gene, *ilvC*, could function for *panE* as had been shown for *E. coli.* Accordingly, the *panE1* and *panE2* disruptions were introduced into a strain, CU550, which is reported to be *trpC2 ilvC4 leuC124.* Both the single *panE1* and the double *panE1, panE2* disruptants were pantothenate auxotrophs on PF medium.

**Table 3. Phenotypes of various panE1 and panE2 mutants on rich and defined media.**

| | | Growth*: | | |
|---|---|---|---|---|
| Strain | Medium | - pan | + pan | |
| PY79 | TBAB | +++ | +++ | |
| | PF | ++ | ++ | |
| PA240 | TBAB spec | + | +++ | |
| | PF | ++ | ++ | |
| PA241 | TBAB cam | +++ | +++ | |
| | PF | ++ | ++ | |
| CU550 | TBAB | +++ | +++ | |
| | PF | ++ | ++ | |
| PA256 | TBAB spec | - | +++ | |
| | PF | - | ++ | |
| PA258 | TBAB spec, cam | - | +++ | |
| | PF | - | ++ | |

| | | | | |
|---|---|---|---|---|
| *Each "+" represents about 1 mm of colony diameter after overnight at 37° C. | | | | |

Thus, mutating both *panE1* and *ilvC* results in pantothenate auxotrophy, while mutating only *panE1* does not, similar to what has been reported for *E.coli* and *S. typhimurium*.

Next, the quantitative effect of *panE1* and *panE2* knockouts in a pantothenate overproducing strain (PA235 described herein) was examined. The *panE1* and *panE2* disruptions were introduced into PA235, either singly or together to produce PA245 (Δ*panE1*::spec), PA248 (Δ*panE2*::cat) and PA244 (Δ*panE1*::cat, Δ*panE2*::spec). The effect of each mutation on pantothenate production was then tested in liquid test tube cultures.

**Table 4. Pantothenate production by PA235 derivatives containing panE1 and panE2 disruptions.**

| Strain | [pan] mg/L | % of PA235 |
|---|---|---|
| PA235 | 990 | (100) |
| PA235 | 940 | 95 |
| PA245 | 59 | 6 |
| PA245 | 82 | 8 |
| PA248 | 1060 | 106 |
| PA248 | 1030 | 104 |
| PA244 | 25 | 3 |
| PA244 | 50 | 5 |

Thus, deletion analysis indicated that the *panE1* gene contributes to over 90% of the pantothenate production, while deletion of *panE2* did not have a significant effect on pantothenate production. It is therefore concluded that *panE1* accounts for most, but not necessarily all, of the ketopantoate reductase activity in *B. subtilis.* The rest of the ketopantoate reductase activity is predicted to be supplied by *ilvC.*

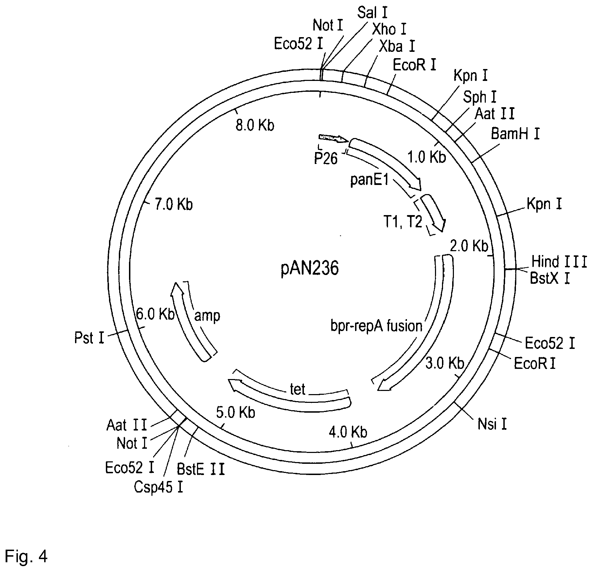

Having identified *panE1* as an important gene for pantothenate production, increased *panE1* expression was tested to determine whether it could enhance pantothenate production in strains such as PA221 or PA235. The *panE1* coding sequence was installed downstream of the *P₂₆* promoter and RBS2 in a vector, pOTP61, designed to integrate and amplify at either the *bpr* locus (a non-essential protease gene) or at the locus of the cloned insert. The resulting plasmid, pAN236 (Figure 4) was transformed into PA221, selecting for resistance to tetracycline at 15 mg/L. The nucleotide sequence of pAN236 is set forth as SEQ ID NO:77. One transformant, named PA236 was chosen for further study.

PA236 was shown to overexpress a protein of about 31,000 daltons, which is close to the expected molecular weight of 33,290 daltons for *panE1* protein. Briefly, whole cell extracts were prepared from PY79, RL-1, PA221, PA221/pOTP61 and PA236 (2 samples). Cell extracts were separated by gel electrophoresis and the gels were coomassie stained to visualize proteins. In cells engineered to overexpress *panE* (PA236-1 and PA236-2), a band was visible having an approximate molecular weight of ∼31,000 daltons (as compared to molecular weight markers). Moreover, PA221 and PA236 expressed increased levels of a ∼29,000 dalton band, corresponding to the *panB* gene product, and a ∼39,000 dalton band, presumably corresponding the *panC* gene product. Furthermore, *E. coli* transformed with pAN006 (Figure 3B) expressed bands correlating to the *panB* and *panC* gene products and *E. coli* transfected with PAN236 expressed a ∼31,000 dalton band corresponding to the *panE* gene product.

Next, PA236 was compared to PA221 carrying the empty vector pOTP61 for pantothenate production in liquid test tube cultures supplemented with 5 g/L β-alanine and 5 g/L α-KIV.

**Table 5. Effect of overexpression of panE1 and panE2 on pantothenate production by engineered strains in liquid test tube cultures.**

| Strain | Additional Plasmid | Gene Overexpressed | [Pantothenate] mg/L |
|---|---|---|---|
| PA221 | pOTP61 | none | 1,000 |
| | | | 940 |
| PA236 | pAN236 | *panE1* | 2,030 |
| | | | 2,050 |
| PA238 | pAN238 | *panE2* | 530 |
| | | | 680 |

Overexpression of *panE1* caused a two-fold increase in pantothenate production when compared to the parent strain *(e.g.,* to slightly over 2 g/L) whereas overexpression of *panE2* resulted in a strain that produced about 35% less pantothenate than the parent strain. The *panE1* nucleotide sequence and predicted amino acid sequence are set forth as SEQ ID NO:29 and SEQ ID NO:30.

### EXAMPLE III: Enhanced Production of a Panto-Compound by Culturing Bacteria Overexpressing panE1 or panBCD in the Presence of Valine.

The ability of valine to function as a media supplement (e.g., as a substitute for α-KIV) in strains engineered to overexpress the *panBCD* operon and *panE1* was evaluated. Valine is closely related to α-KIV by transamination, is less expensive than α-KIV, and is commercially available in kilogram quantities. Valine was substituted for α-KIV in the standard liquid test tube cultures in SVY medium. The concentration of valine was varied from 5 to 50 g/L. Although valine at 5 g/L was slightly less effective than α-KIV in promoting pantothenate production, valine at 10 or 20 g/L equaled or surpassed 5 g/L α-KIV in promoting pantothenate production.

### EXAMPLES IV-X Generation of Microorganisms Capable of Producing Pantothenate in a Precursor-Independent Manner

*B. subtilis* strains such as PA221 and PA235 (engineered to overexpress *panBCD*) and PA236 (engineered to overexpress *panBCD* and *panE1*) need to be fed α-ketoisovalerate (α-KIV) (or valine) and aspartate (or β-alanine) to achieve maximal pantothenate production, as both these precursors are limiting for pantothenate synthesis. Accordingly, manipulated microorganisms were designed to eliminate the need to feed limiting precursors of pantothenate biosynthesis in the production of pantothenate. These strains are also useful in the production of various pantothenate biosynthetic pathway intermediates.

### EXAMPLE IV: Generation of Microorganisms Capable of Producing Pantothenate in an Aspartate- (or β-Alanine) Independent Manner

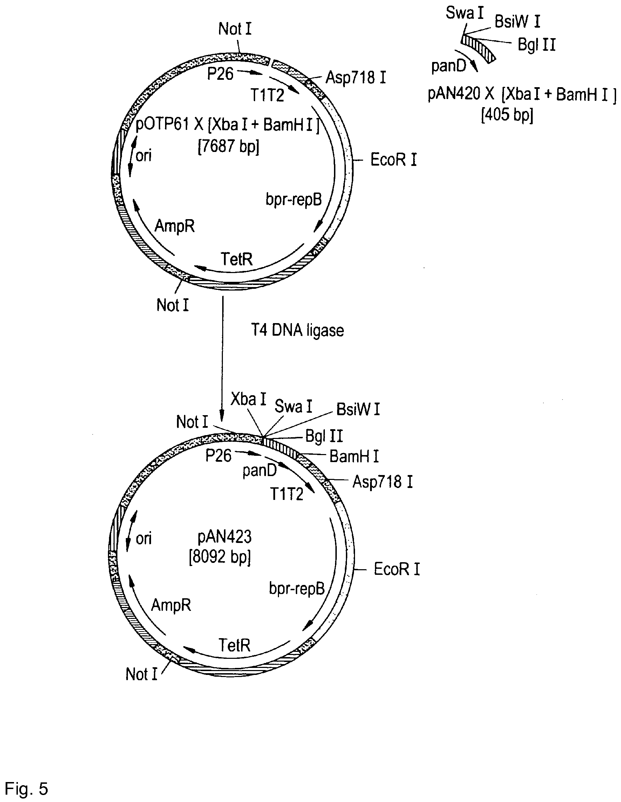

The *panD* gene was cloned into *B. subtilis* expression vector pOTP61 to construct pAN423 (Figure 5). The nucleotide sequence of pAN423 is set forth as SEQ ID NO:78. The *Not*I restriction fragment containing *panD* was isolated from pAN423, self ligated and used to transform PA221. Transformants resistant to Tet¹⁵, Tet³⁰, and Tet⁶⁰ were isolated and saved for further analysis.

Six of the pAN423 transformants plus two control transformants were grown in SVY containing 5 g/l α-KIV with and without 10 g/l aspartate and then assayed for pantothenate production (Table 6).

**Table 6. Effect of overproducing PanD on pantothenate production with and without added aspartate.**

| **Culture* (PA221 transformants)** | **Asp (10 g/L)** | **TetR** (µ g/ml )** | **OD550** | **[pan] (mg/L)** |
|---|---|---|---|---|
| | | | | |
| pOTP61-1 | - | 60 | 8.0 | 76 |
| pOTP61-2 | - | 60 | 7.7 | 91 |
| 423#1-1 | - | 15 | 8.5 | 180 |
| 423#1-2 | - | 15 | 8.0 | 150 |
| 423#1-3 | - | 30 | 8.3 | 220 |
| 423#1-4 | - | 30 | 8.5 | 280 |
| 423#1-5 | - | 60 | 8.9 | 580 |
| 423#1-6 | - | 60 | 8.8 | 280 |
| | | | | |
| pOTP61-1 | + | 60 | 7.5 | 380 |
| pOTP61-2 | + | 60 | 6.9 | 560 |
| 423#1-1 | + | 15 | 8.5 | 1200 |
| 423#1-2 | + | 15 | 8.6 | 1000 |
| 423#1-3 | + | 30 | 8.8 | 1200 |
| 423#1-4 | + | 30 | 9.0 | 1200 |
| 423#1-5 | + | 60 | 9.0 | 1200 |
| 423#1-6 | + | 60 | 9.0 | 1200 |

| | | | | |
|---|---|---|---|---|
| *Test tubes cultures were grown in SVY + α-KIV (5 g/L) with Asp (10 g/L) where indicated. **TetR = Approximate Tet-resistance of transformant | | | | |

The pAN423 transformants produced at least twice the amount of pantothenate as the controls (*i.e.,* to a level at or near that which was obtained in earlier experiments by the addition of β-alanine to the culture medium). The data also show that in the absence of added aspartate, transformants containing additional copies of the *panD* gene expression cassette produce more pantothenate than the control transformants. One of the transformants, 423#1-5, produced about five times as much pantothenate as the controls. These results indicated that increased levels of PanD protein "pull" the conversion of available aspartate towards β-alanine, and that increasing *panD* gene expression can result in enhancement of pantothenate production both in the presence and absence of added aspartate.

Transformant 423#1-5 was re-named strain PA401 and studied further in shake flask fermentations. The shake flask medium was SVY with maltose instead of SVY with glucose. Results of shake flask experiments agreed well with test tube experiments during the first 24 hours. In shake flask experiments without the addition of β-alanine, PA401 produced approximately 1.5 g/l of pantothenate in 24 hours. Addition of β-alanine to the culture medium did not further improve pantothenate titers (Table 7), indicating that with this strain and these fermentation conditions, β-alanine is not limiting pantothenate production. In fact, when no β-alanine is fed, one can observe that PA401 is secreting β-alanine in significant amounts into the medium.

**Table 7. Shake flask cultures with strain PA401 (panD) with and without β -alanine.**

| | Amino acids (g/l) | | 24 hours | | |
|---|---|---|---|---|---|
| Initial | | | | | |
| β-ala Added | β-ala | Val | pH | OD600 | Pantothenate (g/l) |
| 0 | 0.7 | 1.5 | 7.5 | 13.7 | 1.5 |
| 5 g/l | 7.1 | 1.4 | 7.6 | 12.4 | 1.5 |

Each value represents the average of duplicate 250 ml baffled flasks containing 50 ml of medium, incubated at 37° C with shaking (200 rpm).
Base Medium: SVY with 10 g/l α-KIV, 30 g/l maltose
2% Inoculum: SVY with Tet¹⁵ grown 24 hours.

### EXAMPLE V: Engineering the panD gene for Further Increased Synthesis of Aspartate Decarboxylase and Enhanced Production of Pantothenate

This Example describes the generation of improved ribosome binding sites (RBSs) in the *panD* gene to increase the translation of *panD* mRNA.

### Increasing the translation of the panD gene mRNA by generation of synthetic panD RBSs

The RBS (SEQ ID NO:88) used to express *panD* in pAN423 is a synthetic RBS and has been used to successfully produce other proteins in *B. subtilis* at a high level. However, it contains six mismatches when aligned to the "ideal" *B. subtilis* RBS (SEQ ID NO:45) (*e.g.,* an RBS having a sequence which is complementary to the 16S RNA sequence within the *B.subtilis* ribosome). (See *e.g.,* Table 1B, mismatches in bold). Two new RBSs were designed to more closely mimic the ideal RBS. These synthetic RBSs, named new design A (NDA) and new design B (NDB) (also referred to herein as RBS3 and RBS4), are set forth as SEQ ID NO:51 and SEQ ID NO:52 and are aligned with the ideal RBS in Table 1B.

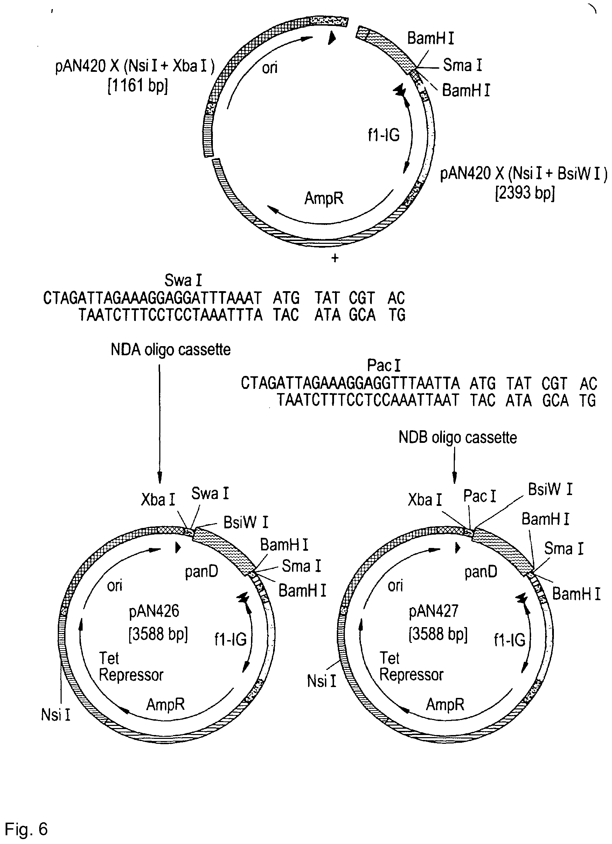

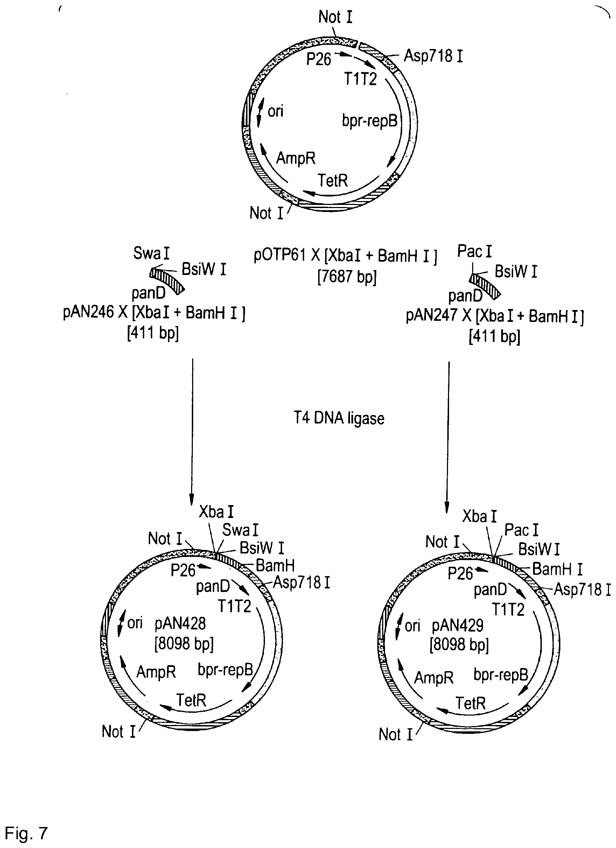

Oligonucleotides corresponding to the top and bottom strands of each new RBS were synthesized, annealed, then used to replace the RBS in pAN420, generating plasmids pAN426 and pAN427. These constructions are illustrated in Figure 6. The presence of the NDA and NDB RBS in pAN426 and pAN427 was confirmed by DNA sequence analysis. Next, the *panD* genes from pAN426 and pAN427 were transferred to *B. subtilis* expression vector pOTP61 as shown in Figure 7, creating pAN428 and pAN429. The nucleotide sequence of pAN429 is set forth as SEQ ID NO:79.

*Not*I restriction fragments lacking the *E. coli* vector sequences were isolated from pAN428 and pAN429, self-ligated, and used to transform strain PA221 to resistance to Tet¹⁵. Four isolates resistant to Tet⁶⁰ were picked from each transformation and assayed for pantothenate and β-alanine production along with PA221 transformed with the empty vector (pOTP61) and PA221 transformed with pAN423 (strain PA401) (see Table 8).

**Table 8. Panthothenate production by test tube cultures of PA221 transformed with pAN428 and pAN429**

| **Plasmid** | | **Medium Supplements** | **OD550** | **Pan** g/l | **β -Ala** g/l |
|---|---|---|---|---|---|
| | | | | | |
| pOTP61 | | α-KIV⁵ | 10 | UND | 0.04 |
| pAN423 | | α-KIV⁵ | 10 | 0.4 | 0.04 |
| | | | | | |
| pAN428-1 | * | α-KIV⁵ | 12 | 0.6 | 0.04 |
| pAN428-2 | | α-KIV⁵ | 11 | 0.5 | 0.03 |
| pAN428-3 | | α-KIV⁵ | 11 | 0.3 | 0.03 |
| pAN428-4 | | α-KIV⁵ | 10 | 0.1 | UND |
| | | | | | |
| pAN429-1 | | α-KIV⁵ | 12 | 0.6 | 0.04 |
| pAN429-2 | | α-KIV⁵ | 11 | 0.5 | 0.04 |
| pAN429-3 | | α-KIV⁵ | 11 | 0.6 | 0.05 |
| pAN429-4 | # | α-KIV⁵ | 12 | 0.8 | 0.10 |
| | | | | | |
| | | | | | |
| pOTP61 | | α-KIV⁵ + Asp¹⁰ | 11 | 0.5 | 0.08 |
| pAN423 | | α-KIV⁵ + Asp¹⁰ | 12 | 0.9 | 1.32 |
| | | | | | |
| pAN428-1 | * | α-KIV⁵ + Asp¹⁰ | 12 | 0.8 | 1.97 |
| pAN428-2 | | α-KIV⁵ + Asp¹⁰ | 12 | 0.8 | 1.51 |
| pAN428-3 | | α-KIV⁵ + Asp¹⁰ | 12 | 0.9 | 1.02 |
| pAN428-4 | | α-KIV⁵ + Asp¹⁰ | 11 | 0.8 | 0.30 |
| | | | | | |
| pAN429-1 | | α-KIV⁵ + Asp¹⁰ | 12 | 0.8 | 1.78 |
| pAN429-2 | | α-KIV⁵ + Asp¹⁰ | 12 | 0.8 | 1.66 |
| pAN429-3 | | α-KIV⁵ + Asp¹⁰ | 12 | 0.8 | 1.78 |
| pAN429-4 | # | α-KIV⁵ + Asp¹⁰ | 13 | 0.8 | 2.28 |

| | | | | | |
|---|---|---|---|---|---|
| UND: Below the limits of detection. * Renamed PA402 # Renamed PA403 | | | | | |

When grown in medium supplemented with α-KIV at 5 g/l (α-KIV⁵), the pAN428-1 transformant and all four of the pAN429 transformants produced more pantothenate than did PA401, suggesting that these transformants contain higher levels of aspartate decarboxylase activity. When grown in medium supplemented with α-KIV⁵ and Asp¹⁰ none of the pAN428 or pAN429 transformants produced more pantothenate than PA401. However, the pAN428-1 transformant and all four of the pAN429 transformants produced significantly more β-alanine than did PA401. It is possible that the excess β-alanine produced from added aspartate causes inhibition of pantothenate production. Alternatively, β-alanine may accumulate because pantoate is limiting in these strains.

The strains that produced the highest level of β-alanine, the pAN428-1 and pAN429-4 transformants, were renamed PA402 and PA403, respectively. These two strains were grown in SVY medium supplemented with various intermediates and reassayed for pantothenate and β-alanine production. PA221 and PA401 were included as controls. The results of the assays are presented in Table 9.

**Table 9. Pantothenate production of PA402 and PA403 in test tube cultures.**

| **Strain** | **Medium Supplements** | **OD550** | **Pan** g/l | **β-Ala** g/l | **Val** g/l |
|---|---|---|---|---|---|
| | | | | | |
| PA221 | α-KIV⁵ | 7.9 | UND | UND | 0.9 |
| PA401 | α-KIV⁵ | 8.7 | 0.3 | 0.04 | 0.9 |
| PA402 | α-KIV⁵ | 8.5 | 0.5 | 0.04 | 0.9 |
| PA403 | α-KIV⁵ | 9.4 | 0.7 | 0.07 | 0.9 |
| | | | | | |
| | | | | | |
| PA221 | α-KIV⁵ + Asp¹⁰ | 9.8 | 0.4 | 0.11 | 0.8 |
| PA401 | α-KIV⁵ + Asp¹⁰ | 9.1 | 0.8 | 1.15 | 0.8 |
| PA402 | α-KIV⁵ + Asp¹⁰ | 9.4 | 0.8 | 2.02 | 0.8 |
| PA403 | α-KIV⁵ + Asp¹⁰ | 9.7 | 0.7 | 2.40 | 0.8 |
| | | | | | |
| | | | | | |
| PA221 | Pantoate⁵ | 8.9 | UND | UND | 0.2 |
| PA401 | Pantoate⁵ | 8.7 | 0.3 | 0.02 | 0.2 |
| PA402 | Pantoate⁵ | 10.6 | 0.5 | 0.02 | 0.2 |
| PA403 | Pantoate⁵ | 10.5 | 0.7 | 0.02 | 0.2 |
| | | | | | |
| | | | | | |
| PA221 | Pantoate⁵ + Asp¹⁰ | 9.5 | 0.4 | 0.06 | 0.2 |
| PA401 | Pantoate⁵ + Asp¹⁰ | 9.2 | 2.2 | 0.62 | 0.2 |
| PA402 | Pantoate⁵ + Asp¹⁰ | 9.1 | 2.8 | 1.17 | 0.2 |
| PA403 | Pantoate⁵ + Asp¹⁰ | 10.2 | 2.9 | 1.58 | 0.2 |

| | | | | | |
|---|---|---|---|---|---|
| UND: Below the limits of detection. | | | | | |

When grown in medium supplemented with either α-KIV⁵ or Pantoate⁵, PA402 and PA403 produced significantly more pantothenate than did PA401. As before, even though PA402 and PA403 produced significantly more β-alanine than PA401 when grown in medium supplemented with α-KIV⁵ and Asp¹⁰, they did not produce a proportional increase in pantothenate. However, when grown in medium supplemented with Pantoate⁵ plus Asp¹⁰, both PA402 and PA403 produced significantly more pantothenate than PA401, about a 30% increase.

It can be concluded from these experiments that the improved NDA and NDB *panD* ribosome binding sites, engineered into pAN428 and pAN429, respectively, lead to increased levels of aspartate decarboxylase activity.

### Increasing the translation of the panD gene mRNA by generation of synthetic panD RBSs within the panBCD operon

The native *B. subtilis panD* gene ribosome binding site (RBS) (SEQ ID NO:43), which is found in the *P₂₆panBCD* operon cassette present in PA221 (and in other engineered pantothenate production strains described herein), is shown in Table 1C aligned with the ideal ribosome binding site (SEQ ID NO:47). The alignment shows mismatches between the native *B. subtilis panD* gene RBS, which is located within the coding sequence for PanC, and the the ideal RBS. Three new RBSs (within the P26 panBCD operon cassette) were generated to increase translation of the *panD* gene mRNA and to yield increased synthesis of aspartate decarboxylase. These synthetic RBSs (termed NDI, NDII, and NDIII, also referred to herein as RBS5, RBS6 and RBS7, respectively) are set forth as SEQ ID NO:55, SEQ ID NO:56 and SEQ ID NO:57, respectively) and are included in Table 1C. It should be noted that although changes in the *panD* RBS within the *panBCD* operon also changes the C-terminal amino acid sequence of the PanC protein encoded by that operon, an alignment of known and suspected PanC protein amino acid sequences showed that the sequence of the last nine amino acids of the *B. subtilis* PanC protein could be altered without affecting any conserved amino acid residues indicating that such changes should not reduce pantothenate synthetase activity or expression. The new RBSs were synthesized and incorporated into the *P₂₆ panBCD* operon expression cassette as follows.

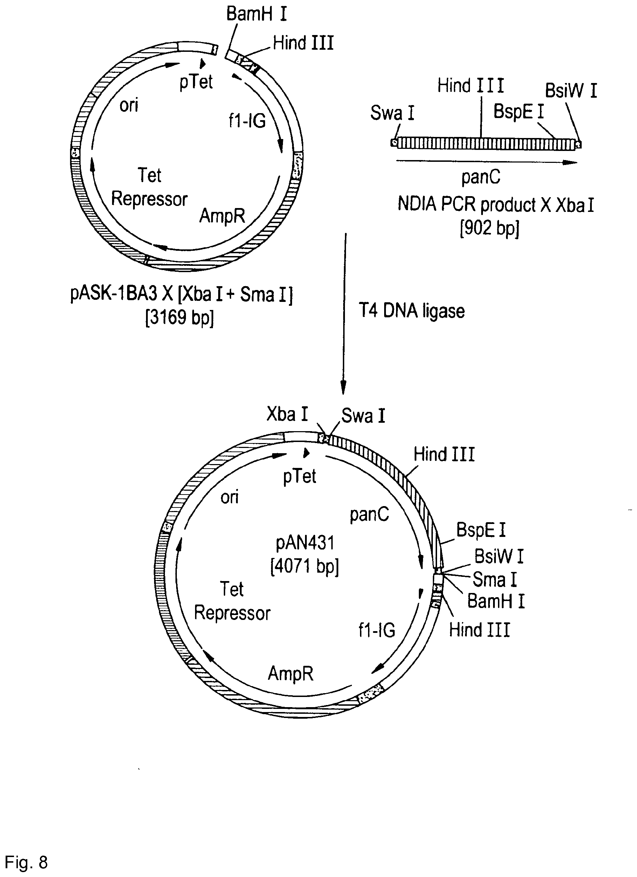

First, PCR primers were designed to contain the following elements: (1) a nucleic acid sequence encoding the first five amino acids of PanD up to and including a unique *Bsi*WI restriction site that had been previously introduced into *panD* by PCR; (2) a stop codon for *panC,* (3) at least one synthetic RBS; and (4) 30-39 bp of nucleic acid sequence having 100% identity with *panC* upstream of the *panD* RBS. The primers were named TP102, TP103, and TP104 and contain the NDI, NDII, and NDIII ribosome binding sites, respectively. These three primers were used in conjunction with the 5' primer TP101, which hybridizes near the start codon of *panC,* in three independent PCR reactions to generate the NDI, NDII, and NDIII PCR products. The PCR products were purified, digested with *Xba*I, then cloned into plasmid vector pASK-1BA3 which had been digested with *Xba*I and *Sma*I. The resulting plasmids were named pAN431, pAN432, and pAN433. The construction of pAN431 is illustrated in Figure 8 and is representative of all three plasmid constructions. The presence of the desired synthetic *panD* gene RBS in each new plasmid was confirmed by DNA sequencing.

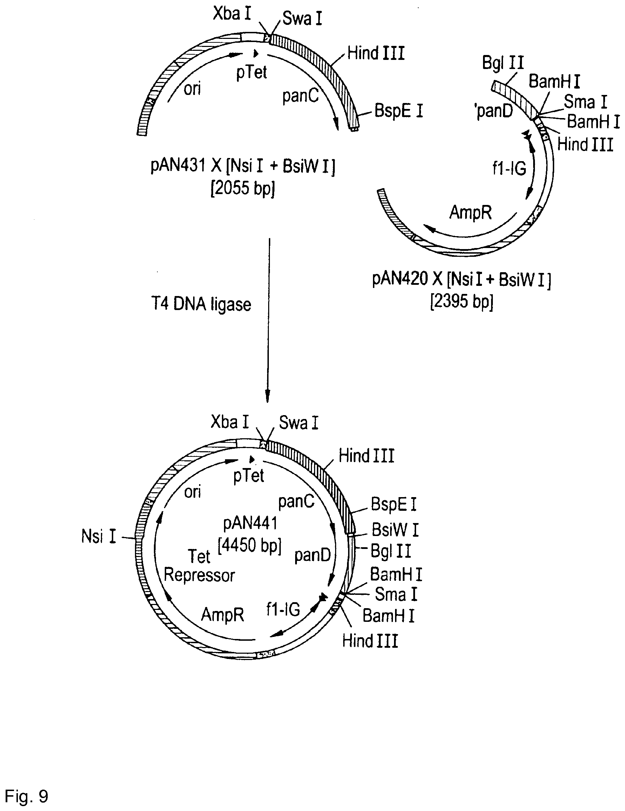

Next, the modified *panC* genes containing the new *panD* RBSs were joined with the *panD* gene utilizing the unique *Bsi*WI restriction site. This was accomplished by isolating the appropriate *Nsi*I*-Bsi*WI restriction fragments from pAN431, pAN432, and pAN433 and ligating them with a 2395 bp *Nsi*I-*Bsi*WI restriction fragment from pAN420, which supplied the *Bsi*WI-modified *panD* gene. These constructions resulted in plasmids pAN441, pAN442, and pAN443, respectively. A representative construction (pAN441) is illustrated in Figure 9. The nucleotide sequence of pAN443 is set forth as SEQ ID NO:80.

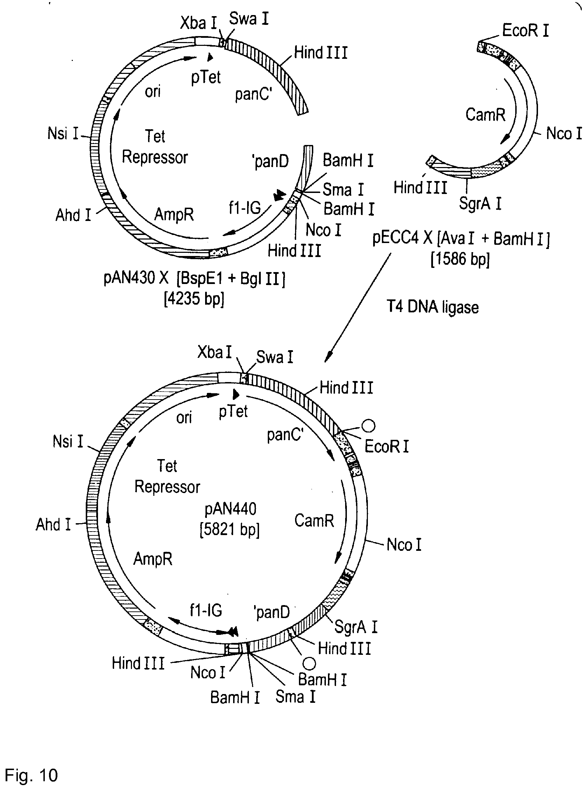

The new *panD* gene RBSs were then substituted into the *P₂₆panBCD* operon expression cassette as follows. First, a deletion-insertion mutation which removes the region of *panC* containing the *panD* RBS was created. This was constructed by digesting pAN430 with a mixture of *Bsp*E1 and *Bgl*II and recovering the 4235 bp fragment which is now missing the 3' end of *panC* and the 5' end of *panD.* This fragment was ligated with an *Ava*I-*Bam*HI restriction fragment from plasmid pECC4, which contains the chloramphenicol acetyl transferase (*cat*) gene. The 5' extension produced by *Ava*I digestion is compatible with that produced by *Bsp*EI while the *Bgl*II and *Bam*HI extensions are also compatible. The resulting plasmid was named pAN440, and its construction is illustrated in Figure 10.

The resulting deletion-insertion mutation was crossed into the *P₂₆ panBCD* operon *via* homologous recombination by transforming PA221 with linearized pAN440 and selecting for resistance to chloramphenicol on Cam⁵ plates containing 1 mM pantothenate. Several transformants were tested, and were all found to require 1 mM pantothenate for growth, as expected. Two of these transformants were remaned PA408A and PA408B and were assayed for pantothenate production. Neither strain synthesized measurable quantities of pantothenate, even when grown in medium containing pantoate and β-alanine at 5 g/l, indicating that the strains are deficient in pantothenate synthetase activity. Next, the new *panD* RBSs were crossed into the *P₂₆ panBCD* operon by transforming PA408 with linearized pAN441, pAN442, and pAN443 plasmid DNA and selecting for growth on TBAB plates without pantothenate supplementation. A transformation with linearized pAN430 (including the native panD RBS) was included as a control and was expected to give rise to transformants identical to PA221 described herein. Four isolates from each transformation were assayed for pantothenate and β-alanine production in SVY medium supplemented with various intermediates (Tables 10 and 11).

**Table 10. Pantothenate production of PA410 - PA413 in test tube cultures.**

| **Strain** | **RBS** | **Medium Supplements** | **OD550** | **Pan** g/l | **β -Ala** g/l |
|---|---|---|---|---|---|
| | | | | | |
| PA221 | native | Pantoate⁵ | 11 | UND | UND |
| | | | | | |
| | | | | | |
| PA410-1 | native | Pantoate⁵ | 12 | UND | UND |
| PA410-2 | | Pantoate⁵ | 12 | UND | UND |
| PA410-3 | | Pantoate⁵ | 12 | UND | UND |
| PA410-4 | | Pantoate⁵ | 12 | UND | UND |
| | | | | | |
| | | | | | |
| PA411-1 | NDI | Pantoate⁵ | 12 | 0.23 | UND |
| PA411-2 | | Pantoate⁵ | 12 | 0.20 | UND |
| PA411-3 | | Pantoate⁵ | 12 | 0.19 | UND |
| PA411-4 | | Pantoate⁵ | 12 | UND | UND |
| | | | | | |
| | | | | | |
| PA412-1 | NDII | Pantoate⁵ | 12 | UND | UND |
| PA412-2 | | Pantoate⁵ | 11 | UND | UND |
| PA412-3 | | Pantoate⁵ | 13 | 0.18 | UND |
| PA412-4 | | Pantoate⁵ | 12 | 0.18 | UND |
| | | | | | |
| | | | | | |
| PA413-1 | NDIII | Pantoate⁵ | 12 | 0.18 | UND |
| PA413-2 | | Pantoate⁵ | 12 | 0.17 | UND |
| PA413-3 | | Pantoate⁵ | 12 | 0.16 | UND |
| PA413-4 | | Pantoate⁵ | 12 | 0.17 | UND |

| | | | | | |
|---|---|---|---|---|---|
| UND: Below the limits of detection. | | | | | |

**Table 11. Pantothenate production of PA410 - PA413 in test tube cultures.**

| **Strain** | **RBS** | **Medium Supplements** | **OD550** | **Pan** g/l | **β -Ala** g/l |
|---|---|---|---|---|---|
| | | | | | |
| PA221 | native | Pantoate⁵ + Asp¹⁰ | 11 | 0.3 | UND |
| | | | | | |
| | | | | | |
| PA410-1 | native | Pantoate⁵ + Asp¹⁰ | 12 | 0.4 | UND |
| PA410-2 | | Pantoate⁵ + Asp¹⁰ | 12 | 0.4 | UND |
| PA410-3 | | Pantoate⁵ + Asp¹⁰ | 12 | 0.4 | UND |
| PA410-4 | | Pantoate⁵ + Asp¹⁰ | 12 | 0.4 | UND |
| | | | | | |
| | | | | | |
| PA411-1 | NDI | Pantoate⁵ + Asp¹⁰ | 13 | 1.7 | 0.4 |
| PA411-2 | | Pantoate⁵ + Asp¹⁰ | 13 | 1.7 | 0.4 |
| PA411-3 | | Pantoate⁵ + Asp¹⁰ | 13 | 1.8 | 0.3 |
| PA411-4 | | Pantoate⁵ + Asp¹⁰ | 13 | 0.4 | UND |
| | | | | | |
| | | | | | |
| PA412-1 | NDII | Pantoate⁵ + Asp¹⁰ | 13 | 0.4 | UND |
| PA412-2 | | Pantoate⁵ + Asp¹⁰ | 12 | 0.4 | UND |
| PA412-3 | | Pantoate⁵ + Asp¹⁰ | 12 | 1.6 | 0.3 |
| PA412-4 | | Pantoate⁵ + Asp¹⁰ | 12 | 1.5 | 0.2 |
| | | | | | |
| | | | | | |
| PA413-1 | NDIII | Pantoate⁵ + Asp¹⁰ | 13 | 1.6 | 0.3 |
| PA413-2 | | Pantoate⁵ + Asp¹⁰ | 13 | 1.6 | 0.4 |
| PA413-3 | | Pantoate⁵ + Asp¹⁰ | 13 | 1.7 | 0.4 |
| PA413-4 | | Pantoate⁵ + Asp¹⁰ | 13 | 1.7 | 0.4 |

| | | | | | |
|---|---|---|---|---|---|
| UND: Below the limits of detection. | | | | | |

As expected from previous experiments using PA221, none of the transformants that contained the native *panD* RBS produced measurable quantities of pantothenate when grown in medium supplemented with pantoate. However, nine of the twelve transformants expected to contain modified *panD* RBSs produced significant quantities of pantothenate (160-230 mg/l) under these conditions, indicating that they possess elevated levels of aspartate decarboxylase activity. When grown in medium supplemented with both pantoate and aspartate, these same nine transformants produced approximately four times more pantothenate than those with the native *panD* RBS. In addition, these nine transformants accumulated measurable quantities of β-alanine (230-410 mg/l). All transformants produced roughly equivalent quantities of pantothenate when grown in medium containing pantoate and β-alanine, demonstrating that each contains a functional pantothenate synthetase.

These data demonstrate that the synthetic *panD* RBSs are about four times more effective than the native *panD* RBS in directing translation of the *panD* gene mRNA and evidence the utility of such synthetic RBSs in enhancing pantothenate production. Additional approaches to increasing pantothenate production can include, for example, increasing the half-life of the *panD* gene mRNA, increasing the strength of the promoter for *panD* transcription and/or increasing the stability of the PanD protein.

### EXAMPLE VI: Construction of Strains Containing an Integrated P₂₆ panE1 Cassette without an Antibiotic Resistance Gene.

Example II describes the identification of the *B. subtilis panE1* gene that encodes the enzyme responsible for the majority of the ketopantoate reductase activity in *B. subtilis.* PA236 (containing the pAN236 plasmid) produced about twice as much pantothenate (2 g/l) as its parent strain, PA221 (1 g/l) in 24 hour SVY test tube cultures. PA236 was presumed to contain an amplified (∼3 copies) integrated pAN236 plasmid based on selection for tetracycline resistance (the tetR gene product being encoded on the pAN236 plasmid in addition to the *P₂₆ panE1* cassette). Also useful in the methodologies of the present disclosure are strains that contain a single integrated unamplifiable copy of *P₂₆ panE1* at the *panE1* locus, for example, without an antibiotic resistance gene in the strain. Such a strain was generated as follows.

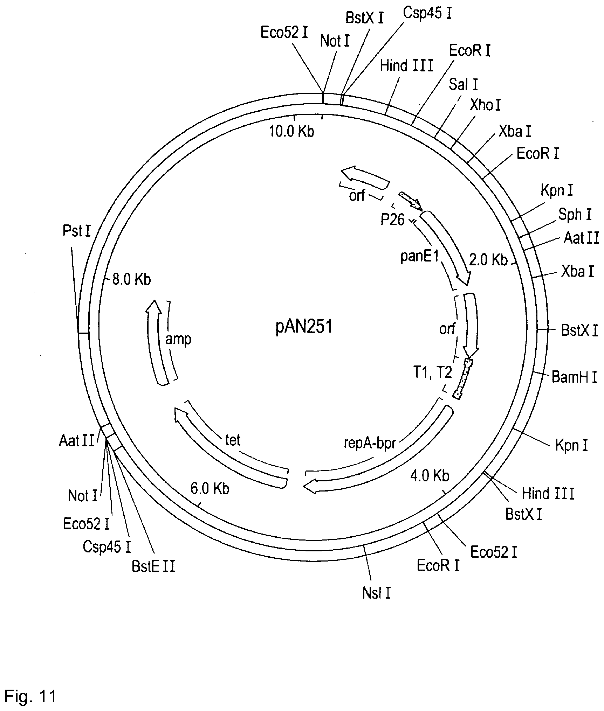

A plasmid named pAN251 was derived from pAN236 by inserting additional chromosomal sequences just upstream and just downstream from the *P₂₆ panE1* cassette. These additional sequences, which provide homology to allow integration of the *P₂₆ panE1* cassette at *panE1* by double crossover, were obtained by PCR from chromosomal DNA as a template. pAN251 is shown in Figure 11. The nucleotide sequence of pAN251 is set forth as SEQ ID NO:81.

Next, a strain was constructed which allowed selection for the incoming *P₂₆ panE1* cassette. The strain included the following three components: (1) *P₂₆ panBCD*; (2) *ΔpanE1*; and (3) *ilvC*⁻, since both *panE1* and *ilvC* must be mutated to have a Pan⁻ phenotype. The starting strain was CU550 (*trpC2*, *ilvC4*, *leuC124*). The *P₂₆ panBCD* cassette from PA221 chromosomal DNA was introduced in two steps to create strain PA290. Next, *ΔpanE1::spec* was transformed into PA290, using chromosomal DNA from strain PA240, to give strain PA294 (*trpC2*, *ilvC4*, *leuC124*, *P₂₆ panBCD*, *ΔpanE1::spec*), which is a strict pantothenate auxotroph. Finally, PA294 was transformed with plasmid pAN251, selecting for pantothenate prototrophy, to give strain PA303. This strain was expected to have the genotype *trpC2*, *ilvC4*, *leuC124*, *P₂₆ panBCD, P₂₆ panE1.* PA303 was checked for the correct chromosomal structure at the *panE*1 locus by PCR using primers that flank the *P₂₆* insertion just upstream of *panE1.* The PCR product from PA303 was of the expected size, with a concomitant loss of the PCR product from the wild type *panE1* gene, consistent with having obtained the desired double crossover event. Furthermore, PA303 was tetracycline sensitive, which is also consistent with the desired double crossover event, as opposed to a Campbell-type single crossover of the plasmids into the chromosome. The *trp*, *ilv*, and *leu* auxotrophies from the parent strain were all maintained in PA303.

In 24 hour liquid SVY test tube cultures, PA303 produced almost the same level of pantothenate as positive control PA236, and about twice as much as PA221, which does not contain engineered *panE1* as indicated in Table 12.

**Table 12. Pantothenate production by 24 hr. test tube cultures of PA303 and controls grown in SVYplus 5 g/l α-KIV and 5 g/l β-alanine.**

| | | Strain | OD600 | [pan] g/l |
|---|---|---|---|---|
| | | | | |
| | | PA221-1 | 10.9 | 0.85 |
| | | PA221-2 | 10.5 | 0.85 |
| | | | | |
| | | PA236-1 | 9.5 | 1.74 |
| | | PA236-2 | 9.3 | 1.70 |
| | | | | |
| | | PA303-1 | 10.8 | 1.66 |
| | | PA303-2 | 10.7 | 1.61 |

### EXAMPLE VII: Generation of Microorganisms Capable of Producing Pantothenate in an α-KIV (or Valine) Independent Manner

α-ketoisovalerate (α-KIV) is a rate limiting intermediate for pantothenate production in certain strains deregulated for pantothenate synthesis. Addition of either α-KIV or valine at 5 g/l increases pantothenate production about 5-fold in test tube cultures with strains such as PA221. In order to alleviate the need to feed either α-KIV or valine, strains were engineered that have an increased capacity to synthesize α-KIV.

α-KIV is produced in *B. subtilis* from pyruvate by the sequential action of three enzymes encoded by four genes, *ilvB* and *ilvN*, *ilvC*, and *ilvD.* In a wild type *B. subtilis*, three of the genes (*ilvB*, *ilvN*, and *ilvC*) are the first three genes of the large *ilv-leu* operon. The fourth gene necessary for α-KIV synthesis, *ilvD*, is located by itself elsewhere on the chromosome. The *B. subtilis ilv-leu* operon is thought to be regulated only by leucine levels. Feeding of exogenous leucine reduces transcription of the *ilv-leu* operon by about 13-fold, probably by an attenuation mechanism (Grandoni et al. (1992) J. Bacteriol. 174: 3212-3219). The only known feedback regulation in the *ilv-leu* pathway is the inhibition of the *leuA* gene product by leucine.

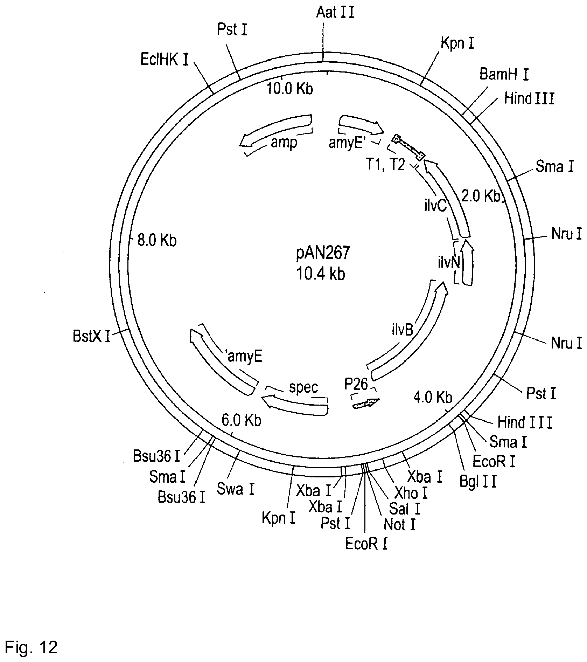

As a first step to deregulate the synthesis of α-KIV, a copy of the *ilvBNC* region from the wild type *B. subtilis ilv-leu* operon was isolated by PCR, and installed adjacent to the *P₂₆* promoter and RBS2 on a vector, pOLL8, that was designed to integrate a single *P₂₆* expression cassette by double recombination at the *amyE* locus. The *amyE* gene encodes a nonessential α-amylase, and is a useful locus for installing expression cassettes. The resulting plasmid, pAN267, is illustrated in Figure 12. The nucleotide sequence of pAN267 is set forth as SEQ ID NO:82. pAN267 readily gave stable transformants by double crossover at the *amyE* locus of *B. subtilis* strains, as described in detail below.

### Construction of pantothenate overproducing strains that are leucine prototrophs

Initially, a *B. subtilis* strain containing *ilvC4* and *ΔpanE1* was used to introduce a single copy of *P₂₆ panE1* into the chromosome without using an antibiotic resistance gene. The double mutant was required to select for the incoming *P₂₆ panE1* cassette because a *ΔpanE1* mutation alone does not result in pantothenate auxotrophy. A strain named CU550 was obtained containing *ilvC4* to be used as a basis for this type of strain construction. However, CU550 also contains a closely linked *leuC124* mutation, so all strains derived from CU550 required leucine. Having shown that the combination of *P₂₆ panBCD* and *P₂₆ panE1* was favorable for pantothenate production, the next step was to reassemble this combination of two cassettes in a leucine prototroph.

Accordingly, the two cassettes were combined in two different strain backgrounds, RL-1 and PY79. To introduce chromosomal *P₂₆ panE1* into the PY79 and RL-1 strain backgrounds without using an antibiotic resistance gene, a strategy was used that did not rely on *ilvC4.* (The strategy took advantage of the observation that the *ΔpanE1* mutation causes a pantothenate bradytrophy, manifested by relatively small colonies on TBAB (rich) plates). First, *ΔpanB::cat* and *ΔpanE::spec* were introduced into both strain backgrounds. Next, the resulting strains were transformed simultaneously with DNA from two strains, PA221 (*P₂₆ panBCD*) and PA303 (*P₂₆ panE1*)*,* selecting for Pan⁺ on TBAB plates. Colonies of two distinct sizes grew on the selective plates, with the larger size comprising about 2% of the colonies. The larger colonies were presumed to represented co-transformants that received both *P₂₆ panBCD* and *P₂₆ panE1*, and that the smaller colonies had received only *P₂₆ panBCD.* Consistent with this prediction, the larger colonies had lost both Cam^{r} and Spec^{r}, while the smaller colonies had lost only the *cat* gene, and retained the *spec* gene. Furthermore, a representative derivative of PY79 named PA327, and a representative derivative of RL-1, named PA328, both produced the elevated levels of pantothenate in test tube cultures which was about 1.6 to 1.7 g/l (Table 13).

**Table 13. Pantothenate production of PA327, PA328, and controls from 24 hr test tube cultures grown in SVYplus 5 g/l α-KIV and β-alanine.**

| Strain | Background | *P26 panE1* copy number | [pan] g/l |
|---|---|---|---|
| | | | |
| PA221-1 | RL-1 | 0 | 0.92 |
| PA221-2 | RL-1 | 0 | 0.95 |
| | | | |
| PA236-1 | RL-1 | amplified (∼3) | 1.60 |
| PA236-2 | RL-1 | amplified (∼3) | 1.73 |
| | | | |
| PA327-1 | PY79 | 1 | 1.66 |
| PA327-2 | PY79 | 1 | 1.65 |
| | | | |
| PA328-1 | RL-1 | 1 | 1.61 |
| PA328-2 | RL-1 | 1 | 1.91 |

Thus, PA327 and PA328 were concluded to contain both *P₂₆ panBCD* and *P₂₆ panE1*, and were used for further constructions as described below. PCR analysis confirmed the presence of the two cassettes.

### Installation of a stable P26 ilvBNC cassette into two lineages of pantothenate overproducing strains

Having constructed PA327 and PA328, derivatives of PY79 and RL-1 that contain *P26 panBCD* and *P26 panE1*, and that are Leu⁺, the next step was to introduce stable copies of *P26 ilvBNC.* This was accomplished by transforming PA327 and PA328 with plasmid pAN267, selecting for Spec^{r}. Screening by PCR showed that about 85% of the obtained transformants contain *P26 ilvBNC* integrated at *amyE* by double crossover. One transformant of PA327, named PA340, and one transformant of PA328, named PA342, were chosen for further study.

In test tube cultures grown in SVY medium plus 5 g/l β-alanine but without added α-KIV, both PA340 and PA342 gave the expected increase in pantothenate production over that of PA327 and PA328, to about 1.3 to 2 g/l (Table 14).

**Table 14. Pantothenate and valine production by PA340 and PA342, both containing P₂₆ ilvBNC in 24 hr test tube cultures grown in SVY with 5 g/l β-alanine and with or without 5 g/l α-KIV**

| Strain | Background | OD600 - α-KIV | + α-KIV | [pan] g/l - α-KIV | + α-KIV | [val] g/l - α-KIV | + α-KIV |
|---|---|---|---|---|---|---|---|
| | | | | | | | |
| PA340-1 | PY79 | 11.8 | 7.1 | 2.02 | 2.10 | 0.38 | 0.90 |
| PA340-2 | PY79 | 10.3 | 7.5 | 1.97 | 2.03 | 0.40 | 0.91 |
| | | | | | | | |
| | | | | | | | |
| PA342-1 | RL-1 | 10.2 | 8.0 | 1.29 | 1.89 | 0.27 | 0.78 |
| PA342-2 | RL-1 | 9.6 | 9.2 | 1.34 | 2.04 | 0.21 | 0.79 |

The two new strains also gave a slight increase in valine secretion, indicating that the *ilvBNC* genes had been deregulated. However, when the same strains were grown with 5 g/l α-KIV added, a further increase in pantothenate production occurred from PA342, suggesting that α-KIV was still rate limiting in this strain background. Similar results, only with more growth and hence higher pantothenate levels, were seen in shake flask cultures (Table 15).

**Table 15. Pantothenate and valine production by PA340 and PA342, both containing P₂₆ ilvBNC in 24 hour shake flask cultures grown in SVY with 5 g/l β-alanine and with or without 5 g/l α-KIV.**

| | | OD600 | | [pan] g/l | | [val] g/l | |
|---|---|---|---|---|---|---|---|
| Strain | Background | - α-KIV | + α-KIV | - α-KIV | + α-KIV | - α-KIV | + α-KIV |
| | | | | | | | |
| PA327 | PY79 | 21 | 22 | 0.6 | 3.0 | 0.5 | 1.3 |
| PA340-1 | PY79 | 20 | 20 | 3.5 | 4.1 | 1.0 | 1.9 |
| PA340-2 | PY79 | 22 | 19 | 3.0 | 2.1 | 0.8 | 1.4 |
| | | | | | | | |
| PA328 | RL-1 | 20 | 16 | 1.4 | 2.7 | 0.6 | 1.3 |
| PA342-1 | RL-1 | 17 | 16 | 3.3 | 3.6 | 0.9 | 1.6 |
| PA342-2 | RL-1 | 18 | 18 | 3.1 | 4.2 | 0.8 | 1.4 |

### EXAMPLE VIII: IncreasingpanD Copy Number in Strains Engineered to Overproduce panE1 and the ilvBNC Gene Products Enhances Pantothenate Production

Experiments where β-alanine was fed to cultures of engineered *B*. *subtilis* strains consistently showed that β-alanine was a rate limiting intermediate in pantothenate synthesis. The effect of adding additional copies of *panD* on pantothenate production in PA340 and PA342 was examined. Strains PA340 and PA342 were transformed with chromosomal DNA isolated from PA401 with selection on plates containing 15 µg/ml of tetracycline (Tet¹⁵ plates). Transformants derived from each parent were patched onto Tet⁶⁰ plates to identify those which were likely to contain multiple copies of the expression cassette. Twelve transformants from each transformation which grew on Tet⁶⁰ were streaked for single colonies on this medium and then assayed in SVY medium test tube cultures for pantothenate production. One transformant from each group was found to produce greater than 300 mg/l pantothenate in 24 hours. These two transformants were saved and named PA404 (PA340 strain background) and PA405 (PA342 strain background). Both strains were resistant to spectinomycin, indicating that the *P₂₆ ilvBNC* expression cassette was still present at *amyE.* PCR analysis of chromosomal DNA isolated from each strain confirmed that the deregulated *panE1* gene had also been retained.

Next, PA404 and PA405 were evaluated in shake flask cultures which were grown in SVY medium containing maltose as the carbon source and supplemented with various intermediates. The cultures were grown for 24 and 48 hours and then assayed for pantothenate, β-alanine, and valine production. The results of this experiment are presented in Table 16. Analogous shake flask culture data for the parent strains (PA340 and PA342) are included in the tables for comparison.

**Table 16. Pantothenate Production by PA404 and PA405 in shake flask cultures after 24 hours**

| **Strain** | **Medium Supplements** | **OD600** | **Pan** g/l | **β - Ala** g/l | **Val** g/l |
|---|---|---|---|---|---|
| | | | | | |
| PA340 | none | 20 | 0.4 | <0.1 | 1.0 |
| PA404 | none | 22 | 1.8 | <0.1 | 0.7 |
| PA342 | none | 19 | 0.3 | 0.2 | 0.7 |
| PA405 | none | 19 | 1.4 | 0.4 | 0.5 |
| | | | | | |
| | | | | | |
| PA340 | β -alanine⁵ | 18 | 3.6 | 3.2 | 0.6 |
| PA404 | β -alanine⁵ | 18 | 2.8 | 5.1 | 0.7 |
| PA342* | β -alanine⁵ | 17 | 3.3 | 3.3 | 0.5 |
| PA405* | β -alanine⁵ | 19 | 1.3 | 6.5 | 0.6 |

| | | | | | |
|---|---|---|---|---|---|
| Values are the average of duplicate flasks except where indicated by *. | | | | | |

In the absence of any medium supplementation, PA404 and PA405 made four to five times more pantothenate in 24 hours compared to their isogenic parent strains (Table 16). The supply of β-alanine was clearly limiting in the parent strains PA340 and PA342. Addition of amplified *P26 panD* greatly increased the supply of β-alanine.

### EXAMPLE IX: Deregulation of the B. subtilis ilvd Gene Enhances Pantothenate Production

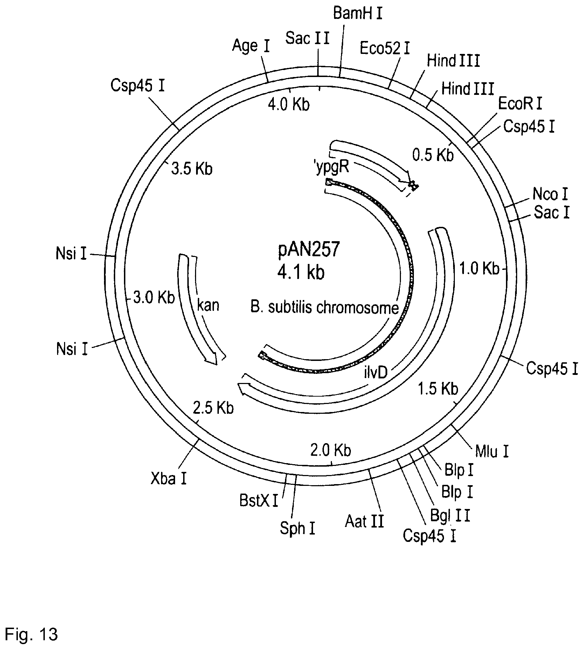

To deregulate expression of the *ilvD gene*, standard procedures (described above) were used to integrate the constitutive *P₂₆* promoter and an artificial ribosome binding site, RBS2, just upstream of the *ilvD* coding region. The *ilvD* gene maps by itself, unlinked to the *ilvBNC* operon. First, a 2.4 kb region of the RL-1 chromosome that contains the *ilvD* coding region and 730 bp of upstream sequence was cloned by PCR into a low copy (about 15 per *E. coli* cell) vector called pOK12, to give plasmid pAN257, shown in Figure 13.

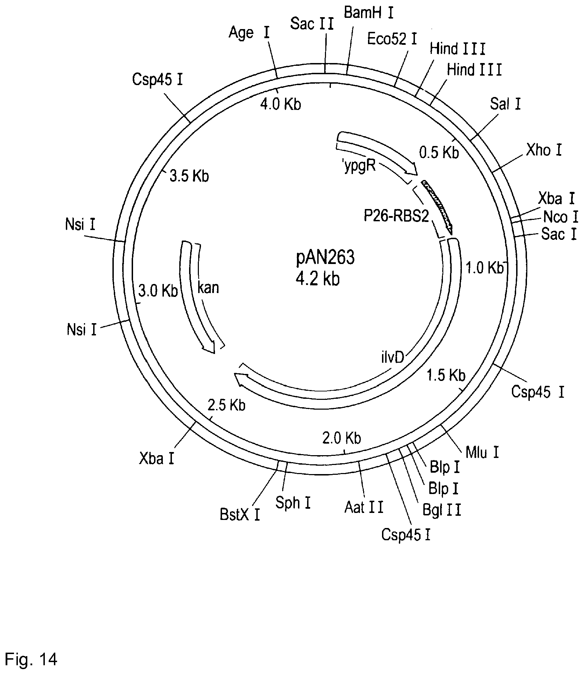

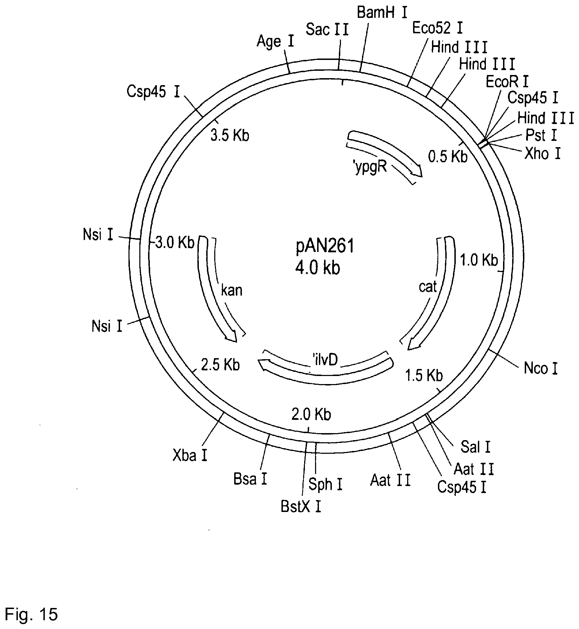

Taking advantage of a natural *Eco*RI site just upstream of the native *ilvD* gene promoter, and a natural *Nco*I site at the *ilvD* start codon, an artificial sequence containing *P₂₆* and RBS2 was inserted into pAN257 to give pAN263 (Figure 14). The nucleotide sequence of pAN263 is set forth as SEQ ID NO:83. In parallel with this construction, the *cat* gene was also inserted into pAN257, between the same upstream *Eco*RI site and a *Bgl*II site in the middle of the *ilvD* coding region, to give pAN261, which is deleted for a large portion of the *ilvD* gene (Figure 15). Using pAN261 and pAN263, the *P₂₆ ilvD* cassette could then be installed in the *B. subtilis* chromosome in two steps. In the first step, pAN261 is introduced by transformation, selecting for chloramphenicol resistance, and then confirming an Ilv⁻ phenotype. In the second step, pAN263 is introduced, selecting for Ilv⁺, checking for chloramphenicol sensitivity, and confirming correct local structure by PCR.

pAN261 was first transformed into strain RL-1 (highly competent) to give strain PA343 (*ΔilvD::cat*), and then chromosomal DNA from PA343 was used to transform PA340 and PA342 to Ilv⁻ auxotrophy, yielding strains named PA348 and PA349, respectively. Chromosomal DNA is inherently more efficient than monomeric plasmid in transforming *B. subtilis.* Similarly, pAN263 DNA was transformed into PA343 (moderately competent) to give strain PA345 (*P₂₆ ilvD*), and then PA345 chromosomal DNA was used to transform PA348 and PA349 to Ilv⁺ prototrophy, yielding strains PA374 and PA354, respectively.

As predicted, PA374 and PA354 gave further increases in pantothenate production, to about 2.5 to 2.9 g/l, in test tube cultures grown in SVY plus 5 g/l β-alanine (Table 17).

**Table 17. Pantothenate and valine production by PA374 and PA354, containing P₂₆ ilvd, and controls, in 24 hr test tube cultures grown in SVY with 5 g/l β-alanine and with or without 5 g/l α-KIV.**

| | | | OD600 | | [pan] g/l | | [val] g/l | |
|---|---|---|---|---|---|---|---|---|
| Strain | Background | *ilvD* status | α-KIV - | + | α-KIV - | + | α-KIV - | + |
| | | | | | | | | |
| PA340 | PY79 | w.t. | 9.2 | 9.0 | 2.14 | 2.23 | 0.38 | 0.90 |
| | | | | | | | | |
| PA348 | PY79 | *ilvD::cat* | 11.7 | 10.0 | 0.19 | 2.23 | 0.19 | 0.91 |
| | | | | | | | | |
| PA374-1 | PY79 | *P26 ilvD* | 9.1 | 7.3 | 2.93 | 2.40 | 0.58 | 0.87 |
| PA374-2 | PY79 | *P26 ilvD* | 8.2 | 7.7 | 2.99 | 2.36 | 0.60 | 0.95 |
| | | | | | | | | |
| PA342 | RL-1 | w.t. | 10.2 | 8.0 | 1.29 | 1.89 | 0.27 | 0.78 |
| | | | | | | | | |
| PA349 | RL-1 | *ilvD::cat* | 8.1 | 7.7 | 0.17 | 1.87 | 0.22 | 0.88 |
| | | | | | | | | |
| PA354-1 | RL-1 | *P26 ilvD* | 9.6 | 9.6 | 2.57 | 2.03 | 0.65 | 1.23 |
| PA354-2 | RL-1 | *P26 ilvD* | 7.5 | 8.2 | 2.48 | 2.24 | 0.64 | 0.97 |

In the absence of added β-alanine, strains PA374 and PA354 produced only about 0.2 g/l pantothenate in test tube cultures, indicating that PanD activity is significantly rate limiting.

To alleviate this limitation, the amplifiable *P₂₆ panD* cassette from strain PA401 was installed. PA401 chromosomal DNA was transformed into PA374 and PA354, selecting for Tet^{r} at 15 mg/l, to yield strains PA377 and PA365, respectively. After transformants were obtained, the strains were streaked on plates containing 30 and 60 mg/l tetracycline to reamplify the copy number of the *P₂₆ panD* cassette integrated at the *bpr* locus. In test tube cultures grown in SVY without α-KIV or β-alanine, a substantial improvement in pantothenate titers over those of PA374 and PA354 was obtained (Tables 18 and 19).

**Table 18. Pantothenate production by PA365, containing amplified P26 panD, and controls, in 24 and 36 hr test tube cultures grown in SVY-glucose without β-alanine or α-KIV.**

| | | OD600 | | [pan] g/l | |
|---|---|---|---|---|---|
| Strain | Relevant genotype | 24 hrs. | 36 hrs | 24 hrs. | 36 hrs. |
| | | | | | |
| PA342-1-1 | w.t. *ilvD* | 11.7 | 8.8 | b.d. | 0.27 |
| PA342-1-2 | w.t. *ilvD* | 12.8 | 8.8 | b.d. | 0.26 |
| | | | | | |
| PA354-1-1 | *P26 ilvD* | n.d. | 11.0 | n.d. | 0.19 |
| PA354-1-2 | *P26 ilvD* | n.d. | 8.4 | n.d. | 0.20 |
| | | | | | |
| PA365-1 | *P26 ilvD, P26 panD* | 9.8 | 10.0 | 1.01 | 2.07 |
| PA365-2 | *P26 ilvD, P26 panD* | 9.9 | 10.4 | 0.96 | 2.09 |

| | | | | | |
|---|---|---|---|---|---|
| n.d. = not determined; b.d. = below detection | | | | | |

**Table 19. Pantothenate production by PA377, containing amplified P₂₆ panD, and controls, in 27 hr test tube cultures grown in SVY-glucose or SVY-maltose, without α-KIV, and with or without β-alanine.**

| | | OD600 | | | |
|---|---|---|---|---|---|
| Strain | Relevant genotype | - β-ala Glucose | + β-ala Glucose | - β-ala Maltose | + β-ala Maltose |
| PA374-1 | *P26 ilvD* | 9.4 | 9.8 | 7.0 | 6.4 |
| PA374-2 | *P26 ilvD* | 9.2 | 9.6 | 6.6 | 6.3 |
| | | | | | |
| PA377-1 | *P26 ilvD*, *P26 panD* | 10.0 | 7.6 | 7.2 | 6.1 |
| PA377-2 | *P26 ilvD, P26 panD* | 10.5 | 7.8 | 9.4 | 5.4 |
| | | [pan] g/l | | | |

| Strain | Relevant genotype | - β-ala Glucose | + β-ala Glucose | - β-ala Maltose | + β-ala Maltose |
|---|---|---|---|---|---|
| PA374-1 | *P26 ilvD* | 0.04 | 2.76 | 0.14 | 1.31 |
| PA374-2 | *P26 ilvD* | 0.10 | 2.65 | 0.15 | 1.33 |
| | | | | | |
| PA377-1 | *P26 ilvD, P26 panD* | 1.25 | 2.76 | 1.26 | 1.10 |
| PA377-2 | *P26 ilvD, P26 panD* | 1.25 | 2.35 | 1.31 | 1.26 |

In SVY with glucose, an increase in pantothenate production can still be achieved by feeding 5 g/l β-alanine suggesting that increasing *panD* expression further might increase pantothenate production. In SVY with maltose, no further increase in pantothenate was obtained by feeding β-alanine suggesting that β-alanine and/or aspartate synthesis is suppressed by glucose. Strains PA377 and PA365 have been evaluated in 10 liter fermentors, where they typically produce above 20 g/l pantothenate in 48 hours without supplemental β-alanine and α-KIV or valine, described in detail below.

### EXAMPLE X: 10 liter Fermentations of Pantothenate-Producing Microbes

Engineering of the *P₂₆ ilvBNC* and *P₂₆ ilvD* cassettes to give strains PA342 and PA354 allowed the production of 22 and 26 g/l of pantothenate, respectively, without the addition of valine or α-KIV to the fermentation medium (Table 20). At 48 hours, both strains had secreted about 0.5 g/l of valine into the medium.

**Table 20. 10-liter fermentations of five pantothenate overproducing strains.**

| Strain | Medium | Feed 40% Glucose plus | OD 600 48 hr | Valine 48 hours g/l | β - ala 48 hr g/l | | Pantothenate g/L 36 hr 48 hr 72 hr | |
|---|---|---|---|---|---|---|---|---|
| PA 236 | SVYG | 50 g/l β - ala 25 g/l α-KIV | 108 | added | added | 16 | 19 | 21 |
| PA 342 | SVYG | 50 g/l β - ala | 92 | 0.5 | added | 17 | 22 | -- |
| PA 354 | SVYG | 50 g/l β - ala | 90 | 0.5 | added | 19 | 26 | -- |
| PA 365 | SVYG | 25g/l YE | 77 | 0.85 | 0.4 | 18 | 21 | 27 |
| PA 377 | SVYG | 25g/l YE | 85 | 1.5 | 0.5 | 18 | 22 | 31 |
| PA 377 | PFMG | 25g/l YE | 96 | 0.8 | 0.4 | 19 | 25 | 29 |
| PA377 | PFMG | - | 71 | 0.7 | 0.1 | 16 | 21 | - |

### Pantothenate synthesis in fermentors

With the addition of the *P₂₆ panD* cassette to strains PA354 and PA374 to create strains PA365 and PA377, neither β-alanine nor α-KIV needed to be added to the fermentors. Strain PA365 produced 21 g/l pantothenate in 48 hours and 27 g/l in 72 hours with no precursors added to the medium (Table 20). PA377 was somewhat better, producing 18 g/l of pantothenate in 36 hours, 22 g/l in 48 hours, and 31 g/l in 72 hours). Valine was measured at 0.85 and 1.5 g/l for strains PA365 and PA377, respectively, at 48 hours in SVYG medium. Strain PA377 maintained valine between 1-1.5 g/l throughout most of the fermentation and β-alanine between 0.2 and 0.5 g/l.

Strain PA377 was further evaluated in 10-liter fermentors in yeast extract based PFMG medium. Pantothenate yields in PFMG and SVYG medium were similar. In PFMG, PA377 produced 19 g/l of pantothenate in 36 hours, 25 g/l in 48 hours, and 29 g/l in 72 hours. In SVYG, PA377 produced 18 g/L pantothenate in 36 hours, 22 g/L in 48 hours and 31 g/L in 72 hours (Table 20).

### EXAMPLE XI: Converting Strain PA377 to a Tryptophan Prototroph

PA377 (Trp⁻) was transformed to Trp⁺ using chromosomal DNA from PY79 to give strain PA824. After re-amplification of the *P₂₆panD* casette, PA824 was compared to PA377 for pantothenate production in test tube cultures grown in SVY glucose with or without 5 g/L β-alanine (Table 21).

**Table 21 : Trp⁺ derivatives of PA377: Pantothenate production in 48 hour test tube cultures grown in SVY glucose, ±β-alanine**

| | | OD₆₀₀ | | [pan] g/L | |
|---|---|---|---|---|---|
| Strain | *trpC* donor | - β-alanine | + β-alanine | - β-alanine | + β-alanine |
| PA377-1 | RL-1 | 8 | 8 | 1.5 | 3.4 |
| PA377-2 | RL-1 | 8 | 9 | 1.6 | 3.6 |
| | | | | | |
| PA824-1 | PY79 | 12 | 10 | 0.7 | 3.7 |
| PA824-2 | PY79 | 11 | 11 | 1.9 | 4.9 |

The Trp+ strains grew to slightly higher densities than PA377. In the absence of exogenous β-alanine, all of the strains produced similar levels of pantothenate, while with the addition of β-alanine, the Trp+ derivatives produced somewhat more pantothenate.

### Fermentor studies with PA824

PA824 was evaluated in CF3000 Chemap 14 liter vessels with 10 liter working volumes. Formulations for two of the media used in the fermentors are given in Tables 22 and 23.

**Table 22: Formulation for PFMG-5 medium BATCH**

| | **MATERIAL** | **g/L (final [])** |
|---|---|---|
| 1 | Amberex 1003 | 10 |
| 2 | Na Glutamate | 5 |
| 3 | (NH₄)₂SO₄ | 8 |
| | | |
| 4 | MAZU DF 37C | 2.5 |

**Added After Sterilization and Cool Down**

| | | |
|---|---|---|
| 1 | KH₂PO₄ | 10 |
| 2 | K₂HPO₄·3H₂O | 20 |
| | | |
| 1 | Glucose | 20 |
| 2 | MgCl₂·6H₂O | 1 |
| 3 | CaCl₂·2H₂O | 0.1 |
| | | |
| 1 | Sodium Citrate | 1 |
| 2 | FeSO₄·7H₂O | 0.01 |
| 3 | SM-1000X | 1.0 ml |
| | | |
| | H₂O | qs to 6000 ml |

**FEED**

| | **MATERIAL** | **g/L** |
|---|---|---|
| 1 | Glucose | 600 |
| 2 | CaCl₂·2H₂O | 0.6 |
| | | |
| | H₂O | qs to 3000 ml |

**Table 23 : Formulation for SVY-4 medium BATCH**

| | **MATERIAL** | **g/L (final [])** |
|---|---|---|
| 1 | Veal Infusion | 25 |
| 2 | Yeast Extract | 5 |
| 3 | Na Glutamate | 5 |
| 4 | (NH₄)₂SO₄ | 4 |
| | | |
| 5 | MAZU DF 37C | 2.5 |
| | | |
| Added After Sterilization and Cool Down | | |
| | | |
| 1 | KH₂PO₄ | 10 |
| 2 | K₂HPO₄·3H₂O | 20 |
| | | |
| 1 | Glucose | 20 |
| 2 | MgCl₂·6H₂O | 1 |
| 3 | CaCl₂·2H₂O | 0.1 |
| | | |
| 1 | Sodium Citrate | 1 |
| 2 | FeSO₄·7H₂O | 0.01 |
| 3 | SM-1000X | 1.0 ml |
| | | |
| | H₂O | qs to 6000 ml |

**FEED**

| | **MATERIAL** | **g/L** |
|---|---|---|
| 1 | Glucose | 600 |
| 2 | CaCl₂·2H₂O | 0.6 |
| | | |
| | H₂O | qs to 3000 ml |

All fermentations were glucose limited fed batch processes. Immediately after inoculation, agitation was set at 200 rpm. The initial batched 2% glucose was consumed during exponential growth. Afterwards, glucose concentrations were maintained between 0.2 and 1.0 g/L by continuous feeding of a 60% glucose solution. The variable rate feed pump was computer controlled and linked to the dissolved oxygen concentration [pO₂] in the tank by an algorithm. When the [pO₂]fell to 30%, computer control began to automatically adjust the agitation rate to maintain a dissolved oxygen concentration between 25 and 30% [pO₂]. Computer control and data recording were by Braun MFCS software.

In one study, PA284 was grown in fermentors at two temperatures (40°C and 43°C) in the medium described in Table 22. Results of two experiments demonstrated that the highest pantothenate titers at early time points were produced at 43°C. The cell mass approached 150 optical density units at OD₆₀₀ and 56 hours at 43°C, and the pantothenate titers were 21 g/L, 28 g/L and 36 g/L at 36, 48 and 72 hours respectively. In the parallel fermentation at 40°C, the cell mass approached 120 optical density units at OD₆₀₀ and 56 hours, and the pantothenate titers were 18 g/L, 26 g/L and 37 g/L at 36, 48 and 72 hours, respectively.

In another study, PA824 was grown in a fermentor at 43°C in the medium described in Table 23. The cell mass exceeded 160 optical density units at OD₆₀₀ and 36 hours, and the pantothenate titers were 23 g/L, 34 g/L, 37 g/L and 40 g/L at 24, 36, 48 and 60 hours, respectively. In other fermentations, increasing the amount of trace elements in the glucose feed (e.g., increasing the concentration of SM from 1X to 2X) resulted in even higher titers of pantothenate.

### EXAMPLE XII: Identification and characterization of the B. subtilis coaA gene product

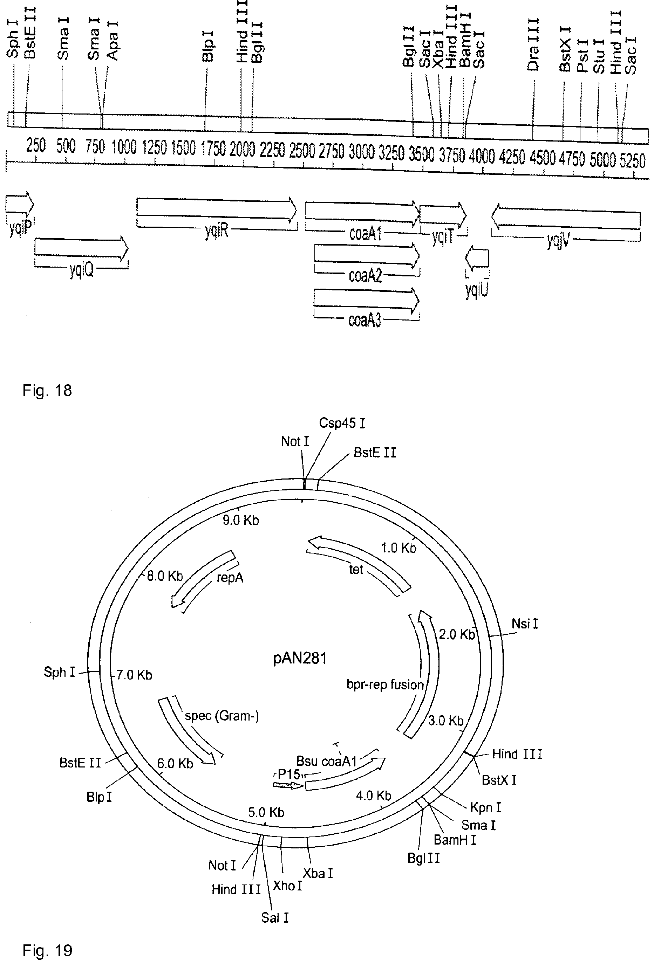

The annotated version of the *B. subtilis* genome sequence available on the "Subtilist" web site contains no gene labeled as *coaA.* However a homology search using the protein sequence of *E. coli* pantothenate kinase as a query sequence gave a good match with *B. subtilis* gene *yqjS*, which is annotated as "unknown; similar to pantothenate kinase." This gene appears to be the penultimate gene in an operon containing five open reading frames (Figure 18). Two of the open reading frames encode proteins which are similar to D-serine dehydratase and to "ketoacyl reductase"; the other two have no known homologies. For the open reading frame corresponding to *coaA*, there are three possible start codons; each having a possible ribosome-binding site (RBS) associated with it. The three potential *coaA* ORFs were named *coaA1, coaA2,* and *coaA3*, from longest to shortest.

All three potential *coaA* open reading frames were cloned along with their respective RBSs by PCR followed by ligation into expression plasmid pAN229. pAN229 is a low copy vector in *E. coli* that provides expression from the SP01 phage *P₁₅* promoter and can integrate by single crossover at *bpr* with tetracycline selection. A representative resulting plasmid, pAN281, is shown in Figure 19.

To determine if the cloned putative *coaA* ORFs actually encode a pantothenate kinase activity, several isolates of all three plasmids were transformed into the *E. coli* strain YH1, that contains the *coaA15(Ts)* allele. Transformants were streaked to plates incubated at 30° and 43°C to test for complementation of the temperature sensitive allele. All isolates of all three *coaA* variants, except for one isolate of pAN282, complemented well at 43°C, indicating that all three plasmid constructs encode an active pantothenate kinase. Accordingly, it can be concluded that the *B. subtilis yqjS* open reading frame codes for an active pantothenate kinase.

### EXAMPLE XIII: Deletion of the coaA gene from the B. subtilis genome

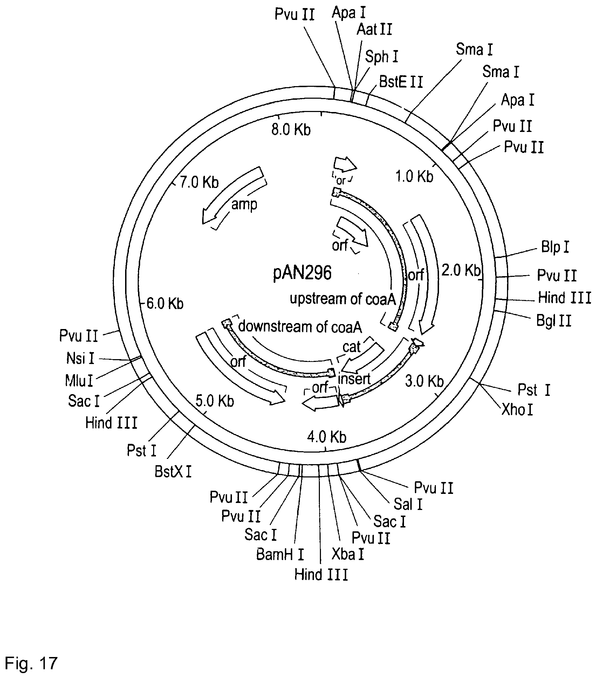

The *coaA* gene of *B. subtilis* (*yqjS*) was deleted from the chromosome of a *B. subtilis* strain by conventional means. The majority of the *coaA* coding sequence was deleted from a plasmid clone and replaced by a chloramphenicol resistance gene (*cat*), while leaving approximately 1 kb of upstream and downstream sequence to allow homologous recombination within the chromosome, to give plasmid pAN296 (see Figure 17). pAN296 was then used to transform a *B. subtilis* strain (PY79), selecting for chloramphenicol resistance. The majority of transformants result from a double crossover event that effectively substitutes the *cat* gene for the *coaA* gene. The transformed strain containing the *coaA* deletion - *cat* insertion grew normally due the presence of a second *B. subtilis* pantothenate kinase encoding gene described herein.

### EXAMPLE XIV: Identification and characterization of a second B. subtilis gene encoding pantothenate kinase activity

As described in detail in the instant specification, in order to maximize pantothenate production, it is necessary to restrict the flow of pantothenate toward Coenzyme A (CoA), for example, by reducing the activity of pantothenate kinase, the first enzyme in the pathway from pantothenate to CoA. After finding that deletion of the *coaA* gene from the chromosome of *B. subtilis* is not a lethal event (see Example XIII), it was concluded that *B. subtilis* must contain a second gene that encodes an active pantothenate kinase, since pantothenate kinase is an essential enzyme activity.

A second pantothenate kinase-encoding gene was identified by complementing the *E. coli* strain YH1 (*coaA15(Ts))* with a *B. subtilis* gene bank and selecting for transformants that were able to grow at 43° C. Found among the transformants were two families of plasmids that had overlapping restriction maps within each family, but not between the families. As expected, the restriction map of one family was identical to that predicted from the *B. subtilis* genome sequence for the homologue of the *E. coli coaA* gene (which we named *coaA* also, see above) and surrounding sequences. The other family had a restriction map that was completely non-overlapping with the first.

DNA sequencing of the ends of the cloned inserts from the second family showed that the clones came from a region of the *B. subtilis* chromosome that includes the 3' end of the *fts*H gene, the 5' end of the *sul* gene, and all of the *yacB, yacC, yacD*, *cysK, pabB*, *pabA* and *pabC* genes. None of the open reading frames of these cloned inserts showed homology to any known pantothenate kinase sequences, either prokaryotic or eukaryotic.

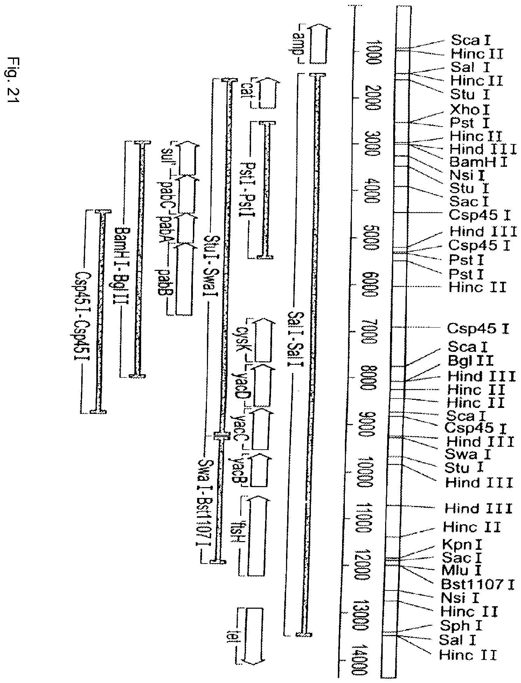

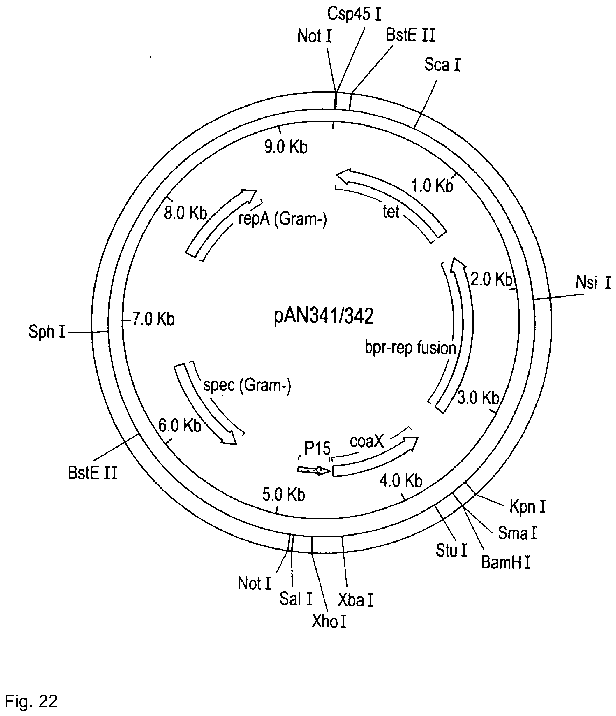

Several deletions were created through the *B. subtilis* genomic sequences in the cloned inserts. Each deletion was tested for complementation of the *E. coli* temperature sensitive pantothenate kinase. In particular, a deletion that removed all DNA between a *Stu* I site in the cloning vector and a *Swa* I site in the *yacC* gene, leaves *yacB* as the only intact open reading frame in the cloned insert (see Figure 21). This deleted plasmid still complemented the *E. coli* pantothenate kinase mutant. However, another deletion that removed DNA from the *Swa* I site in *yacC* through a *Bst*1107I site in the (already truncated) *ftsH* gene, could not complement the *E*. *coli* pantothenate kinase mutant. From these results, it was concluded that the *yacB* open reading frame was responsible for the complementation activity. To confirm that *yacB* is a pantothenate kinase gene, the *yacB* ORF plus 112 base pairs of downstream flanking sequence was amplified by PCR in two independent reactions and cloned downstream of a constitutive promote to give plasmids pAN341 and pAN342 (Figure 22). Both pAN341 and pAN342 complemented the defect in YH1 at 44°C, while a control plasmid, which has the same backbone, but expresses *panBCD* instead of *yacB* did not. This confirmed that the *yacB* open reading frame was responsible for the complementation of YH1.

As such, a novel gene that encodes pantothenate kinase activity in *B. subtilis* has been discovered that is not related by homology to any previously known pantothenate kinase gene. This gene has been renamed *coaX,* as a second, alternative gene that encodes an enzyme that catalyzes the first step in the pathway from pantothenate to CoaA. Deletion of *coaX* by methods described above for deleting *coaA,* in conjunction with reduction in the activity of the CoaA enzyme, provides a means to reduce pantothenate kinase activity to the desired level.

Several homologues of the *B. subtilis coaX gene* were identified by homology searching of various publically available databases using the published *yacB* (*coaX*) open reading frame sequence and predicted amino acid sequence (as set forth in SEQ ID NOs:84 and 85 respectively). In two cases (*Mycobacterium tuberculosis and Streptomyces coelicolor)* the homologous *coaX* genes are adjacent to, or almost adjacent to, pantothenate biosynthetic genes, consistent with these homologs having a role in pantothenate metabolism. The CoaX proteins show no homology to the CoaA family of pantothenate kinases, nor to the eukaryotic family of pantothenate kinases exemplified by PanK of *Saccharomyces cerevisiae.*

Alignment of the amino acid sequences of several bacterial CoaX homologs with the amino acid sequence predicted from translating the *B. subtilis yacB* ORF described in the published *B. subtilis* genome sequence revealed that the CoaX proteins from other bacteria contained additional amino acid residues at their carboxy-terminal ends. Moreover, these extensions beyond the end of the predicted amino acid for the *B. subtilis* gene product contained two relatively well conserved segments of sequence.

Translation of nucleotide sequences just downstream from the stop codon of the *B. subtilis yacB* ORF in a different reading frame revealed the existence of amino acid sequences very similar to the carboxy-terminal extensions of the other bacterial CoaX proteins. It is thus believed that an error exists in the published DNA sequence of the *B. subtilis yacB* ORF sequence that causes a frame shift leading to an artifactual downstream amino acid sequence and premature termination.

The PCR-generated sequences of *B. subtilis CoaX* in pAN341 and pAN342 (described above) contain enough downstream flanking sequence to encode the putative carboxy-terminal extension described above, which is consistent with the result that the clones were functional in the complementation assay. However when the 3' PCR primer was positioned to include only the shorter *yacB* ORF predicted from the published sequence, but not to include the putative carboxy-terminal extension, then the resulting plasmids, pAN329 and pAN330 (similar in structure to pAN341 and pAN342; *see* Figure 22), did not complement the defect in YH1. This result supports the notion that the published *yacB* coding sequence contains a frame-shift error, and that the carboxy-terminal end of CoaX is necessary for pantothenate kinase activity. The predicted correct nucleotide sequence for *B. subtilis coaX* is set forth as SEQ ID NO: 19 and the translated amino acid sequence is set forth as SEQ ID NO:9. A multiple sequence alignment of the CoaX amino acid sequences *of B. subtilis* and 11 homologues thereof is set forth in Figure 23.

### EXAMPLE XV: Generation of mutant coaA genes encoding pantothenate kinase having reduced or temperature sensitive activities

This Example describes strategies for modifying the *coaA* gene *(i.e.,* by introducing point mutations) to reduce the activity of pantothenate kinase after *coaX* is deleted from the genome.

### Cloning and sequencing of the temperature sensitive allele of the E. coli coaA gene.

Two *E. coli* strains, each exhibiting a different mutant CoaA phenotype, were obtained from the *E. coli* Genetic Stock Center. Strain DV62 contains the *coaA15(Ts)* allele, and DV79 contains the *coaA16(Fr)* mutation. DV62 is temperature sensitive at 43°C and produces a pantothenate kinase that is temperature sensitive. DV79 was obtained by reversion of DV62 to temperature resistance, and it produces a temperature stable, feedback resistant pantothenate kinase activity. Since the DNA sequences of these alleles are not available in the literature, the *coaA* genes from the two mutant strains were cloned by PCR and sequenced, in addition to a *coaA* gene from a strain that is wild type at the *coaA* locus, MM294. The PCR primer at the 5' end was designed to include the start codon plus four bases upstream, and added an arbitrarily chosen ribosome binding site (RBS). The three PCR generated fragments were each ligated between the *XbaI* and *BamHI* sites of pAN229 to give pAN284 (from *coaA15(Ts)*), pAN285 (from wild type *coaA*), and pAN286 (from *coaA16(Fr)*). pAN229 is a low copy *E. coli* vector that provides expression from the *P₁₅* promoter and that can integrate by single crossover at *bpr* in *B. subtilis* with tetracycline selection.

All three plasmids were transformed into the *E. coli* strain YH1 for complementation testing. All three plasmids complemented the temperature sensitive *coaA* mutation in *E. coli* YH1. It is presumed that the *coaA15(Ts)* gene in pAN284 is probably significantly overexpressed relative to the normal chromosomal gene, such that the overproduction compensates for the temperature sensitive defect. Complementation of a defect by overproduction is a well-documented phenomenon in *E. coli.*

The *coaA* coding regions from pAN284, 285, and 286 were subcloned into pGEM7 to give pAN306, 307, and 308, respectively, for DNA sequencing. As expected, the DNA sequence of the insert in pAN307 (from wild type *coaA*) matched the *coaA* sequence from the *E. coli* genome database (GenBank™). The sequence from pAN306 contains a single base change that causes a S176L substitution *(i.e.,* a Ser → Leu substitution in the amino acid sequence set forth as SEQ ID NO:2). Interestingly, the DNA sequence of the pAN308 insert, derived from the feedback resistant strain, was identical to that derived from its temperature sensitive parent (represented in pAN306). This is in accord with the genetic data that indicates that the reversion of the temperature sensitive mutation occurred at a second site unlinked to the *coaA* gene.

The S176L mutation, predicted to cause the temperature sensitive defect in *E. coli* pantothenate kinase, changed a serine residue that is conserved in all known or suspected bacterial *coaA* encoded pantothenate kinases, including that of *B. subtilis* (see SEQ ID NO:3 and refer to alignment). Based on this, a serine to leucine change at the homologous residue in the *B. subtilis* pantothenate kinase is predicted to result in either a temperature sensitive enzyme or one which is less active. Accordingly, to produce a mutant *B. subtilis coaA* gene, this specific change was introduced into the *B. subtilis coaA* gene. The mutant version is installed in the chromosome of a *B. subtilis* strain deleted for *coaX,* for example, and the recombinant microorganism is checked for temperature sensitivity (e.g., reduced growth at 43° C). The mutation is then installed into a pantothenate overproducing strain, preferably a strain deleted for the above mentioned *coaX* gene by standard methods to give strains favorable for pantothenate production in *B. subtilis, i.e*., a strain that has reduced pantothenate kinase activity under typical fermentation conditions.

### Additional coaA point mutations resulting in reduced pantothenate kinase activity

Of course it is expected that many other point mutations or combinations of more than one point mutation in *B. subtilis coaA* will also lead to reduced activity. Appropriate mutations can be generated by mutagenic polymerase chain reaction and *in vitro* recombination, and identified by screening for alleles that poorly complement the *E. coli coaA15(Ts)* mutant. An example of such a mutation of this type is a tyrosine to histidine substitution at amino acid 181 of B. *subtilis coaA*, generated by mutagenic polymerase chain reaction (see SEQ ID NO:3 and first line of the alignment of Figure 24).

Isolate pAN282A was derived from the middle-sized *B. subtilis coaA* open reading frame described in Example XII. pAN282A complemented the *E. coli coaA15(Ts)* mutant very poorly, but nonetheless at a level that was detectable above background. As was done for the *E. coli coaA* clones, the open reading frame from pAN282A was subcloned into pGEM7 to give pAN303. The DNA sequence of the insert in pAN303 showed a single base change that led to a tyrosine to histidine amino acid change at the tyrosine corresponding to Y181 of SEQ ID NO:3. This tyrosine residue is conserved in all bacterial *coaA* genes/homologues present in GenBank (Figure 24). This tyrosine residue and the serine that is altered in the *E. coli* temperature sensitive pantothenate kinase described above are separated by only three amino acid residues in a region which is highly conserved in bacterial pantothenate kinases whereas the DNA sequence of a second isolate of the middle-sized open reading frame, from pAN282B, was identical to the wild type sequence from the *B. subtilis* genome sequencing project. The single base change found in pAN303 probably occurred during PCR amplification of the *coaA* gene. If this variant of *coaA2* has sufficient residual biological activity in *B. subtilis,* it may be useful in the future for providing reduced pantothenate kinase activity.

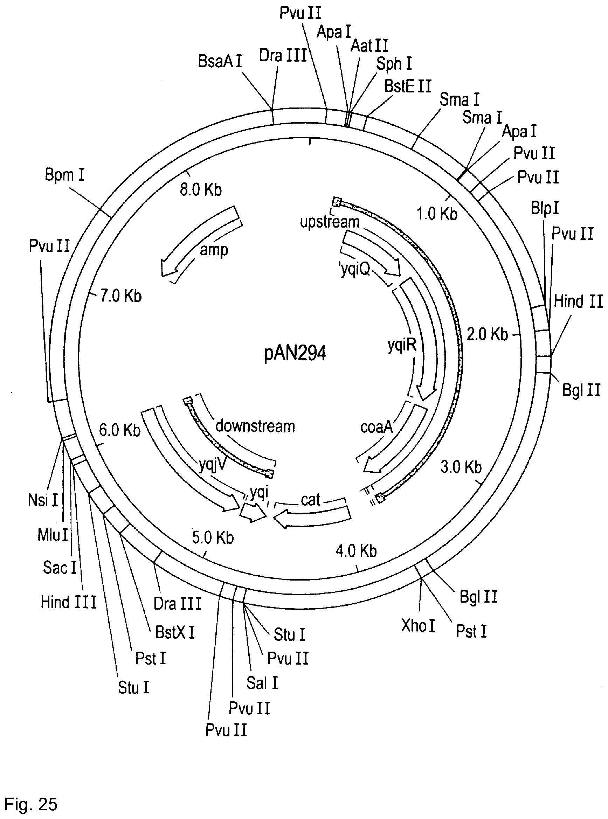

A preferred plasmid that can serve as a basis for mutagenizing the *coaA* open reading frame is pAN294 (see e.g., Figure 25 and Example XII). Briefly, mutagenic PCR is performed using pAN294 as a template and variants of *coaA* having reduced pantothenate kinase activity are screened as described above. Alternatively, mutations such as the one isolated in pAN282A can be installed into pAN294. The desired mutation is then introduced into the chromosome of a *B. subtilis* strain by transformation with the appropriate pAN294 derivative and selected for chloramphenicol resistance at 5 mg/L. Among the resulting transformants will be isolates that contain the desired mutation.

In a similar fashion, mutations that reduce the activity of the CoaX enzyme can be generated and identified, and such mutations used for optimizing pantothenate production by reducing CoA production as described above.

### EXAMPLE XVI: Deleting the second pantothenate kinase gene, coaX gene from B. subtilis

With the knowledge gained above concerning the existence and nature of *coaX,* one can create a deletion of the *coaX* open reading frame from the *B. subtilis* chromosome that will remove the encoded activity, and that will not adversely affect the expression of the genes downstream from *coaX.* In such a deleted strain, the *coaA* gene will be the only gene that encodes pantothenate kinase.

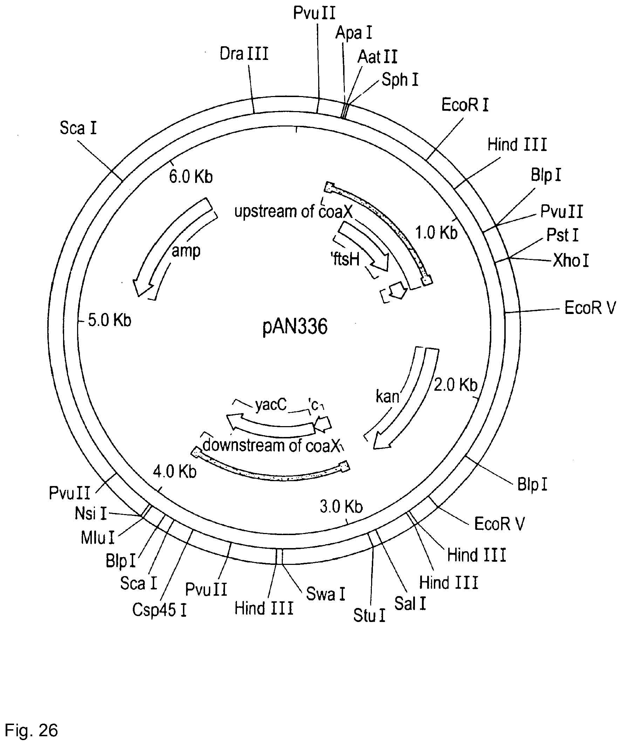

To delete the *coaX* gene from *B. subtilis,* plasmid pAN336 (SEQ ID NO:92), which contains upstream and downstream homology for double crossover, was constructed with a kanamycin resistance gene replacing most of the *coaX* ORF (Figure 26). Strain PY79 was transformed to kanamycin resistance by pAN336, and an isolate confirmed to have resulted from a double crossover by PCR was named PA876. As predicted, deletion of *coaX* by itself is not lethal for *B. subtilis.* Furthermore, chromosomal DNA from PA876 would not transform competent PA861 (PY79 *Δ*coaA ::cat) to kanamycin resistance. These results indicate that it is the combination of *ΔcoaA*::*cat* and *ΔcoaX* :: *kan* that is lethal for *B. subtilis,* confirming that *B. subtilis* contains two unlinked genes that encode pantothenate kanase, *coaA* and *coaX,* and that either gene alone is capable of supplying sufficient pantothenate kinase for a normal rate of growth.

### EXAMPLE XVII: Construction of a plasmid designed to allow directed mutagenesis of the B. subtilis coaA gene

In order to easily introduce mutated *coaA* genes into the *B. subtilis* chromosome, it was necessary to install an antibiotic resistance gene adjacent to the *coaA* gene. This was accomplished by joining together in the vector pGEM5 three DNA fragments: (1) a 3.4 kb DNA sequence containing 2.5 kb of genomic sequence upstream from *coaA* and the *coaA* open reading frame(s); (2) a 1.1 kb DNA sequence containing a chloramphenicol resistance gene (*cat*); and (3) a 1.4 kb DNA sequence comprising a region downstream from the operon that contains *coaA.* The resulting plasmid, named pAN294, effectively replaces the open reading frame *yqjT* (the open reading frame just downstream from *coaA*) with the *cat* gene, with enough homology flanking both sides of the *cat* gene to allow double recombination into the *B. subtilis* chromosome (Figure 25). pAN294 was transformed into *B. subtilis* strain PY79, selecting for chloramphenicol resistance at 5 mg/l to give strains PA836 and PA837, which are presumably identical. PA836 and 837 were checked by diagnostic PCR to show that the *cat* gene had integrated by double crossover, as opposed to single crossover. PA836 and PA837 grow normally, leading to the conclusion that the open reading frame *ygjT* is not essential *(i.e.,* the *yqjT* open reading frame could be deleted from strains PA836 and PA837 with no significant effect on growth or pantothenate production). Thus, variant alleles (*i.e*., mutations) of the *coaA* gane can be introduced into pAN294 and the resulting plasmids can be used to introduce the variant alleles into the chromosome of, for example, a *B. subtilis* strain.

### EXAMPLE XVIII: Generation of mutant coaX genes encoding pantothenate kinase having reduced or temperature sensitive activities

Mutant *coaX* genes are generated by introducing point mutations into the gene and testing the resulting mutants for the ability to complement the *E. coli* YH1 strain as described in Example XII. Preferred mutations in the *coaX* gene seqeunces are those that encode a substitution of a residue conserved among CoaX sequences from a variety of bacterial sources (*e.g*., a conserved residue set forth in Figure 23). Alternatively, random mutations in the *coaX* gene sequence are generated by mutagenic PCR and *in vitro* recombination and identified by screening for alleles that poorly complement the *E. coli coaA15(Ts)* mutant.

Mutants so generated *(i.e.,* mutants having reduced *coaX* activity) can be further engineered such that the endogenous *coaA* gene is deleted (as described in Example XIII). CoaX reduced-activity mutants can also be further engineered to contain reduced-activity CoaA gene products as described in Example XV.

### EXAMPLE XIX: Enhanced Production of Panto-Compounds Using Bacteria Having Deletions in One or More Pantothenate Biosynthetic Enzymes

If the desired panto-compound is not pantothenate, then an appropriate deletion of one or more of the pantothenate biosynthetic genes from a pantothenate overproducing strain will provide a strain that produces said desired panto-compound. In this example, the desired panto-compound is pantoate. Starting with, for example, strain PA236, PA313 or PA824 either one or both of the *pan*C and *panD* genes is deleted. In another example, ketopantoate is the desired panto-compound. Starting with, for example strain PA244, PA245 or PA824 one, two or all of the *ilvC*, *panE1*, *pan*C and *panD* genes are deleted from the starting strain. If β -alanine is the desired panto-compound, then *panB* and *panC* can be deleted, preferably in a fashion that leaves an in frame fusion of a small portion of the 5' end of *panB* with a small portion of the 3' end of *pan*C, from the strain PA221, PA235, PA245, or PA313. In all of the above-mentioned examples, the panto-compound producing strain will be a pantothenate auxotroph. Accordingly, the growth medium requires sufficient pantothenate for adequate growth. Vectors designed to overexpress *panD* as described above are then transformed into the above strains to further enhance β-alanine production.

The above-mentioned deletions are accomplished by methods well-known to those skilled in the art, for example, by insertion of an antibiotic resistance gene and removing sufficient sequence from the target gene(s) to inactivate said target gene(s). Alternatively, removal of targeted sequences is accomplished without simultaneous introduction of an antibiotic resistance gene in said target gene and then introduced by congression (co-transformation with any other appropriate selectable DNA sequence) followed by screening for the loss of function of said target gene by replica plating.

**Table 24 : Strains (and corresponding phenotypes) for panto-compound production**

| **Name** | **Pheno type** | **Drug resist.** | ***panBCD* locus** | ***panE* locus** | ***ilvD* locus** | ***amyE* locus** | ***bpr* locus** | **Parent** |
|---|---|---|---|---|---|---|---|---|
| PA221 | Trp- | | *P26panBCD* | | | | | |
| PA222 | | | *P₁₅panBCD* | | | | | RL-1 |
| PA235 | | | *P₂₆panBCD* | | | | | |
| PA236 | | | *P₂₆panBCD* | *P₂₆panE1* | | | | PA221 |
| PA327 | Trp- | | *P26panBCD* | *P26panE1* | | | | PA221 |
| PA328 | Trp- | | *P26panBCD* | *P26panE1* | | | | PA235 |
| PA340 | Trp- | Spc | *P26panBCD* | *P26panE1* | | *P26ilvBNC* | | PA327 |
| PA342 | Trp- | Spc | *P26panBCD* | *P26panE1* | | *P26ilvBNC* | | PA328 |
| PA354 | Trp- | Spc | *P26panBCD* | *P26panE1* | *P26ilvD* | *P26ilvBNC* | | PA342 |
| PA365 | Trp- | Spc, Tet | *P26panBCD* | *P26panE1* | *P26ilvD* | *P26ilvBNC* | *P26panD423* | PA354 |
| PA374 | Trp- | Spc | *P26panBCD* | *P26panE1* | *P26ilvD* | *P26ilvBNC* | | PA340 |
| PA377 | Trp- | Spc, Tet | *P26panBCD* | *P26panE1* | *P26ilvD* | *P26ilvBNC* | *P26panD423* | PA374 |
| PA401 | Trp- | | *P26panBCD* | | | | *P26panD423* | PA221 |
| PA402 | Trp- | | *P26panBCD* | | | | *P26panD428* | PA221 |
| PA403 | Trp- | | *P26panBCD* | | | | *P26panD429* | PA221 |
| PA404 | Trp- | Spc, Tet | *P26panBCD* | *P26panE1* | | *P26ilvBNC* | *P26panD423* | PA340 |
| PA405 | Trp- | Spc, Tet | *P26panBCD* | *P26panE1* | | *P26ilvBNC* | *P26panD423* | PA342 |
| PA651 | Trp- | Spc | *P26panBC*D* | *P26panE1* | *P26ilvD* | *P26ilvBNC* | | PA374 |
| PA284 | | Spc, Tet | *P26'panBCD* | *P26panE1* | *P26ilvD* | *P26ilvBNC* | *P26panD423* | PA377 |

Equivalents Those skilled in the art will recognize, or be able to ascertain using no more than routine experimentation, many equivalents to the specific embodiments of the disclosure described herein. Such equivalents are intended to be encompassed by the following claims.

### SEQUENCE LISTING

<110> BASF SE
<120> METHODS AND MICROORGANISMS FOR PRODUCTION OF PANTO-COMPOUNDS
<130> PF51386 EP_DIV-1
<140> Current patent application; Divisional of EP 00 966 799
   <141> 2009 12 18
<150> USSN 09/400,494
   <151> 1999-09-21
<150> USSN 60/210,072
   <151> 2000-06-07
<150> USSN 60/221,836
   <151> 2000-07-28
<150> USSN 60/227,860
   <151> 2000-08-24
<160> 98
<170> PatentIn Ver. 2.0
<210> 1
   <211> 311
   <212> PRT
   <213> Haemophilus influenzae
<400> 1
   Met Glu Phe Ser Thr Gln Gln Thr Pro Phe Leu Ser Phe Asn Arg Glu
     1               5                  10                  15
   Gln Trp Ala Glu Leu Arg Lys Ser Val Pro Leu Lys Leu Thr Glu Gln
                20                  25                  30
   Asp Leu Lys Pro Leu Leu Gly Phe Asn Glu Asp Leu Ser Leu Asp Glu
            35                  40                  45
   Val Ser Thr Ile Tyr Leu Pro Leu Thr Arg Leu Ile Asn Tyr Tyr Ile
        50                  55                  60
   Asp Glu Asn Leu His Arg Gln Thr Val Leu His Arg Phe Leu Gly Arg
    65                  70                  75                  80
   Asn Asn Ala Lys Thr Pro Tyr Ile Ile Ser Ile Ala Gly Ser Val Ala
                    85                  90                  95
   Val Gly Lys Ser Thr Ser Ala Arg Ile Leu Gln Ser Leu Leu Ser His
               100                 105                 110
   Trp Pro Thr Glu Arg Lys Val Asp Leu Ile Thr Thr Asp Gly Phe Leu
           115                 120                 125
   Tyr Pro Leu Asn Lys Leu Lys Gln Asp Asn Leu Leu Gln Lys Lys Gly
       130                 135                 140
   Phe Pro Val Ser Tyr Asp Thr Pro Lys Leu Ile Arg Phe Leu Ala Asp
   145                 150                 155                 160
   Val Lys Ser Gly Lys Ser Asn Val Thr Ala Pro Ile Tyr Ser His Leu
                   165                 170                 175
   Thr Tyr Asp Ile Ile Pro Asp Lys Phe Asp Val Val Asp Lys Pro Asp
                   180                 185                 190
   Ile Leu Ile Leu Glu Gly Leu Asn Val Leu Gln Thr Gly Asn Asn Lys
           195                 200                 205
   Thr Asp Gln Thr Phe Val Ser Asp Phe Val Asp Phe Ser Ile Tyr Val
       210                 215                 220
   Asp Ala Glu Glu Lys Leu Leu Lys Glu Trp Tyr Ile Lys Arg Phe Leu
   225                 230                 235                 240
   Lys Phe Arg Glu Ser Ala Phe Asn Asp Pro Asn Ser Tyr Phe Lys His
                   245                 250                 255
   Tyr Ala Ser Leu Ser Lys Glu Glu Ala Ile Ala Thr Ala Ser Lys Ile
               260                 265                 270
   Trp Asp Glu Ile Asn Gly Leu Asn Leu Asn Gln Asn Ile Leu Pro Thr
           275                 280                 285
   Arg Glu Arg Ala Asn Leu Ile Leu Lys Lys Gly His Asn His Gln Val
       290                 295                 300
   Glu Leu Ile Lys Leu Arg Lys
   305                 310
<210> 2
   <211> 316
   <212> PRT
   <213> Escherichia coli
<400> 2
   Met Ser Ile Lys Glu Gln Thr Leu Met Thr Pro Tyr Leu Gln Phe Asp
   1               5                   10                  15
   Arg Asn Gln Trp Ala Ala Leu Arg Asp Ser Val Pro Met Thr Leu Ser
               20                  25                  30
   Glu Asp Glu Ile Ala Arg Leu Lys Gly Ile Asn Glu Asp Leu Ser Leu
           35                  40                  45
   Glu Glu Val Ala Glu Ile Tyr Leu Pro Leu Ser Arg Leu Leu Asn Phe
       50                  55                  60
   Tyr Ile Ser Ser Asn Leu Arg Arg Gln Ala Val Leu Glu Gln Phe Leu
   65                  70                  75                  80
   Gly Thr Asn Gly Gln Arg Ile Pro Tyr Ile Ile Ser Ile Ala Gly Ser
                   85                  90                  95
   Val Ala Val Gly Lys Ser Thr Thr Ala Arg Val Leu Gln Ala Leu Leu
               100                 105                 110
   Ser Arg Trp Pro Glu His Arg Arg Val Glu Leu Ile Thr Thr Asp Gly
           115                 120                 125
   Phe Leu His Pro Asn Gln Val Leu Lys Glu Arg Gly Leu Met Lys Lys
       130                 135                 140
   Lys Gly Phe Pro Glu Ser Tyr Asp Met His Arg Leu Val Lys Phe Val
   145                 150                 155                 160
   Ser Asp Leu Lys Ser Gly Val Pro Asn Val Thr Ala Pro Val Tyr Ser
                   165                 170                 175
   His Leu Ile Tyr Asp Val Ile Pro Asp Gly Asp Lys Thr Val Val Gln
               180                 185                 190
   Pro Asp Ile Leu Ile Leu Glu Gly Leu Asn Val Leu Gln Ser Gly Met
           195                 200                 205
   Asp Tyr Pro His Asp Pro His His Val Phe Val Ser Asp Phe Val Asp
       210                 215                 220
   Phe Ser Ile Tyr Val Asp Ala Pro Glu Asp Leu Leu Gln Thr Trp Tyr
   225                 230                 235                 240
   Ile Asn Arg Phe Leu Lys Phe Arg Glu Gly Ala Phe Thr Asp Pro Asp
                   245                 250                 255
   Ser Tyr Phe His Asn Tyr Ala Lys Leu Thr Lys Glu Glu Ala Ile Lys
               260                 265                 270
   Thr Ala Met Thr Leu Trp Lys Glu Ile Asn Trp Leu Asn Leu Lys Gln
           275                 280                 285
   Asn Ile Leu Pro Thr Arg Glu Arg Ala Ser Leu Ile Leu Thr Lys Ser
       290                 295                 300
   Ala Asn His Ala Val Glu Glu Val Arg Leu Arg Lys
   305                 310                 315
<210> 3
   <211> 319
   <212> PRT
   <213> Bacillus subtilis
<400> 3
   Met Lys Asn Lys Glu Leu Asn Leu His Thr Leu Tyr Thr Gln His Asn
   1 5 10 15
   Arg Glu Ser Trp Ser Gly Phe Gly Gly His Leu Ser Ile Ala Val Ser
   20 25 30
   Glu Glu Glu Ala Lys Ala Val Glu Gly Leu Asn Asp Tyr Leu Ser Val
   35 40 45
   Glu Glu Val Glu Thr Ile Tyr Ile Pro Leu Val Arg Leu Leu His Leu
   50 55 60
   His Val Lys Ser Ala Ala Glu Arg Asn Lys His Val Asn Val Phe Leu
   65 70 75 80
   Lys His Pro His Ser Ala Lys Ile Pro Phe Ile Ile Gly Ile Ala Gly
   85 90 95
   Ser Val Ala Val Gly Lys Ser Thr Thr Ala Arg Ile Leu Gln Lys Leu
   100 105 110
   Leu Ser Arg Leu Pro Asp Arg Pro Lys Val Ser Leu Ile Thr Thr Asp
   115 120 125
   Gly Phe Leu Phe Pro Thr Ala Glu Leu Lys Lys Lys Asn Met Met Ser
   130 135 140
   Arg Lys Gly Phe Pro Glu Ser Tyr Asp Val Lys Ala Leu Leu Glu Phe
   145 150 155 160
   Leu Asn Asp Leu Lys Ser Gly Lys Asp Ser Val Lys Ala Pro Val Tyr
   165 170 175
   Ser His Leu Thr Tyr Asp Arg Glu Glu Gly Val Phe Glu Val Val Glu
   180 185 190
   Gln Ala Asp Ile Val Ile Ile Glu Gly Ile Asn Val Leu Gln Ser Pro
   195 200 205
   Thr Leu Glu Asp Asp Arg Glu Asn Pro Arg Ile Phe Val Ser Asp Phe
   210 215 220
   Phe Asp Phe Ser Ile Tyr Val Asp Ala Glu Glu Ser Arg Ile Phe Thr
   225 230 235 240
   Trp Tyr Leu Glu Arg Phe Arg Leu Leu Arg Glu Thr Ala Phe Gln Asn
   245 250 255
   Pro Asp Ser Tyr Phe His Lys Phe Lys Asp Leu Ser Asp Gln Glu Ala
   260 265 270
   Asp Glu Met Ala Ala Ser Ile Trp Glu Ser Val Asn Arg Pro Asn Leu
   275 280 285
   Tyr Glu Asn Ile Leu Pro Thr Lys Phe Arg Ser Asp Leu Ile Leu Arg
   290 295 300
   Lys Gly Asp Gly His Lys Val Glu Glu Val Leu Val Arg Arg Val
   305 310 315
<210> 4
   <211> 312
   <212> PRT
   <213> Mycobacterium leprae
<400> 4
   Met Pro Arg Leu Ser Glu Pro Ser Pro Tyr Val Glu Phe Asp Arg Lys
   1 5 10 15
   Gln Trp Arg Ala Leu Arg Met Ser Thr Pro Leu Ala Leu Thr Glu Glu
   20 25 30
   Glu Leu Ile Gly Leu Arg Gly Leu Gly Glu Gln Ile Asp Leu Leu Glu
   35 40 45
   Val Glu Glu Val Tyr Leu Pro Leu Ala Arg Leu Ile His Leu Gln Val
   50 55 60
   Ala Ala Arg Gln Arg Leu Phe Ala Ala Thr Ala Glu Phe Leu Gly Glu
   65 70 75 80
   Pro Gln Gln Asn Pro Gly Arg Pro Val Pro Phe Ile Ile Gly Val Ala
   85 90 95
   Gly Ser Val Ala Val Gly Lys Ser Thr Thr Ala Arg Val Leu Gln Ala
   100 105 110
   Leu Leu Ala Arg Trp Asp His His Thr Arg Val Asp Leu Val Thr Thr
   115 120 125
   Asp Gly Phe Leu Tyr Pro Asn Ala Glu Leu Gly Arg Arg Asn Leu Met
   130 135 140
   His Arg Lys Gly Phe Pro Glu Ser Tyr Asn Arg Arg Ala Leu Met Arg
   145 150 155 160
   Phe Val Thr Ser Val Lys Ser Gly Ala Asp Tyr Ala Cys Ala Pro Val
   165 170 175
   Tyr Ser His Leu Arg Tyr Asp Thr Ile Pro Gly Ala Lys His Val Val
   180 185 190
   Arg His Pro Asp Ile Leu Ile Leu Glu Gly Leu Asn Val Leu Gln Thr
   195 200 205
   Gly Pro Thr Leu Met Val Ser Asp Leu Phe Asp Phe Ser Leu Tyr Val
   210 215 220
   Asp Ala Arg Ile Gln Asp Ile Glu Gln Trp Tyr Val Ser Arg Phe Leu
   225 230 235 240
   Ala Met Arg Gly Thr Ala Phe Ala Asp Pro Glu Ser His Phe His His
   245 250 255
   Tyr Ser Ala Leu Thr Asp Ser Lys Ala Ile Ile Ala Ala Arg Glu Ile
   260 265 270
   Trp Arg Ser Ile Asn Arg Pro Asn Leu Val Glu Asn Ile Leu Pro Thr
   275 280 285
   Arg Pro Arg Ala Thr Leu Val Leu Arg Lys Asp Ala Asp His Ser Ile
   290 295 300
   Asn Arg Leu Arg Leu Arg Lys Leu
   305 310
<210> 5
   <211> 312
   <212> PRT
   <213> Mycobacterium tuberculosis
<400> 5
   Met Ser Arg Leu Ser Glu Pro Ser Pro Tyr Val Glu Phe Asp Arg Arg
    1               5                  10                  15
   Gln Trp Arg Ala Leu Arg Met Ser Thr Pro Leu Ala Leu Thr Glu Glu
               20                  25                  30
   Glu Leu Val Gly Leu Arg Gly Leu Gly Glu Gln Ile Asp Leu Leu Glu
           35                  40                  45
   Val Glu Glu Val Tyr Leu Pro Leu Ala Arg Leu Ile His Leu Gln Val
       50                  55                  60
   Ala Ala Arg Gln Arg Leu Phe Ala Ala Thr Ala Glu Phe Leu Gly Glu
   65                  70                  75                  80
   Pro Gln Gln Asn Pro Asp Arg Pro Val Pro Phe Ile Ile Gly Val Ala
                   85                  90                  95
   Gly Ser Val Ala Val Gly Lys Ser Thr Thr Ala Arg Val Leu Gln Ala
               100                 105                 110
   Leu Leu Ala Arg Trp Asp His His Pro Arg Val Asp Leu Val Thr Thr
           115                 120                 125
   Asp Gly Phe Leu Tyr Pro Asn Ala Glu Leu Gln Arg Arg Asn Leu Met
       130                 135                 140
   His Arg Lys Gly Phe Pro Glu Ser Tyr Asn Arg Arg Ala Leu Met Arg
   145                 150                 155                 160
   Phe Val Thr Ser Val Lys Ser Gly Ser Asp Tyr Ala Cys Ala Pro Val
                   165                 170                 175
   Tyr Ser His Leu His Tyr Asp Ile Ile Pro Gly Ala Glu Gln Val Val
               180                 185                 190
   Arg His Pro Asp Ile Leu Ile Leu Glu Gly Leu Asn Val Leu Gln Thr
           195                 200                 205
   Gly Pro Thr Leu Met Val Ser Asp Leu Phe Asp Phe Ser Leu Tyr Val
       210                 215                 220
   Asp Ala Arg Ile Glu Asp Ile Glu Gln Trp Tyr Val Ser Arg Phe Leu
   225                 230                 235                 240
   Ala Met Arg Thr Thr Ala Phe Ala Asp Pro Glu Ser His Phe His His
                   245                 250                 255
   Tyr Ala Ala Phe Ser Asp Ser Gln Ala Val Val Ala Ala Arg Glu Ile
               260                 265                 270
   Trp Arg Thr Ile Asn Arg Pro Asn Leu Val Glu Asn Ile Leu Pro Thr
           275                 280                 285
   Arg Pro Arg Ala Thr Leu Val Leu Arg Lys Asp Ala Asp His Ser Ile
       290                 295                 300
   Asn Arg Leu Arg Leu Arg Lys Leu
   305                 310
<210> 6
   <211> 329
   <212> PRT
   <213> Streptomyces coelicolor
<400> 6
   Met Ile Ser Pro Val Pro Ser Ile Pro Arg Ser Ala His Arg Gln Arg
   1               5                   10                  15
   Pro Glu Ala Thr Pro Tyr Val Asp Leu Thr Arg Pro Glu Trp Ser Ala
               20                  25                  30
   Leu Arg Asp Lys Thr Pro Leu Pro Leu Thr Ala Glu Glu Val Glu Lys
           35                  40                  45
   Leu Arg Gly Leu Gly Asp Val Ile Asp Leu Asp Glu Val Arg Asp Ile
       50                  55                  60
   Tyr Leu Pro Leu Ser Arg Leu Leu Asn Leu Tyr Val Gly Ala Thr Asp
   65                  70                  75                  80
   Gly Leu Arg Gly Ala Leu Asn Thr Phe Leu Gly Glu Gln Gly Ser Gln
                   85                  90                  95
   Ser Gly Thr Pro Phe Val Ile Gly Val Ala Gly Ser Val Ala Val Gly
               100                 105                 110
   Lys Ser Thr Val Ala Arg Leu Leu Gln Ala Leu Leu Ser Arg Trp Pro
           115                 120                 125
   Glu His Pro Arg Val Glu Leu Val Thr Thr Asp Gly Phe Leu Leu Pro
       130                 135                 140
   Thr Arg Glu Leu Glu Ala Arg Gly Leu Met Ser Arg Lys Gly Phe Pro
   145                 150                 155                 160
   Glu Ser Tyr Asp Arg Arg Ala Leu Thr Arg Phe Val Ala Asp Ile Lys
                   165                 170                 175
   Ala Gly Lys Ala Glu Val Thr Ala Pro Val Tyr Ser His Leu Ile Tyr
               180                 185                 190
   Asp Ile Val Pro Asp Gln Arg Leu Val Val Arg Arg Pro Asp Ile Leu
           195                 200                 205
   Ile Val Glu Gly Leu Asn Val Leu Gln Pro Ala Leu Pro Gly Lys Asp
       210                 215                 220
   Gly Arg Thr Arg Val Gly Leu Ala Asp Tyr Phe Asp Phe Ser Val Tyr
   225                 230                 235                 240
   Val Asp Ala Arg Thr Glu Asp Ile Glu Arg Trp Tyr Leu Asn Arg Phe
                   245                 250                 255
   Arg Lys Leu Arg Ala Thr Ala Phe Gln Asn Pro Ser Ser Tyr Phe Arg
               260                 265                 270
   Lys Tyr Thr Gln Val Ser Glu Glu Glu Ala Leu Asp Tyr Ala Arg Thr
           275                 280                 285
   Thr Trp Arg Thr Ile Asn Lys Pro Asn Leu Val Glu Asn Val Ala Pro
       290                 295                 300
   Thr Arg Gly Arg Ala Thr Leu Val Leu Arg Lys Gly Pro Asp His Lys
   305                 310                 315                 320
   Val Gln Arg Leu Ser Leu Arg Lys Leu
                   325
<210> 7
   <211> 265
   <212> PRT
   <213> Streptomyces coelicolor
<400> 7
   Met Leu Leu Thr Ile Asp Val Gly Asn Thr His Thr Val Leu Gly Leu
    1               5                  10                  15
   Phe Asp Gly Glu Asp Ile Val Glu His Trp Arg Ile Ser Thr Asp Ser
               20                  25                  30
   Arg Arg Thr Ala Asp Glu Leu Ala Val Leu Leu Gln Gly Leu Met Gly
           35                  40                  45
   Met His Pro Leu Leu Gly Asp Glu Leu Gly Asp Gly Ile Asp Gly Ile
       50                  55                  60
   Ala Ile Cys Ala Thr Val Pro Ser Val Leu His Glu Leu Arg Glu Val
   65                  70                  75                  80
   Thr Arg Arg Tyr Tyr Gly Asp Val Pro Ala Val Leu Val Glu Pro Gly
                   85                  90                  95
   Val Lys Thr Gly Val Pro Ile Leu Thr Asp His Pro Lys Glu Val Gly
               100                 105                 110
   Ala Asp Arg Ile Ile Asn Ala Val Ala Ala Val Glu Leu Tyr Gly Gly
           115                 120                 125
   Pro Ala Ile Val Val Asp Phe Gly Thr Ala Thr Thr Phe Asp Ala Val
       130                 135                 140
   Ser Ala Arg Gly Glu Tyr Ile Gly Gly Val Ile Ala Pro Gly Ile Glu
   145                 150                 155                 160
   Ile Ser Val Glu Ala Leu Gly Val Lys Gly Ala Gln Leu Arg Lys Ile
                   165                 170                 175
   Glu Val Ala Arg Pro Arg Ser Val Ile Gly Lys Asn Thr Val Glu Ala
               180                 185                 190
   Met Gln Ser Gly Ile Val Tyr Gly Phe Ala Gly Gln Val Asp Gly Val
           195                 200                 205
   Val Asn Arg Met Ala Arg Glu Leu Ala Asp Asp Pro Asp Asp Val Thr
       210                 215                 220
   Val Ile Ala Thr Gly Gly Leu Ala Pro Met Val Leu Gly Glu Ser Ser
   225                 230                 235                 240
   Val Ile Asp Glu His Glu Pro Trp Leu Thr Leu Met Gly Leu Arg Leu
                   245                 250                 255
   Val Tyr Glu Arg Asn Val Ser Arg Met
               260                 265
<210> 8
   <211> 272
   <212> PRT
   <213> Mycobacterium tuberculosis
<400> 8
   Met Leu Leu Ala Ile Asp Val Arg Asn Thr His Thr Val Val Gly Leu
   1 5 10 15
   Leu Ser Gly Met Lys Glu His Ala Lys Val Val Gln Gln Trp Arg Ile
   20 25 30
   Arg Thr Glu Ser Glu Val Thr Ala Asp Glu Leu Ala Leu Thr Ile Asp
   35 40 45
   Gly Leu Ile Gly Glu Asp Ser Glu Arg Leu Thr Gly Thr Ala Ala Leu
   50 55 60
   Ser Thr Val Pro Ser Val Leu His Glu Val Arg Ile Met Leu Asp Gln
   65 70 75 80
   Tyr Trp Pro Ser Val Pro His Val Leu Ile Glu Pro Gly Val Arg Thr
   85 90 95
   Gly Ile Pro Leu Leu Val Asp Asn Pro Lys Glu Val Gly Ala Asp Arg
   100 105 110
   Ile Val Asn Cys Leu Ala Ala Tyr Asp Arg Phe Arg Lys Ala Ala Ile
   115 120 125
   Val Val Asp Phe Gly Ser Ser Ile Cys Val Asp Val Val Ser Ala Lys
   130 135 140
   Gly Glu Phe Leu Gly Gly Ala Ile Ala Pro Gly Val Gln Val Ser Ser
   145 150 155 160
   Asp Ala Ala Ala Ala Arg Ser Ala Ala Leu Arg Arg Val Glu Leu Ala
   165 170 175
   Arg Pro Arg Ser Val Val Gly Lys Asn Thr Val Glu Cys Met Gln Ala
   180 185 190
   Gly Ala Val Phe Gly Phe Ala Gly Leu Val Asp Gly Leu Val Gly Arg
   195 200 205
   Ile Arg Glu Asp Val Ser Gly Phe Ser Val Asp His Asp Val Ala Ile
   210 215 220
   Val Ala Thr Gly His Thr Ala Pro Leu Leu Leu Pro Glu Leu His Thr
   225 230 235 240
   Val Asp His Tyr Asp Gln His Leu Thr Leu Gln Gly Leu Arg Leu Val
   245 250 255
   Phe Glu Arg Asn Leu Glu Val Gln Arg Gly Arg Leu Lys Thr Ala Arg
   260 265 270
<210> 9
   <211> 258
   <212> PRT
   <213> Bacillus subtilis
<400> 9
   Leu Leu Leu Val Ile Asp Val Gly Asn Thr Asn Thr Val Leu Gly Val
   1               5                   10                  15
   Tyr His Asp Gly Lys Leu Glu Tyr His Trp Arg Ile Glu Thr Ser Arg
               20                  25                  30
   His Lys Thr Glu Asp Glu Phe Gly Met Ile Leu Arg Ser Leu Phe Asp
           35                  40                  45
   His Ser Gly Leu Met Phe Glu Gln Ile Asp Gly Ile Ile Ile Ser Ser
       50                  55                  60
   Val Val Pro Pro Ile Met Phe Ala Leu Glu Arg Met Cys Thr Lys Tyr
   65                  70                  75                  80
   Phe His Ile Glu Pro Gln Ile Val Gly Pro Gly Met Lys Thr Gly Leu
                   85                  90                  95
   Asn Ile Lys Tyr Asp Asn Pro Lys Glu Val Gly Ala Asp Arg Ile Val
               100                 105                 110
   Asn Ala Val Ala Ala Ile His Leu Tyr Gly Asn Pro Leu Ile Val Val
           115                 120                 125
   Asp Phe Gly Thr Ala Thr Thr Tyr Cys Tyr Ile Asp Glu Asn Lys Gln
       130                 135                 140
   Tyr Met Gly Gly Ala Ile Ala Pro Gly Ile Thr Ile Ser Thr Glu Ala
   145                 150                 155                 160
   Leu Tyr Ser Arg Ala Ala Lys Leu Pro Arg Ile Glu Ile Thr Arg Pro
                   165                 170                 175
   Asp Asn Ile Ile Gly Lys Asn Thr Val Ser Ala Met Gln Ser Gly Ile
               180                 185                 190
   Leu Phe Gly Tyr Val Gly Gln Val Glu Gly Ile Val Lys Arg Met Lys
           195                 200                 205
   Trp Gln Ala Lys Gln Asp Leu Lys Val Ile Ala Thr Gly Gly Leu Ala
       210                 215                 220
   Pro Leu Ile Ala Asn Glu Ser Asp Cys Ile Asp Ile Val Asp Pro Phe
   225                 230                 235                 240
   Leu Thr Leu Lys Gly Leu Glu Leu Ile Tyr Glu Arg Asn Arg Val Gly
                   245                 250                 255
   Ser Val
<210> 10
   <211> 262
   <212> PRT
   <213> Deinococcus radiopugnans
<400> 10
   Met Pro Ala Phe Pro Leu Leu Ala Val Asp Ile Gly Asn Thr Thr Thr
   1               5                   10                  15
   Val Leu Gly Leu Ala Asp Ala Ser Gly Ala Leu Thr His Thr Trp Arg
                   20                  25                  30
   Ile Arg Thr Asn Arg Glu Met Leu Pro Asp Asp Leu Ala Leu Gln Leu
           35                  40                  45
   His Gly Leu Phe Thr Leu Ala Gly Ala Pro Ile Pro Arg Ala Ala Val
       50                  55                  60
   Leu Ser Ser Val Ala Pro Pro Val Gly Glu Asn Tyr Ala Leu Ala Leu
   65                  70                  75                  80
   Lys Arg His Phe Met Ile Asp Ala Phe Ala Val Ser Ala Glu Asn Leu
                   85                  90                  95
   Pro Asp Val Thr Val Glu Leu Asp Thr Pro Gly Ser Val Gly Ala Asp
               100                 105                 110
   Arg Leu Cys Asn Leu Phe Gly Ala Glu Lys Tyr Leu Gly Gly Leu Asp
           115                 120                 125
   Tyr Ala Val Val Val Asp Phe Gly Thr Ser Thr Asn Phe Asp Val Val
       130                 135                 140
   Gly Arg Gly Arg Arg Phe Leu Gly Gly Ile Leu Ala Thr Gly Ala Gln
   145                 150                 155                 160
   Val Ser Ala Asp Ala Leu Phe Ala Arg Ala Ala Lys Leu Pro Arg Ile
                   165                 170                 175
   Thr Leu Gln Ala Pro Glu Thr Ala Ile Gly Lys Asn Thr Val His Ala
               180                 185                 190
   Leu Gln Ser Gly Leu Val Phe Gly Tyr Ala Glu Met Val Asp Gly Leu
           195                 200                 205
   Leu Arg Arg Ile Arg Ala Glu Leu Pro Gly Glu Ala Val Ala Val Ala
       210                 215                 220
   Thr Gly Gly Phe Ser Arg Thr Val Gln Gly Ile Cys Gln Glu Ile Asp
   225                 230                 235                 240
   Tyr Tyr Asp Glu Thr Leu Thr Leu Arg Gly Leu Val Glu Leu Trp Ala
                   245                 250                 255
   Ser Arg Ser Glu Val Arg
               260
<210> 11
   <211> 212
   <212> PRT
   <213> Desulfovibrio vulgaris
<400> 11
   Met Thr Gln His Phe Leu Leu Phe Asp Ile Gly Asn Thr Asn Val Lys
   1               5                   10                  15
   Ile Gly Ile Ala Val Glu Thr Ala Val Leu Thr Ser Tyr Val Leu Pro
               20                  25                  30
   Thr Asp Pro Gly Gln Thr Thr Asp Ser Ile Gly Leu Arg Leu Leu Glu
           35                  40                  45
   Val Leu Arg His Ala Gly Leu Gly Pro Ala Asp Val Gly Ala Cys Val
       50                  55                  60
   Ala Ser Ser Val Val Pro Gly Val Asn Pro Leu Ile Arg Arg Ala Cys
   65                  70                  75                  80
   Glu Arg Tyr Leu Tyr Arg Lys Leu Leu Phe Ala Pro Gly Asp Ile Ala
                   85                  90                  95
   Ile Pro Leu Asp Asn Arg Tyr Glu Arg Pro Ala Glu Val Gly Ala Asp
               100                 105                 110
   Arg Leu Val Ala Ala Tyr Ala Ala Arg Arg Leu Tyr Pro Gly Pro Arg
           115                 120                 125
   Ser Leu Val Ser Val Asp Phe Gly Thr Ala Thr Thr Phe Asp Cys Val
       130                 135                 140
   Glu Gly Gly Ala Tyr Leu Gly Gly Leu Ile Cys Pro Gly Val Leu Ser
   145                 150                 155                 160
   Ser Ala Gly Ala Leu Ser Ser Arg Thr Ala Lys Leu Pro Arg Ile Ser
                   165                 170                 175
   Leu Glu Val Glu Glu Asp Ser Pro Val Ile Gly Arg Ser Thr Thr Thr
               180                 185                 190
   Ser Leu Asn His Gly Phe Ile Phe Gly Phe Ala Ala Met Thr Glu Gly
           195                 200                 205
   Val Leu Ala Ala
       210
<210> 12
   <211> 246
   <212> PRT
   <213> Thermotoga maritima
<400> 12
   Met Tyr Leu Leu Val Asp Val Gly Asn Thr His Ser Val Phe Ser Ile
   1 5 10 15
   Thr Glu Asp Gly Lys Thr Phe Arg Arg Trp Arg Leu Ser Thr Gly Val
   20 25 30
   Phe Gln Thr Glu Asp Glu Leu Phe Ser His Leu His Pro Leu Leu Gly
   35 40 45
   Asp Ala Met Arg Glu Ile Lys Gly Ile Gly Val Ala Ser Val Val Pro
   50 55 60
   Thr Gln Asn Thr Val Ile Glu Arg Phe Ser Gln Lys Tyr Phe His Ile
   65 70 75 80
   Ser Pro Ile Trp Val Lys Ala Lys Asn Gly Cys Val Lys Trp Asn Val
   85 90 95
   Lys Asn Pro Ser Glu Val Gly Ala Asp Arg Val Ala Asn Val Val Ala
   100 105 110
   Phe Val Lys Glu Tyr Gly Lys Asn Gly Ile Ile Ile Asp Met Gly Thr
   115 120 125
   Ala Thr Thr Val Asp Leu Val Val Asn Gly Ser Tyr Glu Gly Gly Ala
   130 135 140
   Ile Leu Pro Gly Phe Phe Met Met Val His Ser Leu Phe Arg Gly Thr
   145 150 155 160
   Ala Lys Leu Pro Leu Val Glu Val Lys Pro Ala Asp Phe Val Val Gly
   165 170 175
   Lys Asp Thr Glu Glu Asn Ile Arg Leu Gly Val Val Asn Gly Ser Val
   180 185 190
   Tyr Ala Leu Glu Gly Ile Ile Gly Arg Ile Lys Glu Val Tyr Gly Asp
   195 200 205
   Leu Pro Val Val Leu Thr Gly Gly Gln Ser Lys Ile Val Lys Asp Met
   210 215 220
   Ile Lys His Glu Ile Phe Asp Glu Asp Leu Thr Ile Lys Gly Val Tyr
   225 230 235 240
   His Phe Cys Phe Gly Asp
   245
<210> 13
   <211> 273
   <212> PRT
   <213> Treponema pallidum
<400> 13
   Met Leu Leu Ile Asp Val Gly Asn Ser His Val Val Phe Gly Ile Gln
   1               5                   10                  15
   Gly Glu Asn Gly Gly Arg Val Cys Val Arg Glu Leu Phe Arg Leu Ala
               20                  25                  30
   Pro Asp Ala Arg Lys Thr Gln Asp Glu Tyr Ser Leu Leu Ile His Ala
           35                  40                  45
   Leu Cys Glu Arg Ala Gly Val Gly Arg Ala Ser Leu Arg Asp Ala Phe
       50                  55                  60
   Ile Ser Ser Val Val Pro Val Leu Thr Lys Thr Ile Ala Asp Ala Val
   65                  70                  75                  80
   Ala Gln Ile Ser Gly Val Gln Pro Val Val Phe Gly Pro Trp Ala Tyr
                   85                  90                  95
   Glu His Leu Pro Val Arg Ile Pro Glu Pro Val Arg Ala Glu Ile Gly
               100                 105                 110
   Thr Asp Leu Val Ala Asn Ala Val Ala Ala Tyr Val His Phe Arg Ser
           115                 120                 125
   Ala Cys Val Val Val Asp Cys Gly Thr Ala Leu Thr Phe Thr Ala Val
       130                 135                 140
   Asp Gly Thr Gly Leu Ile Gln Gly Val Ala Ile Ala Pro Gly Leu Arg
   145                 150                 155                 160
   Thr Ala Val Gln Ser Leu His Thr Gly Thr Ala Gln Leu Pro Leu Val
                   165                 170                 175
   Pro Leu Ala Leu Pro Asp Ser Val Leu Gly Lys Asp Thr Thr His Ala
               180                 185                 190
   Val Gln Ala Gly Val Val Arg Gly Thr Leu Phe Val Ile Arg Ala Met
           195                 200                 205
   Ile Ala Gln Cys Gln Lys Glu Leu Gly Cys Arg Cys Ala Ala Val Ile
       210                 215                 220
   Thr Gly Gly Leu Ser Arg Leu Phe Ser Ser Glu Val Asp Phe Pro Pro
   225                 230                 235                 240
   Ile Asp Ala Gln Leu Thr Leu Ser Gly Leu Ala His Ile Ala Arg Leu
                   245                 250                 255
   Val Pro Thr Ser Leu Leu Pro Pro Ala Thr Val Ser Gly Ser Ser Gly
               260                 265                 270
   Asn
<210> 14
   <211> 262
   <212> PRT
   <213> Borrelia burgdorferi
<400> 14
   Met Asn Lys Pro Leu Leu Ser Glu Leu Ile Ile Asp Ile Gly Asn Thr
   1               5                   10                  15
   Ser Ile Ala Phe Ala Leu Phe Lys Asp Asn Gln Val Asn Leu Phe Ile
               20                  25                  30
   Lys Met Lys Thr Asn Leu Met Leu Arg Tyr Asp Glu Val Tyr Ser Phe
           35                  40                  45
   Phe Glu Glu Asn Phe Asp Phe Asn Val Asn Lys Val Phe Ile Ser Ser
       50                  55                  60
   Val Val Pro Ile Leu Asn Glu Thr Phe Lys Asn Val Ile Phe Ser Phe
   65                  70                  75                  80
   Phe Lys Ile Lys Pro Leu Phe Ile Gly Phe Asp Leu Asn Tyr Asp Leu
                   85                  90                  95
   Thr Phe Asn Pro Tyr Lys Ser Asp Lys Phe Leu Leu Gly Ser Asp Val
               100                 105                 110
   Phe Ala Asn Leu Val Ala Ala Ile Glu Asn Tyr Ser Phe Glu Asn Val
           115                 120                 125
   Leu Val Val Asp Leu Gly Thr Ala Cys Thr Ile Phe Ala Val Ser Arg
       130                 135                 140
   Gln Asp Gly Ile Leu Gly Gly Ile Ile Asn Ser Gly Pro Leu Ile Asn
   145                 150                 155                 160
   Phe Asn Ser Leu Leu Asp Asn Ala Tyr Leu Ile Lys Lys Phe Pro Ile
                   165                 170                 175
   Ser Thr Pro Asn Asn Leu Leu Glu Arg Thr Thr Ser Gly Ser Val Asn
               180                 185                 190
   Ser Gly Leu Phe Tyr Gln Tyr Lys Tyr Leu Ile Glu Gly Val Tyr Arg
           195                 200                 205
   Asp Ile Lys Gln Met Tyr Lys Lys Lys Phe Asn Leu Ile Ile Thr Gly
       210                 215                 220
   Gly Asn Ala Asp Leu Ile Leu Ser Leu Ile Glu Ile Glu Phe Ile Phe
   225                 230                 235                 240
   Asn Ile His Leu Thr Val Glu Gly Val Arg Ile Leu Gly Asn Ser Ile
                   245                 250                 255
   Asp Phe Lys Phe Val Asn
               260
<210> 15
   <211> 229
   <212> PRT
   <213> Aquifex aeolicus
<400> 15
   Met Arg Phe Leu Thr Val Asp Val Gly Asn Ser Ser Val Asp Ile Ala
   1               5                   10                  15
   Leu Trp Glu Gly Lys Lys Val Lys Asp Phe Leu Lys Leu Ser His Glu
               20                  25                  30
   Glu Phe Leu Lys Glu Glu Phe Pro Lys Leu Lys Ala Leu Gly Ile Ser
           35                  40                  45
   Val Lys Gln Ser Phe Ser Glu Lys Val Arg Gly Lys Ile Pro Lys Ile
       50                  55                  60
   Lys Phe Leu Lys Lys Glu Asn Phe Pro Ile Gln Val Asp Tyr Lys Thr
   65                  70                  75                  80
   Pro Glu Thr Leu Gly Thr Asp Arg Val Ala Leu Ala Tyr Ser Ala Lys
                   85                  90                  95
   Lys Phe Tyr Gly Lys Asn Val Val Val Ile Ser Ala Gly Thr Ala Leu
               100                 105                 110
   Val Ile Asp Leu Val Leu Glu Gly Lys Phe Lys Gly Gly Phe Ile Thr
           115                 120                 125
   Leu Gly Leu Gly Lys Lys Leu Lys Ile Leu Ser Asp Leu Ala Glu Gly
       130                 135                 140
   Ile Pro Glu Phe Phe Pro Glu Glu Val Glu Ile Phe Leu Gly Arg Ser
   145                 150                 155                 160
   Thr Arg Glu Cys Val Leu Gly Gly Ala Tyr Arg Glu Ser Thr Glu Phe
                   165                 170                 175
   Ile Lys Ser Thr Leu Lys Leu Trp Arg Lys Val Phe Lys Arg Lys Phe
               180                 185                 190
   Lys Val Val Ile Thr Gly Gly Glu Gly Lys Tyr Phe Ser Lys Phe Gly
           195                 200                 205
   Ile Tyr Asp Pro Leu Leu Val His Arg Gly Met Arg Asn Leu Leu Tyr
       210                 215                 220
   Leu Tyr His Arg Ile
   225
<210> 16
   <211> 257
   <212> PRT
   <213> Synechocystis sp.
<400> 16
   Met Glu Thr Ser Lys Pro Gly Cys Gly Leu Ala Leu Asp Asn Asp Lys
   1               5                   10                  15
   Gln Lys Pro Trp Leu Gly Leu Met Ile Gly Asn Ser Arg Leu His Trp
               20                  25                  30
   Ala Tyr Cys Ser Gly Asn Ala Pro Leu Gln Thr Trp Val Thr Asp Tyr
           35                  40                  45
   Asn Pro Lys Ser Ala Gln Leu Pro Val Leu Leu Gly Lys Val Pro Leu
       50                  55                  60
   Met Leu Ala Ser Val Val Pro Glu Gln Thr Glu Val Trp Arg Val Tyr
   65                  70                  75                  80
   Gln Pro Lys Ile Leu Thr Leu Lys Asn Leu Pro Leu Val Asn Leu Tyr
                   85                  90                  95
   Pro Ser Phe Gly Ile Asp Arg Ala Leu Ala Gly Leu Gly Thr Gly Leu
               100                 105                 110
   Thr Tyr Gly Phe Pro Cys Leu Val Val Asp Gly Gly Thr Ala Leu Thr
           115                 120                 125
   Ile Thr Gly Phe Asp Gln Asp Lys Lys Leu Val Gly Gly Ala Ile Leu
       130                 135                 140
   Pro Gly Leu Gly Leu Gln Leu Ala Thr Leu Gly Asp Arg Leu Ala Ala
   145                 150                 155                 160
   Leu Pro Lys Leu Glu Met Asp Gln Leu Thr Glu Leu Pro Asp Arg Trp
                   165                 170                 175
   Ala Leu Asp Thr Pro Ser Ala Ile Phe Ser Gly Val Val Tyr Gly Val
               180                 185                 190
   Leu Gly Ala Leu Gln Ser Tyr Leu Gln Asp Trp Gln Lys Leu Phe Pro
           195                 200                 205
   Gly Ala Ala Met Val Ile Thr Gly Gly Asp Gly Lys Ile Leu His Gly
       210                 215                 220
   Phe Leu Lys Glu His Ser Pro Asn Leu Ser Val Ala Trp Asp Asp Asn
   225                 230                 235                 240
   Leu Ile Phe Leu Gly Met Ala Ala Ile His His Gly Asp Arg Pro Ile
                   245                 250                 255
   Cys
<210> 17
   <211> 223
   <212> PRT
   <213> Helicobacter pylori
<400> 17
   Met Pro Ala Arg Gln Ser Phe Thr Asp Leu Lys Asn Leu Val Leu Cys
   1 5 10 15
   Asp Ile Gly Asn Thr Arg Ile His Phe Ala Gln Asn Tyr Gln Leu Phe
   20 25 30
   Ser Ser Ala Lys Glu Asp Leu Lys Arg Leu Gly Ile Gln Lys Glu Ile
   35 40 45
   Phe Tyr Ile Ser Val Asn Glu Glu Asn Glu Lys Ala Leu Leu Asn Cys
   50 55 60
   Tyr Pro Asn Ala Lys Asn Ile Ala Gly Phe Phe His Leu Glu Thr Asp
   65 70 75 80
   Tyr Val Gly Leu Gly Ile Asp Arg Gln Met Ala Cys Leu Ala Val Asn
   85 90 95
   Asn Gly Val Val Val Asp Ala Gly Ser Ala Ile Thr Ile Asp Leu Ile
   100 105 110
   Lys Glu Gly Lys His Leu Gly Gly Cys Ile Leu Pro Gly Leu Ala Gln
   115 120 125
   Tyr Ile His Ala Tyr Lys Lys Ser Ala Lys Ile Leu Glu Gln Pro Phe
   130 135 140
   Lys Ala Leu Asp Ser Leu Glu Val Leu Pro Lys Ser Thr Arg Asp Ala
   145 150 155 160
   Val Asn Tyr Gly Met Val Leu Ser Val Ile Ala Cys Ile Gln His Leu
   165 170 175
   Ala Lys Asn Gln Lys Ile Tyr Leu Cys Gly Gly Asp Ala Lys Tyr Leu
   180 185 190
   Ser Ala Phe Leu Pro His Ser Val Cys Lys Glu Arg Leu Val Phe Asp
   195 200 205
   Gly Met Glu Ile Ala Leu Lys Lys Ala Gly Ile Leu Glu Cys Lys
   210 215 220
<210> 18
   <211> 267
   <212> PRT
   <213> Bordetella pertussis
<400> 18
   Met Ile Ile Leu Ile Asp Ser Gly Asn Ser Arg Leu Lys Val Gly Trp
   1               5                   10                  15
   Phe Asp Pro Asp Ala Pro Gln Ala Ala Arg Glu Pro Ala Pro Val Ala
               20                  25                  30
   Phe Asp Asn Leu Asp Leu Asp Ala Leu Gly Arg Trp Leu Ala Thr Leu
           35                  40                  45
   Pro Arg Arg Pro Gln Arg Ala Leu Gly Val Asn Val Ala Gly Leu Ala
       50                  55                  60
   Arg Gly Glu Ala Ile Ala Ala Thr Leu Arg Ala Gly Gly Cys Asp Ile
   65                  70                  75                  80
   Arg Trp Leu Arg Ala Gln Pro Leu Ala Met Gly Leu Arg Asn Gly Tyr
                   85                  90                  95
   Arg Asn Pro Asp Gln Leu Gly Ala Asp Arg Trp Ala Cys Met Val Gly
               100                 105                 110
   Val Leu Ala Arg Gln Pro Ser Val His Pro Pro Leu Leu Val Ala Ser
           115                 120                 125
   Phe Gly Thr Ala Thr Thr Leu Asp Thr Ile Gly Pro Asp Asn Val Phe
       130                 135                 140
   Pro Gly Gly Leu Ile Leu Pro Gly Pro Ala Met Met Arg Gly Ala Leu
   145                 150                 155                 160
   Ala Tyr Gly Thr Ala His Leu Pro Leu Ala Asp Gly Leu Val Ala Asp
                   165                 170                 175
   Tyr Pro Ile Asp Thr His Gln Ala Ile Ala Ser Gly Ile Ala Ala Ala
               180                 185                 190
   Gln Ala Gly Ala Ile Val Arg Gln Trp Leu Ala Gly Arg Gln Arg Tyr
           195                 200                 205
   Gly Gln Ala Pro Glu Ile Tyr Val Ala Gly Gly Gly Trp Pro Glu Val
       210                 215                 220
   Arg Gln Glu Ala Glu Arg Leu Leu Ala Val Thr Gly Ala Ala Phe Gly
   225                 230                 235                 240
   Ala Thr Pro Gln Pro Thr Tyr Leu Asp Ser Pro Val Leu Asp Gly Leu
                   245                 250                 255
   Ala Ala Leu Ala Ala Gln Gly Ala Pro Thr Ala
               260                 265
<210> 19
   <211> 777
   <212> DNA
   <213> Bacillus subtilis
<220>
   <221> CDS
   <222> (1)..(774)
<400> 19
   ttg tta ctg gtt atc gat gtg ggg aac acc aat act gta ctt ggt gta      48
   Leu Leu Leu Val Ile Asp Val Gly Asn Thr Asn Thr Val Leu Gly Val
   1               5                   10                  15
   tat cat gat gga aaa tta gaa tat cac tgg cgt ata gaa aca agc agg      96
   Tyr His Asp Gly Lys Leu Glu Tyr His Trp Arg Ile Glu Thr Ser Arg
               20                  25                  30
   cat aaa aca gaa gat gag ttt ggg atg att ttg cgc tcc tta ttt gat      144
   His Lys Thr Glu Asp Glu Phe Gly Met Ile Leu Arg Ser Leu Phe Asp
           35                  40                  45
   cac tcc ggg ctt atg ttt gaa cag ata gat ggc att att att tcg tca      192
   His Ser Gly Leu Met Phe Glu Gln Ile Asp Gly Ile Ile Ile Ser Ser
       50                  55                  60
   gta gtg ccg cca atc atg ttt gcg tta gaa aga atg tgc aca aaa tac      240
   Val Val Pro Pro Ile Met Phe Ala Leu Glu Arg Met Cys Thr Lys Tyr
   65                  70                  75                  80
   ttt cat atc gag cct caa att gtt ggt cca ggt atg aaa acc ggt tta      288
   Phe His Ile Glu Pro Gln Ile Val Gly Pro Gly Met Lys Thr Gly Leu
                   85                  90                  95
   aat ata aaa tat gac aat ccg aaa gaa gta ggg gca gac aga atc gta      336
   Asn Ile Lys Tyr Asp Asn Pro Lys Glu Val Gly Ala Asp Arg Ile Val
               100                 105                 110
   aat gct gtc gct gcg ata cac ttg tac ggc aat cca tta att gtt gtc      384
   Asn Ala Val Ala Ala Ile His Leu Tyr Gly Asn Pro Leu Ile Val Val
           115                 120                 125
   gat ttc gga acc gcc aca acg tac tgc tat att gat gaa aac aaa caa      432
   Asp Phe Gly Thr Ala Thr Thr Tyr Cys Tyr Ile Asp Glu Asn Lys Gln
       130                 135                 140
   tac atg ggc ggg gcg att gcc cct ggg att aca att tcg aca gag gcg      480
   Tyr Met Gly Gly Ala Ile Ala Pro Gly Ile Thr Ile Ser Thr Glu Ala
   145                 150                 155                 160
   ctt tac tcg cgt gca gca aag ctt cct cgt atc gaa atc acc cgg ccc      528
   Leu Tyr Ser Arg Ala Ala Lys Leu Pro Arg Ile Glu Ile Thr Arg Pro
                   165                 170                 175
   gac aat att atc gga aaa aac act gtt agc gcg atg caa tct gga att      576
   Asp Asn Ile Ile Gly Lys Asn Thr Val Ser Ala Met Gln Ser Gly Ile
               180                 185                 190
   tta ttt ggc tat gtc ggc caa gtg gaa gga atc gtt aag cga atg aaa      624
   Leu Phe Gly Tyr Val Gly Gln Val Glu Gly Ile Val Lys Arg Met Lys
           195                 200                 205
   tgg cag gca aaa cag gac ctc aag gtc att gcg aca gga ggc ctg gcg      672
   Trp Gln Ala Lys Gln Asp Leu Lys Val Ile Ala Thr Gly Gly Leu Ala
       210                 215                 220
   ccg ctc att gcg aac gaa tca gat tgt ata gac atc gtt gat cca ttc      720
   Pro Leu Ile Ala Asn Glu Ser Asp Cys Ile Asp Ile Val Asp Pro Phe
   225                 230                 235                 240
   tta acc cta aaa ggg ctg gaa ttg att tat gaa aga aac cgc gta gga      768
   Leu Thr Leu Lys Gly Leu Glu Leu Ile Tyr Glu Arg Asn Arg Val Gly
                   245                 250                 255
   agt gta tag                                                          777
   Ser Val
<210> 20
   <211> 960
   <212> DNA
   <213> Bacillus subtilis
<220>
   <221> CDS
   <222> (1)..(957)
<400> 20
   gtg aaa aat aaa gaa ctt aac cta cat act tta tat aca cag cac aat      48
   Met Lys Asn Lys Glu Leu Asn Leu His Thr Leu Tyr Thr Gln His Asn
   1               5                   10                  15
   cgg gag tct tgg tct ggt ttt ggg ggg cat ttg tcg att gct gta tct      96
   Arg Glu Ser Trp Ser Gly Phe Gly Gly His Leu Ser Ile Ala Val Ser
               20                  25                  30
   gaa gaa gag gca aaa gct gtg gaa gga ttg aat gat tat cta tct gtt      144
   Glu Glu Glu Ala Lys Ala Val Glu Gly Leu Asn Asp Tyr Leu Ser Val
           35                  40                  45
   gaa gaa gtg gag acg atc tat att ccg ctt gtt cgc ttg ctt cat tta      192
   Glu Glu Val Glu Thr Ile Tyr Ile Pro Leu Val Arg Leu Leu His Leu
       50                  55                  60
   cat gtc aag tct gcg gct gaa cgc aat aag cat gtc aat gtt ttt ttg      240
   His Val Lys Ser Ala Ala Glu Arg Asn Lys His Val Asn Val Phe Leu
   65                  70                  75                  80
   aag cac cca cat tca gcc aaa att ccg ttt att atc ggc att gcc ggc      288
   Lys His Pro His Ser Ala Lys Ile Pro Phe Ile Ile Gly Ile Ala Gly
                   85                  90                  95
   agt gtc gca gtc gga aaa agc acg acg gcg cgg atc ttg cag aag ctg      336
   Ser Val Ala Val Gly Lys Ser Thr Thr Ala Arg Ile Leu Gln Lys Leu
               100                 105                 110
   ctt tcg cgt ttg cct gac cgt cca aaa gtg agc ctt atc acg aca gat      384
   Leu Ser Arg Leu Pro Asp Arg Pro Lys Val Ser Leu Ile Thr Thr Asp
           115                 120                 125
   ggt ttt tta ttt cct act gcc gag ctg aaa aag aaa aat atg atg tca      432
   Gly Phe Leu Phe Pro Thr Ala Glu Leu Lys Lys Lys Asn Met Met Ser
       130                 135                 140
   aga aaa gga ttt cct gaa agc tat gat gta aag gcg ctg ctc gaa ttt      480
   Arg Lys Gly Phe Pro Glu Ser Tyr Asp Val Lys Ala Leu Leu Glu Phe
   145                 150                 155                 160
   ttg aat gac tta aaa tca gga aag gac agc gta aag gcc ccg gtg tat      528
   Leu Asn Asp Leu Lys Ser Gly Lys Asp Ser Val Lys Ala Pro Val Tyr
                   165                 170                 175
   tcc cat cta acc tat gac cgc gag gaa ggt gtg ttc gag gtt gta gaa      576
   Ser His Leu Thr Tyr Asp Arg Glu Glu Gly Val Phe Glu Val Val Glu
               180                 185                 190
   cag gcg gat att gtg att att gaa ggc att aat gtt ctt cag tcg ccc      624
   Gln Ala Asp Ile Val Ile Ile Glu Gly Ile Asn Val Leu Gln Ser Pro
           195                 200                 205
   acc ttg gag gat gac cgg gaa aac ccg cgt att ttt gtt tcc gat ttc      672
   Thr Leu Glu Asp Asp Arg Glu Asn Pro Arg Ile Phe Val Ser Asp Phe
       210                 215                 220
   ttt gat ttt tcg att tat gtg gat gcg gag gaa agc cgg att ttc act      720
   Phe Asp Phe Ser Ile Tyr Val Asp Ala Glu Glu Ser Arg Ile Phe Thr
   225                 230                 235                 240
   tgg tat tta gag cgt ttt cgc ctg ctt cgg gaa aca gct ttt caa aat      768
   Trp Tyr Leu Glu Arg Phe Arg Leu Leu Arg Glu Thr Ala Phe Gln Asn
                   245                 250                 255
   cct gat tca tat ttt cat aaa ttt aaa gac ttg tcc gat cag gag gct      816
   Pro Asp Ser Tyr Phe His Lys Phe Lys Asp Leu Ser Asp Gln Glu Ala
               260                 265                 270
   gac gag atg gca gcc tcg att tgg gag agt gtc aac cgg ccg aat tta      864
   Asp Glu Met Ala Ala Ser Ile Trp Glu Ser Val Asn Arg Pro Asn Leu
           275                 280                 285
   tat gaa aat att ttg cca act aaa ttc agg tca gat ctc att ttg cgt      912
   Tyr Glu Asn Ile Leu Pro Thr Lys Phe Arg Ser Asp Leu Ile Leu Arg
       290                 295                 300
   aag gga gac ggg cat aag gtc gag gaa gtg ttg gta agg agg gta tga      960
   Lys Gly Asp Gly His Lys Val Glu Glu Val Leu Val Arg Arg Val
   305                 310                 315
<210> 21
   <211> 882
   <212> DNA
   <213> Bacillus subtilis
<220>
   <221> CDS
   <222> (1)..(879)
<400> 21
   ttg tcg att gct gta tct gaa gaa gag gca aaa gct gtg gaa gga ttg      48
   Met Ser Ile Ala Val Ser Glu Glu Glu Ala Lys Ala Val Glu Gly Leu
     1               5                  10                  15
   aat gat tat cta tct gtt gaa gaa gtg gag acg atc tat att ccg ctt      96
   Asn Asp Tyr Leu Ser Val Glu Glu Val Glu Thr Ile Tyr Ile Pro Leu
                20                  25                  30
   gtt cgc ttg ctt cat tta cat gtc aag tct gcg gct gaa cgc aat aag     144
   Val Arg Leu Leu His Leu His Val Lys Ser Ala Ala Glu Arg Asn Lys
            35                  40                  45
   cat gtc aat gtt ttt ttg aag cac cca cat tca gcc aaa att ccg ttt     192
   His Val Asn Val Phe Leu Lys His Pro His Ser Ala Lys Ile Pro Phe
        50                  55                  60
   att atc ggc att gcc ggc agt gtc gca gtc gga aaa agc acg acg gcg     240
   Ile Ile Gly Ile Ala Gly Ser Val Ala Val Gly Lys Ser Thr Thr Ala
    65                  70                  75                  80
   cgg atc ttg cag aag ctg ctt tcg cgt ttg cct gac cgt cca aaa gtg     288
   Arg Ile Leu Gln Lys Leu Leu Ser Arg Leu Pro Asp Arg Pro Lys Val
                   85                  90                  95
   agc ctt atc acg aca gat ggt ttt tta ttt cct act gcc gag ctg aaa      336
   Ser Leu Ile Thr Thr Asp Gly Phe Leu Phe Pro Thr Ala Glu Leu Lys
               100                 105                 110
   aag aaa aat atg atg tca aga aaa gga ttt cct gaa agc tat gat gta      384
   Lys Lys Asn Met Met Ser Arg Lys Gly Phe Pro Glu Ser Tyr Asp Val
           115                 120                 125
   aag gcg ctg ctc gaa ttt ttg aat gac tta aaa tca gga aag gac agc      432
   Lys Ala Leu Leu Glu Phe Leu Asn Asp Leu Lys Ser Gly Lys Asp Ser
       130                 135                 140
   gta aag gcc ccg gtg tat tcc cat cta acc tat gac cgc gag gaa ggt      480
   Val Lys Ala Pro Val Tyr Ser His Leu Thr Tyr Asp Arg Glu Glu Gly
   145                 150                 155                 160
   gtg ttc gag gtt gta gaa cag gcg gat att gtg att att gaa ggc att      528
   Val Phe Glu Val Val Glu Gln Ala Asp Ile Val Ile Ile Glu Gly Ile
                   165                 170                 175
   aat gtt ctt cag tcg ccc acc ttg gag gat gac cgg gaa aac ccg cgt      576
   Asn Val Leu Gln Ser Pro Thr Leu Glu Asp Asp Arg Glu Asn Pro Arg
               180                 185                 190
   att ttt gtt tcc gat ttc ttt gat ttt tcg att tat gtg gat gcg gag      624
   Ile Phe Val Ser Asp Phe Phe Asp Phe Ser Ile Tyr Val Asp Ala Glu
           195                 200                 205
   gaa agc cgg att ttc act tgg tat tta gag cgt ttt cgc ctg ctt cgg      672
   Glu Ser Arg Ile Phe Thr Trp Tyr Leu Glu Arg Phe Arg Leu Leu Arg
       210                 215                 220
   gaa aca gct ttt caa aat cct gat tca tat ttt cat aaa ttt aaa gac      720
   Glu Thr Ala Phe Gln Asn Pro Asp Ser Tyr Phe His Lys Phe Lys Asp
   225                 230                 235                 240
   ttg tcc gat cag gag gct gac gag atg gca gcc tcg att tgg gag agt      768
   Leu Ser Asp Gln Glu Ala Asp Glu Met Ala Ala Ser Ile Trp Glu Ser
                   245                 250                 255
   gtc aac cgg ccg aat tta tat gaa aat att ttg cca act aaa ttc agg      816
   Val Asn Arg Pro Asn Leu Tyr Glu Asn Ile Leu Pro Thr Lys Phe Arg
               260                 265                 270
   tca gat ctc att ttg cgt aag gga gac ggg cat aag gtc gag gaa gtg      864
   Ser Asp Leu Ile Leu Arg Lys Gly Asp Gly His Lys Val Glu Glu Val
           275                 280                 285
   ttg gta agg agg gta tga                                              882
   Leu Val Arg Arg Val
       290
<210> 22
   <211> 846
   <212> DNA
   <213> Bacillus subtilis
<220>
   <221> CDS
   <222> (1)..(843)
<400> 22
   gtg gaa gga ttg aat gat tat cta tct gtt gaa gaa gtg gag acg atc 48
   Met Glu Gly Leu Asn Asp Tyr Leu Ser Val Glu Glu Val Glu Thr Ile
   1               5                    10                   15
   tat att ccg ctt gtt cgc ttg ctt cat tta cat gtc aag tct gcg gct     96
   Tyr Ile Pro Leu Val Arg Leu Leu His Leu His Val Lys Ser Ala Ala
               20                  25                  30
   gaa cgc aat aag cat gtc aat gtt ttt ttg aag cac cca cat tca gcc    144
   Glu Arg Asn Lys His Val Asn Val Phe Leu Lys His Pro His Ser Ala
           35                  40                  45
   aaa att ccg ttt att atc ggc att gcc ggc agt gtc gca gtc gga aaa    192
   Lys Ile Pro Phe Ile Ile Gly Ile Ala Gly Ser Val Ala Val Gly Lys
       50                  55                  60
   agc acg acg gcg cgg atc ttg cag aag ctg ctt tcg cgt ttg cct gac    240
   Ser Thr Thr Ala Arg Ile Leu Gln Lys Leu Leu Ser Arg Leu Pro Asp
   65                  70                  75                  80
   cgt cca aaa gtg agc ctt atc acg aca gat ggt ttt tta ttt cct act    288
   Arg Pro Lys Val Ser Leu Ile Thr Thr Asp Gly Phe Leu Phe Pro Thr
                   85                  90                  95
   gcc gag ctg aaa aag aaa aat atg atg tca aga aaa gga ttt cct gaa    336
   Ala Glu Leu Lys Lys Lys Asn Met Met Ser Arg Lys Gly Phe Pro Glu
               100                 105                 110
   agc tat gat gta aag gcg ctg ctc gaa ttt ttg aat gac tta aaa tca    384
   Ser Tyr Asp Val Lys Ala Leu Leu Glu Phe Leu Asn Asp Leu Lys Ser
           115                 120                 125
   gga aag gac agc gta aag gcc ccg gtg tat tcc cat cta acc tat gac    432
   Gly Lys Asp Ser Val Lys Ala Pro Val Tyr Ser His Leu Thr Tyr Asp
       130                 135                 140
   cgc gag gaa ggt gtg ttc gag gtt gta gaa cag gcg gat att gtg att    480
   Arg Glu Glu Gly Val Phe Glu Val Val Glu Gln Ala Asp Ile Val Ile
   145                 150                 155                 160
   att gaa ggc att aat gtt ctt cag tcg ccc acc ttg gag gat gac cgg    528
   Ile Glu Gly Ile Asn Val Leu Gln Ser Pro Thr Leu Glu Asp Asp Arg
                   165                 170                 175
   gaa aac ccg cgt att ttt gtt tcc gat ttc ttt gat ttt tcg att tat    576
   Glu Asn Pro Arg Ile Phe Val Ser Asp Phe Phe Asp Phe Ser Ile Tyr
               180                 185                 190
   gtg gat gcg gag gaa agc cgg att ttc act tgg tat tta gag cgt ttt    624
   Val Asp Ala Glu Glu Ser Arg Ile Phe Thr Trp Tyr Leu Glu Arg Phe
           195                 200                 205
   cgc ctg ctt cgg gaa aca gct ttt caa aat cct gat tca tat ttt cat    672
   Arg Leu Leu Arg Glu Thr Ala Phe Gln Asn Pro Asp Ser Tyr Phe His
       210                 215                 220
   aaa ttt aaa gac ttg tcc gat cag gag gct gac gag atg gca gcc tcg    720
   Lys Phe Lys Asp Leu Ser Asp Gln Glu Ala Asp Glu Met Ala Ala Ser
   225                 230                 235                 240
   att tgg gag agt gtc aac cgg ccg aat tta tat gaa aat att ttg cca    768
   Ile Trp Glu Ser Val Asn Arg Pro Asn Leu Tyr Glu Asn Ile Leu Pro
                   245                 250                 255
   act aaa ttc agg tca gat ctc att ttg cgt aag gga gac ggg cat aag    816
   Thr Lys Phe Arg Ser Asp Leu Ile Leu Arg Lys Gly Asp Gly His Lys
               260                 265                 270
   gtc gag gaa gtg ttg gta agg agg gta tga                            846
   Val Glu Glu Val Leu Val Arg Arg Val
           275                 280
<210> 23
   <211> 831
   <212> DNA
   <213> Bacillus subtilis
<220>
   <221> CDS
   <222> (1)..(831)
<400> 23
   atg aaa aca aaa ctg gat ttt cta aaa atg aag gag tct gaa gaa ccg 48
   Met Lys Thr Lys Leu Asp Phe Leu Lys Met Lys Glu Ser Glu Glu Pro
   1 5 10 15
   att gtc atg ctg acc gct tat gat tat ccg gca gct aaa ctt gct gaa 96
   Ile Val Met Leu Thr Ala Tyr Asp Tyr Pro Ala Ala Lys Leu Ala Glu
   20 25 30
   caa gcg gga gtt gac atg att tta gtc ggt gat tca ctt gga atg gtc 144
   Gln Ala Gly Val Asp Met Ile Leu Val Gly Asp Ser Leu Gly Met Val
   35 40 45
   gtc ctc ggc ctt gat tca act gtc ggt gtg aca gtt gcg gac atg atc 192
   Val Leu Gly Leu Asp Ser Thr Val Gly Val Thr Val Ala Asp Met Ile
   50 55 60
   cat cat aca aaa gcc gtt aaa agg ggt gcg ccg aat acc ttt att gtg 240
   His His Thr Lys Ala Val Lys Arg Gly Ala Pro Asn Thr Phe Ile Val
   65 70 75 80
   aca gat atg ccg ttt atg tct tat cac ctg tct aag gaa gat acg ctg 288
   Thr Asp Met Pro Phe Met Ser Tyr His Leu Ser Lys Glu Asp Thr Leu
   85 90 95
   aaa aat gca gcg gct atc gtt cag gaa agc gga gct gac gca ctg aag 336
   Lys Asn Ala Ala Ala Ile Val Gln Glu Ser Gly Ala Asp Ala Leu Lys
   100 105 110
   ctt gag ggc gga gaa ggc gtg ttt gaa tcc att cgc gca ttg acg ctt 384
   Leu Glu Gly Gly Glu Gly Val Phe Glu Ser Ile Arg Ala Leu Thr Leu
   115 120 125
   gga ggc att cca gta gtc agt cac tta ggt ttg aca ccg cag tca gtc 432
   Gly Gly Ile Pro Val Val Ser His Leu Gly Leu Thr Pro Gln Ser Val
   130 135 140
   ggc gta ctg ggc ggc tat aaa gta cag ggc aaa gac gaa caa agc gcc 480
   Gly Val Leu Gly Gly Tyr Lys Val Gln Gly Lys Asp Glu Gln Ser Ala
   145 150 155 160
   aaa aaa tta ata gaa gac agt ata aaa tgc gaa gaa gca gga gct atg 528
   Lys Lys Leu Ile Glu Asp Ser Ile Lys Cys Glu Glu Ala Gly Ala Met
   165 170 175
   atg ctt gtg ctg gaa tgt gtg ccg gca gaa ctc aca gcc aaa att gcc 576
   Met Leu Val Leu Glu Cys Val Pro Ala Glu Leu Thr Ala Lys Ile Ala
   180 185 190
   gag acg cta agc ata ccg gtc att gga atc ggg gct ggt gtg aaa gcg 624
   Glu Thr Leu Ser Ile Pro Val Ile Gly Ile Gly Ala Gly Val Lys Ala
   195 200 205
   gac gga caa gtt ctc gtt tat cat gat att atc ggc cac ggt gtt gag 672
   Asp Gly Gln Val Leu Val Tyr His Asp Ile Ile Gly His Gly Val Glu
   210 215 220
   aga aca cct aaa ttt gta aag caa tat acg cgc att gat gaa acc atc 720
   Arg Thr Pro Lys Phe Val Lys Gln Tyr Thr Arg Ile Asp Glu Thr Ile
   225                 230                 235                 240
   gaa aca gca atc agc gga tat gtt cag gat gta aga cat cgt gct ttc      768
   Glu Thr Ala Ile Ser Gly Tyr Val Gln Asp Val Arg His Arg Ala Phe
                   245                 250                 255
   cct gaa caa aag cat tcc ttt caa atg aac cag aca gtg ctt gac ggc      816
   Pro Glu Gln Lys His Ser Phe Gln Met Asn Gln Thr Val Leu Asp Gly
               260                 265                 270
   ttg tac ggg gga aaa                                                  831
   Leu Tyr Gly Gly Lys
           275
<210> 24
   <211> 277
   <212> PRT
   <213> Bacillus subtilis
<400> 24
   Met Lys Thr Lys Leu Asp Phe Leu Lys Met Lys Glu Ser Glu Glu Pro
   1 5 10 15
   Ile Val Met Leu Thr Ala Tyr Asp Tyr Pro Ala Ala Lys Leu Ala Glu
   20 25 30
   Gln Ala Gly Val Asp Met Ile Leu Val Gly Asp Ser Leu Gly Met Val
   35 40 45
   Val Leu Gly Leu Asp Ser Thr Val Gly Val Thr Val Ala Asp Met Ile
   50 55 60
   His His Thr Lys Ala Val Lys Arg Gly Ala Pro Asn Thr Phe Ile Val
   65 70 75 80
   Thr Asp Met Pro Phe Met Ser Tyr His Leu Ser Lys Glu Asp Thr Leu
   85 90 95
   Lys Asn Ala Ala Ala Ile Val Gln Glu Ser Gly Ala Asp Ala Leu Lys
   100 105 110
   Leu Glu Gly Gly Glu Gly Val Phe Glu Ser Ile Arg Ala Leu Thr Leu
   115 120 125
   Gly Gly Ile Pro Val Val Ser His Leu Gly Leu Thr Pro Gln Ser Val
   130 135 140
   Gly Val Leu Gly Gly Tyr Lys Val Gln Gly Lys Asp Glu Gln Ser Ala
   145 150 155 160
   Lys Lys Leu Ile Glu Asp Ser Ile Lys Cys Glu Glu Ala Gly Ala Met
   165 170 175
   Met Leu Val Leu Glu Cys Val Pro Ala Glu Leu Thr Ala Lys Ile Ala
   180 185 190
   Glu Thr Leu Ser Ile Pro Val Ile Gly Ile Gly Ala Gly Val Lys Ala
   195 200 205
   Asp Gly Gln Val Leu Val Tyr His Asp Ile Ile Gly His Gly Val Glu
   210 215 220
   Arg Thr Pro Lys Phe Val Lys Gln Tyr Thr Arg Ile Asp Glu Thr Ile
   225 230 235 240
   Glu Thr Ala Ile Ser Gly Tyr Val Gln Asp Val Arg His Arg Ala Phe
   245 250 255
   Pro Glu Gln Lys His Ser Phe Gln Met Asn Gln Thr Val Leu Asp Gly
   260 265 270
   Leu Tyr Gly Gly Lys
   275
<210> 25
   <211> 858
   <212> DNA
   <213> Bacillus subtilis
<220>
   <221> CDS
   <222> (1)..(858)
<400> 25
   atg aga cag att act gat att tca cag ctg aaa gaa gcc ata aaa caa 48
   Met Arg Gln Ile Thr Asp Ile Ser Gln Leu Lys Glu Ala Ile Lys Gln
   1 5 10 15
   tac cat tca gag ggc aag tca atc gga ttt gtt ccg acg atg ggg ttt 96
   Tyr His Ser Glu Gly Lys Ser Ile Gly Phe Val Pro Thr Met Gly Phe
   20 25 30
   ctg cat gag ggg cat tta acc tta gca gac aaa gca aga caa gaa aac 144
   Leu His Glu Gly His Leu Thr Leu Ala Asp Lys Ala Arg Gln Glu Asn
   35 40 45
   gac gcc gtt att atg agt att ttt gtg aat cct gca caa ttc ggc cct 192
   Asp Ala Val Ile Met Ser Ile Phe Val Asn Pro Ala Gln Phe Gly Pro
   50 55 60
   aat gaa gat ttt gaa gca tat ccg cgc gat att gag cgg gat gca gct 240
   Asn Glu Asp Phe Glu Ala Tyr Pro Arg Asp Ile Glu Arg Asp Ala Ala
   65 70 75 80
   ctt gca gaa aac gcc gga gtc gat att ctt ttt acg cca gat gct cat 288
   Leu Ala Glu Asn Ala Gly Val Asp Ile Leu Phe Thr Pro Asp Ala His
   85 90 95
   gat atg tat ccc ggt gaa aag aat gtc acg att cat gta gaa aga cgc 336
   Asp Met Tyr Pro Gly Glu Lys Asn Val Thr Ile His Val Glu Arg Arg
   100 105 110
   aca gac gtg tta tgc ggg cgc tca aga gaa gga cat ttt gac ggg gtc 384
   Thr Asp Val Leu Cys Gly Arg Ser Arg Glu Gly His Phe Asp Gly Val
   115 120 125
   gcg atc gta ctg acg aag ctt ttc aat cta gtc aag ccg act cgt gcc 432
   Ala Ile Val Leu Thr Lys Leu Phe Asn Leu Val Lys Pro Thr Arg Ala
   130 135 140
   tat ttc ggt tta aaa gat gcg cag cag gta gct gtt gtt gat ggg tta 480
   Tyr Phe Gly Leu Lys Asp Ala Gln Gln Val Ala Val Val Asp Gly Leu
   145 150 155 160
   atc agc gac ttc ttc atg gat att gaa ttg gtt cct gtc gat acg gtc 528
   Ile Ser Asp Phe Phe Met Asp Ile Glu Leu Val Pro Val Asp Thr Val
   165 170 175
   aga gag gaa gac ggc tta gcc aaa agc tct cgc aat gta tac tta aca 576
   Arg Glu Glu Asp Gly Leu Ala Lys Ser Ser Arg Asn Val Tyr Leu Thr
   180 185 190
   gct gag gaa aga aaa gaa gcg cct aag ctg tat cgg gcc ctt caa aca 624
   Ala Glu Glu Arg Lys Glu Ala Pro Lys Leu Tyr Arg Ala Leu Gln Thr
           195                 200                 205
   agt gcg gaa ctt gtc caa gcc ggt gaa aga gat cct gaa gcg gtg ata      672
   Ser Ala Glu Leu Val Gln Ala Gly Glu Arg Asp Pro Glu Ala Val Ile
       210                 215                 220
   aaa gct gca aaa gat atc att gaa acg act agc gga acc ata gac tat      720
   Lys Ala Ala Lys Asp Ile Ile Glu Thr Thr Ser Gly Thr Ile Asp Tyr
   225                 230                 235                 240
   gta gag ctt tat tcc tat ccg gaa ctc gag cct gtg aat gaa att gct      768
   Val Glu Leu Tyr Ser Tyr Pro Glu Leu Glu Pro Val Asn Glu Ile Ala
                   245                 250                 255
   gga aag atg att ctc gct gtt gca gtt gct ttt tca aaa gcg cgt tta      816
   Gly Lys Met Ile Leu Ala Val Ala Val Ala Phe Ser Lys Ala Arg Leu
               260                 265                 270
   ata gat aat atc att att gat att cga gaa atg gag aga ata              858
   Ile Asp Asn Ile Ile Ile Asp Ile Arg Glu Met Glu Arg Ile
           275                 280                 285
<210> 26
   <211> 286
   <212> PRT
   <213> Bacillus subtilis
<400> 26
   Met Arg Gln Ile Thr Asp Ile Ser Gln Leu Lys Glu Ala Ile Lys Gln
   1               5                   10                  15
   Tyr His Ser Glu Gly Lys Ser Ile Gly Phe Val Pro Thr Met Gly Phe
               20                  25                  30
   Leu His Glu Gly His Leu Thr Leu Ala Asp Lys Ala Arg Gln Glu Asn
           35                  40                  45
   Asp Ala Val Ile Met Ser Ile Phe Val Asn Pro Ala Gln Phe Gly Pro
       50                  55                  60
   Asn Glu Asp Phe Glu Ala Tyr Pro Arg Asp Ile Glu Arg Asp Ala Ala
   65                  70                  75                  80
   Leu Ala Glu Asn Ala Gly Val Asp Ile Leu Phe Thr Pro Asp Ala His
                   85                  90                  95
   Asp Met Tyr Pro Gly Glu Lys Asn Val Thr Ile His Val Glu Arg Arg
               100                 105                 110
   Thr Asp Val Leu Cys Gly Arg Ser Arg Glu Gly His Phe Asp Gly Val
           115                 120                 125
   Ala Ile Val Leu Thr Lys Leu Phe Asn Leu Val Lys Pro Thr Arg Ala
       130                 135                 140
   Tyr Phe Gly Leu Lys Asp Ala Gln Gln Val Ala Val Val Asp Gly Leu
   145                 150                 155                 160
   Ile Ser Asp Phe Phe Met Asp Ile Glu Leu Val Pro Val Asp Thr Val
                   165                 170                 175
   Arg Glu Glu Asp Gly Leu Ala Lys Ser Ser Arg Asn Val Tyr Leu Thr
               180                 185                 190
   Ala Glu Glu Arg Lys Glu Ala Pro Lys Leu Tyr Arg Ala Leu Gln Thr
           195                 200                 205
   Ser Ala Glu Leu Val Gln Ala Gly Glu Arg Asp Pro Glu Ala Val Ile
       210                 215                 220
   Lys Ala Ala Lys Asp Ile Ile Glu Thr Thr Ser Gly Thr Ile Asp Tyr
   225                 230                 235                 240
   Val Glu Leu Tyr Ser Tyr Pro Glu Leu Glu Pro Val Asn Glu Ile Ala
                   245                 250                 255
   Gly Lys Met Ile Leu Ala Val Ala Val Ala Phe Ser Lys Ala Arg Leu
               260                 265                 270
   Ile Asp Asn Ile Ile Ile Asp Ile Arg Glu Met Glu Arg Ile
           275                 280                 285
<210> 27
   <211> 381
   <212> DNA
   <213> Bacillus subtilis
<220>
   <221> CDS
   <222> (1)..(381)
<400> 27
   atg tat cga aca atg atg agc ggc aaa ctt cac agg gca act gtt acg 48
   Met Tyr Arg Thr Met Met Ser Gly Lys Leu His Arg Ala Thr Val Thr
   1 5 10 15
   gaa gca aac ctg aac tat gtg gga agc att aca att gat gaa gat ctc 96
   Glu Ala Asn Leu Asn Tyr Val Gly Ser Ile Thr Ile Asp Glu Asp Leu
   20 25 30
   att gat gct gtg gga atg ctt cct aat gaa aaa gta caa att gtg aat 144
   Ile Asp Ala Val Gly Met Leu Pro Asn Glu Lys Val Gln Ile Val Asn
   35 40 45
   aat aat aat gga gca cgt ctt gaa acg tat att att cct ggt aaa cgg 192
   Asn Asn Asn Gly Ala Arg Leu Glu Thr Tyr Ile Ile Pro Gly Lys Arg
   50 55 60
   gga agc ggc gtc ata tgc tta aac ggt gca gcc gca cgc ctt gtg cag 240
   Gly Ser Gly Val Ile Cys Leu Asn Gly Ala Ala Ala Arg Leu Val Gln
   65 70 75 80
   gaa gga gat aag gtc att att att tcc tac aaa atg atg tct gat caa 288
   Glu Gly Asp Lys Val Ile Ile Ile Ser Tyr Lys Met Met Ser Asp Gln
   85 90 95
   gaa gcg gca agc cat gag ccg aaa gtg gct gtt ctg aat gat caa aac 336
   Glu Ala Ala Ser His Glu Pro Lys Val Ala Val Leu Asn Asp Gln Asn
   100 105 110
   aaa att gaa caa atg ctg ggg aac gaa cca gcc cgt aca att ttg 381
   Lys Ile Glu Gln Met Leu Gly Asn Glu Pro Ala Arg Thr Ile Leu
   115 120 125
<210> 28
   <211> 127
   <212> PRT
   <213> Bacillus subtilis
<400> 28
   Met Tyr Arg Thr Met Met Ser Gly Lys Leu His Arg Ala Thr Val Thr
   1 5 10 15
   Glu Ala Asn Leu Asn Tyr Val Gly Ser Ile Thr Ile Asp Glu Asp Leu
   20 25 30
   Ile Asp Ala Val Gly Met Leu Pro Asn Glu Lys Val Gln Ile Val Asn
   35 40 45
   Asn Asn Asn Gly Ala Arg Leu Glu Thr Tyr Ile Ile Pro Gly Lys Arg
   50 55 60
   Gly Ser Gly Val Ile Cys Leu Asn Gly Ala Ala Ala Arg Leu Val Gln
   65 70 75 80
   Glu Gly Asp Lys Val Ile Ile Ile Ser Tyr Lys Met Met Ser Asp Gln
   85 90 95
   Glu Ala Ala Ser His Glu Pro Lys Val Ala Val Leu Asn Asp Gln Asn
   100 105 110
   Lys Ile Glu Gln Met Leu Gly Asn Glu Pro Ala Arg Thr Ile Leu
   115 120 125
<210> 29
   <211> 894
   <212> DNA
   <213> Bacillus subtilis
<220>
   <221> CDS
   <222> (1)..(894)
<400> 29
   atg aaa att gga att atc ggc gga ggc tcc gtt ggt ctt tta tgc gcc      48
   Met Lys Ile Gly Ile Ile Gly Gly Gly Ser Val Gly Leu Leu Cys Ala
   1               5                   10                  15
   tat tat ttg tca ctt tat cac gac gtg act gtt gtg acg agg cgg caa      96
   Tyr Tyr Leu Ser Leu Tyr His Asp Val Thr Val Val Thr Arg Arg Gln
               20                  25                  30
   gaa cag gct gcg gcc att cag tct gaa gga atc cgg ctt tat aaa ggc     144
   Glu Gln Ala Ala Ala Ile Gln Ser Glu Gly Ile Arg Leu Tyr Lys Gly
           35                  40                  45
   ggg gag gaa ttc agg gct gat tgc agt gcg gac acg agt atc aat tcg     192
   Gly Glu Glu Phe Arg Ala Asp Cys Ser Ala Asp Thr Ser Ile Asn Ser
       50                  55                  60
   gac ttt gac ctg ctt gtc gtg aca gtg aag cag cat cag ctt caa tct     240
   Asp Phe Asp Leu Leu Val Val Thr Val Lys Gln His Gln Leu Gln Ser
   65                  70                  75                  80
   gtt ttt tcg tcg ctt gaa cga atc ggg aag acg aat ata tta ttt ttg     288
   Val Phe Ser Ser Leu Glu Arg Ile Gly Lys Thr Asn Ile Leu Phe Leu
                   85                  90                  95
   caa aac ggc atg ggg cat atc cac gac cta aaa gac tgg cac gtt ggc     336
   Gln Asn Gly Met Gly His Ile His Asp Leu Lys Asp Trp His Val Gly
               100                 105                 110
   cat tcc att tat gtt gga atc gtt gag cac gga gct gta aga aaa tcg     384
   His Ser Ile Tyr Val Gly Ile Val Glu His Gly Ala Val Arg Lys Ser
           115                 120                 125
   gat aca gct gtt gat cat aca ggc cta ggt gcg ata aaa tgg agc gcg     432
   Asp Thr Ala Val Asp His Thr Gly Leu Gly Ala Ile Lys Trp Ser Ala
       130                 135                 140
   ttc gac gat gct gaa cca gac cgg ctg aac atc ttg ttt cag cat aac     480
   Phe Asp Asp Ala Glu Pro Asp Arg Leu Asn Ile Leu Phe Gln His Asn
   145                 150                 155                 160
   cat tcg gat ttt ccg att tat tat gag acg gat tgg tac cgt ctg ctg      528
   His Ser Asp Phe Pro Ile Tyr Tyr Glu Thr Asp Trp Tyr Arg Leu Leu
                   165                 170                 175
   acg ggc aag ctg att gta aat gcg tgt att aat cct tta act gcg tta      576
   Thr Gly Lys Leu Ile Val Asn Ala Cys Ile Asn Pro Leu Thr Ala Leu
               180                 185                 190
   ttg caa gtg aaa aat gga gaa ctg ctg aca acg cca gct tat ctg gct      624
   Leu Gln Val Lys Asn Gly Glu Leu Leu Thr Thr Pro Ala Tyr Leu Ala
           195                 200                 205
   ttt atg aag ctg gta ttt cag gag gca tgc cgc att tta aaa ctt gaa      672
   Phe Met Lys Leu Val Phe Gln Glu Ala Cys Arg Ile Leu Lys Leu Glu
       210                 215                 220
   aat gaa gaa aag gct tgg gag cgg gtt cag gcc gtt tgt ggg caa acg      720
   Asn Glu Glu Lys Ala Trp Glu Arg Val Gln Ala Val Cys Gly Gln Thr
   225                 230                 235                 240
   aaa gag aat cgt tca tca atg ctg gtt gac gtc att gga ggc cgg cag      768
   Lys Glu Asn Arg Ser Ser Met Leu Val Asp Val Ile Gly Gly Arg Gln
                   245                 250                 255
   acg gaa gct gac gcc att atc gga tac tta ttg aag gaa gca agt ctt      816
   Thr Glu Ala Asp Ala Ile Ile Gly Tyr Leu Leu Lys Glu Ala Ser Leu
               260                 265                 270
   caa ggt ctt gat gcc gtc cac cta gag ttt tta tat ggc agc atc aaa      864
   Gln Gly Leu Asp Ala Val His Leu Glu Phe Leu Tyr Gly Ser Ile Lys
           275                 280                 285
   gca ttg gag cga aat aca aac aaa gtc ttt                              894
   Ala Leu Glu Arg Asn Thr Asn Lys Val Phe
       290                 295
<210> 30
   <211> 298
   <212> PRT
   <213> Bacillus subtilis
<400> 30
   Met Lys Ile Gly Ile Ile Gly Gly Gly Ser Val Gly Leu Leu Cys Ala
   1               5                   10                  15
   Tyr Tyr Leu Ser Leu Tyr His Asp Val Thr Val Val Thr Arg Arg Gln
               20                  25                  30
   Glu Gln Ala Ala Ala Ile Gln Ser Glu Gly Ile Arg Leu Tyr Lys Gly
           35                  40                  45
   Gly Glu Glu Phe Arg Ala Asp Cys Ser Ala Asp Thr Ser Ile Asn Ser
       50                  55                  60
   Asp Phe Asp Leu Leu Val Val Thr Val Lys Gln His Gln Leu Gln Ser
   65                  70                  75                  80
   Val Phe Ser Ser Leu Glu Arg Ile Gly Lys Thr Asn Ile Leu Phe Leu
                   85                  90                  95
   Gln Asn Gly Met Gly His Ile His Asp Leu Lys Asp Trp His Val Gly
               100                 105                 110
   His Ser Ile Tyr Val Gly Ile Val Glu His Gly Ala Val Arg Lys Ser
           115                 120                 125
   Asp Thr Ala Val Asp His Thr Gly Leu Gly Ala Ile Lys Trp Ser Ala
       130                 135                 140
   Phe Asp Asp Ala Glu Pro Asp Arg Leu Asn Ile Leu Phe Gln His Asn
   145                 150                 155                 160
   His Ser Asp Phe Pro Ile Tyr Tyr Glu Thr Asp Trp Tyr Arg Leu Leu
                   165                 170                 175
   Thr Gly Lys Leu Ile Val Asn Ala Cys Ile Asn Pro Leu Thr Ala Leu
               180                 185                 190
   Leu Gln Val Lys Asn Gly Glu Leu Leu Thr Thr Pro Ala Tyr Leu Ala
           195                 200                 205
   Phe Met Lys Leu Val Phe Gln Glu Ala Cys Arg Ile Leu Lys Leu Glu
       210                 215                 220
   Asn Glu Glu Lys Ala Trp Glu Arg Val Gln Ala Val Cys Gly Gln Thr
   225                 230                 235                 240
   Lys Glu Asn Arg Ser Ser Met Leu Val Asp Val Ile Gly Gly Arg Gln
                   245                 250                 255
   Thr Glu Ala Asp Ala Ile Ile Gly Tyr Leu Leu Lys Glu Ala Ser Leu
               260                 265                 270
   Gln Gly Leu Asp Ala Val His Leu Glu Phe Leu Tyr Gly Ser Ile Lys
           275                 280                 285
   Ala Leu Glu Arg Asn Thr Asn Lys Val Phe
       290                 295
<210> 31
   <211> 1725
   <212> DNA
   <213> Bacillus subtilis
<220>
   <221> CDS
   <222> (1)..(1722)
<400> 31
   atg ggg act aat gta cag gtg gat tca gca tct gcc gaa tgt aca cag      48
   Met Gly Thr Asn Val Gln Val Asp Ser Ala Ser Ala Glu Cys Thr Gln
   1               5                   10                  15
   acg atg agc gga gca tta atg ctg att gaa tca tta aaa aaa gag aaa      96
   Thr Met Ser Gly Ala Leu Met Leu Ile Glu Ser Leu Lys Lys Glu Lys
               20                  25                  30
   gta gaa atg atc ttc ggt tat ccg ggc ggg gct gtg ctt ccg att tac      144
   Val Glu Met Ile Phe Gly Tyr Pro Gly Gly Ala Val Leu Pro Ile Tyr
           35                  40                  45
   gat aag cta tac aat tca ggg ttg gta cat atc ctt ccc cgt cac gaa      192
   Asp Lys Leu Tyr Asn Ser Gly Leu Val His Ile Leu Pro Arg His Glu
       50                  55                  60
   caa gga gca att cat gca gcg gag gga tac gca agg gtc tcc gga aaa      240
   Gln Gly Ala Ile His Ala Ala Glu Gly Tyr Ala Arg Val Ser Gly Lys
   65                  70                  75                  80
   ccg ggt gtc gtc att gcc acg tca ggg ccg gga gcg aca aac ctt gtt      288
   Pro Gly Val Val Ile Ala Thr Ser Gly Pro Gly Ala Thr Asn Leu Val
                   85                  90                  95
   aca ggc ctt gct gat gcc atg att gat tca ttg ccg tta gtc gtc ttt      336
   Thr Gly Leu Ala Asp Ala Met Ile Asp Ser Leu Pro Leu Val Val Phe
               100                 105                 110
   aca ggg cag gta gca acc tct gta atc ggg agc gat gca ttt cag gaa      384
   Thr Gly Gln Val Ala Thr Ser Val Ile Gly Ser Asp Ala Phe Gln Glu
           115                 120                 125
   gca gac att tta ggg att acg atg cca gta aca aaa cac agc tac cag      432
   Ala Asp Ile Leu Gly Ile Thr Met Pro Val Thr Lys His Ser Tyr Gln
       130                 135                 140
   gtt cgc cag ccg gaa gat ctg ccg cgc atc att aaa gaa gcg ttc cat      480
   Val Arg Gln Pro Glu Asp Leu Pro Arg Ile Ile Lys Glu Ala Phe His
   145                 150                 155                 160
   att gca aca act gga aga ccc gga cct gta ttg att gat att ccg aaa      528
   Ile Ala Thr Thr Gly Arg Pro Gly Pro Val Leu Ile Asp Ile Pro Lys
                   165                 170                 175
   gat gta gca aca att gaa gga gaa ttc agc tac gat cat gag atg aat      576
   Asp Val Ala Thr Ile Glu Gly Glu Phe Ser Tyr Asp His Glu Met Asn
               180                 185                 190
   ctc ccg gga tac cag ccg aca aca gag ccg aat tat ttg cag atc cgc      624
   Leu Pro Gly Tyr Gln Pro Thr Thr Glu Pro Asn Tyr Leu Gln Ile Arg
           195                 200                 205
   aag ctt gtg gaa gcc gtg agc agt gcg aaa aaa ccg gtg atc ctg gcg      672
   Lys Leu Val Glu Ala Val Ser Ser Ala Lys Lys Pro Val Ile Leu Ala
       210                 215                 220
   ggt gcg ggc gta ctg cac gga aaa gcg tca gaa gaa tta aaa aat tat      720
   Gly Ala Gly Val Leu His Gly Lys Ala Ser Glu Glu Leu Lys Asn Tyr
   225                 230                 235                 240
   gct gaa cag cag caa atc cct gtg gca cac acc ctt ttg ggg ctc gga      768
   Ala Glu Gln Gln Gln Ile Pro Val Ala His Thr Leu Leu Gly Leu Gly
                   245                 250                 255
   ggc ttc ccg gct gac cat ccg ctt ttc cta ggg atg gcg gga atg cac      816
   Gly Phe Pro Ala Asp His Pro Leu Phe Leu Gly Met Ala Gly Met His
               260                 265                 270
   ggt act tat aca gcc aat atg gcc ctt cat gaa tgt gat cta tta atc      864
   Gly Thr Tyr Thr Ala Asn Met Ala Leu His Glu Cys Asp Leu Leu Ile
           275                 280                 285
   agt atc ggc gcc cgt ttt gat gac cgt gtc aca gga aac ctg aaa cac      912
   Ser Ile Gly Ala Arg Phe Asp Asp Arg Val Thr Gly Asn Leu Lys His
       290                 295                 300
   ttt gcc aga aac gca aag ata gcc cac atc gat att gat cca gct gaa      960
   Phe Ala Arg Asn Ala Lys Ile Ala His Ile Asp Ile Asp Pro Ala Glu
   305                 310                 315                 320
   atc gga aaa atc atg aaa aca cag att cct gta gtc gga gac agc aaa      1008
   Ile Gly Lys Ile Met Lys Thr Gln Ile Pro Val Val Gly Asp Ser Lys
                   325                 330                 335
   att gtc ctg cag gag ctg atc aaa caa gac ggc aaa caa agc gat tca      1056
   Ile Val Leu Gln Glu Leu Ile Lys Gln Asp Gly Lys Gln Ser Asp Ser
               340                 345                 350
   agc gaa tgg aaa aaa cag ctc gca gaa tgg aaa gaa gag tat ccg ctc      1104
   Ser Glu Trp Lys Lys Gln Leu Ala Glu Trp Lys Glu Glu Tyr Pro Leu
           355                 360                 365
   tgg tat gta gat aat gaa gaa gaa ggt ttt aaa cct cag aaa ttg att      1152
   Trp Tyr Val Asp Asn Glu Glu Glu Gly Phe Lys Pro Gln Lys Leu Ile
       370                 375                 380
   gaa tat att cat caa ttt aca aaa gga gag gcc att gtc gca acg gat      1200
   Glu Tyr Ile His Gln Phe Thr Lys Gly Glu Ala Ile Val Ala Thr Asp
   385                 390                 395                 400
   gta ggc cag cat caa atg tgg tca gcg caa ttt tat ccg ttc caa aaa      1248
   Val Gly Gln His Gln Met Trp Ser Ala Gln Phe Tyr Pro Phe Gln Lys
                   405                 410                 415
   gca gat aaa tgg gtc acg tca ggc gga ctt gga acg atg gga ttc ggt      1296
   Ala Asp Lys Trp Val Thr Ser Gly Gly Leu Gly Thr Met Gly Phe Gly
               420                 425                 430
   ctt ccg gcg gcg atc ggc gca cag ctg gcc gaa aaa gat gct act gtt      1344
   Leu Pro Ala Ala Ile Gly Ala Gln Leu Ala Glu Lys Asp Ala Thr Val
           435                 440                 445
   gtc gcg gtt gtc gga gac ggc gga ttc caa atg acg ctt caa gaa ctc      1392
   Val Ala Val Val Gly Asp Gly Gly Phe Gln Met Thr Leu Gln Glu Leu
       450                 455                 460
   gat gtt att cgc gaa tta aat ctt ccg gtc aag gta gtg att tta aat      1440
   Asp Val Ile Arg Glu Leu Asn Leu Pro Val Lys Val Val Ile Leu Asn
   465                 470                 475                 480
   aac gct tgt ctc gga atg gtc aga cag tgg cag gaa att ttc tat gaa      1488
   Asn Ala Cys Leu Gly Met Val Arg Gln Trp Gln Glu Ile Phe Tyr Glu
                   485                 490                 495
   gaa cgt tat tca gaa tct aaa ttc gct tct cag cct gac ttc gtc aaa      1536
   Glu Arg Tyr Ser Glu Ser Lys Phe Ala Ser Gln Pro Asp Phe Val Lys
               500                 505                 510
   ttg tcc gaa gca tac ggc att aaa ggc atc aga att tca tca gaa gcg      1584
   Leu Ser Glu Ala Tyr Gly Ile Lys Gly Ile Arg Ile Ser Ser Glu Ala
           515                 520                 525
   gaa gca aag gaa aag ctg gaa gag gca tta aca tca aga gaa cct gtt      1632
   Glu Ala Lys Glu Lys Leu Glu Glu Ala Leu Thr Ser Arg Glu Pro Val
       530                 535                 540
   gtc att gac gtg cgg gtt gcc agc gaa gaa aaa gta ttc ccg atg gtg      1680
   Val Ile Asp Val Arg Val Ala Ser Glu Glu Lys Val Phe Pro Met Val
   545                 550                 555                 560
   gct ccg ggg aaa ggg ctg cat gaa atg gtg ggg gtg aaa cct tga          1725
   Ala Pro Gly Lys Gly Leu His Glu Met Val Gly Val Lys Pro
                   565                 570
<210> 32
   <211> 574
   <212> PRT
   <213> Bacillus subtilis
<400> 32
   Met Gly Thr Asn Val Gln Val Asp Ser Ala Ser Ala Glu Cys Thr Gln
    1               5                  10                  15
   Thr Met Ser Gly Ala Leu Met Leu Ile Glu Ser Leu Lys Lys Glu Lys
               20                  25                  30
   Val Glu Met Ile Phe Gly Tyr Pro Gly Gly Ala Val Leu Pro Ile Tyr
           35                  40                  45
   Asp Lys Leu Tyr Asn Ser Gly Leu Val His Ile Leu Pro Arg His Glu
       50                  55                  60
   Gln Gly Ala Ile His Ala Ala Glu Gly Tyr Ala Arg Val Ser Gly Lys
    65                  70                  75                  80
   Pro Gly Val Val Ile Ala Thr Ser Gly Pro Gly Ala Thr Asn Leu Val
                    85                  90                  95
   Thr Gly Leu Ala Asp Ala Met Ile Asp Ser Leu Pro Leu Val Val Phe
               100                 105                 110
   Thr Gly Gln Val Ala Thr Ser Val Ile Gly Ser Asp Ala Phe Gln Glu
           115                 120                 125
   Ala Asp Ile Leu Gly Ile Thr Met Pro Val Thr Lys His Ser Tyr Gln
       130                 135                 140
   Val Arg Gln Pro Glu Asp Leu Pro Arg Ile Ile Lys Glu Ala Phe His
   145                 150                 155                 160
   Ile Ala Thr Thr Gly Arg Pro Gly Pro Val Leu Ile Asp Ile Pro Lys
                   165                 170                 175
   Asp Val Ala Thr Ile Glu Gly Glu Phe Ser Tyr Asp His Glu Met Asn
               180                 185                 190
   Leu Pro Gly Tyr Gln Pro Thr Thr Glu Pro Asn Tyr Leu Gln Ile Arg
           195                 200                 205
   Lys Leu Val Glu Ala Val Ser Ser Ala Lys Lys Pro Val Ile Leu Ala
       210                 215                 220
   Gly Ala Gly Val Leu His Gly Lys Ala Ser Glu Glu Leu Lys Asn Tyr
   225                 230                 235                 240
   Ala Glu Gln Gln Gln Ile Pro Val Ala His Thr Leu Leu Gly Leu Gly
                   245                 250                 255
   Gly Phe Pro Ala Asp His Pro Leu Phe Leu Gly Met Ala Gly Met His
               260                 265                 270
   Gly Thr Tyr Thr Ala Asn Met Ala Leu His Glu Cys Asp Leu Leu Ile
           275                 280                 285
   Ser Ile Gly Ala Arg Phe Asp Asp Arg Val Thr Gly Asn Leu Lys His
       290                 295                 300
   Phe Ala Arg Asn Ala Lys Ile Ala His Ile Asp Ile Asp Pro Ala Glu
   305                 310                 315                 320
   Ile Gly Lys Ile Met Lys Thr Gln Ile Pro Val Val Gly Asp Ser Lys
                   325                 330                 335
   Ile Val Leu Gln Glu Leu Ile Lys Gln Asp Gly Lys Gln Ser Asp Ser
               340                 345                 350
   Ser Glu Trp Lys Lys Gln Leu Ala Glu Trp Lys Glu Glu Tyr Pro Leu
           355                 360                 365
   Trp Tyr Val Asp Asn Glu Glu Glu Gly Phe Lys Pro Gln Lys Leu Ile
       370                 375                 380
   Glu Tyr Ile His Gln Phe Thr Lys Gly Glu Ala Ile Val Ala Thr Asp
   385                 390                 395                 400
   Val Gly Gln His Gln Met Trp Ser Ala Gln Phe Tyr Pro Phe Gln Lys
                   405                 410                 415
   Ala Asp Lys Trp Val Thr Ser Gly Gly Leu Gly Thr Met Gly Phe Gly
               420                 425                 430
   Leu Pro Ala Ala Ile Gly Ala Gln Leu Ala Glu Lys Asp Ala Thr Val
           435                 440                 445
   Val Ala Val Val Gly Asp Gly Gly Phe Gln Met Thr Leu Gln Glu Leu
       450                 455                 460
   Asp Val Ile Arg Glu Leu Asn Leu Pro Val Lys Val Val Ile Leu Asn
   465                 470                 475                 480
   Asn Ala Cys Leu Gly Met Val Arg Gln Trp Gln Glu Ile Phe Tyr Glu
                   485                 490                 495
   Glu Arg Tyr Ser Glu Ser Lys Phe Ala Ser Gln Pro Asp Phe Val Lys
               500                 505                 510
   Leu Ser Glu Ala Tyr Gly Ile Lys Gly Ile Arg Ile Ser Ser Glu Ala
           515                 520                 525
   Glu Ala Lys Glu Lys Leu Glu Glu Ala Leu Thr Ser Arg Glu Pro Val
       530                 535                 540
   Val Ile Asp Val Arg Val Ala Ser Glu Glu Lys Val Phe Pro Met Val
   545                 550                 555                 560
   Ala Pro Gly Lys Gly Leu His Glu Met Val Gly Val Lys Pro
                   565                 570
<210> 33
   <211> 525
   <212> DNA
   <213> Bacillus subtilis
<220>
   <221> CDS
   <222> (1)..(522)
<400> 33
   ttg aaa aga att atc aca ttg act gtg gtg aac cgc tcc ggg gtg tta      48
   Met Lys Arg Ile Ile Thr Leu Thr Val Val Asn Arg Ser Gly Val Leu
    1               5                  10                  15
   aac cgg atc acc ggt cta ttc aca aaa agg cat tac aac att gaa agc      96
   Asn Arg Ile Thr Gly Leu Phe Thr Lys Arg His Tyr Asn Ile Glu Ser
               20                  25                  30
   att aca gtt gga cac aca gaa aca gcc ggc gtt tcc aga atc acc ttc      144
   Ile Thr Val Gly His Thr Glu Thr Ala Gly Val Ser Arg Ile Thr Phe
           35                  40                  45
   gtc gtt cat gtt gaa ggt gaa aat gat gtt gaa cag tta acg aaa cag      192
   Val Val His Val Glu Gly Glu Asn Asp Val Glu Gln Leu Thr Lys Gln
       50                  55                  60
   ctc aac aaa cag att gat gtg ctg aaa gtc aca gac atc aca aat caa      240
   Leu Asn Lys Gln Ile Asp Val Leu Lys Val Thr Asp Ile Thr Asn Gln
   65                  70                  75                  80
   tcg att gtc cag agg gag ctg gcc tta atc aag gtt gtc tcc gca cct      288
   Ser Ile Val Gln Arg Glu Leu Ala Leu Ile Lys Val Val Ser Ala Pro
                   85                  90                  95
   tca aca aga aca gag att aat gga atc ata gaa ccg ttt aga gcc tct      336
   Ser Thr Arg Thr Glu Ile Asn Gly Ile Ile Glu Pro Phe Arg Ala Ser
               100                 105                 110
   gtc gtt gat gtc agc aga gac agc atc gtt gtt cag gtg aca ggt gaa      384
   Val Val Asp Val Ser Arg Asp Ser Ile Val Val Gln Val Thr Gly Glu
           115                 120                 125
   tct aac aaa att gaa gcg ctt att gag tta tta aaa cct tat ggc att      432
   Ser Asn Lys Ile Glu Ala Leu Ile Glu Leu Leu Lys Pro Tyr Gly Ile
       130                 135                 140
   aaa gaa atc gcg aga aca ggt aca acg gct ttt gcg agg gga acc agc      480
   Lys Glu Ile Ala Arg Thr Gly Thr Thr Ala Phe Ala Arg Gly Thr Ser
   145                 150                 155                 160
   aaa agg cgt cat cca ata aaa caa tat cta ttg tat aaa aca taa          525
   Lys Arg Arg His Pro Ile Lys Gln Tyr Leu Leu Tyr Lys Thr
                   165                 170
<210> 34
   <211> 174
   <212> PRT
   <213> Bacillus subtilis
<400> 34
   Met Lys Arg Ile Ile Thr Leu Thr Val Val Asn Arg Ser Gly Val Leu
   1 5 10 15
   Asn Arg Ile Thr Gly Leu Phe Thr Lys Arg His Tyr Asn Ile Glu Ser
   20 25 30
   Ile Thr Val Gly His Thr Glu Thr Ala Gly Val Ser Arg Ile Thr Phe
   35 40 45
   Val Val His Val Glu Gly Glu Asn Asp Val Glu Gln Leu Thr Lys Gln
   50 55 60
   Leu Asn Lys Gln Ile Asp Val Leu Lys Val Thr Asp Ile Thr Asn Gln
   65 70 75 80
   Ser Ile Val Gln Arg Glu Leu Ala Leu Ile Lys Val Val Ser Ala Pro
   85 90 95
   Ser Thr Arg Thr Glu Ile Asn Gly Ile Ile Glu Pro Phe Arg Ala Ser
   100 105 110
   Val Val Asp Val Ser Arg Asp Ser Ile Val Val Gln Val Thr Gly Glu
   115 120 125
   Ser Asn Lys Ile Glu Ala Leu Ile Glu Leu Leu Lys Pro Tyr Gly Ile
   130 135 140
   Lys Glu Ile Ala Arg Thr Gly Thr Thr Ala Phe Ala Arg Gly Thr Ser
   145 150 155 160
   Lys Arg Arg His Pro Ile Lys Gln Tyr Leu Leu Tyr Lys Thr
   165 170
<210> 35
   <211> 1029
   <212> DNA
   <213> Bacillus subtilis
<220>
   <221> CDS
   <222> (1)..(1026)
<400> 35
   atg gta aaa gta tat tat aac ggt gat atc aaa gag aac gta ttg gct 48
   Met Val Lys Val Tyr Tyr Asn Gly Asp Ile Lys Glu Asn Val Leu Ala
   1 5 10 15
   gga aaa aca gta gcg gtt atc ggg tac ggt tcg caa ggc cac gca cat 96
   Gly Lys Thr Val Ala Val Ile Gly Tyr Gly Ser Gln Gly His Ala His
   20 25 30
   gcc ctg aac ctt aaa gaa agc gga gta gac gtg atc gtc ggt gtt aga 144
   Ala Leu Asn Leu Lys Glu Ser Gly Val Asp Val Ile Val Gly Val Arg
   35 40 45
   caa gga aaa tct ttc act caa gcc caa gaa gac gga cat aaa gta ttt 192
   Gln Gly Lys Ser Phe Thr Gln Ala Gln Glu Asp Gly His Lys Val Phe
   50 55 60
   tca gta aaa gaa gcg gca gcc caa gcc gaa atc atc atg gtt ctg ctt 240
   Ser Val Lys Glu Ala Ala Ala Gln Ala Glu Ile Ile Met Val Leu Leu
   65 70 75 80
   ccg gat gag cag cag caa aaa gta tac gaa gct gaa atc aaa gat gaa 288
   Pro Asp Glu Gln Gln Gln Lys Val Tyr Glu Ala Glu Ile Lys Asp Glu
   85 90 95
   ttg aca gca gga aaa tca tta gta ttc gct cat gga ttt aac gtg cat 336
   Leu Thr Ala Gly Lys Ser Leu Val Phe Ala His Gly Phe Asn Val His
   100 105 110
   ttc cat caa att gtt cct ccg gcg gat gta gat gta ttc tta gtg gcc 384
   Phe His Gln Ile Val Pro Pro Ala Asp Val Asp Val Phe Leu Val Ala
   115 120 125
   cct aaa ggc ccg gga cac ttg gta aga aga aca tat gag caa gga gct 432
   Pro Lys Gly Pro Gly His Leu Val Arg Arg Thr Tyr Glu Gln Gly Ala
   130 135 140
   ggc gta cct gca ttg ttc gca atc tat caa gat gtg act gga gaa gca 480
   Gly Val Pro Ala Leu Phe Ala Ile Tyr Gln Asp Val Thr Gly Glu Ala
   145 150 155 160
   aga gac aaa gcc ctc gct tat gct aaa gga atc ggc ggc gca aga gcg 528
   Arg Asp Lys Ala Leu Ala Tyr Ala Lys Gly Ile Gly Gly Ala Arg Ala
   165 170 175
   ggc gta tta gaa acg aca ttt aaa gaa gaa aca gaa aca gat ttg ttc 576
   Gly Val Leu Glu Thr Thr Phe Lys Glu Glu Thr Glu Thr Asp Leu Phe
   180 185 190
   ggt gag caa gca gtt ctt tgc ggc gga tta agc gcg ctt gtc aaa gcc 624
   Gly Glu Gln Ala Val Leu Cys Gly Gly Leu Ser Ala Leu Val Lys Ala
   195 200 205
   gga ttt gaa acc tta act gaa gca ggt tat cag cct gaa ctt gca tac 672
   Gly Phe Glu Thr Leu Thr Glu Ala Gly Tyr Gln Pro Glu Leu Ala Tyr
   210 215 220
   ttc gag tgt ctt cat gag ctg aaa tta atc gta gac ctt atg tac gaa 720
   Phe Glu Cys Leu His Glu Leu Lys Leu Ile Val Asp Leu Met Tyr Glu
   225 230 235 240
   gaa gga ctt gca gga atg aga tat tca atc tct gac aca gca cag tgg 768
   Glu Gly Leu Ala Gly Met Arg Tyr Ser Ile Ser Asp Thr Ala Gln Trp
   245 250 255
   gga gat ttc gta tca ggc cct cgc gtt gtg gac gcc aaa gta aaa gaa 816
   Gly Asp Phe Val Ser Gly Pro Arg Val Val Asp Ala Lys Val Lys Glu
   260 265 270
   tct atg aaa gaa gta tta aaa gat atc caa aac ggt aca ttc gca aaa 864
   Ser Met Lys Glu Val Leu Lys Asp Ile Gln Asn Gly Thr Phe Ala Lys
   275 280 285
   gag tgg atc gtc gaa aac caa gta aac cgt cct cgt ttc aac gct atc      912
   Glu Trp Ile Val Glu Asn Gln Val Asn Arg Pro Arg Phe Asn Ala Ile
       290                 295                 300
   aat gca agc gag aac gaa cat caa atc gaa gta gtg gga aga aag ctt      960
   Asn Ala Ser Glu Asn Glu His Gln Ile Glu Val Val Gly Arg Lys Leu
   305                 310                 315                 320
   cgt gaa atg atg ccg ttt gtg aaa caa ggc aag aag aag gaa gcg gtg      1008
   Arg Glu Met Met Pro Phe Val Lys Gln Gly Lys Lys Lys Glu Ala Val
                   325                 330                 335
   gtc tcc gtt gcg caa aat taa                                          1029
   Val Ser Val Ala Gln Asn
               340
<210> 36
   <211> 342
   <212> PRT
   <213> Bacillus subtilis
<400> 36
   Met Val Lys Val Tyr Tyr Asn Gly Asp Ile Lys Glu Asn Val Leu Ala
   1 5 10 15
   Gly Lys Thr Val Ala Val Ile Gly Tyr Gly Ser Gln Gly His Ala His
   20 25 30
   Ala Leu Asn Leu Lys Glu Ser Gly Val Asp Val Ile Val Gly Val Arg
   35 40 45
   Gln Gly Lys Ser Phe Thr Gln Ala Gln Glu Asp Gly His Lys Val Phe
   50 55 60
   Ser Val Lys Glu Ala Ala Ala Gln Ala Glu Ile Ile Met Val Leu Leu
   65 70 75 80
   Pro Asp Glu Gln Gln Gln Lys Val Tyr Glu Ala Glu Ile Lys Asp Glu
   85 90 95
   Leu Thr Ala Gly Lys Ser Leu Val Phe Ala His Gly Phe Asn Val His
   100 105 110
   Phe His Gln Ile Val Pro Pro Ala Asp Val Asp Val Phe Leu Val Ala
   115 120 125
   Pro Lys Gly Pro Gly His Leu Val Arg Arg Thr Tyr Glu Gln Gly Ala
   130 135 140
   Gly Val Pro Ala Leu Phe Ala Ile Tyr Gln Asp Val Thr Gly Glu Ala
   145 150 155 160
   Arg Asp Lys Ala Leu Ala Tyr Ala Lys Gly Ile Gly Gly Ala Arg Ala
   165 170 175
   Gly Val Leu Glu Thr Thr Phe Lys Glu Glu Thr Glu Thr Asp Leu Phe
   180 185 190
   Gly Glu Gln Ala Val Leu Cys Gly Gly Leu Ser Ala Leu Val Lys Ala
   195 200 205
   Gly Phe Glu Thr Leu Thr Glu Ala Gly Tyr Gln Pro Glu Leu Ala Tyr
   210 215 220
   Phe Glu Cys Leu His Glu Leu Lys Leu Ile Val Asp Leu Met Tyr Glu
   225 230 235 240
   Glu Gly Leu Ala Gly Met Arg Tyr Ser Ile Ser Asp Thr Ala Gln Trp
   245 250 255
   Gly Asp Phe Val Ser Gly Pro Arg Val Val Asp Ala Lys Val Lys Glu
   260 265 270
   Ser Met Lys Glu Val Leu Lys Asp Ile Gln Asn Gly Thr Phe Ala Lys
   275 280 285
   Glu Trp Ile Val Glu Asn Gln Val Asn Arg Pro Arg Phe Asn Ala Ile
   290 295 300
   Asn Ala Ser Glu Asn Glu His Gln Ile Glu Val Val Gly Arg Lys Leu
   305 310 315 320
   Arg Glu Met Met Pro Phe Val Lys Gln Gly Lys Lys Lys Glu Ala Val
   325 330 335
   Val Ser Val Ala Gln Asn
   340
<210> 37
   <211> 1674
   <212> DNA
   <213> Bacillus subtilis
<220>
   <221> CDS
   <222> (1)..(1674)
<400> 37
   atg gca gaa tta cgc agt aat atg atc aca caa gga atc gat aga gct      48
   Met Ala Glu Leu Arg Ser Asn Met Ile Thr Gln Gly Ile Asp Arg Ala
    1               5                  10                  15
   ccg cac cgc agt ttg ctt cgt gca gca ggg gta aaa gaa gag gat ttc      96
   Pro His Arg Ser Leu Leu Arg Ala Ala Gly Val Lys Glu Glu Asp Phe
               20                  25                  30
   ggc aag ccg ttt att gcg gtg tgt aat tca tac att gat atc gtt ccc     144
   Gly Lys Pro Phe Ile Ala Val Cys Asn Ser Tyr Ile Asp Ile Val Pro
           35                  40                  45
   ggt cat gtt cac ttg cag gag ttt ggg aaa atc gta aaa gaa gca atc     192
   Gly His Val His Leu Gln Glu Phe Gly Lys Ile Val Lys Glu Ala Ile
       50                  55                  60
   aga gaa gca ggg ggc gtt ccg ttt gaa ttt aat acc att ggg gta gat     240
   Arg Glu Ala Gly Gly Val Pro Phe Glu Phe Asn Thr Ile Gly Val Asp
   65                  70                  75                  80
   gat ggc atc gca atg ggg cat atc ggt atg aga tat tcg ctg cca agc     288
   Asp Gly Ile Ala Met Gly His Ile Gly Met Arg Tyr Ser Leu Pro Ser
                   85                  90                  95
   cgt gaa att atc gca gac tct gtg gaa acg gtt gta tcc gca cac tgg     336
   Arg Glu Ile Ile Ala Asp Ser Val Glu Thr Val Val Ser Ala His Trp
               100                 105                 110
   ttt gac gga atg gtc tgt att ccg aac tgc gac aaa atc aca ccg gga     384
   Phe Asp Gly Met Val Cys Ile Pro Asn Cys Asp Lys Ile Thr Pro Gly
           115                 120                 125
   atg ctt atg gcg gca atg cgc atc aac att ccg acg att ttt gtc agc     432
   Met Leu Met Ala Ala Met Arg Ile Asn Ile Pro Thr Ile Phe Val Ser
       130                 135                 140
   ggc gga ccg atg gcg gca gga aga aca agt tac ggg cga aaa atc tcc     480
   Gly Gly Pro Met Ala Ala Gly Arg Thr Ser Tyr Gly Arg Lys Ile Ser
   145                 150                 155                 160
   ctt tcc tca gta ttc gaa ggg gta ggc gcc tac caa gca ggg aaa atc     528
   Leu Ser Ser Val Phe Glu Gly Val Gly Ala Tyr Gln Ala Gly Lys Ile
                   165                 170                 175
   aac gaa aac gag ctt caa gaa cta gag cag ttc gga tgc cca acg tgc     576
   Asn Glu Asn Glu Leu Gln Glu Leu Glu Gln Phe Gly Cys Pro Thr Cys
               180                 185                 190
   ggg tct tgc tca ggc atg ttt acg gcg aac tca atg aac tgt ctg tca     624
   Gly Ser Cys Ser Gly Met Phe Thr Ala Asn Ser Met Asn Cys Leu Ser
           195                 200                 205
   gaa gca ctt ggt ctt gct ttg ccg ggt aat gga acc att ctg gca aca     672
   Glu Ala Leu Gly Leu Ala Leu Pro Gly Asn Gly Thr Ile Leu Ala Thr
       210                 215                 220
   tct ccg gaa cgc aaa gag ttt gtg aga aaa tcg gct gcg caa tta atg     720
   Ser Pro Glu Arg Lys Glu Phe Val Arg Lys Ser Ala Ala Gln Leu Met
   225                 230                 235                 240
   gaa acg att cgc aaa gat atc aaa ccg cgt gat att gtt aca gta aaa     768
   Glu Thr Ile Arg Lys Asp Ile Lys Pro Arg Asp Ile Val Thr Val Lys
                   245                 250                 255
   gcg att gat aac gcg ttt gca ctc gat atg gcg ctc gga ggt tct aca     816
   Ala Ile Asp Asn Ala Phe Ala Leu Asp Met Ala Leu Gly Gly Ser Thr
               260                 265                 270
   aat acc gtt ctt cat acc ctt gcc ctt gca aac gaa gcc ggc gtt gaa     864
   Asn Thr Val Leu His Thr Leu Ala Leu Ala Asn Glu Ala Gly Val Glu
           275                 280                 285
   tac tct tta gaa cgc att aac gaa gtc gct gag cgc gtg ccg cac ttg     912
   Tyr Ser Leu Glu Arg Ile Asn Glu Val Ala Glu Arg Val Pro His Leu
       290                 295                 300
   gct aag ctg gcg cct gca tcg gat gtg ttt att gaa gat ctt cac gaa     960
   Ala Lys Leu Ala Pro Ala Ser Asp Val Phe Ile Glu Asp Leu His Glu
   305                 310                 315                 320
   gcg ggc ggc gtt tca gcg gct ctg aat gag ctt tcg aag aaa gaa gga     1008
   Ala Gly Gly Val Ser Ala Ala Leu Asn Glu Leu Ser Lys Lys Glu Gly
                   325                 330                 335
   gcg ctt cat tta gat gcg ctg act gtt aca gga aaa act ctt gga gaa     1056
   Ala Leu His Leu Asp Ala Leu Thr Val Thr Gly Lys Thr Leu Gly Glu
               340                 345                 350
   acc att gcc gga cat gaa gta aag gat tat gac gtc att cac ccg ctg     1104
   Thr Ile Ala Gly His Glu Val Lys Asp Tyr Asp Val Ile His Pro Leu
           355                 360                 365
   gat caa cca ttc act gaa aag gga ggc ctt gct gtt tta ttc ggt aat     1152
   Asp Gln Pro Phe Thr Glu Lys Gly Gly Leu Ala Val Leu Phe Gly Asn
       370                 375                 380
   cta gct ccg gac ggc gct atc att aaa aca ggc ggc gta cag aat ggg     1200
   Leu Ala Pro Asp Gly Ala Ile Ile Lys Thr Gly Gly Val Gln Asn Gly
   385                 390                 395                 400
   att aca aga cac gaa ggg ccg gct gtc gta ttc gat tct cag gac gag     1248
   Ile Thr Arg His Glu Gly Pro Ala Val Val Phe Asp Ser Gln Asp Glu
                   405                 410                 415
   gcg ctt gac ggc att atc aac cga aaa gta aaa gaa ggc gac gtt gtc     1296
   Ala Leu Asp Gly Ile Ile Asn Arg Lys Val Lys Glu Gly Asp Val Val
               420                 425                 430
   atc atc aga tac gaa ggg cca aaa ggc gga cct ggc atg ccg gaa atg      1344
   Ile Ile Arg Tyr Glu Gly Pro Lys Gly Gly Pro Gly Met Pro Glu Met
           435                 440                 445
   ctg gcg cca aca tcc caa atc gtt gga atg gga ctc ggg cca aaa gtg      1392
   Leu Ala Pro Thr Ser Gln Ile Val Gly Met Gly Leu Gly Pro Lys Val
       450                 455                 460
   gca ttg att acg gac gga cgt ttt tcc gga gcc tcc cgt ggc ctc tca      1440
   Ala Leu Ile Thr Asp Gly Arg Phe Ser Gly Ala Ser Arg Gly Leu Ser
   465                 470                 475                 480
   atc ggc cac gta tca cct gag gcc gct gag ggc ggg ccg ctt gcc ttt      1488
   Ile Gly His Val Ser Pro Glu Ala Ala Glu Gly Gly Pro Leu Ala Phe
                   485                 490                 495
   gtt gaa aac gga gac cat att atc gtt gat att gaa aaa cgc atc ttg      1536
   Val Glu Asn Gly Asp His Ile Ile Val Asp Ile Glu Lys Arg Ile Leu
               500                 505                 510
   gat gta caa gtg cca gaa gaa gag tgg gaa aaa cga aaa gcg aac tgg      1584
   Asp Val Gln Val Pro Glu Glu Glu Trp Glu Lys Arg Lys Ala Asn Trp
           515                 520                 525
   aaa ggt ttt gaa ccg aaa gtg aaa acc ggc tac ctg gca cgt tat tct      1632
   Lys Gly Phe Glu Pro Lys Val Lys Thr Gly Tyr Leu Ala Arg Tyr Ser
       530                 535                 540
   aaa ctt gtg aca agt gcc aac acc ggc ggt att atg aaa atc              1674
   Lys Leu Val Thr Ser Ala Asn Thr Gly Gly Ile Met Lys Ile
   545                 550                 555
<210> 38
   <211> 558
   <212> PRT
   <213> Bacillus subtilis
<400> 38
   Met Ala Glu Leu Arg Ser Asn Met Ile Thr Gln Gly Ile Asp Arg Ala
   1 5 10 15
   Pro His Arg Ser Leu Leu Arg Ala Ala Gly Val Lys Glu Glu Asp Phe
   20 25 30
   Gly Lys Pro Phe Ile Ala Val Cys Asn Ser Tyr Ile Asp Ile Val Pro
   35 40 45
   Gly His Val His Leu Gln Glu Phe Gly Lys Ile Val Lys Glu Ala Ile
   50 55 60
   Arg Glu Ala Gly Gly Val Pro Phe Glu Phe Asn Thr Ile Gly Val Asp
   65 70 75 80
   Asp Gly Ile Ala Met Gly His Ile Gly Met Arg Tyr Ser Leu Pro Ser
   85 90 95
   Arg Glu Ile Ile Ala Asp Ser Val Glu Thr Val Val Ser Ala His Trp
   100 105 110
   Phe Asp Gly Met Val Cys Ile Pro Asn Cys Asp Lys Ile Thr Pro Gly
   115 120 125
   Met Leu Met Ala Ala Met Arg Ile Asn Ile Pro Thr Ile Phe Val Ser
   130 135 140
   Gly Gly Pro Met Ala Ala Gly Arg Thr Ser Tyr Gly Arg Lys Ile Ser
   145 150 155 160
   Leu Ser Ser Val Phe Glu Gly Val Gly Ala Tyr Gln Ala Gly Lys Ile
                   165                 170                 175
   Asn Glu Asn Glu Leu Gln Glu Leu Glu Gln Phe Gly Cys Pro Thr Cys
               180                 185                 190
   Gly Ser Cys Ser Gly Met Phe Thr Ala Asn Ser Met Asn Cys Leu Ser
           195                 200                 205
   Glu Ala Leu Gly Leu Ala Leu Pro Gly Asn Gly Thr Ile Leu Ala Thr
       210                 215                 220
   Ser Pro Glu Arg Lys Glu Phe Val Arg Lys Ser Ala Ala Gln Leu Met
   225                 230                 235                 240
   Glu Thr Ile Arg Lys Asp Ile Lys Pro Arg Asp Ile Val Thr Val Lys
                   245                 250                 255
   Ala Ile Asp Asn Ala Phe Ala Leu Asp Met Ala Leu Gly Gly Ser Thr
               260                 265                 270
   Asn Thr Val Leu His Thr Leu Ala Leu Ala Asn Glu Ala Gly Val Glu
           275                 280                 285
   Tyr Ser Leu Glu Arg Ile Asn Glu Val Ala Glu Arg Val Pro His Leu
       290                 295                 300
   Ala Lys Leu Ala Pro Ala Ser Asp Val Phe Ile Glu Asp Leu His Glu
   305                 310                 315                 320
   Ala Gly Gly Val Ser Ala Ala Leu Asn Glu Leu Ser Lys Lys Glu Gly
                   325                 330                 335
   Ala Leu His Leu Asp Ala Leu Thr Val Thr Gly Lys Thr Leu Gly Glu
               340                 345                 350
   Thr Ile Ala Gly His Glu Val Lys Asp Tyr Asp Val Ile His Pro Leu
           355                 360                 365
   Asp Gln Pro Phe Thr Glu Lys Gly Gly Leu Ala Val Leu Phe Gly Asn
       370                 375                 380
   Leu Ala Pro Asp Gly Ala Ile Ile Lys Thr Gly Gly Val Gln Asn Gly
   385                 390                 395                 400
   Ile Thr Arg His Glu Gly Pro Ala Val Val Phe Asp Ser Gln Asp Glu
                   405                 410                 415
   Ala Leu Asp Gly Ile Ile Asn Arg Lys Val Lys Glu Gly Asp Val Val
               420                 425                 430
   Ile Ile Arg Tyr Glu Gly Pro Lys Gly Gly Pro Gly Met Pro Glu Met
           435                 440                 445
   Leu Ala Pro Thr Ser Gln Ile Val Gly Met Gly Leu Gly Pro Lys Val
       450                 455                 460
   Ala Leu Ile Thr Asp Gly Arg Phe Ser Gly Ala Ser Arg Gly Leu Ser
   465                 470                 475                 480
   Ile Gly His Val Ser Pro Glu Ala Ala Glu Gly Gly Pro Leu Ala Phe
                   485                 490                 495
   Val Glu Asn Gly Asp His Ile Ile Val Asp Ile Glu Lys Arg Ile Leu
               500                 505                 510
   Asp Val Gln Val Pro Glu Glu Glu Trp Glu Lys Arg Lys Ala Asn Trp
           515                 520                 525
   Lys Gly Phe Glu Pro Lys Val Lys Thr Gly Tyr Leu Ala Arg Tyr Ser
       530                 535                 540
   Lys Leu Val Thr Ser Ala Asn Thr Gly Gly Ile Met Lys Ile
   545                 550                 555
<210> 39
   <211> 194
   <212> DNA
   <213> Artificial Sequence
<220>
   <223> Description of Artificial Sequence:promoter sequence
<220>
   <221> -35_signal
   <222> (136)..(141)
<220>
   <221> -10_signal
   <222> (159)..(164)
<400> 39
   gctattgacg acagctatgg ttcactgtcc accaaccaaa actgtgctca gtaccgccaa 60
   tatttctccc ttgaggggta caaagaggtg tccctagaag agatccacgc tgtgtaaaaa 120
   ttttacaaaa aggtattgac tttccctaca gggtgtgtaa taatttaatt acaggcgggg 180
   gcaaccccgc ctgt                                                   194
<210> 40
   <211> 163
   <212> DNA
   <213> Artificial Sequence
<220>
   <223> Description of Artificial Sequence:promoter sequence
<220>
   <221> -35_signal
   <222> (113)..(118)
<220>
   <221> -10_signal
   <222> (136)..(141)
<400> 40
   gcctacctag cttccaagaa agatatccta acagcacaag agcggaaaga tgttttgttc 60
   tacatccaga acaacctctg ctaaaattcc tgaaaaattt tgcaaaaagt tgttgacttt 120
   atctacaagg tgtggtataa taatcttaac aacagcagga cgc                    163
<210> 41
   <211> 127
   <212> DNA
   <213> Artificial Sequence
<220>
   <223> Description of Artificial Sequence:promoter sequence
<220>
   <221> -35_signal
   <222> (34)..(39)
<220>
   <221> -10_signal
   <222> (58)..(63)
<220>
   <221> -35_signal
   <222> (75)..(80)
<220>
   <221> -10_signal
   <222> (98)..(103)
<400> 41
   gaggaatcat agaattttgt caaaataatt ttattgacaa cgtcttatta acgttgatat 60
   aatttaaatt ttatttgaca aaaatgggct cgtgttgtac aataaatgta gtgaggtgga 120
   tgcaatg 127
<210> 42
   <211> 24
   <212> DNA
   <213> Artificial Sequence
<220>
   <223> Description of Artificial sequence:ribosome binding site
<400> 42
   taaacatgag gaggagaaaa catg 24
<210> 43
   <211> 28
   <212> DNA
   <213> Artificial Sequence
<220>
   <223> Description of Artificial sequence:ribosome binding site
<400> 43
   attcgagaaa tggagagaat ataatatg 28
<210> 44
   <211> 13
   <212> DNA
   <213> Artificial Sequence
<220>
   <223> Description of Artificial sequence:ribosome binding site
<400> 44
   agaaaggagg tga 13
<210> 45
   <211> 23
   <212> DNA
   <213> Artificial Sequence
<220>
   <223> Description of Artificial sequence:ribosome binding site
<220>
   <221> misc_feature
   <222> 17-20
   <223> n = a, c, g, or t
<400> 45
   ttaagaaagg aggtgannnn atg 23
<210> 46
   <211> 23
   <212> DNA
   <213> Artificial Sequence
<220>
   <223> Description of Artificial sequence:ribosome binding site
<220>
   <221> misc_feature
   <222> 16-20
   <223> n = a, c, g, or t
<400> 46
   ttagaaagga ggtgannnnn atg 23
<210> 47
   <211> 23
   <212> DNA
   <213> Artificial Sequence
<220>
   <223> Description of Artificial sequence:ribosome binding site
<220>
   <221> misc_feature
   <222> 14-20
   <223> n = a, c, g, or t
<400> 47
   agaaaggagg tgannnnnnn atg 23
<210> 48
   <211> 22
   <212> DNA
   <213> Artificial Sequence
<220>
   <223> Description of Artificial sequence:ribosome binding site
<220>
   <221> misc_feature
   <222> 14-19
   <223> n = a, c, g, or t
<400> 48
   agaaaggagg tgannnnnna tg 22
<210> 49
   <211> 25
   <212> DNA
   <213> Artificial Sequence
<220>
   <223> Description of Artificial Sequence:ribosome binding site
<400> 49
   ccctctagaa ggaggagaaa acatg 25
<210> 50
   <211> 24
   <212> DNA
   <213> Artificial Sequence
<220>
   <223> Description of Artificial Sequence:ribosome binding site
<400> 50
   ccctctagag gaggagaaaa catg 24
<210> 51
   <211> 23
   <212> DNA
   <213> Artificial Sequence
<220>
   <223> Description of Artificial Sequence:ribosome binding site
<400> 51
   ttagaaagga ggatttaaat atg 23
<210> 52
   <211> 23
   <212> DNA
   <213> Artificial Sequence
<220>
   <223> Description of Artificial Sequence:ribosome binding site
<400> 52
   ttagaaagga ggtttaatta atg 23
<210> 53
   <211> 23
   <212> DNA
   <213> Artificial Sequence
<220>
   <223> Description of Artificial Sequence:ribosome binding site
<400> 53
   ttagaaagga ggtgatttaa atg 23
<210> 54
   <211> 23
   <212> DNA
   <213> Artificial Sequence
<220>
   <223> Description of Artificial Sequence:ribosome binding site
<400> 54
   ttagaaagga ggtgtttaaa atg 23
<210> 55
   <211> 28
   <212> DNA
   <213> Artificial Sequence
<220>
   <223> Description of Artificial Sequence:ribosome binding site
<400> 55
   attcgagaaa ggaggtgaat ataatatg 28
<210> 56
   <211> 27
   <212> DNA
   <213> Artificial Sequence
<220>
   <223> Description of Artificial Sequence:ribosome binding site
<400> 56
   attcgagaaa ggaggtgaat aataatg 27
<210> 57
   <211> 28
   <212> DNA
   <213> Artificial Sequence
<220>
   <223> Description of Artificial Sequence:ribosome binding site
<400> 57
   attcgtagaa aggaggtgaa ttaatatg 28
<210> 58
   <211> 3291
   <212> DNA
   <213> Bacillus subtilis
<400> 58
   atggggacta atgtacaggt ggattcagca tctgccgaat gtacacagac gatgagcgga 60
   gcattaatgc tgattgaatc attaaaaaaa gagaaagtag aaatgatctt cggttatccg 120
   ggcggggctg tgcttccgat ttacgataag ctatacaatt cagggttggt acatatcctt 180
   ccccgtcacg aacaaggagc aattcatgca gcggagggat acgcaagggt ctccggaaaa 240
   ccgggtgtcg tcattgccac gtcagggccg ggagcgacaa accttgttac aggccttgct 300
   gatgccatga ttgattcatt gccgttagtc gtctttacag ggcaggtagc aacctctgta 360
   atcgggagcg atgcatttca ggaagcagac attttaggga ttacgatgcc agtaacaaaa 420
   cacagctacc aggttcgcca gccggaagat ctgccgcgca tcattaaaga agcgttccat 480
   attgcaacaa ctggaagacc cggacctgta ttgattgata ttccgaaaga tgtagcaaca 540
   attgaaggag aattcagcta cgatcatgag atgaatctcc cgggatacca gccgacaaca 600
   gagccgaatt atttgcagat ccgcaagctt gtggaagccg tgagcagtgc gaaaaaaccg 660
   gtgatcctgg cgggtgcggg cgtactgcac ggaaaagcgt cagaagaatt aaaaaattat 720
   gctgaacagc agcaaatccc tgtggcacac acccttttgg ggctcggagg cttcccggct 780
   gaccatccgc ttttcctagg gatggcggga atgcacggta cttatacagc caatatggcc 840
   cttcatgaat gtgatctatt aatcagtatc ggcgcccgtt ttgatgaccg tgtcacagga 900
   aacctgaaac actttgccag aaacgcaaag atagcccaca tcgatattga tccagctgaa 960
   atcggaaaaa tcatgaaaac acagattcct gtagtcggag acagcaaaat tgtcctgcag 1020
   gagctgatca aacaagacgg caaacaaagc gattcaagcg aatggaaaaa acagctcgca 1080
   gaatggaaag aagagtatcc gctctggtat gtagataatg aagaagaagg ttttaaacct 1140
   cagaaattga ttgaatatat tcatcaattt acaaaaggag aggccattgt cgcaacggat 1200
   gtaggccagc atcaaatgtg gtcagcgcaa ttttatccgt tccaaaaagc agataaatgg 1260
   gtcacgtcag gcggacttgg aacgatggga ttcggtcttc cggcggcgat cggcgcacag 1320
   ctggccgaaa aagatgctac tgttgtcgcg gttgtcggag acggcggatt ccaaatgacg 1380
   cttcaagaac tcgatgttat tcgcgaatta aatcttccgg tcaaggtagt gattttaaat 1440
   aacgcttgtc tcggaatggt cagacagtgg caggaaattt tctatgaaga acgttattca 1500
   gaatctaaat tcgcttctca gcctgacttc gtcaaattgt ccgaagcata cggcattaaa 1560
   ggcatcagaa tttcatcaga agcggaagca aaggaaaagc tggaagaggc attaacatca 1620
   agagaacctg ttgtcattga cgtgcgggtt gccagcgaag aaaaagtatt cccgatggtg 1680
   gctccgggga aagggctgca tgaaatggtg ggggtgaaac cttgaaaaga attatcacat 1740
   tgactgtggt gaaccgctcc ggggtgttaa accggatcac cggtctattc acaaaaaggc 1800
   attacaacat tgaaagcatt acagttggac acacagaaac agccggcgtt tccagaatca 1860
   ccttcgtcgt tcatgttgaa ggtgaaaatg atgttgaaca gttaacgaaa cagctcaaca 1920
   aacagattga tgtgctgaaa gtcacagaca tcacaaatca atcgattgtc cagagggagc 1980
   tggccttaat caaggttgtc tccgcacctt caacaagaac agagattaat ggaatcatag 2040
   aaccgtttag agcctctgtc gttgatgtca gcagagacag catcgttgtt caggtgacag 2100
   gtgaatctaa caaaattgaa gcgcttattg agttattaaa accttatggc attaaagaaa 2160
   tcgcgagaac aggtacaacg gcttttgcga ggggaaccag caaaaggcgt catccaataa 2220
   aacaatatct attgtataaa acataacaag ggagagattg aaatggtaaa agtatattat 2280
   aacggtgata tcaaagagaa cgtattggct ggaaaaacag tagcggttat cgggtacggt 2340
   tcgcaaggcc acgcacatgc cctgaacctt aaagaaagcg gagtagacgt gatcgtcggt 2400
   gttagacaag gaaaatcttt cactcaagcc caagaagacg gacataaagt attttcagta 2460
   aaagaagcgg cagcccaagc cgaaatcatc atggttctgc ttccggatga gcagcagcaa 2520
   aaagtatacg aagctgaaat caaagatgaa ttgacagcag gaaaatcatt agtattcgct 2580
   catggattta acgtgcattt ccatcaaatt gttcctccgg cggatgtaga tgtattctta 2640
   gtggccccta aaggcccggg acacttggta agaagaacat atgagcaagg agctggcgta 2700
   cctgcattgt tcgcaatcta tcaagatgtg actggagaag caagagacaa agccctcgct 2760
   tatgctaaag gaatcggcgg cgcaagagcg ggcgtattag aaacgacatt taaagaagaa 2820
   acagaaacag atttgttcgg tgagcaagca gttctttgcg gcggattaag cgcgcttgtc 2880
   aaagccggat ttgaaacctt aactgaagca ggttatcagc ctgaacttgc atacttcgag 2940
   tgtcttcatg agctgaaatt aatcgtagac cttatgtacg aagaaggact tgcaggaatg 3000
   agatattcaa tctctgacac agcacagtgg ggagatttcg tatcaggccc tcgcgttgtg 3060
   gacgccaaag taaaagaatc tatgaaagaa gtattaaaag atatccaaaa cggtacattc 3120
   gcaaaagagt ggatcgtcga aaaccaagta aaccgtcctc gtttcaacgc tatcaatgca 3180
   agcgagaacg aacatcaaat cgaagtagtg ggaagaaagc ttcgtgaaat gatgccgttt 3240
   gtgaaacaag gcaagaagaa ggaagcggtg gtctccgttg cgcaaaatta a 3291
<210> 59
   <211> 2363
   <212> DNA
   <213> Bacillus subtilis
<220>
   <221> CDS
   <222> (242)..(1072)
<220>
   <221> CDS
   <222> (1077)..(1934)
<220>
   <221> CDS
   <222> (1939)..(2319)
<400> 59
<210> 60
   <211> 293
   <212> PRT
   <213> Bacillus subtilis
<400> 60
   Met Ser Ile Ala Val Ser Glu Glu Glu Ala Lys Ala Val Glu Gly Leu
   1               5                   10                  15
   Asn Asp Tyr Leu Ser Val Glu Glu Val Glu Thr Ile Tyr Ile Pro Leu
               20                  25                  30
   Val Arg Leu Leu His Leu His Val Lys Ser Ala Ala Glu Arg Asn Lys
           35                  40                  45
   His Val Asn Val Phe Leu Lys His Pro His Ser Ala Lys Ile Pro Phe
       50                  55                  60
   Ile Ile Gly Ile Ala Gly Ser Val Ala Val Gly Lys Ser Thr Thr Ala
   65                  70                  75                  80
   Arg Ile Leu Gln Lys Leu Leu Ser Arg Leu Pro Asp Arg Pro Lys Val
                   85                  90                  95
   Ser Leu Ile Thr Thr Asp Gly Phe Leu Phe Pro Thr Ala Glu Leu Lys
               100                 105                 110
   Lys Lys Asn Met Met Ser Arg Lys Gly Phe Pro Glu Ser Tyr Asp Val
           115                 120                 125
   Lys Ala Leu Leu Glu Phe Leu Asn Asp Leu Lys Ser Gly Lys Asp Ser
       130                 135                 140
   Val Lys Ala Pro Val Tyr Ser His Leu Thr Tyr Asp Arg Glu Glu Gly
   145                 150                 155                 160
   Val Phe Glu Val Val Glu Gln Ala Asp Ile Val Ile Ile Glu Gly Ile
                   165                 170                 175
   Asn Val Leu Gln Ser Pro Thr Leu Glu Asp Asp Arg Glu Asn Pro Arg
               180                 185                 190
   Ile Phe Val Ser Asp Phe Phe Asp Phe Ser Ile Tyr Val Asp Ala Glu
           195                 200                 205
   Glu Ser Arg Ile Phe Thr Trp Tyr Leu Glu Arg Phe Arg Leu Leu Arg
       210                 215                 220
   Glu Thr Ala Phe Gln Asn Pro Asp Ser Tyr Phe His Lys Phe Lys Asp
   225                 230                 235                 240
   Leu Ser Asp Gln Glu Ala Asp Glu Met Ala Ala Ser Ile Trp Glu Ser
                   245                 250                 255
   Val Asn Arg Pro Asn Leu Tyr Glu Asn Ile Leu Pro Thr Lys Phe Arg
               260                 265                 270
   Ser Asp Leu Ile Leu Arg Lys Gly Asp Gly His Lys Val Glu Glu Val
           275                 280                 285
   Leu Val Arg Arg Val
       290
<210> 61
   <211> 281
   <212> PRT
   <213> Bacillus subtilis
<400> 61
   Met Glu Gly Leu Asn Asp Tyr Leu Ser Val Glu Glu Val Glu Thr Ile
   1               5                   10                  15
   Tyr Ile Pro Leu Val Arg Leu Leu His Leu His Val Lys Ser Ala Ala
               20                  25                  30
   Glu Arg Asn Lys His Val Asn Val Phe Leu Lys His Pro His Ser Ala
           35                  40                  45
   Lys Ile Pro Phe Ile Ile Gly Ile Ala Gly Ser Val Ala Val Gly Lys
       50                  55                  60
   Ser Thr Thr Ala Arg Ile Leu Gln Lys Leu Leu Ser Arg Leu Pro Asp
   65                  70                  75                  80
   Arg Pro Lys Val Ser Leu Ile Thr Thr Asp Gly Phe Leu Phe Pro Thr
                   85                  90                  95
   Ala Glu Leu Lys Lys Lys Asn Met Met Ser Arg Lys Gly Phe Pro Glu
               100                 105                 110
   Ser Tyr Asp Val Lys Ala Leu Leu Glu Phe Leu Asn Asp Leu Lys Ser
           115                 120                 125
   Gly Lys Asp Ser Val Lys Ala Pro Val Tyr Ser His Leu Thr Tyr Asp
       130                 135                 140
   Arg Glu Glu Gly Val Phe Glu Val Val Glu Gln Ala Asp Ile Val Ile
   145                 150                 155                 160
   Ile Glu Gly Ile Asn Val Leu Gln Ser Pro Thr Leu Glu Asp Asp Arg
                   165                 170                 175
   Glu Asn Pro Arg Ile Phe Val Ser Asp Phe Phe Asp Phe Ser Ile Tyr
               180                 185                 190
   Val Asp Ala Glu Glu Ser Arg Ile Phe Thr Trp Tyr Leu Glu Arg Phe
           195                 200                 205
   Arg Leu Leu Arg Glu Thr Ala Phe Gln Asn Pro Asp Ser Tyr Phe His
       210                 215                 220
   Lys Phe Lys Asp Leu Ser Asp Gln Glu Ala Asp Glu Met Ala Ala Ser
   225                 230                 235                 240
   Ile Trp Glu Ser Val Asn Arg Pro Asn Leu Tyr Glu Asn Ile Leu Pro
                   245                 250                 255
   Thr Lys Phe Arg Ser Asp Leu Ile Leu Arg Lys Gly Asp Gly His Lys
               260                 265                 270
   Val Glu Glu Val Leu Val Arg Arg Val
           275                 280
<210> 62
   <211> 1092
   <212> DNA
   <213> Bacillus subtilis
<220>
   <221> CDS
   <222> (1)..(1089)
<400> 62
   atg act aaa caa aca att cgc gtt gaa ttg aca tca aca aaa aaa ccg    48
   Met Thr Lys Gln Thr Ile Arg Val Glu Leu Thr Ser Thr Lys Lys Pro
     1               5                  10                  15
   aaa cca gac cca aat cag ctt tcg ttc gga aga gtg ttt aca gac cac    96
   Lys Pro Asp Pro Asn Gln Leu Ser Phe Gly Arg Val Phe Thr Asp His
                20                  25                  30
   atg ttt gta atg gac tat gcc gca gat aaa ggt tgg tac gat cca aga   144
   Met Phe Val Met Asp Tyr Ala Ala Asp Lys Gly Trp Tyr Asp Pro Arg
            35                  40                  45
   atc att cct tat caa ccc tta tca atg gat cca act gca atg gtc tat   192
   Ile Ile Pro Tyr Gln Pro Leu Ser Met Asp Pro Thr Ala Met Val Tyr
        50                  55                  60
   cac tac ggc caa acc gtg ttt gaa ggg tta aag gct tac gtg tca gag   240
   His Tyr Gly Gln Thr Val Phe Glu Gly Leu Lys Ala Tyr Val Ser Glu
    65                  70                  75                  80
   gat gac cat gtt ctg ctt ttc aga ccg gaa aaa aat atg gaa cgc ctg   288
   Asp Asp His Val Leu Leu Phe Arg Pro Glu Lys Asn Met Glu Arg Leu
                    85                  90                  95
   aat caa tca aac gac cgc ctc tgc atc ccg caa att gat gaa gaa cag   336
   Asn Gln Ser Asn Asp Arg Leu Cys Ile Pro Gln Ile Asp Glu Glu Gln
               100                 105                 110
   gtt ctt gaa ggc tta aag cag ctt gtc gca att gat aaa gac tgg att   384
   Val Leu Glu Gly Leu Lys Gln Leu Val Ala Ile Asp Lys Asp Trp Ile
           115                 120                 125
   cca aat gcg gag ggc acg tcc ctt tac atc cgt ccg ttc atc atc gca   432
   Pro Asn Ala Glu Gly Thr Ser Leu Tyr Ile Arg Pro Phe Ile Ile Ala
       130                 135                 140
   acc gag cct ttc ctt ggt gtt gcg gca tct cat acg tat aag ctc ttg   480
   Thr Glu Pro Phe Leu Gly Val Ala Ala Ser His Thr Tyr Lys Leu Leu
   145                 150                 155                 160
   atc att ctt tct ccg gtc ggc tct tat tac aaa gaa ggc att aag ccg   528
   Ile Ile Leu Ser Pro Val Gly Ser Tyr Tyr Lys Glu Gly Ile Lys Pro
                   165                 170                 175
   gtc aaa atc gct gtt gaa agt gaa ttt gtc cgt gcg gta aaa ggc gga   576
   Val Lys Ile Ala Val Glu Ser Glu Phe Val Arg Ala Val Lys Gly Gly
               180                 185                 190
   aca gga aat gcc aaa acc gca gga aac tat gct tca agc tta aaa gcg   624
   Thr Gly Asn Ala Lys Thr Ala Gly Asn Tyr Ala Ser Ser Leu Lys Ala
           195                 200                 205
   cag cag gta gcc gaa gag aaa gga ttt tct caa gta ctc tgg ctg gac   672
   Gln Gln Val Ala Glu Glu Lys Gly Phe Ser Gln Val Leu Trp Leu Asp
       210                 215                 220
   ggc att gag aag aaa tac atc gaa gaa gtc gga agc atg aac atc ttc   720
   Gly Ile Glu Lys Lys Tyr Ile Glu Glu Val Gly Ser Met Asn Ile Phe
   225                 230                 235                 240
   ttc aaa atc aac ggt gaa atc gta aca ccg atg ctg aac ggg agc atc      768
   Phe Lys Ile Asn Gly Glu Ile Val Thr Pro Met Leu Asn Gly Ser Ile
                   245                 250                 255
   ctg gaa ggc att acg cgc aat tca gtc atc gcc ttg ctt aag cat tgg      816
   Leu Glu Gly Ile Thr Arg Asn Ser Val Ile Ala Leu Leu Lys His Trp
               260                 265                 270
   ggc ctt caa gtt tca gaa cga aaa att gcg atc gat gag gtc atc caa      864
   Gly Leu Gln Val Ser Glu Arg Lys Ile Ala Ile Asp Glu Val Ile Gln
           275                 280                 285
   gcc cat aaa gac ggc atc ctg gaa gaa gcc ttc gga aca ggt aca gca      912
   Ala His Lys Asp Gly Ile Leu Glu Glu Ala Phe Gly Thr Gly Thr Ala
       290                 295                 300
   gct gtt att tcc cca gtc ggc gag ctg atc tgg cag gat gaa aca ctt      960
   Ala Val Ile Ser Pro Val Gly Glu Leu Ile Trp Gln Asp Glu Thr Leu
   305                 310                 315                 320
   tcg atc aac aac ggt gaa aca gga gaa atc gca aaa aaa cta tat gac      1008
   Ser Ile Asn Asn Gly Glu Thr Gly Glu Ile Ala Lys Lys Leu Tyr Asp
                   325                 330                 335
   acg att aca ggc att caa aaa ggc gct gtc gca gac gaa ttc gga tgg      1056
   Thr Ile Thr Gly Ile Gln Lys Gly Ala Val Ala Asp Glu Phe Gly Trp
               340                 345                 350
   acg acc gaa gtc gca gcg ctg act gaa agc aag taa                      1092
   Thr Thr Glu Val Ala Ala Leu Thr Glu Ser Lys
           355                 360
<210> 63
   <211> 363
   <212> PRT
   <213> Bacillus subtilis
<400> 63
   Met Thr Lys Gln Thr Ile Arg Val Glu Leu Thr Ser Thr Lys Lys Pro
    1               5                  10                  15
   Lys Pro Asp Pro Asn Gln Leu Ser Phe Gly Arg Val Phe Thr Asp His
               20                  25                  30
   Met Phe Val Met Asp Tyr Ala Ala Asp Lys Gly Trp Tyr Asp Pro Arg
           35                  40                  45
   Ile Ile Pro Tyr Gln Pro Leu Ser Met Asp Pro Thr Ala Met Val Tyr
       50                  55                  60
   His Tyr Gly Gln Thr Val Phe Glu Gly Leu Lys Ala Tyr Val Ser Glu
   65                  70                  75                  80
   Asp Asp His Val Leu Leu Phe Arg Pro Glu Lys Asn Met Glu Arg Leu
                   85                  90                  95
   Asn Gln Ser Asn Asp Arg Leu Cys Ile Pro Gln Ile Asp Glu Glu Gln
               100                 105                 110
   Val Leu Glu Gly Leu Lys Gln Leu Val Ala Ile Asp Lys Asp Trp Ile
           115                 120                 125
   Pro Asn Ala Glu Gly Thr Ser Leu Tyr Ile Arg Pro Phe Ile Ile Ala
       130                 135                 140
   Thr Glu Pro Phe Leu Gly Val Ala Ala Ser His Thr Tyr Lys Leu Leu
   145                 150                 155                 160
   Ile Ile Leu Ser Pro Val Gly Ser Tyr Tyr Lys Glu Gly Ile Lys Pro
                   165                 170                 175
   Val Lys Ile Ala Val Glu Ser Glu Phe Val Arg Ala Val Lys Gly Gly
               180                 185                 190
   Thr Gly Asn Ala Lys Thr Ala Gly Asn Tyr Ala Ser Ser Leu Lys Ala
           195                 200                 205
   Gln Gln Val Ala Glu Glu Lys Gly Phe Ser Gln Val Leu Trp Leu Asp
       210                 215                 220
   Gly Ile Glu Lys Lys Tyr Ile Glu Glu Val Gly Ser Met Asn Ile Phe
   225                 230                 235                 240
   Phe Lys Ile Asn Gly Glu Ile Val Thr Pro Met Leu Asn Gly Ser Ile
                   245                 250                 255
   Leu Glu Gly Ile Thr Arg Asn Ser Val Ile Ala Leu Leu Lys His Trp
               260                 265                 270
   Gly Leu Gln Val Ser Glu Arg Lys Ile Ala Ile Asp Glu Val Ile Gln
           275                 280                 285
   Ala His Lys Asp Gly Ile Leu Glu Glu Ala Phe Gly Thr Gly Thr Ala
       290                 295                 300
   Ala Val Ile Ser Pro Val Gly Glu Leu Ile Trp Gln Asp Glu Thr Leu
   305                 310                 315                 320
   Ser Ile Asn Asn Gly Glu Thr Gly Glu Ile Ala Lys Lys Leu Tyr Asp
                   325                 330                 335
   Thr Ile Thr Gly Ile Gln Lys Gly Ala Val Ala Asp Glu Phe Gly Trp
               340                 345                 350
   Thr Thr Glu Val Ala Ala Leu Thr Glu Ser Lys
           355                 360
<210> 64
   <211> 1071
   <212> DNA
   <213> Bacillus subtilis
<220>
   <221> CDS
   <222> (1)..(1068)
<400> 64
   ttg aat aag ctt att gaa cga gaa aaa act gta tat tat aag gaa aag      48
   Met Asn Lys Leu Ile Glu Arg Glu Lys Thr Val Tyr Tyr Lys Glu Lys
     1               5                  10                  15
   ccc gac ccg tct tcc ttg ggg ttt gga caa tat ttt aca gat tat atg      96
   Pro Asp Pro Ser Ser Leu Gly Phe Gly Gln Tyr Phe Thr Asp Tyr Met
                20                  25                  30
   ttt gtg atg gac tac gaa gag ggg att gga tgg cat cat ccg aga att     144
   Phe Val Met Asp Tyr Glu Glu Gly Ile Gly Trp His His Pro Arg Ile
            35                  40                  45
   gcg ccg tac gca ccg ctt acg ctt gat ccg tct tca tct gtt ttt cat     192
   Ala Pro Tyr Ala Pro Leu Thr Leu Asp Pro Ser Ser Ser Val Phe His
        50                  55                  60
   tac ggc cag gct gtt ttt gaa gga tta aaa gca tac aga aca gac gac     240
   Tyr Gly Gln Ala Val Phe Glu Gly Leu Lys Ala Tyr Arg Thr Asp Asp
    65                  70                  75                  80
   ggc agg gtg ctg ctg ttc cgt ccg gat caa aat atc aaa cgg ctg aac      288
   Gly Arg Val Leu Leu Phe Arg Pro Asp Gln Asn Ile Lys Arg Leu Asn
                    85                  90                  95
   aga tcg tgt gag cgc atg agc atg ccc cct tta gac gaa gag ctg gtg      336
   Arg Ser Cys Glu Arg Met Ser Met Pro Pro Leu Asp Glu Glu Leu Val
               100                 105                 110
   ctt gag gca ttg acg caa tta gtt gag ctg gag aaa gat tgg gtt cca      384
   Leu Glu Ala Leu Thr Gln Leu Val Glu Leu Glu Lys Asp Trp Val Pro
           115                 120                 125
   aag gaa aaa gga acg tca ctg tat att cgt cct ttt gtc att gcc aca      432
   Lys Glu Lys Gly Thr Ser Leu Tyr Ile Arg Pro Phe Val Ile Ala Thr
       130                 135                 140
   gaa ccg agt ctc ggt gtg aag gca tcc agg agc tat aca ttt atg atc      480
   Glu Pro Ser Leu Gly Val Lys Ala Ser Arg Ser Tyr Thr Phe Met Ile
   145                 150                 155                 160
   gtg ctt tcg cct gtc ggc tcc tat tat ggc gac gat cag ctg aag ccg      528
   Val Leu Ser Pro Val Gly Ser Tyr Tyr Gly Asp Asp Gln Leu Lys Pro
                   165                 170                 175
   gtt aga atc tat gtc gaa gat gag tat gtg agg gcg gtc aac gga gga      576
   Val Arg Ile Tyr Val Glu Asp Glu Tyr Val Arg Ala Val Asn Gly Gly
               180                 185                 190
   gtc ggg ttt gca aaa acg gct gga aac tat gcc gcc agt ctt cag gca      624
   Val Gly Phe Ala Lys Thr Ala Gly Asn Tyr Ala Ala Ser Leu Gln Ala
           195                 200                 205
   cag cgg aaa gcg aat gaa ctg ggc tat gac cag gta ctg tgg ctg gac      672
   Gln Arg Lys Ala Asn Glu Leu Gly Tyr Asp Gln Val Leu Trp Leu Asp
       210                 215                 220
   gcc atc gaa aag aaa tat gtg gaa gaa gta ggg agc atg aac atc ttt      720
   Ala Ile Glu Lys Lys Tyr Val Glu Glu Val Gly Ser Met Asn Ile Phe
   225                 230                 235                 240
   ttc gtc ata aac ggg gaa gct gtc aca cct gct tta agc gga agc att      768
   Phe Val Ile Asn Gly Glu Ala Val Thr Pro Ala Leu Ser Gly Ser Ile
                   245                 250                 255
   tta agc ggg gtt aca cgt gcg tct gcg att gaa ttg att cga agc tgg      816
   Leu Ser Gly Val Thr Arg Ala Ser Ala Ile Glu Leu Ile Arg Ser Trp
               260                 265                 270
   ggc att ccg gtt cgt gaa gag aga ata tcg att gat gag gtg tat gcg      864
   Gly Ile Pro Val Arg Glu Glu Arg Ile Ser Ile Asp Glu Val Tyr Ala
           275                 280                 285
   gcc tct gca cgc gga gaa ttg aca gag gtc ttt ggc aca ggc acg gca      912
   Ala Ser Ala Arg Gly Glu Leu Thr Glu Val Phe Gly Thr Gly Thr Ala
       290                 295                 300
   gca gtc gtt acg cct gtc ggt gaa ctc aac atc cat gga aaa acg gtg      960
   Ala Val Val Thr Pro Val Gly Glu Leu Asn Ile His Gly Lys Thr Val
   305                 310                 315                 320
   att gta ggc gac ggg caa atc ggg gac ctc tcg aaa aag ctg tat gaa     1008
   Ile Val Gly Asp Gly Gln Ile Gly Asp Leu Ser Lys Lys Leu Tyr Glu
                   325                 330                 335
   acg ata aca gat att cag ctt ggc aag gta aaa ggc ccg ttt aac tgg     1056
   Thr Ile Thr Asp Ile Gln Leu Gly Lys Val Lys Gly Pro Phe Asn Trp
               340                 345                 350
   aca gtg gaa gtg tga                                                   1071
   Thr Val Glu Val
           355
<210> 65
   <211> 356
   <212> PRT
   <213> Bacillus subtilis
<400> 65
   Met Asn Lys Leu Ile Glu Arg Glu Lys Thr Val Tyr Tyr Lys Glu Lys
   1               5                   10                  15
   Pro Asp Pro Ser Ser Leu Gly Phe Gly Gln Tyr Phe Thr Asp Tyr Met
               20                  25                  30
   Phe Val Met Asp Tyr Glu Glu Gly Ile Gly Trp His His Pro Arg Ile
           35                  40                  45
   Ala Pro Tyr Ala Pro Leu Thr Leu Asp Pro Ser Ser Ser Val Phe His
       50                  55                  60
   Tyr Gly Gln Ala Val Phe Glu Gly Leu Lys Ala Tyr Arg Thr Asp Asp
   65                  70                  75                  80
   Gly Arg Val Leu Leu Phe Arg Pro Asp Gln Asn Ile Lys Arg Leu Asn
                   85                  90                  95
   Arg Ser Cys Glu Arg Met Ser Met Pro Pro Leu Asp Glu Glu Leu Val
               100                 105                 110
   Leu Glu Ala Leu Thr Gln Leu Val Glu Leu Glu Lys Asp Trp Val Pro
           115                 120                 125
   Lys Glu Lys Gly Thr Ser Leu Tyr Ile Arg Pro Phe Val Ile Ala Thr
       130                 135                 140
   Glu Pro Ser Leu Gly Val Lys Ala Ser Arg Ser Tyr Thr Phe Met Ile
   145                 150                 155                 160
   Val Leu Ser Pro Val Gly Ser Tyr Tyr Gly Asp Asp Gln Leu Lys Pro
                   165                 170                 175
   Val Arg Ile Tyr Val Glu Asp Glu Tyr Val Arg Ala Val Asn Gly Gly
               180                 185                 190
   Val Gly Phe Ala Lys Thr Ala Gly Asn Tyr Ala Ala Ser Leu Gln Ala
           195                 200                 205
   Gln Arg Lys Ala Asn Glu Leu Gly Tyr Asp Gln Val Leu Trp Leu Asp
       210                 215                 220
   Ala Ile Glu Lys Lys Tyr Val Glu Glu Val Gly Ser Met Asn Ile Phe
   225                 230                 235                 240
   Phe Val Ile Asn Gly Glu Ala Val Thr Pro Ala Leu Ser Gly Ser Ile
                   245                 250                 255
   Leu Ser Gly Val Thr Arg Ala Ser Ala Ile Glu Leu Ile Arg Ser Trp
               260                 265                 270
   Gly Ile Pro Val Arg Glu Glu Arg Ile Ser Ile Asp Glu Val Tyr Ala
           275                 280                 285
   Ala Ser Ala Arg Gly Glu Leu Thr Glu Val Phe Gly Thr Gly Thr Ala
       290                 295                 300
   Ala Val Val Thr Pro Val Gly Glu Leu Asn Ile His Gly Lys Thr Val
   305 310 315 320
   Ile Val Gly Asp Gly Gln Ile Gly Asp Leu Ser Lys Lys Leu Tyr Glu
   325 330 335
   Thr Ile Thr Asp Ile Gln Leu Gly Lys Val Lys Gly Pro Phe Asn Trp
   340 345 350
   Thr Val Glu Val
   355
<210> 66
   <211> 1428
   <212> DNA
   <213> Bacillus subtilis
<220>
   <221> CDS
   <222> (1)..(1425)
<400> 66
   atg tta aac ggc caa aaa gaa tat cgc gtg gaa aaa gac ttc ctt ggg     48
   Met Leu Asn Gly Gln Lys Glu Tyr Arg Val Glu Lys Asp Phe Leu Gly
    1               5                  10                  15
   gaa aaa caa att gaa gca gat gtt tat tac gga att cag acg ctc cgt     96
   Glu Lys Gln Ile Glu Ala Asp Val Tyr Tyr Gly Ile Gln Thr Leu Arg
               20                  25                  30
   gct tct gaa aat ttt ccg atc aca gga tac aaa atc cat gag gaa atg     144
   Ala Ser Glu Asn Phe Pro Ile Thr Gly Tyr Lys Ile His Glu Glu Met
           35                  40                  45
   att aac gca ctg gcg att gtg aaa aaa gct gcg gct ctt gcc aac atg     192
   Ile Asn Ala Leu Ala Ile Val Lys Lys Ala Ala Ala Leu Ala Asn Met
       50                  55                  60
   gac gtg aaa cgg ctg tat gaa gga att ggc caa gct atc gta caa gcc     240
   Asp Val Lys Arg Leu Tyr Glu Gly Ile Gly Gln Ala Ile Val Gln Ala
   65                  70                  75                  80
   gct gac gag att ctg gaa ggc aag tgg cac gat cag ttt atc gtc gat     288
   Ala Asp Glu Ile Leu Glu Gly Lys Trp His Asp Gln Phe Ile Val Asp
                   85                  90                  95
   ccg att cag ggc ggt gcc gga act tct atg aac atg aac gcg aat gag     336
   Pro Ile Gln Gly Gly Ala Gly Thr Ser Met Asn Met Asn Ala Asn Glu
               100                 105                 110
   gtt atc gga aac cgg gcg ctt gaa atc atg gga cat aaa aag gga gat     384
   Val Ile Gly Asn Arg Ala Leu Glu Ile Met Gly His Lys Lys Gly Asp
           115                 120                 125
   tat atc cat tta agt cca aac aca cat gtg aac atg tca cag tct cag     432
   Tyr Ile His Leu Ser Pro Asn Thr His Val Asn Met Ser Gln Ser Gln
       130                 135                 140
   aac gat gtg ttc ccg act gct atc cat att tcc aca ttg aag ctc tta     480
   Asn Asp Val Phe Pro Thr Ala Ile His Ile Ser Thr Leu Lys Leu Leu
   145                 150                 155                 160
   gaa aaa ctg ctg aaa aca atg gaa gat atg cat agt gtg ttt aaa caa     528
   Glu Lys Leu Leu Lys Thr Met Glu Asp Met His Ser Val Phe Lys Gln
                   165                 170                 175
   aaa gca cag gag ttt cac tct gtt att aaa atg ggc cgg aca cac ctt     576
   Lys Ala Gln Glu Phe His Ser Val Ile Lys Met Gly Arg Thr His Leu
               180                 185                 190
   caa gat gcg gtt ccg atc cgt ctt ggc cag gaa ttc gaa gct tac agc      624
   Gln Asp Ala Val Pro Ile Arg Leu Gly Gln Glu Phe Glu Ala Tyr Ser
           195                 200                 205
   cgt gtt ctc gag cgt gat atc aaa cga atc aag caa tcg cgc cag cac      672
   Arg Val Leu Glu Arg Asp Ile Lys Arg Ile Lys Gln Ser Arg Gln His
       210                 215                 220
   ctg tat gaa gtc aac atg ggc gca act gct gtt ggt aca ggg ctg aac      720
   Leu Tyr Glu Val Asn Met Gly Ala Thr Ala Val Gly Thr Gly Leu Asn
   225                 230                 235                 240
   gct gat cct gaa tat atc aaa cag gta gta aag cac ctt gct gat att      768
   Ala Asp Pro Glu Tyr Ile Lys Gln Val Val Lys His Leu Ala Asp Ile
                   245                 250                 255
   agc ggg ctt cct ctt gtc ggc gct gat cat ctt gtt gat gcg aca caa      816
   Ser Gly Leu Pro Leu Val Gly Ala Asp His Leu Val Asp Ala Thr Gln
               260                 265                 270
   aat aca gat gcc tat aca gag gta tca gct tca tta aaa gtc tgc atg      864
   Asn Thr Asp Ala Tyr Thr Glu Val Ser Ala Ser Leu Lys Val Cys Met
           275                 280                 285
   atg aac atg tcg aag atc gca aac gac ctg cgc tta atg gcg tcg gga      912
   Met Asn Met Ser Lys Ile Ala Asn Asp Leu Arg Leu Met Ala Ser Gly
       290                 295                 300
   ccg cgc gcc gga ctt gcg gaa att tct ctg cct gca cgt cag ccg ggt      960
   Pro Arg Ala Gly Leu Ala Glu Ile Ser Leu Pro Ala Arg Gln Pro Gly
   305                 310                 315                 320
   tca tct att atg ccg ggg aaa gtc aat ccg gtt atg gcg gag ctg atc      1008
   Ser Ser Ile Met Pro Gly Lys Val Asn Pro Val Met Ala Glu Leu Ile
                   325                 330                 335
   aac caa att gcg ttc cag gtt atc gga aat gac aat aca atc tgc ctt      1056
   Asn Gln Ile Ala Phe Gln Val Ile Gly Asn Asp Asn Thr Ile Cys Leu
               340                 345                 350
   gct tca gaa gcc ggc cag ctt gag ttg aac gtc atg gag ccc gtg ctt      1104
   Ala Ser Glu Ala Gly Gln Leu Glu Leu Asn Val Met Glu Pro Val Leu
           355                 360                 365
   gtc ttt aat ttg ctt caa tcc atc agc atc atg aac aac ggc ttc cgt      1152
   Val Phe Asn Leu Leu Gln Ser Ile Ser Ile Met Asn Asn Gly Phe Arg
       370                 375                 380
   tcg ttc act gac aac tgc tta aaa ggc att gaa gcc aac gaa aag cgt      1200
   Ser Phe Thr Asp Asn Cys Leu Lys Gly Ile Glu Ala Asn Glu Lys Arg
   385                 390                 395                 400
   atg aag caa tac gta gaa aaa agc gca ggc gtg atc aca gct gtc aat      1248
   Met Lys Gln Tyr Val Glu Lys Ser Ala Gly Val Ile Thr Ala Val Asn
                   405                 410                 415
   ccg cat ctt ggg tat gaa gcg gca gct aga att gcc agg gaa gca att      1296
   Pro His Leu Gly Tyr Glu Ala Ala Ala Arg Ile Ala Arg Glu Ala Ile
               420                 425                 430
   atg aca ggg caa tct gtc cgg gat ctt tgt ctg cag cat gat gtg ctg      1344
   Met Thr Gly Gln Ser Val Arg Asp Leu Cys Leu Gln His Asp Val Leu
           435                 440                 445
   act gaa gaa gaa ttg gat att att tta aac cca tat gag atg acc aaa      1392
   Thr Glu Glu Glu Leu Asp Ile Ile Leu Asn Pro Tyr Glu Met Thr Lys
       450                 455                 460
   cca ggt atc gca ggg aaa gaa cta tta gaa aaa taa                    1428
   Pro Gly Ile Ala Gly Lys Glu Leu Leu Glu Lys
   465                 470                 475
<210> 67
   <211> 475
   <212> PRT
   <213> Bacillus subtilis
<400> 67
   Met Leu Asn Gly Gln Lys Glu Tyr Arg Val Glu Lys Asp Phe Leu Gly
   1               5                   10                  15
   Glu Lys Gln Ile Glu Ala Asp Val Tyr Tyr Gly Ile Gln Thr Leu Arg
               20                  25                  30
   Ala Ser Glu Asn Phe Pro Ile Thr Gly Tyr Lys Ile His Glu Glu Met
           35                  40                  45
   Ile Asn Ala Leu Ala Ile Val Lys Lys Ala Ala Ala Leu Ala Asn Met
       50                  55                  60
   Asp Val Lys Arg Leu Tyr Glu Gly Ile Gly Gln Ala Ile Val Gln Ala
   65                  70                  75                  80
   Ala Asp Glu Ile Leu Glu Gly Lys Trp His Asp Gln Phe Ile Val Asp
                   85                  90                  95
   Pro Ile Gln Gly Gly Ala Gly Thr Ser Met Asn Met Asn Ala Asn Glu
               100                 105                 110
   Val Ile Gly Asn Arg Ala Leu Glu Ile Met Gly His Lys Lys Gly Asp
           115                 120                 125
   Tyr Ile His Leu Ser Pro Asn Thr His Val Asn Met Ser Gln Ser Gln
       130                 135                 140
   Asn Asp Val Phe Pro Thr Ala Ile His Ile Ser Thr Leu Lys Leu Leu
   145                 150                 155                 160
   Glu Lys Leu Leu Lys Thr Met Glu Asp Met His Ser Val Phe Lys Gln
                   165                 170                 175
   Lys Ala Gln Glu Phe His Ser Val Ile Lys Met Gly Arg Thr His Leu
               180                 185                 190
   Gln Asp Ala Val Pro Ile Arg Leu Gly Gln Glu Phe Glu Ala Tyr Ser
           195                 200                 205
   Arg Val Leu Glu Arg Asp Ile Lys Arg Ile Lys Gln Ser Arg Gln His
       210                 215                 220
   Leu Tyr Glu Val Asn Met Gly Ala Thr Ala Val Gly Thr Gly Leu Asn
   225                 230                 235                 240
   Ala Asp Pro Glu Tyr Ile Lys Gln Val Val Lys His Leu Ala Asp Ile
                   245                 250                 255
   Ser Gly Leu Pro Leu Val Gly Ala Asp His Leu Val Asp Ala Thr Gln
               260                 265                 270
   Asn Thr Asp Ala Tyr Thr Glu Val Ser Ala Ser Leu Lys Val Cys Met
           275                 280                 285
   Met Asn Met Ser Lys Ile Ala Asn Asp Leu Arg Leu Met Ala Ser Gly
       290                 295                 300
   Pro Arg Ala Gly Leu Ala Glu Ile Ser Leu Pro Ala Arg Gln Pro Gly
   305                 310                 315                 320
   Ser Ser Ile Met Pro Gly Lys Val Asn Pro Val Met Ala Glu Leu Ile
                   325                 330                 335
   Asn Gln Ile Ala Phe Gln Val Ile Gly Asn Asp Asn Thr Ile Cys Leu
               340                 345                 350
   Ala Ser Glu Ala Gly Gln Leu Glu Leu Asn Val Met Glu Pro Val Leu
           355                 360                 365
   Val Phe Asn Leu Leu Gln Ser Ile Ser Ile Met Asn Asn Gly Phe Arg
       370                 375                 380
   Ser Phe Thr Asp Asn Cys Leu Lys Gly Ile Glu Ala Asn Glu Lys Arg
   385                 390                 395                 400
   Met Lys Gln Tyr Val Glu Lys Ser Ala Gly Val Ile Thr Ala Val Asn
                   405                 410                 415
   Pro His Leu Gly Tyr Glu Ala Ala Ala Arg Ile Ala Arg Glu Ala Ile
               420                 425                 430
   Met Thr Gly Gln Ser Val Arg Asp Leu Cys Leu Gln His Asp Val Leu
           435                 440                 445
   Thr Glu Glu Glu Leu Asp Ile Ile Leu Asn Pro Tyr Glu Met Thr Lys
       450                 455                 460
   Pro Gly Ile Ala Gly Lys Glu Leu Leu Glu Lys
   465                 470                 475
<210> 68
   <211> 768
   <212> DNA
   <213> Bacillus subtilis
<220>
   <221> CDS
   <222> (1)..(765)
<400> 68
   atg aaa cga gaa agc aac att caa gtg ctc agc cgt ggt caa aaa gat    48
   Met Lys Arg Glu Ser Asn Ile Gln Val Leu Ser Arg Gly Gln Lys Asp
    1               5                  10                  15
   cag cct gtg agc cag att tat caa gta tca aca atg act tct cta tta    96
   Gln Pro Val Ser Gln Ile Tyr Gln Val Ser Thr Met Thr Ser Leu Leu
               20                  25                  30
   gac gga gta tat gac gga gat ttt gaa ctg tca gag att ccg aaa tat   144
   Asp Gly Val Tyr Asp Gly Asp Phe Glu Leu Ser Glu Ile Pro Lys Tyr
           35                  40                  45
   gga gac ttc ggt atc gga acc ttt aac aag ctt gac gga gag ctg att   192
   Gly Asp Phe Gly Ile Gly Thr Phe Asn Lys Leu Asp Gly Glu Leu Ile
       50                  55                  60
   ggg ttt gac ggc gaa ttt tac cgt ctt cgc tca gac gga acc gcg aca   240
   Gly Phe Asp Gly Glu Phe Tyr Arg Leu Arg Ser Asp Gly Thr Ala Thr
   65                  70                  75                  80
   ccg gtc caa aat gga gac cgt tca ccg ttc tgt tca ttt acg ttc ttt   288
   Pro Val Gln Asn Gly Asp Arg Ser Pro Phe Cys Ser Phe Thr Phe Phe
                   85                  90                  95
   aca ccg gac atg acg cac aaa att gat gcg aaa atg aca cgc gaa gac   336
   Thr Pro Asp Met Thr His Lys Ile Asp Ala Lys Met Thr Arg Glu Asp
               100                 105                 110
   ttt gaa aaa gag atc aac agc atg ctg cca agc aga aac tta ttt tat   384
   Phe Glu Lys Glu Ile Asn Ser Met Leu Pro Ser Arg Asn Leu Phe Tyr
           115                 120                 125
   gca att cgc att gac gga ttg ttt aaa aag gtg cag aca aga aca gta   432
   Ala Ile Arg Ile Asp Gly Leu Phe Lys Lys Val Gln Thr Arg Thr Val
       130                 135                 140
   gaa ctt caa gaa aaa cct tac gtg cca atg gtt gaa gcg gtc aaa aca   480
   Glu Leu Gln Glu Lys Pro Tyr Val Pro Met Val Glu Ala Val Lys Thr
   145                 150                 155                 160
   cag ccg att ttc aac ttc gac aac gtg aga gga acg att gta ggt ttc   528
   Gln Pro Ile Phe Asn Phe Asp Asn Val Arg Gly Thr Ile Val Gly Phe
                   165                 170                 175
   ttg aca cca gct tat gca aac gga atc gcc gtt tct ggc tat cac ctg   576
   Leu Thr Pro Ala Tyr Ala Asn Gly Ile Ala Val Ser Gly Tyr His Leu
               180                 185                 190
   cac ttc att gac gaa gga cgc aat tca ggc gga cac gtt ttt gac tat   624
   His Phe Ile Asp Glu Gly Arg Asn Ser Gly Gly His Val Phe Asp Tyr
           195                 200                 205
   gtg ctt gag gat tgc acg gtt acg att tct caa aaa atg aac atg aat   672
   Val Leu Glu Asp Cys Thr Val Thr Ile Ser Gln Lys Met Asn Met Asn
       210                 215                 220
   ctc aga ctt ccg aac aca gcg gat ttc ttt aat gcg aat ctg gat aac   720
   Leu Arg Leu Pro Asn Thr Ala Asp Phe Phe Asn Ala Asn Leu Asp Asn
   225                 230                 235                 240
   cct gat ttt gcg aaa gat atc gaa aca act gaa gga agc cct gaa taa   768
   Pro Asp Phe Ala Lys Asp Ile Glu Thr Thr Glu Gly Ser Pro Glu
                   245                 250                 255
<210> 69
   <211> 255
   <212> PRT
   <213> Bacillus subtilis
<400> 69
   Met Lys Arg Glu Ser Asn Ile Gln Val Leu Ser Arg Gly Gln Lys Asp
   1               5                   10                  15
   Gln Pro Val Ser Gln Ile Tyr Gln Val Ser Thr Met Thr Ser Leu Leu
               20                  25                  30
   Asp Gly Val Tyr Asp Gly Asp Phe Glu Leu Ser Glu Ile Pro Lys Tyr
           35                  40                  45
   Gly Asp Phe Gly Ile Gly Thr Phe Asn Lys Leu Asp Gly Glu Leu Ile
       50                  55                  60
   Gly Phe Asp Gly Glu Phe Tyr Arg Leu Arg Ser Asp Gly Thr Ala Thr
   65                  70                  75                  80
   Pro Val Gln Asn Gly Asp Arg Ser Pro Phe Cys Ser Phe Thr Phe Phe
                   85                  90                  95
   Thr Pro Asp Met Thr His Lys Ile Asp Ala Lys Met Thr Arg Glu Asp
               100                 105                 110
   Phe Glu Lys Glu Ile Asn Ser Met Leu Pro Ser Arg Asn Leu Phe Tyr
           115                 120                 125
   Ala Ile Arg Ile Asp Gly Leu Phe Lys Lys Val Gln Thr Arg Thr Val
       130                 135                 140
   Glu Leu Gln Glu Lys Pro Tyr Val Pro Met Val Glu Ala Val Lys Thr
   145                 150                 155                 160
   Gln Pro Ile Phe Asn Phe Asp Asn Val Arg Gly Thr Ile Val Gly Phe
                   165                 170                 175
   Leu Thr Pro Ala Tyr Ala Asn Gly Ile Ala Val Ser Gly Tyr His Leu
               180                 185                 190
   His Phe Ile Asp Glu Gly Arg Asn Ser Gly Gly His Val Phe Asp Tyr
           195                 200                 205
   Val Leu Glu Asp Cys Thr Val Thr Ile Ser Gln Lys Met Asn Met Asn
       210                 215                 220
   Leu Arg Leu Pro Asn Thr Ala Asp Phe Phe Asn Ala Asn Leu Asp Asn
   225                 230                 235                 240
   Pro Asp Phe Ala Lys Asp Ile Glu Thr Thr Glu Gly Ser Pro Glu
                   245                 250                 255
<210> 70
   <211> 1254
   <212> DNA
   <213> Escherichia coli
<220>
   <221> CDS
   <222> (1)..(1251)
<400> 70
   atg aca ttc tcc ctt ttt ggt gac aaa ttt acc cgc cac tcc ggc att     48
   Met Thr Phe Ser Leu Phe Gly Asp Lys Phe Thr Arg His Ser Gly Ile
     1               5                  10                  15
   acg ctg ttg atg gaa gat ctg aac gac ggt tta cgc acg cct ggc gcg     96
   Thr Leu Leu Met Glu Asp Leu Asn Asp Gly Leu Arg Thr Pro Gly Ala
                20                  25                  30
   att atg ctc ggc ggc ggt aat ccg gcg cag atc ccg gaa atg cag gac    144
   Ile Met Leu Gly Gly Gly Asn Pro Ala Gln Ile Pro Glu Met Gln Asp
            35                  40                  45
   tac ttc cag acg cta ctg acc gac atg ctg gaa agt ggc aaa gcg act    192
   Tyr Phe Gln Thr Leu Leu Thr Asp Met Leu Glu Ser Gly Lys Ala Thr
        50                  55                  60
   gat gca ctg tgt aac tac gac ggt cca cag ggg aaa acg gag cta ctc    240
   Asp Ala Leu Cys Asn Tyr Asp Gly Pro Gln Gly Lys Thr Glu Leu Leu
    65                  70                  75                  80
   aca ctg ctt gcc gga atg ctg cgc gag aag ttg ggt tgg gat atc gaa    288
   Thr Leu Leu Ala Gly Met Leu Arg Glu Lys Leu Gly Trp Asp Ile Glu
                    85                  90                  95
   cca cag aat att gca cta aca aac ggc agc cag agc gcg ttt ttc tac    336
   Pro Gln Asn Ile Ala Leu Thr Asn Gly Ser Gln Ser Ala Phe Phe Tyr
               100                 105                 110
   tta ttt aac ctg ttt gcc gga cgc cgt gcc gat ggt cgg gtc aaa aaa    384
   Leu Phe Asn Leu Phe Ala Gly Arg Arg Ala Asp Gly Arg Val Lys Lys
   115 120 125
   gtg ctg ttc ccg ctt gca ccg gaa tac att ggc tat gct gac gcc gga 432
   Val Leu Phe Pro Leu Ala Pro Glu Tyr Ile Gly Tyr Ala Asp Ala Gly
   130 135 140
   ctg gaa gaa gat ctg ttt gtc tct gcg cgt ccg aat att gaa ctg ctg 480
   Leu Glu Glu Asp Leu Phe Val Ser Ala Arg Pro Asn Ile Glu Leu Leu
   145 150 155 160
   ccg gaa ggc cag ttt aaa tac cac gtc gat ttt gag cat ctg cat att 528
   Pro Glu Gly Gln Phe Lys Tyr His Val Asp Phe Glu His Leu His Ile
   165 170 175
   ggc gaa gaa acc ggg atg att tgc gtc tcc cgg ccg acg aat cca aca 576
   Gly Glu Glu Thr Gly Met Ile Cys Val Ser Arg Pro Thr Asn Pro Thr
   180 185 190
   ggc aat gtg att act gac gaa gag ttg ctg aag ctt gac gcg ctg ggc 624
   Gly Asn Val Ile Thr Asp Glu Glu Leu Leu Lys Leu Asp Ala Leu Gly
   195 200 205
   aat caa cac ggc att ccg ctg gtg att gat aac gct tat ggc gtc ccg 672
   Asn Gln His Gly Ile Pro Leu Val Ile Asp Asn Ala Tyr Gly Val Pro
   210 215 220
   ttc ccg ggt atc atc ttc agt gaa gcg cgc ccg cta tgg aat ccg aat 720
   Phe Pro Gly Ile Ile Phe Ser Glu Ala Arg Pro Leu Trp Asn Pro Asn
   225 230 235 240
   atc gtg ctg tgc atg agt ctt tcc aag ctg ggt cta cct ggc tcc cgc 768
   Ile Val Leu Cys Met Ser Leu Ser Lys Leu Gly Leu Pro Gly Ser Arg
   245 250 255
   tgc ggc att atc atc gcc aat gaa aaa atc atc acc gcc atc acc aat 816
   Cys Gly Ile Ile Ile Ala Asn Glu Lys Ile Ile Thr Ala Ile Thr Asn
   260 265 270
   atg aac ggc att atc agc ctg gca cct ggc ggt att ggt ccg gcg atg 864
   Met Asn Gly Ile Ile Ser Leu Ala Pro Gly Gly Ile Gly Pro Ala Met
   275 280 285
   atg tgt gaa atg att aag cgt aac gat ctg ctg cgc ctg tct gaa aca 912
   Met Cys Glu Met Ile Lys Arg Asn Asp Leu Leu Arg Leu Ser Glu Thr
   290 295 300
   gtc atc aaa ccg ttt tac tac cag cgt gtt cag gaa act atc gcc atc 960
   Val Ile Lys Pro Phe Tyr Tyr Gln Arg Val Gln Glu Thr Ile Ala Ile
   305 310 315 320
   att cgc cgc tat tta ccg gaa aat cgc tgc ctg att cat aaa ccg gaa 1008
   Ile Arg Arg Tyr Leu Pro Glu Asn Arg Cys Leu Ile His Lys Pro Glu
   325 330 335
   gga gcc att ttc ctc tgg cta tgg ttt aag gat ttg ccc att acg acc 1056
   Gly Ala Ile Phe Leu Trp Leu Trp Phe Lys Asp Leu Pro Ile Thr Thr
   340 345 350
   aag cag ctc tat cag cgc ctg aaa gca cgc ggc gtg ctg atg gtg ccg 1104
   Lys Gln Leu Tyr Gln Arg Leu Lys Ala Arg Gly Val Leu Met Val Pro
   355 360 365
   ggg cac aac ttc ttc cca ggg ctg gat aaa ccg tgg ccg cat acg cat 1152
   Gly His Asn Phe Phe Pro Gly Leu Asp Lys Pro Trp Pro His Thr His
   370 375 380
   caa tgt atg cgc atg aac tac gta cca gag ccg gag aaa att gag gcg 1200
   Gln Cys Met Arg Met Asn Tyr Val Pro Glu Pro Glu Lys Ile Glu Ala
   385                 390                 395                 400
   ggg gtg aag att ctg gcg gaa gag ata gaa aga gcc tgg gct gaa agt    1248
   Gly Val Lys Ile Leu Ala Glu Glu Ile Glu Arg Ala Trp Ala Glu Ser
                   405                 410                 415
   cac taa                                                            1254
   His
<210> 71
   <211> 417
   <212> PRT
   <213> Escherichia coli
<400> 71
   Met Thr Phe Ser Leu Phe Gly Asp Lys Phe Thr Arg His Ser Gly Ile
    1               5                  10                  15
   Thr Leu Leu Met Glu Asp Leu Asn Asp Gly Leu Arg Thr Pro Gly Ala
               20                  25                  30
   Ile Met Leu Gly Gly Gly Asn Pro Ala Gln Ile Pro Glu Met Gln Asp
           35                  40                  45
   Tyr Phe Gln Thr Leu Leu Thr Asp Met Leu Glu Ser Gly Lys Ala Thr
       50                  55                  60
   Asp Ala Leu Cys Asn Tyr Asp Gly Pro Gln Gly Lys Thr Glu Leu Leu
   65                  70                  75                  80
   Thr Leu Leu Ala Gly Met Leu Arg Glu Lys Leu Gly Trp Asp Ile Glu
                   85                  90                  95
   Pro Gln Asn Ile Ala Leu Thr Asn Gly Ser Gln Ser Ala Phe Phe Tyr
               100                 105                 110
   Leu Phe Asn Leu Phe Ala Gly Arg Arg Ala Asp Gly Arg Val Lys Lys
           115                 120                 125
   Val Leu Phe Pro Leu Ala Pro Glu Tyr Ile Gly Tyr Ala Asp Ala Gly
       130                 135                 140
   Leu Glu Glu Asp Leu Phe Val Ser Ala Arg Pro Asn Ile Glu Leu Leu
   145                 150                 155                 160
   Pro Glu Gly Gln Phe Lys Tyr His Val Asp Phe Glu His Leu His Ile
                   165                 170                 175
   Gly Glu Glu Thr Gly Met Ile Cys Val Ser Arg Pro Thr Asn Pro Thr
               180                 185                 190
   Gly Asn Val Ile Thr Asp Glu Glu Leu Leu Lys Leu Asp Ala Leu Gly
           195                 200                 205
   Asn Gln His Gly Ile Pro Leu Val Ile Asp Asn Ala Tyr Gly Val Pro
       210                 215                 220
   Phe Pro Gly Ile Ile Phe Ser Glu Ala Arg Pro Leu Trp Asn Pro Asn
   225                 230                 235                 240
   Ile Val Leu Cys Met Ser Leu Ser Lys Leu Gly Leu Pro Gly Ser Arg
                   245                 250                 255
   Cys Gly Ile Ile Ile Ala Asn Glu Lys Ile Ile Thr Ala Ile Thr Asn
               260                 265                 270
   Met Asn Gly Ile Ile Ser Leu Ala Pro Gly Gly Ile Gly Pro Ala Met
           275                 280                 285
   Met Cys Glu Met Ile Lys Arg Asn Asp Leu Leu Arg Leu Ser Glu Thr
       290                 295                 300
   Val Ile Lys Pro Phe Tyr Tyr Gln Arg Val Gln Glu Thr Ile Ala Ile
   305                 310                 315                 320
   Ile Arg Arg Tyr Leu Pro Glu Asn Arg Cys Leu Ile His Lys Pro Glu
                   325                 330                 335
   Gly Ala Ile Phe Leu Trp Leu Trp Phe Lys Asp Leu Pro Ile Thr Thr
               340                 345                 350
   Lys Gln Leu Tyr Gln Arg Leu Lys Ala Arg Gly Val Leu Met Val Pro
           355                 360                 365
   Gly His Asn Phe Phe Pro Gly Leu Asp Lys Pro Trp Pro His Thr His
       370                 375                 380
   Gln Cys Met Arg Met Asn Tyr Val Pro Glu Pro Glu Lys Ile Glu Ala
   385                 390                 395                 400
   Gly Val Lys Ile Leu Ala Glu Glu Ile Glu Arg Ala Trp Ala Glu Ser
                   405                 410                 415
   His
<210> 72
   <211> 8803
   <212> DNA
   <213> Artificial Sequence
<220>
   <223> Description of Artificial Sequence: Recombinant pAN294 plasmid
<400> 72
   tgcgccgcta cagggcgcgt ccattcgcca ttcaggctgc gcaactgttg ggaagggcga 60
   tcggtgcggg cctcttcgct attacgccag ctggcgaaag ggggatgtgc tgcaaggcga 120
   ttaagttggg taacgccagg gttttcccag tcacgacgtt gtaaaacgac ggccagtgaa 180
   ttgtaatacg actcactata gggcgaattg ggcccgacgt cgcatgctgg atgaaaagcc 240
   gatgaccgct tttcaggtct gtcagcagct ttttcctgct gtatatgaaa aggaattgtt 300
   tttaacgatg tcagaaacgg caggtcacct tgatgtgttg gaggctgaag aagccatcac 360
   gtcatattgg gaaggaaata ccgtatactt taaaacaatg aagaggtgaa atgggtgaaa 420
   catatagcgg gaaaaaggat ttggataacc ggcgcttcag gagggcttgg agaaagaatc 480
   gcatacttat gcgcggctga aggagcccat gtcctgctgt cggctagacg cgaggatcgt 540
   ttgatagaaa tcaaaaggaa aataaccgag gaatggagcg gacagtgtga gatttttcct 600
   ctggatgtcg gccgcctaga ggatatcgcc cgggtccgcg atcagatcgg ctcgattgat 660
   gtactgatta acaatgcagg cttcggtata tttgaaacgg ttttagactc tacattggat 720
   gacatgaaag cgatgtttga tgtgaatgtc ttcggcctga tcgcctgtac aaaagcggtg 780
   cttccgcaaa tgcttgagca aaaaaaggga catatcatca atatcgcctc tcaagcgggg 840
   aaaatcgcca caccgaagtc tagcctgtat tccgcgacca aacatgccgt gttaggttac 900
   tcaaacgctt tgcggatgga gctttcggga accggcattt atgtgacaac agtcaacccg 960
   ggcccgattc agacggactt tttttccatt gctgataaag gcggggacta cgccaaaaat 1020
   gtcggccgct ggatgcttga tcctgatgac gtggcagctc aaattacagc tgcaattttt 1080
   acgaaaaagc gggagatcaa tcttccgcgt ttaatgaatg ccggcactaa gctgtatcag 1140
   ctgtttccag ctcttgtaga aaagctggca ggacgcgcgc tcatgaaaaa ataatgatag 1200
   aactgcctgt ggtggagtgg cttgtttctc acggggcagt ttttgatagt ggaagggaga 1260
   gattgttgaa tgtcagttca ttcagaagtc cttcatgctc tgcttaaaga tccgtttatt 1320
   cagaaactga ttgatgcaga gcctgtattc tgggcaaatt caggcaagaa agaggggcca 1380
   ttaccccgtg cagatgagtg ggcaaccgag atagcggaag cggaaaaaag aatgcagcgg 1440
   tttgcacctt acattgccga ggtgtttcct gagacgaaag gcgctaaagg aatcatcgag 1500
   tctccgcttt ttgaggtgca gcatatgaag ggaaagctgg aagcggcata tcagcagcca 1560
   tttcccggaa gatggctttt aaagtgcgac catgagcttc cgatttcagg atcgattaaa 1620
   gcgaggggcg ggatttatga agtgttaaag tatgctgaaa atctcgcgct tcaagaagga 1680
   atgcttcagg aaaccgatga ttaccgcatc ttacaggaag agcggtttac cgggtttttc 1740
   tcccgctatt cgattgctgt cggttcgaca ggaaatctag gtttaagcat cggcatcatc 1800
   ggcgcggcac tcgggtttcg cgtgacagtg catatgtccg ccgatgctaa gcagtggaaa 1860
   aaggatctcc tccgccaaaa gggagtcact gttatggagt acgaaacaga ttacagtgaa 1920
   gcggtgaacg aagggagacg gcaggcggaa caagatccat tctgttattt tattgatgat 1980
   gaacattctc gtcagctgtt cttaggatat gctgttgctg caagccgatt aaaaacacag 2040
   cttgactgta tgaatataaa gccaagtctt gagacgccct tgtttgtgta tctgccgtgc 2100
   ggagtcggcg gaggaccggg cggtgtagca tttgggctga agcttttata cggagatgat 2160
   gttcatgtgt ttttcgcaga accaactcat tcaccttgta tgctgttagg gctttattca 2220
   ggacttcacg agaagatctc cgtccaggat atcggcctgg ataatcagac ggctgctgac 2280
   ggacttgccg tagggaggcc gtcaggattt gtcggcaagc tgattgaacc gcttctgagc 2340
   ggctgttata cggtagagga caatacgctt tatactttgc ttcatatgct ggctgtatct 2400
   gaagataaat atttagagcc ctctgctctt gctggcatgt tcgggccggt tcagcttttt 2460
   tcgacagaag agggaaggcg ctatgctcag aaatataaga tggaacatgc cgtacatgtc 2520
   gtctggggaa cgggaggaag catggttcca aaagatgaaa tggctgcgta taaccgaatc 2580
   ggtgctgatt tgctaaaaaa acgaaatgga aaataagcag acagtgaaaa ggttttccgt 2640
   tacaatcttt gtaagggttt taacctacag agagtcaggt gtaaacagtg aaaaataaag 2700
   aacttaacct acatacttta tatacacagc acaatcggga gtcttggtct ggttttgggg 2760
   ggcatttgtc gattgctgta tctgaagaag aggcaaaagc tgtggaagga ttgaatgatt 2820
   atctatctgt tgaagaagtg gagacgatct atattccgct tgttcgcttg cttcatttac 2880
   atgtcaagtc tgcggctgaa cgcaataagc atgtcaatgt ttttttgaag cacccacatt 2940
   cagccaaaat tccgtttatt atcggcattg ccggcagtgt cgcagtcgga aaaagcacga 3000
   cggcgcggat cttgcagaag ctgctttcgc gtttgcctga ccgtccaaaa gtgagcctta 3060
   tcacgacaga tggtttttta tttcctactg ccgagctgaa aaagaaaaat atgatgtcaa 3120
   gaaaaggatt tcctgaaagc tatgatgtaa aggcgctgct cgaatttttg aatgacttaa 3180
   aatcaggaaa ggacagcgta aaggccccgg tgtattccca tctaacctat gaccgcgagg 3240
   aaggtgtgtt cgaggttgta gaacaggcgg atattgtgat tattgaaggc attaatgttc 3300
   ttcagtcgcc caccttggag gatgaccggg aaaacccgcg tatttttgtt tccgatttct 3360
   ttgatttttc gatttatgtg gatgcggagg aaagccggat tttcacttgg tatttagagc 3420
   gttttcgcct gcttcgggaa acagcttttc aaaatcctga ttcatatttt cataaattta 3480
   aagacttgtc cgatcaggag gctgacgaga tggcagcctc gatttgggag agtgtcaacc 3540
   ggccgaattt atatgaaaat attttgccaa ctaaattcag gtcagatctc attttgcgta 3600
   agggagacgg gcataaggtc gaggaagtgt tggtaaggag ggtatgaaat gtgctgcagc 3660
   tcgagcaata gttaccctta ttatcaagat aagaaagaaa aggatttttc gctacgctca 3720
   aatcctttaa aaaaacacaa aagaccacat tttttaatgt ggtctttatt cttcaactaa 3780
   agcacccatt agttcaacaa acgaaaattg gataaagtgg gatattttta aaatatatat 3840
   ttatgttaca gtaatattga cttttaaaaa aggattgatt ctaatgaaga aagcagacaa 3900
   gtaagcctcc taaattcact ttagataaaa atttaggagg catatcaaat gaactttaat 3960
   aaaattgatt tagacaattg gaagagaaaa gagatattta atcattattt gaaccaacaa 4020
   acgactttta gtataaccac agaaattgat attagtgttt tataccgaaa cataaaacaa 4080
   gaaggatata aattttaccc tgcatttatt ttcttagtga caagggtgat aaactcaaat 4140
   acagctttta gaactggtta caatagcgac ggagagttag gttattggga taagttagag 4200
   ccactttata caatttttga tggtgtatct aaaacattct ctggtatttg gactcctgta 4260
   aagaatgact tcaaagagtt ttatgattta tacctttctg atgtagagaa atataatggt 4320
   tcggggaaat tgtttcccaa aacacctata cctgaaaatg ctttttctct ttctattatt 4380
   ccatggactt catttactgg gtttaactta aatatcaata ataatagtaa ttaccttcta 4440
   cccattatta cagcaggaaa attcattaat aaaggtaatt caatatattt accgctatct 4500
   ttacaggtac atcattctgt ttgtgatggt tatcatgcag gattgtttat gaactctatt 4560
   caggaattgt cagataggcc taatgactgg cttttataat atgagataat gccgactgta 4620
   ctttttacag tcggttttct aatgtcacta acctgccccg ttagttgaag aaggttttta 4680
   tattacagct gtcgactcgt gatcttcgga caggctgttc agctttttct caatgcgatc 4740
   cagctgcgct tttcggtttt tcgcatactt gaagcctgta acagccgcaa agacgacagc 4800
   ggcaaatata ataaatacaa acagctgaaa catcacatca cctatattca tgttcttcac 4860
   ctcatgtttg cgggagagat tcattctctt ccgtttttta tttaaagcgg cttttccaga 4920
   cgggaacggt gttttgtggt ctccattttc atttgccgat aggcgaacgc taaaaatggc 4980
   aggccgagca gggtaatgcc gctcaggaca gaaaaaatat aaatcggccg gccagcgcca 5040
   aacaggtcta tacatatccc cccgacccaa gggccgatga cgtttccgag ctgtggaaaa 5100
   ccgattgccc cgaaataagt gccttttaat cctggttttg caatctggtc tacatacaaa 5160
   tccatcatag agaataaaag cacttcgccg attgtaaatg tgatgacaat catcacaatt 5220
   gatggaacac cgtgtgatac ggtgaaaatg gccatgctga tgctaaccat cacattaccg 5280
   agcatcagag aacaaagcgg cgaaaaccgt tttgcaaaat ggacaatggg aaattgcgtc 5340
   gccaacacaa cgattgcgtt taatgtcagc atcagcccat acagcttcgt tccattgccg 5400
   atcaaggggt tctgcgccat atactgaggg aatgtggaac tgaattgtga gtagccgaag 5460
   gtgcatagcg taatgccgac caaagcaatg gtaaaaagat aatccttttg cgtgaccata 5520
   aacgcttccc gcacgctcat atttcgggac tgggctggtg ctgataagga tggatgtttt 5580
   ttaaattgga gggcaagcac aattccgtat agtccgtaaa tgactgcagg caccaaaaag 5640
   ggcgtagtcg attgcgatga gccgaaatat aggccaagca caggtccgaa gacaacgccg 5700
   atattaatag ccgcatagcg taaattaaaa actagcagtc tcgttttttc ttctgtcata 5760
   tcagacaaca aggcctttga agcgggctca aacagtgatt tgcaaagacc gtttaatgcg 5820
   tttactacaa aaaacaccca gagattagat gctgccgcaa agcctgcaaa taccagcatc 5880
   catccgaaaa tcgatacaag catcatgttt tttctgccga atttatctga gatatatccg 5940
   ccgtaaaagc ttgcgaggat gccgactgat gagctcgcgg cgatgaccag ccctgcatag 6000
   gaagctgatg cgccttggac ggctgtcaaa taaatcgcta aaaaaggaat gctcatcgat 6060
   gttgccattc tgccgaaaat ggttccgatt ataattgtac gcgttggatg catagcttga 6120
   gtattctata gtgtcaccta aatagcttgg cgtaatcatg gtcatagctg tttcctgtgt 6180
   gaaattgtta tccgctcaca attccacaca acatacgagc cggaagcata aagtgtaaag 6240
   cctggggtgc ctaatgagtg agctaactca cattaattgc gttgcgctca ctgcccgctt 6300
   tccagtcggg aaacctgtcg tgccagctgc attaatgaat cggccaacgc gcggggagag 6360
   gcggtttgcg tattgggcgc tcttccgctt cctcgctcac tgactcgctg cgctcggtcg 6420
   ttcggctgcg gcgagcggta tcagctcact caaaggcggt aatacggtta tccacagaat 6480
   caggggataa cgcaggaaag aacatgtgag caaaaggcca gcaaaaggcc aggaaccgta 6540
   aaaaggccgc gttgctggcg tttttcgata ggctccgccc ccctgacgag catcacaaaa 6600
   atcgacgctc aagtcagagg tggcgaaacc cgacaggact ataaagatac caggcgtttc 6660
   cccctggaag ctccctcgtg cgctctcctg ttccgaccct gccgcttacc ggatacctgt 6720
   ccgcctttct cccttcggga agcgtggcgc tttctcatag ctcacgctgt aggtatctca 6780
   gttcggtgta ggtcgttcgc tccaagctgg gctgtgtgca cgaacccccc gttcagcccg 6840
   accgctgcgc cttatccggt aactatcgtc ttgagtccaa cccggtaaga cacgacttat 6900
   cgccactggc agcagccact ggtaacagga ttagcagagc gaggtatgta ggcggtgcta 6960
   cagagttctt gaagtggtgg cctaactacg gctacactag aaggacagta tttggtatct 7020
   gcgctctgct gaagccagtt accttcggaa aaagagttgg tagctcttga tccggcaaac 7080
   aaaccaccgc tggtagcggt ggtttttttg tttgcaagca gcagattacg cgcagaaaaa 7140
   aaggatctca agaagatcct ttgatctttt ctacggggtc tgacgctcag tggaacgaaa 7200
   actcacgtta agggattttg gtcatgagat tatcaaaaag gatcttcacc tagatccttt 7260
   taaattaaaa atgaagtttt aaatcaatct aaagtatata tgagtaaact tggtctgaca 7320
   gttaccaatg cttaatcagt gaggcaccta tctcagcgat ctgtctattt cgttcatcca 7380
   tagttgcctg actccccgtc gtgtagataa ctacgatacg ggagggctta ccatctggcc 7440
   ccagtgctgc aatgataccg cgagacccac gctcaccggc tccagattta tcagcaataa 7500
   accagccagc cggaagggcc gagcgcagaa gtggtcctgc aactttatcc gcctccatcc 7560
   agtctattaa ttgttgccgg gaagctagag taagtagttc gccagttaat agtttgcgca 7620
   acgttgttgg cattgctaca ggcatcgtgg tgtcacgctc gtcgtttggt atggcttcat 7680
   tcagctccgg ttcccaacga tcaaggcgag ttacatgatc ccccatgttg tgcaaaaaag 7740
   cggttagctc cttcggtcct ccgatcgttg tcagaagtaa gttggccgca gtgttatcac 7800
   tcatggttat ggcagcactg cataattctc ttactgtcat gccatccgta agatgctttt 7860
   ctgtgactgg tgagtactca accaagtcat tctgagaata ccgcgcccgg cgaccgagtt 7920
   gctcttgccc ggcgtcaata cgggataata gtgtatgaca tagcagaact ttaaaagtgc 7980
   tcatcattgg aaaacgttct tcggggcgaa aactctcaag gatcttaccg ctgttgagat 8040
   ccagttcgat gtaacccact cgtgcaccca actgatcttc agcatctttt actttcacca 8100
   gcgtttctgg gtgagcaaaa acaggaaggc aaaatgccgc aaaaaaggga ataagggcga 8160
   cacggaaatg ttgaatactc atactcttcc tttttcaata ttattgaagc atttatcagg 8220
   gttattgtct catgagcgga tacatatttg aatgtattta gaaaaataaa caaatagggg 8280
   ttccgcgcac atttccccga aaagtgccac ctgtatgcgg tgtgaaatac cgcacagatg 8340
   cgtaaggaga aaataccgca tcaggcgaaa ttgtaaacgt taatattttg ttaaaattcg 8400
   cgttaaatat ttgttaaatc agctcatttt ttaaccaata ggccgaaatc ggcaaaatcc 8460
   cttataaatc aaaagaatag accgagatag ggttgagtgt tgttccagtt tggaacaaga 8520
   gtccactatt aaagaacgtg gactccaacg tcaaagggcg aaaaaccgtc tatcagggcg 8580
   atggcccact acgtgaacca tcacccaaat caagtttttt gcggtcgagg tgccgtaaag 8640
   ctctaaatcg gaaccctaaa gggagccccc gatttagagc ttgacgggga aagccggcga 8700
   acgtggcgag aaaggaaggg aagaaagcga aaggagcggg cgctagggcg ctggcaagtg 8760
   tagcggtcac gctgcgcgta accaccacac ccgccgcgct taa 8803
<210> 73
   <211> 8320
   <212> DNA
   <213> Artificial Sequence
<220>
   <223> Description of Artificial Sequence: Recombinant pAN296 plasmid
<400> 73
   tgcgccgcta cagggcgcgt ccattcgcca ttcaggctgc gcaactgttg ggaagggcga 60
   tcggtgcggg cctcttcgct attacgccag ctggcgaaag ggggatgtgc tgcaaggcga 120
   ttaagttggg taacgccagg gttttcccag tcacgacgtt gtaaaacgac ggccagtgaa 180
   ttgtaatacg actcactata gggcgaattg ggcccgacgt cgcatgctgg atgaaaagcc 240
   gatgaccgct tttcaggtct gtcagcagct ttttcctgct gtatatgaaa aggaattgtt 300
   tttaacgatg tcagaaacgg caggtcacct tgatgtgttg gaggctgaag aagccatcac 360
   gtcatattgg gaaggaaata ccgtatactt taaaacaatg aagaggtgaa atgggtgaaa 420
   catatagcgg gaaaaaggat ttggataacc ggcgcttcag gagggcttgg agaaagaatc 480
   gcatacttat gcgcggctga aggagcccat gtcctgctgt cggctagacg cgaggatcgt 540
   ttgatagaaa tcaaaaggaa aataaccgag gaatggagcg gacagtgtga gatttttcct 600
   ctggatgtcg gccgcctaga ggatatcgcc cgggtccgcg atcagatcgg ctcgattgat 660
   gtactgatta acaatgcagg cttcggtata tttgaaacgg ttttagactc tacattggat 720
   gacatgaaag cgatgtttga tgtgaatgtc ttcggcctga tcgcctgtac aaaagcggtg 780
   cttccgcaaa tgcttgagca aaaaaaggga catatcatca atatcgcctc tcaagcgggg 840
   aaaatcgcca caccgaagtc tagcctgtat tccgcgacca aacatgccgt gttaggttac 900
   tcaaacgctt tgcggatgga gctttcggga accggcattt atgtgacaac agtcaacccg 960
   ggcccgattc agacggactt tttttccatt gctgataaag gcggggacta cgccaaaaat 1020
   gtcggccgct ggatgcttga tcctgatgac gtggcagctc aaattacagc tgcaattttt 1080
   acgaaaaagc gggagatcaa tcttccgcgt ttaatgaatg ccggcactaa gctgtatcag 1140
   ctgtttccag ctcttgtaga aaagctggca ggacgcgcgc tcatgaaaaa ataatgatag 1200
   aactgcctgt ggtggagtgg cttgtttctc acggggcagt ttttgatagt ggaagggaga 1260
   gattgttgaa tgtcagttca ttcagaagtc cttcatgctc tgcttaaaga tccgtttatt 1320
   cagaaactga ttgatgcaga gcctgtattc tgggcaaatt caggcaagaa agaggggcca 1380
   ttaccccgtg cagatgagtg ggcaaccgag atagcggaag cggaaaaaag aatgcagcgg 1440
   tttgcacctt acattgccga ggtgtttcct gagacgaaag gcgctaaagg aatcatcgag 1500
   tctccgcttt ttgaggtgca gcatatgaag ggaaagctgg aagcggcata tcagcagcca 1560
   tttcccggaa gatggctttt aaagtgcgac catgagcttc cgatttcagg atcgattaaa 1620
   gcgaggggcg ggatttatga agtgttaaag tatgctgaaa atctcgcgct tcaagaagga 1680
   atgcttcagg aaaccgatga ttaccgcatc ttacaggaag agcggtttac cgggtttttc 1740
   tcccgctatt cgattgctgt cggttcgaca ggaaatctag gtttaagcat cggcatcatc 1800
   ggcgcggcac tcgggtttcg cgtgacagtg catatgtccg ccgatgctaa gcagtggaaa 1860
   aaggatctcc tccgccaaaa gggagtcact gttatggagt acgaaacaga ttacagtgaa 1920
   gcggtgaacg aagggagacg gcaggcggaa caagatccat tctgttattt tattgatgat 1980
   gaacattctc gtcagctgtt cttaggatat gctgttgctg caagccgatt aaaaacacag 2040
   cttgactgta tgaatataaa gccaagtctt gagacgccct tgtttgtgta tctgccgtgc 2100
   ggagtcggcg gaggaccggg cggtgtagca tttgggctga agcttttata cggagatgat 2160
   gttcatgtgt ttttcgcaga accaactcat tcaccttgta tgctgttagg gctttattca 2220
   ggacttcacg agaagatctc cgtccaggat atcggcctgg ataatcagac ggctgctgac 2280
   ggacttgccg tagggaggcc gtcaggattt gtcggcaagc tgattgaacc gcttctgagc 2340
   ggctgttata cggtagagga caatacgctt tatactttgc ttcatatgct ggctgtatct 2400
   gaagataaat atttagagcc ctctgctctt gctggcatgt tcgggccggt tcagcttttt 2460
   tcgacagaag agggaaggcg ctatgctcag aaatataaga tggaacatgc cgtacatgtc 2520
   gtctggggaa cgggaggaag catggttcca aaagatgaaa tggctgcgta taaccgaatc 2580
   ggtgctgatt tgctaaaaaa acgaaatgga aaataagcag acagtgaaaa ggttttccgt 2640
   tacaatcttt gtaagggttt taacctacag agagtcaggt gtaaacagtg aaaaataaag 2700
   aacttaacct acatacttta tatacacagc acaatcggga gtcttctgca gctcgagcaa 2760
   tagttaccct tattatcaag ataagaaaga aaaggatttt tcgctacgct caaatccttt 2820
   aaaaaaacac aaaagaccac attttttaat gtggtcttta ttcttcaact aaagcaccca 2880
   ttagttcaac aaacgaaaat tggataaagt gggatatttt taaaatatat atttatgtta 2940
   cagtaatatt gacttttaaa aaaggattga ttctaatgaa gaaagcagac aagtaagcct 3000
   cctaaattca ctttagataa aaatttagga ggcatatcaa atgaacttta ataaaattga 3060
   tttagacaat tggaagagaa aagagatatt taatcattat ttgaaccaac aaacgacttt 3120
   tagtataacc acagaaattg atattagtgt tttataccga aacataaaac aagaaggata 3180
   taaattttac cctgcattta ttttcttagt gacaagggtg ataaactcaa atacagcttt 3240
   tagaactggt tacaatagcg acggagagtt aggttattgg gataagttag agccacttta 3300
   tacaattttt gatggtgtat ctaaaacatt ctctggtatt tggactcctg taaagaatga 3360
   cttcaaagag ttttatgatt tatacctttc tgatgtagag aaatataatg gttcggggaa 3420
   attgtttccc aaaacaccta tacctgaaaa tgctttttct ctttctatta ttccatggac 3480
   ttcatttact gggtttaact taaatatcaa taataatagt aattaccttc tacccattat 3540
   tacagcagga aaattcatta ataaaggtaa ttcaatatat ttaccgctat ctttacaggt 3600
   acatcattct gtttgtgatg gttatcatgc aggattgttt atgaactcta ttcaggaatt 3660
   gtcagatagg cctaatgact ggcttttata atatgagata atgccgactg tactttttac 3720
   agtcggtttt ctaatgtcac taacctgccc cgttagttga agaaggtttt tatattacag 3780
   ctgtcgacta aggtcgagga agtgttggta aggagggtat gaaatgtgca tcatattgaa 3840
   ctgtatgtct ctgatttgga ggcgtctagg cggttttggg gctggttctt aaaagaactt 3900
   ggttataaag agtatcaaaa atggagctca ggcatcagct ggaagaaaga tcgtttttac 3960
   ctagtgattg tgcaggcgaa agagccattt ctagagccgg aataccatag atgccgagtc 4020
   ggtctgaacc atctcgcatt tcatgctgaa tccaagcttc aagtcgatca gatgactgaa 4080
   aaattgacgg caaaaggcta tcgtgtgttg taccgagaca ggcatccttt tgccggagga 4140
   gacgggcatt atgcagtctt ttgtgaggat ccagaccgga ttaaggtaga gctcgttgcc 4200
   ccaagctgtt aatcgtgatc ttcggacagg ctgttcagct ttttctcaat gcgatccagc 4260
   tgcgcttttc ggtttttcgc atacttgaag cctgtaacag ccgcaaagac gacagcggca 4320
   aatataataa atacaaacag ctgaaacatc acatcaccta tattcatgtt cttcacctca 4380
   tgtttgcggg agagattcat tctcttccgt tttttattta aagcggcttt tccagacggg 4440
   aacggtgttt tgtggtctcc attttcattt gccgataggc gaacgctaaa aatggcaggc 4500
   cgagcagggt aatgccgctc aggacagaaa aaatataaat cggccggcca gcgccaaaca 4560
   ggtctataca tatccccccg acccaagggc cgatgacgtt tccgagctgt ggaaaaccga 4620
   ttgccccgaa ataagtgcct tttaatcctg gttttgcaat ctggtctaca tacaaatcca 4680
   tcatagagaa taaaagcact tcgccgattg taaatgtgat gacaatcatc acaattgatg 4740
   gaacaccgtg tgatacggtg aaaatggcca tgctgatgct aaccatcaca ttaccgagca 4800
   tcagagaaca aagcggcgaa aaccgttttg caaaatggac aatgggaaat tgcgtcgcca 4860
   acacaacgat tgcgtttaat gtcagcatca gcccatacag cttcgttcca ttgccgatca 4920
   aggggttctg cgccatatac tgagggaatg tggaactgaa ttgtgagtag ccgaaggtgc 4980
   atagcgtaat gccgaccaaa gcaatggtaa aaagataatc cttttgcgtg accataaacg 5040
   cttcccgcac gctcatattt cgggactggg ctggtgctga taaggatgga tgttttttaa 5100
   attggagggc aagcacaatt ccgtatagtc cgtaaatgac tgcaggcacc aaaaagggcg 5160
   tagtcgattg cgatgagccg aaatataggc caagcacagg tccgaagaca acgccgatat 5220
   taatagccgc atagcgtaaa ttaaaaacta gcagtctcgt tttttcttct gtcatatcag 5280
   acaacaaggc ctttgaagcg ggctcaaaca gtgatttgca aagaccgttt aatgcgttta 5340
   ctacaaaaaa cacccagaga ttagatgctg ccgcaaagcc tgcaaatacc agcatccatc 5400
   cgaaaatcga tacaagcatc atgttttttc tgccgaattt atctgagata tatccgccgt 5460
   aaaagcttgc gaggatgccg actgatgagc tcgcggcgat gaccagccct gcataggaag 5520
   ctgatgcgcc ttggacggct gtcaaataaa tcgctaaaaa aggaatgctc atcgatgttg 5580
   ccattctgcc gaaaatggtt ccgattataa ttgtaacgcg ttggatgcat agcttgagta 5640
   ttctatagtg tcacctaaat agcttggcgt aatcatggtc atagctgttt cctgtgtgaa 5700
   attgttatcc gctcacaatt ccacacaaca tacgagccgg aagcataaag tgtaaagcct 5760
   ggggtgccta atgagtgagc taactcacat taattgcgtt gcgctcactg cccgctttcc 5820
   agtcgggaaa cctgtcgtgc cagctgcatt aatgaatcgg ccaacgcgcg gggagaggcg 5880
   gtttgcgtat tgggcgctct tccgcttcct cgctcactga ctcgctgcgc tcggtcgttc 5940
   ggctgcggcg agcggtatca gctcactcaa aggcggtaat acggttatcc acagaatcag 6000
   gggataacgc aggaaagaac atgtgagcaa aaggccagca aaaggccagg aaccgtaaaa 6060
   aggccgcgtt gctggcgttt ttcgataggc tccgcccccc tgacgagcat cacaaaaatc 6120
   gacgctcaag tcagaggtgg cgaaacccga caggactata aagataccag gcgtttcccc 6180
   ctggaagctc cctcgtgcgc tctcctgttc cgaccctgcc gcttaccgga tacctgtccg 6240
   cctttctccc ttcgggaagc gtggcgcttt ctcatagctc acgctgtagg tatctcagtt 6300
   cggtgtaggt cgttcgctcc aagctgggct gtgtgcacga accccccgtt cagcccgacc 6360
   gctgcgcctt atccggtaac tatcgtcttg agtccaaccc ggtaagacac gacttatcgc 6420
   cactggcagc agccactggt aacaggatta gcagagcgag gtatgtaggc ggtgctacag 6480
   agttcttgaa gtggtggcct aactacggct acactagaag gacagtattt ggtatctgcg 6540
   ctctgctgaa gccagttacc ttcggaaaaa gagttggtag ctcttgatcc ggcaaacaaa 6600
   ccaccgctgg tagcggtggt ttttttgttt gcaagcagca gattacgcgc agaaaaaaag 6660
   gatctcaaga agatcctttg atcttttcta cggggtctga cgctcagtgg aacgaaaact 6720
   cacgttaagg gattttggtc atgagattat caaaaaggat cttcacctag atccttttaa 6780
   attaaaaatg aagttttaaa tcaatctaaa gtatatatga gtaaacttgg tctgacagtt 6840
   accaatgctt aatcagtgag gcacctatct cagcgatctg tctatttcgt tcatccatag 6900
   ttgcctgact ccccgtcgtg tagataacta cgatacggga gggcttacca tctggcccca 6960
   gtgctgcaat gataccgcga gacccacgct caccggctcc agatttatca gcaataaacc 7020
   agccagccgg aagggccgag cgcagaagtg gtcctgcaac tttatccgcc tccatccagt 7080
   ctattaattg ttgccgggaa gctagagtaa gtagttcgcc agttaatagt ttgcgcaacg 7140
   ttgttggcat tgctacaggc atcgtggtgt cacgctcgtc gtttggtatg gcttcattca 7200
   gctccggttc ccaacgatca aggcgagtta catgatcccc catgttgtgc aaaaaagcgg 7260
   ttagctcctt cggtcctccg atcgttgtca gaagtaagtt ggccgcagtg ttatcactca 7320
   tggttatggc agcactgcat aattctctta ctgtcatgcc atccgtaaga tgcttttctg 7380
   tgactggtga gtactcaacc aagtcattct gagaataccg cgcccggcga ccgagttgct 7440
   cttgcccggc gtcaatacgg gataatagtg tatgacatag cagaacttta aaagtgctca 7500
   tcattggaaa acgttcttcg gggcgaaaac tctcaaggat cttaccgctg ttgagatcca 7560
   gttcgatgta acccactcgt gcacccaact gatcttcagc atcttttact ttcaccagcg 7620
   tttctgggtg agcaaaaaca ggaaggcaaa atgccgcaaa aaagggaata agggcgacac 7680
   ggaaatgttg aatactcata ctcttccttt ttcaatatta ttgaagcatt tatcagggtt 7740
   attgtctcat gagcggatac atatttgaat gtatttagaa aaataaacaa ataggggttc 7800
   cgcgcacatt tccccgaaaa gtgccacctg tatgcggtgt gaaataccgc acagatgcgt 7860
   aaggagaaaa taccgcatca ggcgaaattg taaacgttaa tattttgtta aaattcgcgt 7920
   taaatatttg ttaaatcagc tcatttttta accaataggc cgaaatcggc aaaatccctt 7980
   ataaatcaaa agaatagacc gagatagggt tgagtgttgt tccagtttgg aacaagagtc 8040
   cactattaaa gaacgtggac tccaacgtca aagggcgaaa aaccgtctat cagggcgatg 8100
   gcccactacg tgaaccatca cccaaatcaa gttttttgcg gtcgaggtgc cgtaaagctc 8160
   taaatcggaa ccctaaaggg agcccccgat ttagagcttg acggggaaag ccggcgaacg 8220
   tggcgagaaa ggaagggaag aaagcgaaag gagcgggcgc tagggcgctg gcaagtgtag 8280
   cggtcacgct gcgcgtaacc accacacccg ccgcgcttaa                       8320
<210> 74
   <211> 250
   <212> PRT
   <213> Clostridium acetobutylicum
<400> 74
   Asn Lys Arg Ala Ala Phe Met Leu Leu Leu Phe Leu Arg Ser Val Leu
   1               5                   10                  15
   Lys Val Ile Leu Val Leu Asp Val Gly Asn Thr Asn Ile Val Leu Gly
               20                  25                  30
   Ile Tyr Asn Asp Thr Lys Leu Thr Ala Glu Trp Arg Leu Ser Thr Asp
           35                  40                  45
   Val Leu Arg Ser Ala Asp Glu Tyr Gly Ile Gln Val Met Asn Leu Phe
       50                  55                  60
   Gln Gln Asp Lys Leu Asp Pro Thr Leu Val Glu Gly Val Ile Ile Ser
   65                  70                  75                  80
   Ser Val Val Pro Asn Ile Met Tyr Ser Leu Glu His Met Ile Arg Lys
                   85                  90                  95
   Tyr Phe Lys Ile Asn Pro Leu Val Val Gly Pro Gly Ile Lys Thr Gly
               100                 105                 110
   Ile Asn Ile Lys Tyr Asp Asn Pro Lys Glu Val Gly Ala Asp Arg Ile
           115                 120                 125
   Val Asn Ala Val Ala Ala His Glu Ile Tyr Lys Arg Ser Leu Ile Ile
       130                 135                 140
   Ile Asp Phe Gly Thr Ala Thr Thr Phe Cys Ala Val Arg Glu Asn Gly
   145                 150                 155                 160
   Asp Tyr Leu Gly Gly Ala Ile Cys Pro Gly Ile Lys Val Ser Ser Glu
                   165                 170                 175
   Ala Leu Phe Glu Lys Ala Ala Lys Leu Pro Arg Val Glu Leu Ile Lys
               180                 185                 190
   Pro Ala Tyr Ala Ile Cys Lys Asn Thr Ile Ser Ser Ile Gln Ser Gly
           195                 200                 205
   Ile Val Tyr Arg Tyr Leu Arg Gln Val Lys Tyr Leu Phe Glu Lys Leu
       210                 215                 220
   Lys Glu Asn Leu Pro Asp Gly Arg Arg Thr Arg Thr Ser Leu Val Leu
   225                 230                 235                 240
   Ala Thr Gly Gly Leu Ala Lys Leu Ile Asn
                   245                 250
<210> 75
   <211> 258
   <212> PRT
   <213> Rhodobacter capsulatus
<400> 75
   Met Leu Leu Cys Ile Asp Cys Gly Asn Thr Asn Thr Val Phe Ser Val
   1 5 10 15
   Trp Asp Gly Thr Asp Phe Ala Ala Thr Trp Arg Ile Ala Thr Asp His
   20 25 30
   Arg Arg Thr Ala Asp Glu Tyr Phe Val Trp Leu Asn Thr Leu Met Gln
   35 40 45
   Leu Lys Gly Leu Gln Gly Arg Ile Ser Glu Ala Ile Ile Ser Ser Thr
   50 55 60
   Ala Pro Arg Val Val Phe Asn Leu Arg Val Leu Cys Asn Arg Tyr Phe
   65 70 75 80
   Asp Cys Arg Pro Tyr Val Val Gly Lys Pro Gly Cys Glu Leu Pro Val
   85 90 95
   Ala Pro Arg Val Asp Pro Gly Thr Thr Val Gly Pro Asp Arg Leu Val
   100 105 110
   Asn Thr Val Ala Gly Tyr Asp Arg His Gly Gly Asp Leu Ile Val Val
   115 120 125
   Asp Phe Gly Thr Ala Thr Thr Phe Asp Val Val Ala Pro Asp Gly Ala
   130 135 140
   Tyr Ile Gly Gly Val Ile Ala Pro Gly Val Asn Leu Ser Leu Glu Ala
   145 150 155 160
   Leu His Met Ala Ala Ala Ala Leu Pro His Val Asp Val Thr Lys Pro
   165 170 175
   Gln Gly Val Ile Gly Thr Asn Thr Val Ala Cys Ile Gln Ser Gly Val
   180 185 190
   Tyr Trp Gly Tyr Ile Gly Leu Val Glu Gly Ile Val Arg Gln Ile Arg
   195 200 205
   Met Glu Arg Asp Arg Pro Met Lys Val Ile Ala Thr Gly Gly Leu Ala
   210 215 220
   Ser Leu Phe Asp Leu Gly Phe Asp Leu Phe Asp Lys Val Glu Asp Asp
   225 230 235 240
   Leu Thr Met His Gly Leu Arg Leu Ile Phe Asp Tyr Asn Lys Gly Leu
   245 250 255
   Gly Ala
<210> 76
   <211> 10801
   <212> DNA
   <213> Artificial Sequence
<220>
   <223> Description of Artificial Sequence: Recombinant pAN240 plasmid
<400> 76
<210> 77
   <211> 8654
   <212> DNA
   <213> Artificial Sequence
<220>
   <223> Description of Artificial Sequence: Recombinant pAN236 plasmid
<400> 77
   ctcgaggcct acctagcttc caagaaagat atcctaacag cacaagagcg gaaagatgtt 60
   ttgttctaca tccagaacaa cctctgctaa aattcctgaa aaattttgca aaaagttgtt 120
   gactttatct acaaggtgtg gtataataat cttaacaaca gcaggacgct ctagaggagg 180
   agacatcatg aaaattggaa ttatcggcgg aggctccgtt ggtcttttat gcgcctatta 240
   tttgtcactt tatcacgacg tgactgttgt gacgaggcgg caagaacagg ctgcggccat 300
   tcagtctgaa ggaatccggc tttataaagg cggggaggaa ttcagggctg attgcagtgc 360
   ggacacgagt atcaattcgg actttgacct gcttgtcgtg acagtgaagc agcatcagct 420
   tcaatctgtt ttttcgtcgc ttgaacgaat cgggaagacg aatatattat ttttgcaaaa 480
   cggcatgggg catatccacg acctaaaaga ctggcacgtt ggccattcca tttatgttgg 540
   aatcgttgag cacggagctg taagaaaatc ggatacagct gttgatcata caggcctagg 600
   tgcgataaaa tggagcgcgt tcgacgatgc tgaaccagac cggctgaaca tcttgtttca 660
   gcataaccat tcggattttc cgatttatta tgagacggat tggtaccgtc tgctgacggg 720
   caagctgatt gtaaatgcgt gtattaatcc tttaactgcg ttattgcaag tgaaaaatgg 780
   agaactgctg acaacgccag cttatctggc ttttatgaag ctggtatttc aggaggcatg 840
   ccgcatttta aaacttgaaa atgaagaaaa ggcttgggag cgggttcagg ccgtttgtgg 900
   gcaaacgaaa gagaatcgtt catcaatgct ggttgacgtc attggaggcc ggcagacgga 960
   agctgacgcc attatcggat acttattgaa ggaagcaagt cttcaaggtc ttgatgccgt 1020
   ccacctagag tttttatatg gcagcatcaa agcattggag cgaaatacaa acaaagtctt 1080
   ttgagctttt tcggtaacat gctatactca tttcggatca ctaactattt attggaggat 1140
   cctgttttgg cggatgagag aagattttca gcctgataca gattaaatca gaacgcagaa 1200
   gcggtctgat aaaacagaat ttgcctggcg gcagtagcgc ggtggtccca cctgacccca 1260
   tgccgaactc agaagtgaaa cgccgtagcg ccgatggtag tgtggggtct ccccatgcga 1320
   gagtagggaa ctgccaggca tcaaataaaa cgaaaggctc agtcgaaaga ctgggccttt 1380
   cgttttatct gttgtttgtc ggtgaacgct ctcctgagta ggacaaatcc gccgggagcg 1440
   gatttgaacg ttgcgaagca acggcccgga gggtggcggg caggacgccc gccataaact 1500
   gccaggcatc aaattaagca gaaggccatc ctgacggatg gcctttttgc gtttctacaa 1560
   actcttggta cccagaaaaa gcggcaaaag cggctgttaa aaaagcgaaa tcgaagaagc 1620
   tgtctgccgc taagacggaa tatcaaaagc gttctgctgt tgtgtcatct ttaaaagtca 1680
   cagccgatga atcccagcaa gatgtcctaa aatacttgaa cacccagaaa gataaaggaa 1740
   atgcagacca aattcattct tattatgtgg tgaacgggat tgctgttcat gcctcaaaag 1800
   aggttatgga aaaagtggtg cagtttcccg aagtggaaaa ggtgcttcct aatgagaaac 1860
   ggcagctttt taagtcatcc tccccattta atatgaaaaa agcacagaaa gctattaaag 1920
   caactgacgg tgtggaatgg aatgtagacc aaatcgatgc cccaaaagct tgggcacttg 1980
   gatatgatgg aactggcacg gttgttgcgt ccattgatac cggggtggaa tggaatcatc 2040
   cggcattaaa agagaaatat cgcggatata atccggaaaa tcctaatgag cctgaaaatg 2100
   aaatgaactg gtatgatgcc gtagcaggcg aggcaagccc ttatgatgat ttggctcatg 2160
   gaacccacgt gacaggcacg atggtgggct ctgaacctga tggaacaaat caaatcggtg 2220
   tagcacctgg cgcaaaatgg attgctgtta aagcgttctc tgaagatggc ggcactgatg 2280
   ctgacatttt ggaagctggt gaatgggttt tagcaccaaa ggacgcggaa ggaaatcccc 2340
   acccggaaat ggctcctgat gttgtcaata actcatgggg agggggctct ggacttgatg 2400
   aatggtacag agacatggtc aatgcctggc gttcggccga tattttccct gagttttcag 2460
   cggggaatac ggatctcttt attcccggcg ggcctggttc tatcgcaaat ccggcaaact 2520
   atccagaatc gtttgcaact ggagcgactg agaattccaa ttccccatgg agagaaaaga 2580
   aaatcgctaa tgttgattac tttgaacttc tgcatattct tgaatttaaa aaggctgaaa 2640
   gagtaaaaga ttgtgctgaa atattagagt ataaacaaaa tcgtgaaaca ggcgaaagaa 2700
   agttgtatcg agtgtggttt tgtaaatcca ggctttgtcc aatgtgcaac tggaggagag 2760
   caatgaaaca tggcattcag tcacaaaagg ttgttgctga agttattaaa caaaagccaa 2820
   cagttcgttg gttgtttctc acattaacag ttaaaaatgt ttatgatggc gaagaattaa 2880
   ataagagttt gtcagatatg gctcaaggat ttcgccgaat gatgcaatat aaaaaaatta 2940
   ataaaaatct tgttggtttt atgcgtgcaa cggaagtgac aataaataat aaagataatt 3000
   cttataatca gcacatgcat gtattggtat gtgtggaacc aacttatttt aagaatacag 3060
   aaaactacgt gaatcaaaaa caatggattc aattttggaa aaaggcaatg aaattagact 3120
   atgatccaaa tgtaaaagtt caaatgattc gaccgaaaaa taaatataaa tcggatatac 3180
   aatcggcaat tgacgaaact gcaaaatatc ctgtaaagga tacggatttt atgaccgatg 3240
   atgaagaaaa gaatttgaaa cgtttgtctg atttggagga aggtttacac cgtaaaaggt 3300
   taatctccta tggtggtttg ttaaaagaaa tacataaaaa attaaacctt gatgacacag 3360
   aagaaggcga tttgattcat acagatgatg acgaaaaagc cgatgaagat ggattttcta 3420
   ttattgcaat gtggaattgg gaacggaaaa attattttat taaagagtag ttcaacaaac 3480
   gggccatatt gttgtataag tgatgaaata ctgaatttaa aacttagttt atatgtggta 3540
   aaatgtttta atcaagttta ggaggaatta attatgaagt gtaatgaatg taacagggtt 3600
   caattaaaag agggaagcgt atcattaacc ctataaacta cgtctgccct cattattgga 3660
   gggtgaaatg tgaatacatc ctattcacaa tcgaatttac gacacaacca aattttaatt 3720
   tggctttgca ttttatcttt ttttagcgta ttaaatgaaa tggttttgaa cgtctcatta 3780
   cctgatattg caaatgattt taataaacca cctgcgagta caaactgggt gaacacagcc 3840
   tttatgttaa ccttttccat tggaacagct gtatatggaa agctatctga tcaattaggc 3900
   atcaaaaggt tactcctatt tggaattata ataaattgtt tcgggtcggt aattgggttt 3960
   gttggccatt ctttcttttc cttacttatt atggctcgtt ttattcaagg ggctggtgca 4020
   gctgcatttc cagcactcgt aatggttgta gttgcgcgct atattccaaa ggaaaatagg 4080
   ggtaaagcat ttggtcttat tggatcgata gtagccatgg gagaaggagt cggtccagcg 4140
   attggtggaa tgatagccca ttatattcat tggtcctatc ttctactcat tcctatgata 4200
   acaattatca ctgttccgtt tcttatgaaa ttattaaaga aagaagtaag gataaaaggt 4260
   cattttgata tcaaaggaat tatactaatg tctgtaggca ttgtattttt tatgttgttt 4320
   acaacatcat atagcatttc ttttcttatc gttagcgtgc tgtcattcct gatatttgta 4380
   aaacatatca ggaaagtaac agatcctttt gttgatcccg gattagggaa aaatatacct 4440
   tttatgattg gagttctttg tgggggaatt atatttggaa cagtagcagg gtttgtctct 4500
   atggttcctt atatgatgaa agatgttcac cagctaagta ctgccgaaat cggaagtgta 4560
   attattttcc ctggaacaat gagtgtcatt attttcggct acattggtgg gatacttgtt 4620
   gatagaagag gtcctttata cgtgttaaac atcggagtta catttctttc tgttagcttt 4680
   ttaactgctt cctttctttt agaaacaaca tcatggttca tgacaattat aatcgtattt 4740
   gttttaggtg ggctttcgtt caccaaaaca gttatatcaa caattgtttc aagtagcttg 4800
   aaacagcagg aagctggtgc tggaatgagt ttgcttaact ttaccagctt tttatcagag 4860
   ggaacaggta ttgcaattgt aggtggttta ttatccatac ccttacttga tcaaaggttg 4920
   ttacctatgg aagttgatca gtcaacttat ctgtatagta atttgttatt acttttttca 4980
   ggaatcattg tcattagttg gctggttacc ttgaatgtat ataaacattc tcaaagggat 5040
   ttctaaatcg ttaagggatc aactttggga gagagttcaa aattgatcct ttttttataa 5100
   cagttcgaag cggccgcaat tcttgaagac gaaagggcct cgtgatacgc ctatttttat 5160
   aggttaatgt catgataata atggtttctt agacgtcagg tggcactttt cggggaaatg 5220
   tgcgcggaac ccctatttgt ttatttttct aaatacattc aaatatgtat ccgctcatga 5280
   gacaataacc ctgataaatg cttcaataat attgaaaaag gaagagtatg agtattcaac 5340
   atttccgtgt cgcccttatt cccttttttg cggcattttg ccttcctgtt tttgctcacc 5400
   cagaaacgct ggtgaaagta aaagatgctg aagatcagtt gggtgcacga gtgggttaca 5460
   tcgaactgga tctcaacagc ggtaagatcc ttgagagttt tcgccccgaa gaacgttttc 5520
   caatgatgag cacttttaaa gttctgctat gtggcgcggt attatcccgt attgacgccg 5580
   ggcaagagca actcggtcgc cgcatacact attctcagaa tgacttggtt gagtactcac 5640
   cagtcacaga aaagcatctt acggatggca tgacagtaag agaattatgc agtgctgcca 5700
   taaccatgag tgataacact gcggccaact tacttctgac aacgatcgga ggaccgaagg 5760
   agctaaccgc ttttttgcac aacatggggg atcatgtaac tcgccttgat cgttgggaac 5820
   cggagctgaa tgaagccata ccaaacgacg agcgtgacac cacgatgcct gcagcaatgg 5880
   caacaacgtt gcgcaaacta ttaactggcg aactacttac tctagcttcc cggcaacaat 5940
   taatagactg gatggaggcg gataaagttg caggaccact tctgcgctcg gcccttccgg 6000
   ctggctggtt tattgctgat aaatctggag ccggtgagcg tgggtctcgc ggtatcattg 6060
   cagcactggg gccagatggt aagccctccc gtatcgtagt tatctacacg acggggagtc 6120
   aggcaactat ggatgaacga aatagacaga tcgctgagat aggtgcctca ctgattaagc 6180
   attggtaact gtcagaccaa gtttactcat atatacttta gattgattta aaacttcatt 6240
   tttaatttaa aaggatctag gtgaagatcc tttttgataa tctcatgacc aaaatccctt 6300
   aacgtgagtt ttcgttccac tgagcgtcag accccgtaga aaagatcaaa ggatcttctt 6360
   gagatccttt ttttctgcgc gtaatctgct gcttgcaaac aaaaaaacca ccgctaccag 6420
   cggtggtttg tttgccggat caagagctac caactctttt tccgaaggta actggcttca 6480
   gcagagcgca gataccaaat actgtccttc tagtgtagcc gtagttaggc caccacttca 6540
   agaactctgt agcaccgcct acatacctcg ctctgctaat cctgttacca gtggctgctg 6600
   ccagtggcga taagtcgtgt cttaccgggt tggactcaag acgatagtta ccggataagg 6660
   cgcagcggtc gggctgaacg gggggttcgt gcacacagcc cagcttggag cgaacgacct 6720
   acaccgaact gagataccta cagcgtgagc tatgagaaag cgccacgctt cccgaaggga 6780
   gaaaggcgga caggtatccg gtaagcggca gggtcggaac aggagagcgc acgagggagc 6840
   ttccaggggg aaacgcctgg tatctttata gtcctgtcgg gtttcgccac ctctgacttg 6900
   agcgtcgatt tttgtgatgc tcgtcagggg ggcggagcct atggaaaaac gccagcaacg 6960
   cggccttttt acggttcctg gccttttgct ggccttttgc tcacatgttc tttcctgcgt 7020
   tatcccctga ttctgtggat aaccgtatta ccgcctttga gtgagctgat accgctcgcc 7080
   gcagccgaac gaccgagcgc agcgagtcag tgagcgagga agcggaagag cgcctgatgc 7140
   ggtattttct ccttacgcat ctgtgcggta tttcacaccg catatggtgc actctcagta 7200
   caatctgctc tgatgccgca tagttaagcc agtatacact ccgctatcgc tacgtgactg 7260
   ggtcatggct gcgccccgac acccgccaac acccgctgac gcgccctgac gggcttgtct 7320
   gctcccggca tccgcttaca gacaagctgt gaccgtctcc gggagctgca tgtgtcagag 7380
   gttttcaccg tcatcaccga aacgcgcgag gcagctgcgg taaagctcat cagcgtggtc 7440
   gtgaagcgat tcacagatgt ctgcctgttc atccgcgtcc agctcgttga gtttctccag 7500
   aagcgttaat gtctggcttc tgataaagcg ggccatgtta agggcggttt tttcctgttt 7560
   ggtcacttga tgcctccgtg taagggggaa tttctgttca tgggggtaat gataccgatg 7620
   aaacgagaga ggatgctcac gatacgggtt actgatgatg aacatgcccg gttactggaa 7680
   cgttgtgagg gtaaacaact ggcggtatgg atgcggcggg accagagaaa aatcactcag 7740
   ggtcaatgcc agcgcttcgt taatacagat gtaggtgttc cacagggtag ccagcagcat 7800
   cctgcgatgc agatccggaa cataatggtg cagggcgctg acttccgcgt ttccagactt 7860
   tacgaaacac ggaaaccgaa gaccattcat gttgttgctc aggtcgcaga cgttttgcag 7920
   cagcagtcgc ttcacgttcg ctcgcgtatc ggtgattcat tctgctaacc agtaaggcaa 7980
   ccccgccagc ctagccgggt cctcaacgac aggagcacga tcatgcgcac ccgtggccag 8040
   gacccaacgc tgcccgagat gcgccgcgtg cggctgctgg agatggcgga cgcgatggat 8100
   atgttctgcc aagggttggt ttgcgcattc acagttctcc gcaagaattg attggctcca 8160
   attcttggag tggtgaatcc gttagcgagg tgccgccggc ttccattcag gtcgaggtgg 8220
   cccggctcca tgcaccgcga cgcaacgcgg ggaggcagac aaggtatagg gcggcgccta 8280
   caatccatgc caacccgttc catgtgctcg ccgaggcggc ataaatcgcc gtgacgatca 8340
   gcggtccagt gatcgaagtt aggctggtaa gagccgcgag cgatccttga agctgtccct 8400
   gatggtcgtc atctacctgc ctggacagca tggcctgcaa cgcgggcatc ccgatgccgc 8460
   cggaagcgag aagaatcata atggggaagg ccatccagcc tcgcgtcggc ggccgcttcg 8520
   tcgaccgaaa cagcagttat aaggcatgaa gctgtccggt ttttgcaaaa gtggctgtga 8580
   ctgtaaaaag aaatcgaaaa agaccgtttt gtgtgaaaac ggtctttttg tttcctttta 8640
   accaactgcc ataa 8654
<210> 78
   <211> 8093
   <212> DNA
   <213> Artificial Sequence
<220>
   <223> Description of Artificial Sequence: Recombinant pAN423 plasmid
<400> 78
   ggcggccgct tcgtcgaccg aaacagcagt tataaggcat gaagctgtcc ggtttttgca 60
   aaagtggctg tgactgtaaa aagaaatcga aaaagaccgt tttgtgtgaa aacggtcttt 120
   ttgtttcctt ttaaccaact gccataactc gaggcctacc tagcttccaa gaaagatatc 180
   ctaacagcac aagagcggaa agatgttttg ttctacatcc agaacaacct ctgctaaaat 240
   tcctgaaaaa ttttgcaaaa agttgttgac tttatctaca aggtgtggta taataatctt 300
   aacaacagca ggacgctcta gaaaaggagg aatttaaatg tatcgtacga tgatgagcgg 360
   caaacttcac agggcaactg ttacggaagc aaacctgaac tatgtgggaa gcattacaat 420
   tgatgaagat ctcattgatg ctgtgggaat gcttcctaat gaaaaagtac aaattgtgaa 480
   taataataat ggagcacgtc ttgaaacgta tattattcct ggtaaacggg gaagcggcgt 540
   catatgctta aacggtgcag ccgcacgcct tgtgcaggaa ggagataagg tcattattat 600
   ttcctacaaa atgatgtctg atcaagaagc ggcaagccat gagccgaaag tggctgttct 660
   gaatgatcaa aacaaaattg aacaaatgct ggggaacgaa ccagcccgta caattttgta 720
   aaggatcctg ttttggcgga tgagagaaga ttttcagcct gatacagatt aaatcagaac 780
   gcagaagcgg tctgataaaa cagaatttgc ctggcggcag tagcgcggtg gtcccacctg 840
   accccatgcc gaactcagaa gtgaaacgcc gtagcgccga tggtagtgtg gggtctcccc 900
   atgcgagagt agggaactgc caggcatcaa ataaaacgaa aggctcagtc gaaagactgg 960
   gcctttcgtt ttatctgttg tttgtcggtg aacgctctcc tgagtaggac aaatccgccg 1020
   ggagcggatt tgaacgttgc gaagcaacgg cccggagggt ggcgggcagg acgcccgcca 1080
   taaactgcca ggcatcaaat taagcagaag gccatcctga cggatggcct ttttgcgttt 1140
   ctacaaactc ttggtaccca gaaaaagcgg caaaagcggc tgttaaaaaa gcgaaatcga 1200
   agaagctgtc tgccgctaag acggaatatc aaaagcgttc tgctgttgtg tcatctttaa 1260
   aagtcacagc cgatgaatcc cagcaagatg tcctaaaata cttgaacacc cagaaagata 1320
   aaggaaatgc agaccaaatt cattcttatt atgtggtgaa cgggattgct gttcatgcct 1380
   caaaagaggt tatggaaaaa gtggtgcagt ttcccgaagt ggaaaaggtg cttcctaatg 1440
   agaaacggca gctttttaag tcatcctccc catttaatat gaaaaaagca cagaaagcta 1500
   ttaaagcaac tgacggtgtg gaatggaatg tagaccaaat cgatgcccca aaagcttggg 1560
   cacttggata tgatggaact ggcacggttg ttgcgtccat tgataccggg gtggaatgga 1620
   atcatccggc attaaaagag aaatatcgcg gatataatcc ggaaaatcct aatgagcctg 1680
   aaaatgaaat gaactggtat gatgccgtag caggcgaggc aagcccttat gatgatttgg 1740
   ctcatggaac ccacgtgaca ggcacgatgg tgggctctga acctgatgga acaaatcaaa 1800
   tcggtgtagc acctggcgca aaatggattg ctgttaaagc gttctctgaa gatggcggca 1860
   ctgatgctga cattttggaa gctggtgaat gggttttagc accaaaggac gcggaaggaa 1920
   atccccaccc ggaaatggct cctgatgttg tcaataactc atggggaggg ggctctggac 1980
   ttgatgaatg gtacagagac atggtcaatg cctggcgttc ggccgatatt ttccctgagt 2040
   tttcagcggg gaatacggat ctctttattc ccggcgggcc tggttctatc gcaaatccgg 2100
   caaactatcc agaatcgttt gcaactggag cgactgagaa ttccaattcc ccatggagag 2160
   aaaagaaaat cgctaatgtt gattactttg aacttctgca tattcttgaa tttaaaaagg 2220
   ctgaaagagt aaaagattgt gctgaaatat tagagtataa acaaaatcgt gaaacaggcg 2280
   aaagaaagtt gtatcgagtg tggttttgta aatccaggct ttgtccaatg tgcaactgga 2340
   ggagagcaat gaaacatggc attcagtcac aaaaggttgt tgctgaagtt attaaacaaa 2400
   agccaacagt tcgttggttg tttctcacat taacagttaa aaatgtttat gatggcgaag 2460
   aattaaataa gagtttgtca gatatggctc aaggatttcg ccgaatgatg caatataaaa 2520
   aaattaataa aaatcttgtt ggttttatgc gtgcaacgga agtgacaata aataataaag 2580
   ataattctta taatcagcac atgcatgtat tggtatgtgt ggaaccaact tattttaaga 2640
   atacagaaaa ctacgtgaat caaaaacaat ggattcaatt ttggaaaaag gcaatgaaat 2700
   tagactatga tccaaatgta aaagttcaaa tgattcgacc gaaaaataaa tataaatcgg 2760
   atatacaatc ggcaattgac gaaactgcaa aatatcctgt aaaggatacg gattttatga 2820
   ccgatgatga agaaaagaat ttgaaacgtt tgtctgattt ggaggaaggt ttacaccgta 2880
   aaaggttaat ctcctatggt ggtttgttaa aagaaataca taaaaaatta aaccttgatg 2940
   acacagaaga aggcgatttg attcatacag atgatgacga aaaagccgat gaagatggat 3000
   tttctattat tgcaatgtgg aattgggaac ggaaaaatta ttttattaaa gagtagttca 3060
   acaaacgggc catattgttg tataagtgat gaaatactga atttaaaact tagtttatat 3120
   gtggtaaaat gttttaatca agtttaggag gaattaatta tgaagtgtaa tgaatgtaac 3180
   agggttcaat taaaagaggg aagcgtatca ttaaccctat aaactacgtc tgccctcatt 3240
   attggagggt gaaatgtgaa tacatcctat tcacaatcga atttacgaca caaccaaatt 3300
   ttaatttggc tttgcatttt atcttttttt agcgtattaa atgaaatggt tttgaacgtc 3360
   tcattacctg atattgcaaa tgattttaat aaaccacctg cgagtacaaa ctgggtgaac 3420
   acagccttta tgttaacctt ttccattgga acagctgtat atggaaagct atctgatcaa 3480
   ttaggcatca aaaggttact cctatttgga attataataa attgtttcgg gtcggtaatt 3540
   gggtttgttg gccattcttt cttttcctta cttattatgg ctcgttttat tcaaggggct 3600
   ggtgcagctg catttccagc actcgtaatg gttgtagttg cgcgctatat tccaaaggaa 3660
   aataggggta aagcatttgg tcttattgga tcgatagtag ccatgggaga aggagtcggt 3720
   ccagcgattg gtggaatgat agcccattat attcattggt cctatcttct actcattcct 3780
   atgataacaa ttatcactgt tccgtttctt atgaaattat taaagaaaga agtaaggata 3840
   aaaggtcatt ttgatatcaa aggaattata ctaatgtctg taggcattgt attttttatg 3900
   ttgtttacaa catcatatag catttctttt cttatcgtta gcgtgctgtc attcctgata 3960
   tttgtaaaac atatcaggaa agtaacagat ccttttgttg atcccggatt agggaaaaat 4020
   atacctttta tgattggagt tctttgtggg ggaattatat ttggaacagt agcagggttt 4080
   gtctctatgg ttccttatat gatgaaagat gttcaccagc taagtactgc cgaaatcgga 4140
   agtgtaatta ttttccctgg aacaatgagt gtcattattt tcggctacat tggtgggata 4200
   cttgttgata gaagaggtcc tttatacgtg ttaaacatcg gagttacatt tctttctgtt 4260
   agctttttaa ctgcttcctt tcttttagaa acaacatcat ggttcatgac aattataatc 4320
   gtatttgttt taggtgggct ttcgttcacc aaaacagtta tatcaacaat tgtttcaagt 4380
   agcttgaaac agcaggaagc tggtgctgga atgagtttgc ttaactttac cagcttttta 4440
   tcagagggaa caggtattgc aattgtaggt ggtttattat ccataccctt acttgatcaa 4500
   aggttgttac ctatggaagt tgatcagtca acttatctgt atagtaattt gttattactt 4560
   ttttcaggaa tcattgtcat tagttggctg gttaccttga atgtatataa acattctcaa 4620
   agggatttct aaatcgttaa gggatcaact ttgggagaga gttcaaaatt gatccttttt 4680
   ttataacagt tcgaagcggc cgcaattctt gaagacgaaa gggcctcgtg atacgcctat 4740
   ttttataggt taatgtcatg ataataatgg tttcttagac gtcaggtggc acttttcggg 4800
   gaaatgtgcg cggaacccct atttgtttat ttttctaaat acattcaaat atgtatccgc 4860
   tcatgagaca ataaccctga taaatgcttc aataatattg aaaaaggaag agtatgagta 4920
   ttcaacattt ccgtgtcgcc cttattccct tttttgcggc attttgcctt cctgtttttg 4980
   ctcacccaga aacgctggtg aaagtaaaag atgctgaaga tcagttgggt gcacgagtgg 5040
   gttacatcga actggatctc aacagcggta agatccttga gagttttcgc cccgaagaac 5100
   gttttccaat gatgagcact tttaaagttc tgctatgtgg cgcggtatta tcccgtattg 5160
   acgccgggca agagcaactc ggtcgccgca tacactattc tcagaatgac ttggttgagt 5220
   actcaccagt cacagaaaag catcttacgg atggcatgac agtaagagaa ttatgcagtg 5280
   ctgccataac catgagtgat aacactgcgg ccaacttact tctgacaacg atcggaggac 5340
   cgaaggagct aaccgctttt ttgcacaaca tgggggatca tgtaactcgc cttgatcgtt 5400
   gggaaccgga gctgaatgaa gccataccaa acgacgagcg tgacaccacg atgcctgcag 5460
   caatggcaac aacgttgcgc aaactattaa ctggcgaact acttactcta gcttcccggc 5520
   aacaattaat agactggatg gaggcggata aagttgcagg accacttctg cgctcggccc 5580
   ttccggctgg ctggtttatt gctgataaat ctggagccgg tgagcgtggg tctcgcggta 5640
   tcattgcagc actggggcca gatggtaagc cctcccgtat cgtagttatc tacacgacgg 5700
   ggagtcaggc aactatggat gaacgaaata gacagatcgc tgagataggt gcctcactga 5760
   ttaagcattg gtaactgtca gaccaagttt actcatatat actttagatt gatttaaaac 5820
   ttcattttta atttaaaagg atctaggtga agatcctttt tgataatctc atgaccaaaa 5880
   tcccttaacg tgagttttcg ttccactgag cgtcagaccc cgtagaaaag atcaaaggat 5940
   cttcttgaga tccttttttt ctgcgcgtaa tctgctgctt gcaaacaaaa aaaccaccgc 6000
   taccagcggt ggtttgtttg ccggatcaag agctaccaac tctttttccg aaggtaactg 6060
   gcttcagcag agcgcagata ccaaatactg tccttctagt gtagccgtag ttaggccacc 6120
   acttcaagaa ctctgtagca ccgcctacat acctcgctct gctaatcctg ttaccagtgg 6180
   ctgctgccag tggcgataag tcgtgtctta ccgggttgga ctcaagacga tagttaccgg 6240
   ataaggcgca gcggtcgggc tgaacggggg gttcgtgcac acagcccagc ttggagcgaa 6300
   cgacctacac cgaactgaga tacctacagc gtgagctatg agaaagcgcc acgcttcccg 6360
   aagggagaaa ggcggacagg tatccggtaa gcggcagggt cggaacagga gagcgcacga 6420
   gggagcttcc agggggaaac gcctggtatc tttatagtcc tgtcgggttt cgccacctct 6480
   gacttgagcg tcgatttttg tgatgctcgt caggggggcg gagcctatgg aaaaacgcca 6540
   gcaacgcggc ctttttacgg ttcctggcct tttgctggcc ttttgctcac atgttctttc 6600
   ctgcgttatc ccctgattct gtggataacc gtattaccgc ctttgagtga gctgataccg 6660
   ctcgccgcag ccgaacgacc gagcgcagcg agtcagtgag cgaggaagcg gaagagcgcc 6720
   tgatgcggta ttttctcctt acgcatctgt gcggtatttc acaccgcata tggtgcactc 6780
   tcagtacaat ctgctctgat gccgcatagt taagccagta tacactccgc tatcgctacg 6840
   tgactgggtc atggctgcgc cccgacaccc gccaacaccc gctgacgcgc cctgacgggc 6900
   ttgtctgctc ccggcatccg cttacagaca agctgtgacc gtctccggga gctgcatgtg 6960
   tcagaggttt tcaccgtcat caccgaaacg cgcgaggcag ctgcggtaaa gctcatcagc 7020
   gtggtcgtga agcgattcac agatgtctgc ctgttcatcc gcgtccagct cgttgagttt 7080
   ctccagaagc gttaatgtct ggcttctgat aaagcgggcc atgttaaggg cggttttttc 7140
   ctgtttggtc acttgatgcc tccgtgtaag ggggaatttc tgttcatggg ggtaatgata 7200
   ccgatgaaac gagagaggat gctcacgata cgggttactg atgatgaaca tgcccggtta 7260
   ctggaacgtt gtgagggtaa acaactggcg gtatggatgc ggcgggacca gagaaaaatc 7320
   actcagggtc aatgccagcg cttcgttaat acagatgtag gtgttccaca gggtagccag 7380
   cagcatcctg cgatgcagat ccggaacata atggtgcagg gcgctgactt ccgcgtttcc 7440
   agactttacg aaacacggaa accgaagacc attcatgttg ttgctcaggt cgcagacgtt 7500
   ttgcagcagc agtcgcttca cgttcgctcg cgtatcggtg attcattctg ctaaccagta 7560
   aggcaacccc gccagcctag ccgggtcctc aacgacagga gcacgatcat gcgcacccgt 7620
   ggccaggacc caacgctgcc cgagatgcgc cgcgtgcggc tgctggagat ggcggacgcg 7680
   atggatatgt tctgccaagg gttggtttgc gcattcacag ttctccgcaa gaattgattg 7740
   gctccaattc ttggagtggt gaatccgtta gcgaggtgcc gccggcttcc attcaggtcg 7800
   aggtggcccg gctccatgca ccgcgacgca acgcggggag gcagacaagg tatagggcgg 7860
   cgcctacaat ccatgccaac ccgttccatg tgctcgccga ggcggcataa atcgccgtga 7920
   cgatcagcgg tccagtgatc gaagttaggc tggtaagagc cgcgagcgat ccttgaagct 7980
   gtccctgatg gtcgtcatct acctgcctgg acagcatggc ctgcaacgcg ggcatcccga 8040
   tgccgccgga agcgagaaga atcataatgg ggaaggccat ccagcctcgc gtc 8093
<210> 79
   <211> 8098
   <212> DNA
   <213> Artificial Sequence
<220>
   <223> Description of Artificial Sequence: Recombinant pAN429 plasmid
<400> 79
   ggcggccgct tcgtcgaccg aaacagcagt tataaggcat gaagctgtcc ggtttttgca 60
   aaagtggctg tgactgtaaa aagaaatcga aaaagaccgt tttgtgtgaa aacggtcttt 120
   ttgtttcctt ttaaccaact gccataactc gaggcctacc tagcttccaa gaaagatatc 180
   ctaacagcac aagagcggaa agatgttttg ttctacatcc agaacaacct ctgctaaaat 240
   tcctgaaaaa ttttgcaaaa agttgttgac tttatctaca aggtgtggta taataatctt 300
   aacaacagca ggacgctcta gattagaaag gaggtttaat taatgtatcg tacgatgatg 360
   agcggcaaac ttcacagggc aactgttacg gaagcaaacc tgaactatgt gggaagcatt 420
   acaattgatg aagatctcat tgatgctgtg ggaatgcttc ctaatgaaaa agtacaaatt 480
   gtgaataata ataatggagc acgtcttgaa acgtatatta ttcctggtaa acggggaagc 540
   ggcgtcatat gcttaaacgg tgcagccgca cgccttgtgc aggaaggaga taaggtcatt 600
   attatttcct acaaaatgat gtctgatcaa gaagcggcaa gccatgagcc gaaagtggct 660
   gttctgaatg atcaaaacaa aattgaacaa atgctgggga acgaaccagc ccgtacaatt 720
   ttgtaaagga tcctgttttg gcggatgaga gaagattttc agcctgatac agattaaatc 780
   agaacgcaga agcggtctga taaaacagaa tttgcctggc ggcagtagcg cggtggtccc 840
   acctgacccc atgccgaact cagaagtgaa acgccgtagc gccgatggta gtgtggggtc 900
   tccccatgcg agagtaggga actgccaggc atcaaataaa acgaaaggct cagtcgaaag 960
   actgggcctt tcgttttatc tgttgtttgt cggtgaacgc tctcctgagt aggacaaatc 1020
   cgccgggagc ggatttgaac gttgcgaagc aacggcccgg agggtggcgg gcaggacgcc 1080
   cgccataaac tgccaggcat caaattaagc agaaggccat cctgacggat ggcctttttg 1140
   cgtttctaca aactcttggt acccagaaaa agcggcaaaa gcggctgtta aaaaagcgaa 1200
   atcgaagaag ctgtctgccg ctaagacgga atatcaaaag cgttctgctg ttgtgtcatc 1260
   tttaaaagtc acagccgatg aatcccagca agatgtccta aaatacttga acacccagaa 1320
   agataaagga aatgcagacc aaattcattc ttattatgtg gtgaacggga ttgctgttca 1380
   tgcctcaaaa gaggttatgg aaaaagtggt gcagtttccc gaagtggaaa aggtgcttcc 1440
   taatgagaaa cggcagcttt ttaagtcatc ctccccattt aatatgaaaa aagcacagaa 1500
   agctattaaa gcaactgacg gtgtggaatg gaatgtagac caaatcgatg ccccaaaagc 1560
   ttgggcactt ggatatgatg gaactggcac ggttgttgcg tccattgata ccggggtgga 1620
   atggaatcat ccggcattaa aagagaaata tcgcggatat aatccggaaa atcctaatga 1680
   gcctgaaaat gaaatgaact ggtatgatgc cgtagcaggc gaggcaagcc cttatgatga 1740
   tttggctcat ggaacccacg tgacaggcac gatggtgggc tctgaacctg atggaacaaa 1800
   tcaaatcggt gtagcacctg gcgcaaaatg gattgctgtt aaagcgttct ctgaagatgg 1860
   cggcactgat gctgacattt tggaagctgg tgaatgggtt ttagcaccaa aggacgcgga 1920
   aggaaatccc cacccggaaa tggctcctga tgttgtcaat aactcatggg gagggggctc 1980
   tggacttgat gaatggtaca gagacatggt caatgcctgg cgttcggccg atattttccc 2040
   tgagttttca gcggggaata cggatctctt tattcccggc gggcctggtt ctatcgcaaa 2100
   tccggcaaac tatccagaat cgtttgcaac tggagcgact gagaattcca attccccatg 2160
   gagagaaaag aaaatcgcta atgttgatta ctttgaactt ctgcatattc ttgaatttaa 2220
   aaaggctgaa agagtaaaag attgtgctga aatattagag tataaacaaa atcgtgaaac 2280
   aggcgaaaga aagttgtatc gagtgtggtt ttgtaaatcc aggctttgtc caatgtgcaa 2340
   ctggaggaga gcaatgaaac atggcattca gtcacaaaag gttgttgctg aagttattaa 2400
   acaaaagcca acagttcgtt ggttgtttct cacattaaca gttaaaaatg tttatgatgg 2460
   cgaagaatta aataagagtt tgtcagatat ggctcaagga tttcgccgaa tgatgcaata 2520
   taaaaaaatt aataaaaatc ttgttggttt tatgcgtgca acggaagtga caataaataa 2580
   taaagataat tcttataatc agcacatgca tgtattggta tgtgtggaac caacttattt 2640
   taagaataca gaaaactacg tgaatcaaaa acaatggatt caattttgga aaaaggcaat 2700
   gaaattagac tatgatccaa atgtaaaagt tcaaatgatt cgaccgaaaa ataaatataa 2760
   atcggatata caatcggcaa ttgacgaaac tgcaaaatat cctgtaaagg atacggattt 2820
   tatgaccgat gatgaagaaa agaatttgaa acgtttgtct gatttggagg aaggtttaca 2880
   ccgtaaaagg ttaatctcct atggtggttt gttaaaagaa atacataaaa aattaaacct 2940
   tgatgacaca gaagaaggcg atttgattca tacagatgat gacgaaaaag ccgatgaaga 3000
   tggattttct attattgcaa tgtggaattg ggaacggaaa aattatttta ttaaagagta 3060
   gttcaacaaa cgggccatat tgttgtataa gtgatgaaat actgaattta aaacttagtt 3120
   tatatgtggt aaaatgtttt aatcaagttt aggaggaatt aattatgaag tgtaatgaat 3180
   gtaacagggt tcaattaaaa gagggaagcg tatcattaac cctataaact acgtctgccc 3240
   tcattattgg agggtgaaat gtgaatacat cctattcaca atcgaattta cgacacaacc 3300
   aaattttaat ttggctttgc attttatctt tttttagcgt attaaatgaa atggttttga 3360
   acgtctcatt acctgatatt gcaaatgatt ttaataaacc acctgcgagt acaaactggg 3420
   tgaacacagc ctttatgtta accttttcca ttggaacagc tgtatatgga aagctatctg 3480
   atcaattagg catcaaaagg ttactcctat ttggaattat aataaattgt ttcgggtcgg 3540
   taattgggtt tgttggccat tctttctttt ccttacttat tatggctcgt tttattcaag 3600
   gggctggtgc agctgcattt ccagcactcg taatggttgt agttgcgcgc tatattccaa 3660
   aggaaaatag gggtaaagca tttggtctta ttggatcgat agtagccatg ggagaaggag 3720
   tcggtccagc gattggtgga atgatagccc attatattca ttggtcctat cttctactca 3780
   ttcctatgat aacaattatc actgttccgt ttcttatgaa attattaaag aaagaagtaa 3840
   ggataaaagg tcattttgat atcaaaggaa ttatactaat gtctgtaggc attgtatttt 3900
   ttatgttgtt tacaacatca tatagcattt cttttcttat cgttagcgtg ctgtcattcc 3960
   tgatatttgt aaaacatatc aggaaagtaa cagatccttt tgttgatccc ggattaggga 4020
   aaaatatacc ttttatgatt ggagttcttt gtgggggaat tatatttgga acagtagcag 4080
   ggtttgtctc tatggttcct tatatgatga aagatgttca ccagctaagt actgccgaaa 4140
   tcggaagtgt aattattttc cctggaacaa tgagtgtcat tattttcggc tacattggtg 4200
   ggatacttgt tgatagaaga ggtcctttat acgtgttaaa catcggagtt acatttcttt 4260
   ctgttagctt tttaactgct tcctttcttt tagaaacaac atcatggttc atgacaatta 4320
   taatcgtatt tgttttaggt gggctttcgt tcaccaaaac agttatatca acaattgttt 4380
   caagtagctt gaaacagcag gaagctggtg ctggaatgag tttgcttaac tttaccagct 4440
   ttttatcaga gggaacaggt attgcaattg taggtggttt attatccata cccttacttg 4500
   atcaaaggtt gttacctatg gaagttgatc agtcaactta tctgtatagt aatttgttat 4560
   tacttttttc aggaatcatt gtcattagtt ggctggttac cttgaatgta tataaacatt 4620
   ctcaaaggga tttctaaatc gttaagggat caactttggg agagagttca aaattgatcc 4680
   tttttttata acagttcgaa gcggccgcaa ttcttgaaga cgaaagggcc tcgtgatacg 4740
   cctattttta taggttaatg tcatgataat aatggtttct tagacgtcag gtggcacttt 4800
   tcggggaaat gtgcgcggaa cccctatttg tttatttttc taaatacatt caaatatgta 4860
   tccgctcatg agacaataac cctgataaat gcttcaataa tattgaaaaa ggaagagtat 4920
   gagtattcaa catttccgtg tcgcccttat tccctttttt gcggcatttt gccttcctgt 4980
   ttttgctcac ccagaaacgc tggtgaaagt aaaagatgct gaagatcagt tgggtgcacg 5040
   agtgggttac atcgaactgg atctcaacag cggtaagatc cttgagagtt ttcgccccga 5100
   agaacgtttt ccaatgatga gcacttttaa agttctgcta tgtggcgcgg tattatcccg 5160
   tattgacgcc gggcaagagc aactcggtcg ccgcatacac tattctcaga atgacttggt 5220
   tgagtactca ccagtcacag aaaagcatct tacggatggc atgacagtaa gagaattatg 5280
   cagtgctgcc ataaccatga gtgataacac tgcggccaac ttacttctga caacgatcgg 5340
   aggaccgaag gagctaaccg cttttttgca caacatgggg gatcatgtaa ctcgccttga 5400
   tcgttgggaa ccggagctga atgaagccat accaaacgac gagcgtgaca ccacgatgcc 5460
   tgcagcaatg gcaacaacgt tgcgcaaact attaactggc gaactactta ctctagcttc 5520
   ccggcaacaa ttaatagact ggatggaggc ggataaagtt gcaggaccac ttctgcgctc 5580
   ggcccttccg gctggctggt ttattgctga taaatctgga gccggtgagc gtgggtctcg 5640
   cggtatcatt gcagcactgg ggccagatgg taagccctcc cgtatcgtag ttatctacac 5700
   gacggggagt caggcaacta tggatgaacg aaatagacag atcgctgaga taggtgcctc 5760
   actgattaag cattggtaac tgtcagacca agtttactca tatatacttt agattgattt 5820
   aaaacttcat ttttaattta aaaggatcta ggtgaagatc ctttttgata atctcatgac 5880
   caaaatccct taacgtgagt tttcgttcca ctgagcgtca gaccccgtag aaaagatcaa 5940
   aggatcttct tgagatcctt tttttctgcg cgtaatctgc tgcttgcaaa caaaaaaacc 6000
   accgctacca gcggtggttt gtttgccgga tcaagagcta ccaactcttt ttccgaaggt 6060
   aactggcttc agcagagcgc agataccaaa tactgtcctt ctagtgtagc cgtagttagg 6120
   ccaccacttc aagaactctg tagcaccgcc tacatacctc gctctgctaa tcctgttacc 6180
   agtggctgct gccagtggcg ataagtcgtg tcttaccggg ttggactcaa gacgatagtt 6240
   accggataag gcgcagcggt cgggctgaac ggggggttcg tgcacacagc ccagcttgga 6300
   gcgaacgacc tacaccgaac tgagatacct acagcgtgag ctatgagaaa gcgccacgct 6360
   tcccgaaggg agaaaggcgg acaggtatcc ggtaagcggc agggtcggaa caggagagcg 6420
   cacgagggag cttccagggg gaaacgcctg gtatctttat agtcctgtcg ggtttcgcca 6480
   cctctgactt gagcgtcgat ttttgtgatg ctcgtcaggg gggcggagcc tatggaaaaa 6540
   cgccagcaac gcggcctttt tacggttcct ggccttttgc tggccttttg ctcacatgtt 6600
   ctttcctgcg ttatcccctg attctgtgga taaccgtatt accgcctttg agtgagctga 6660
   taccgctcgc cgcagccgaa cgaccgagcg cagcgagtca gtgagcgagg aagcggaaga 6720
   gcgcctgatg cggtattttc tccttacgca tctgtgcggt atttcacacc gcatatggtg 6780
   cactctcagt acaatctgct ctgatgccgc atagttaagc cagtatacac tccgctatcg 6840
   ctacgtgact gggtcatggc tgcgccccga cacccgccaa cacccgctga cgcgccctga 6900
   cgggcttgtc tgctcccggc atccgcttac agacaagctg tgaccgtctc cgggagctgc 6960
   atgtgtcaga ggttttcacc gtcatcaccg aaacgcgcga ggcagctgcg gtaaagctca 7020
   tcagcgtggt cgtgaagcga ttcacagatg tctgcctgtt catccgcgtc cagctcgttg 7080
   agtttctcca gaagcgttaa tgtctggctt ctgataaagc gggccatgtt aagggcggtt 7140
   ttttcctgtt tggtcacttg atgcctccgt gtaaggggga atttctgttc atgggggtaa 7200
   tgataccgat gaaacgagag aggatgctca cgatacgggt tactgatgat gaacatgccc 7260
   ggttactgga acgttgtgag ggtaaacaac tggcggtatg gatgcggcgg gaccagagaa 7320
   aaatcactca gggtcaatgc cagcgcttcg ttaatacaga tgtaggtgtt ccacagggta 7380
   gccagcagca tcctgcgatg cagatccgga acataatggt gcagggcgct gacttccgcg 7440
   tttccagact ttacgaaaca cggaaaccga agaccattca tgttgttgct caggtcgcag 7500
   acgttttgca gcagcagtcg cttcacgttc gctcgcgtat cggtgattca ttctgctaac 7560
   cagtaaggca accccgccag cctagccggg tcctcaacga caggagcacg atcatgcgca 7620
   cccgtggcca ggacccaacg ctgcccgaga tgcgccgcgt gcggctgctg gagatggcgg 7680
   acgcgatgga tatgttctgc caagggttgg tttgcgcatt cacagttctc cgcaagaatt 7740
   gattggctcc aattcttgga gtggtgaatc cgttagcgag gtgccgccgg cttccattca 7800
   ggtcgaggtg gcccggctcc atgcaccgcg acgcaacgcg gggaggcaga caaggtatag 7860
   ggcggcgcct acaatccatg ccaacccgtt ccatgtgctc gccgaggcgg cataaatcgc 7920
   cgtgacgatc agcggtccag tgatcgaagt taggctggta agagccgcga gcgatccttg 7980
   aagctgtccc tgatggtcgt catctacctg cctggacagc atggcctgca acgcgggcat 8040
   cccgatgccg ccggaagcga gaagaatcat aatggggaag gccatccagc ctcgcgtc 8098
<210> 80
   <211> 4450
   <212> DNA
   <213> Artificial Sequence
<220>
   <223> Description of Artificial Sequence: Recombinant pAN443 plasmid
<400> 80
   ccatcgaatg gccagatgat taattcctaa tttttgttga cactctatca ttgatagagt 60
   tattttacca ctccctatca gtgatagaga aaagtgaaat gaatagttcg acaaaaatct 120
   agattagaaa ggaggattta aatatgagac agattactga tatttcacag ctgaaagaag 180
   ccataaaaca ataccattca gagggcaagt caatcggatt tgttccgacg atggggtttc 240
   tgcatgaggg gcatttaacc ttagcagaca aagcaagaca agaaaacgac gccgttatta 300
   tgagtatttt tgtgaatcct gcacaattcg gccctaatga agattttgaa gcatatccgc 360
   gcgatattga gcgggatgca gctcttgcag aaaacgccgg agtcgatatt ctttttacgc 420
   cagatgctca tgatatgtat cccggtgaaa agaatgtcac gattcatgta gaaagacgca 480
   cagacgtgtt atgcgggcgc tcaagagaag gacattttga cggggtcgcg atcgtactga 540
   cgaagctttt caatctagtc aagccgactc gtgcctattt cggtttaaaa gatgcgcagc 600
   aggtagctgt tgttgatggg ttaatcagcg acttcttcat ggatattgaa ttggttcctg 660
   tcgatacggt cagagaggaa gacggcttag ccaaaagctc tcgcaatgta tacttaacag 720
   ctgaggaaag aaaagaagcg cctaagctgt atcgggccct tcaaacaagt gcggaacttg 780
   tccaagccgg tgaaagagat cctgaagcgg tgataaaagc tgcaaaagat atcattgaaa 840
   cgactagcgg aaccatagac tatgtagagc tttattccta tccggaactc gagcctgtga 900
   atgaaattgc tggaaagatg attctcgctg ttgcagttgc tttttcaaaa gcgcgtttaa 960
   tagataatat cattattgat attcgtagaa aggaggtgaa ttaatatgta tcgtacgatg 1020
   atgagcggca aacttcacag ggcaactgtt acggaagcaa acctgaacta tgtgggaagc 1080
   attacaattg atgaagatct cattgatgct gtgggaatgc ttcctaatga aaaagtacaa 1140
   attgtgaata ataataatgg agcacgtctt gaaacgtata ttattcctgg taaacgggga 1200
   agcggcgtca tatgcttaaa cggtgcagcc gcacgccttg tgcaggaagg agataaggtc 1260
   attattattt cctacaaaat gatgtctgat caagaagcgg caagccatga gccgaaagtg 1320
   gctgttctga atgatcaaaa caaaattgaa caaatgctgg ggaacgaacc agcccgtaca 1380
   attttgtaaa ggatcccccg gggatccctc gaggtcgacc tgcaggggga ccatggtctc 1440
   agcgcttgga gccacccgca gttcgaaaaa taataagctt gacctgtgaa gtgaaaaatg 1500
   gcgcacattg tgcgacattt tttttgtctg ccgtttaccg ctactgcgtc acggatctcc 1560
   acgcgccctg tagcggcgca ttaagcgcgg cgggtgtggt ggttacgcgc agcgtgaccg 1620
   ctacacttgc cagcgcccta gcgcccgctc ctttcgcttt cttcccttcc tttctcgcca 1680
   cgttcgccgg ctttccccgt caagctctaa atcgggggct ccctttaggg ttccgattta 1740
   gtgctttacg gcacctcgac cccaaaaaac ttgattaggg tgatggttca cgtagtgggc 1800
   catcgccctg atagacggtt tttcgccctt tgacgttgga gtccacgttc tttaatagtg 1860
   gactcttgtt ccaaactgga acaacactca accctatctc ggtctattct tttgatttat 1920
   aagggatttt gccgatttcg gcctattggt taaaaaatga gctgatttaa caaaaattta 1980
   acgcgaattt taacaaaata ttaacgctta caatttcagg tggcactttt cggggaaatg 2040
   tgcgcggaac ccctatttgt ttatttttct aaatacattc aaatatgtat ccgctcatga 2100
   gacaataacc ctgataaatg cttcaataat attgaaaaag gaagagtatg agtattcaac 2160
   atttccgtgt cgcccttatt cccttttttg cggcattttg ccttcctgtt tttgctcacc 2220
   cagaaacgct ggtgaaagta aaagatgctg aagatcagtt gggtgcacga gtgggttaca 2280
   tcgaactgga tctcaacagc ggtaagatcc ttgagagttt tcgccccgaa gaacgttttc 2340
   caatgatgag cacttttaaa gttctgctat gtggcgcggt attatcccgt attgacgccg 2400
   ggcaagagca actcggtcgc cgcatacact attctcagaa tgacttggtt gagtactcac 2460
   cagtcacaga aaagcatctt acggatggca tgacagtaag agaattatgc agtgctgcca 2520
   taaccatgag tgataacact gcggccaact tacttctgac aacgatcgga ggaccgaagg 2580
   agctaaccgc ttttttgcac aacatggggg atcatgtaac tcgccttgat cgttgggaac 2640
   cggagctgaa tgaagccata ccaaacgacg agcgtgacac cacgatgcct gtagcaatgg 2700
   caacaacgtt gcgcaaacta ttaactggcg aactacttac tctagcttcc cggcaacaat 2760
   tgatagactg gatggaggcg gataaagttg caggaccact tctgcgctcg gcccttccgg 2820
   ctggctggtt tattgctgat aaatctggag ccggtgagcg tggctctcgc ggtatcattg 2880
   cagcactggg gccagatggt aagccctccc gtatcgtagt tatctacacg acggggagtc 2940
   aggcaactat ggatgaacga aatagacaga tcgctgagat aggtgcctca ctgattaagc 3000
   attggtagga attaatgatg tctcgtttag ataaaagtaa agtgattaac agcgcattag 3060
   agctgcttaa tgaggtcgga atcgaaggtt taacaacccg taaactcgcc cagaagctag 3120
   gtgtagagca gcctacattg tattggcatg taaaaaataa gcgggctttg ctcgacgcct 3180
   tagccattga gatgttagat aggcaccata ctcacttttg ccctttagaa ggggaaagct 3240
   ggcaagattt tttacgtaat aacgctaaaa gttttagatg tgctttacta agtcatcgcg 3300
   atggagcaaa agtacattta ggtacacggc ctacagaaaa acagtatgaa actctcgaaa 3360
   atcaattagc ctttttatgc caacaaggtt tttcactaga gaatgcatta tatgcactca 3420
   gcgcagtggg gcattttact ttaggttgcg tattggaaga tcaagagcat caagtcgcta 3480
   aagaagaaag ggaaacacct actactgata gtatgccgcc attattacga caagctatcg 3540
   aattatttga tcaccaaggt gcagagccag ccttcttatt cggccttgaa ttgatcatat 3600
   gcggattaga aaaacaactt aaatgtgaaa gtgggtctta aaagcagcat aacctttttc 3660
   cgtgatggta acttcactag tttaaaagga tctaggtgaa gatccttttt gataatctca 3720
   tgaccaaaat cccttaacgt gagttttcgt tccactgagc gtcagacccc gtagaaaaga 3780
   tcaaaggatc ttcttgagat cctttttttc tgcgcgtaat ctgctgcttg caaacaaaaa 3840
   aaccaccgct accagcggtg gtttgtttgc cggatcaaga gctaccaact ctttttccga 3900
   aggtaactgg cttcagcaga gcgcagatac caaatactgt ccttctagtg tagccgtagt 3960
   taggccacca cttcaagaac tctgtagcac cgcctacata cctcgctctg ctaatcctgt 4020
   taccagtggc tgctgccagt ggcgataagt cgtgtcttac cgggttggac tcaagacgat 4080
   agttaccgga taaggcgcag cggtcgggct gaacgggggg ttcgtgcaca cagcccagct 4140
   tggagcgaac gacctacacc gaactgagat acctacagcg tgagctatga gaaagcgcca 4200
   cgcttcccga agggagaaag gcggacaggt atccggtaag cggcagggtc ggaacaggag 4260
   agcgcacgag ggagcttcca gggggaaacg cctggtatct ttatagtcct gtcgggtttc 4320
   gccacctctg acttgagcgt cgatttttgt gatgctcgtc aggggggcgg agcctatgga 4380
   aaaacgccag caacgcggcc tttttacggt tcctggcctt ttgctggcct tttgctcaca 4440
   tgacccgaca 4450
<210> 81
   <211> 10212
   <212> DNA
   <213> Artificial Sequence
<220>
   <223> Description of Artificial Sequence: Recombinant pAN251 plasmid
<400> 81
   gcggccgcta aaaagagctt gaggatttgc ggagtgaaaa tcagacattg cggaatcagc 60
   tagagatgac agaagaggat tacaaggcac tgatcgatat catggatcgg gccagaaaaa 120
   tggttgtttc gaaggaagac ggaagaatga aaaaagcggc tcaagaaacg taaagaaacg 180
   cctgaaatga accggcccta tagtaagaat aggccggttg ttttgatttc tatgcagact 240
   ctcccggtgt catttcgcga tccatatcag gatgccagat gagcgggtct tcccctttgt 300
   cccgcgccat atcatactta acagttttaa agttcatttt gttccaaaat tccgctgatt 360
   tcattctcgg atttgtccgg atcggcattt tgaatgattt tgcaaattca accaaggctc 420
   tcccgtatcc cctgttctgg tagcccggaa gaacctcaag cttccacagc tccaaataat 480
   cctggcggtt gtcaaaatag ggattcgatt tgccgttaac ttgatacaga ctcattcgtg 540
   ctacaagttt atcgccaaaa taaatcccgt aaaaaggcga ggtgctgtca ttttcaataa 600
   tattatcctg aagttcttca agcattgaaa gctcctgaat gccgtattct ttgaatttct 660
   tgaattcttc cagcgtttta tagttgataa gcagacgttc tacctttgtc aaacaaatct 720
   ccccctttgt tgtttctaca tatattgtaa acgctttatt taaaaaatcc aaatatttaa 780
   actttaattt taagcacatg ggatctttga gaagtaattt cttcttactt ctgctatgat 840
   aatacgtaaa tgcgtcgacc gaaacagcag ttataaggca tgaagctgtc cggtttttgc 900
   aaaagtggct gtgactgtaa aaagaaatcg aaaaagaccg ttttgtgtga aaacggtctt 960
   tttgtttcct tttaaccaac tgccataact cgaggcctac ctagcttcca agaaagatat 1020
   cctaacagca caagagcgga aagatgtttt gttctacatc cagaacaacc tctgctaaaa 1080
   ttcctgaaaa attttgcaaa aagttgttga ctttatctac aaggtgtggt ataataatct 1140
   taacaacagc aggacgctct agaggaggag acatcatgaa aattggaatt atcggcggag 1200
   gctccgttgg tcttttatgc gcctattatt tgtcacttta tcacgacgtg actgttgtga 1260
   cgaggcggca agaacaggct gcggccattc agtctgaagg aatccggctt tataaaggcg 1320
   gggaggaatt cagggctgat tgcagtgcgg acacgagtat caattcggac tttgacctgc 1380
   ttgtcgtgac agtgaagcag catcagcttc aatctgtttt ttcgtcgctt gaacgaatcg 1440
   ggaagacgaa tatattattt ttgcaaaacg gcatggggca tatccacgac ctaaaagact 1500
   ggcacgttgg ccattccatt tatgttggaa tcgttgagca cggagctgta agaaaatcgg 1560
   atacagctgt tgatcataca ggcctaggtg cgataaaatg gagcgcgttc gacgatgctg 1620
   aaccagaccg gctgaacatc ttgtttcagc ataaccattc ggattttccg atttattatg 1680
   agacggattg gtaccgtctg ctgacgggca agctgattgt aaatgcgtgt attaatcctt 1740
   taactgcgtt attgcaagtg aaaaatggag aactgctgac aacgccagct tatctggctt 1800
   ttatgaagct ggtatttcag gaggcatgcc gcattttaaa acttgaaaat gaagaaaagg 1860
   cttgggagcg ggttcaggcc gtttgtgggc aaacgaaaga gaatcgttca tcaatgctgg 1920
   ttgacgtcat tggaggccgg cagacggaag ctgacgccat tatcggatac ttattgaagg 1980
   aagcaagtct tcaaggtctt gatgccgtcc acctagagtt tttatatggc agcatcaaag 2040
   cattggagcg aaatacaaac aaagtctttt gagctttttc ggtaacatgc tatactcatt 2100
   tcggatcact aactatttat tggagaaagg aagttctaga agatgcagct aactgaactt 2160
   tccatcaaaa atcagaatgt gtttgtacag cactatatag atggcaaaga agaaatgtct 2220
   tctttttttg attacagtat tcatcataag gacatgtggc gcgaaagact ggaagactta 2280
   tcttcccggt ttttcgcaag agaggaattg gcggcgtact taacctctta ccataataaa 2340
   ttcggttcaa gtgcgatgca gtctgctatt gagaagctga aggacccgtc aagtgccgct 2400
   gtagtcggcg gacagcaggc aggactttta acaggaccgc tttacaccat acataaaatc 2460
   atttcaatca ttgttttagc aaagcaacaa gaaaaggaac tgcaagtgcc tgtcatacca 2520
   atcttctggg tggctggaga agaccacgat ttggatgaga ttaattttgt tcacacatct 2580
   gaagagaatg ggcctgtgaa aaaaaagctg cctcagtctt attggaagaa atcatcagca 2640
   gcgagtacat cgcttgatca ggaaaagtgt gccgcgtgga tagatgatgt ttttgccgct 2700
   tttgaagaaa cagaccatac gaatacactt ctcgacaatg tgaaacgatg tttaagggaa 2760
   tctgttacgt ttactgactt ctttgaactg ctgatcgcgg atttgttcca agaagagggc 2820
   ttagttttat taaattctgg ggatcctgtt ttggcggatg agagaagatt ttcagcctga 2880
   tacagattaa atcagaacgc agaagcggtc tgataaaaca gaatttgcct ggcggcagta 2940
   gcgcggtggt cccacctgac cccatgccga actcagaagt gaaacgccgt agcgccgatg 3000
   gtagtgtggg gtctccccat gcgagagtag ggaactgcca ggcatcaaat aaaacgaaag 3060
   gctcagtcga aagactgggc ctttcgtttt atctgttgtt tgtcggtgaa cgctctcctg 3120
   agtaggacaa atccgccggg agcggatttg aacgttgcga agcaacggcc cggagggtgg 3180
   cgggcaggac gcccgccata aactgccagg catcaaatta agcagaaggc catcctgacg 3240
   gatggccttt ttgcgtttct acaaactctt ggtacccaga aaaagcggca aaagcggctg 3300
   ttaaaaaagc gaaatcgaag aagctgtctg ccgctaagac ggaatatcaa aagcgttctg 3360
   ctgttgtgtc atctttaaaa gtcacagccg atgaatccca gcaagatgtc ctaaaatact 3420
   tgaacaccca gaaagataaa ggaaatgcag accaaattca ttcttattat gtggtgaacg 3480
   ggattgctgt tcatgcctca aaagaggtta tggaaaaagt ggtgcagttt cccgaagtgg 3540
   aaaaggtgct tcctaatgag aaacggcagc tttttaagtc atcctcccca tttaatatga 3600
   aaaaagcaca gaaagctatt aaagcaactg acggtgtgga atggaatgta gaccaaatcg 3660
   atgccccaaa agcttgggca cttggatatg atggaactgg cacggttgtt gcgtccattg 3720
   ataccggggt ggaatggaat catccggcat taaaagagaa atatcgcgga tataatccgg 3780
   aaaatcctaa tgagcctgaa aatgaaatga actggtatga tgccgtagca ggcgaggcaa 3840
   gcccttatga tgatttggct catggaaccc acgtgacagg cacgatggtg ggctctgaac 3900
   ctgatggaac aaatcaaatc ggtgtagcac ctggcgcaaa atggattgct gttaaagcgt 3960
   tctctgaaga tggcggcact gatgctgaca ttttggaagc tggtgaatgg gttttagcac 4020
   caaaggacgc ggaaggaaat ccccacccgg aaatggctcc tgatgttgtc aataactcat 4080
   ggggaggggg ctctggactt gatgaatggt acagagacat ggtcaatgcc tggcgttcgg 4140
   ccgatatttt ccctgagttt tcagcgggga atacggatct ctttattccc ggcgggcctg 4200
   gttctatcgc aaatccggca aactatccag aatcgtttgc aactggagcg actgagaatt 4260
   ccaattcccc atggagagaa aagaaaatcg ctaatgttga ttactttgaa cttctgcata 4320
   ttcttgaatt taaaaaggct gaaagagtaa aagattgtgc tgaaatatta gagtataaac 4380
   aaaatcgtga aacaggcgaa agaaagttgt atcgagtgtg gttttgtaaa tccaggcttt 4440
   gtccaatgtg caactggagg agagcaatga aacatggcat tcagtcacaa aaggttgttg 4500
   ctgaagttat taaacaaaag ccaacagttc gttggttgtt tctcacatta acagttaaaa 4560
   atgtttatga tggcgaagaa ttaaataaga gtttgtcaga tatggctcaa ggatttcgcc 4620
   gaatgatgca atataaaaaa attaataaaa atcttgttgg ttttatgcgt gcaacggaag 4680
   tgacaataaa taataaagat aattcttata atcagcacat gcatgtattg gtatgtgtgg 4740
   aaccaactta ttttaagaat acagaaaact acgtgaatca aaaacaatgg attcaatttt 4800
   ggaaaaaggc aatgaaatta gactatgatc caaatgtaaa agttcaaatg attcgaccga 4860
   aaaataaata taaatcggat atacaatcgg caattgacga aactgcaaaa tatcctgtaa 4920
   aggatacgga ttttatgacc gatgatgaag aaaagaattt gaaacgtttg tctgatttgg 4980
   aggaaggttt acaccgtaaa aggttaatct cctatggtgg tttgttaaaa gaaatacata 5040
   aaaaattaaa ccttgatgac acagaagaag gcgatttgat tcatacagat gatgacgaaa 5100
   aagccgatga agatggattt tctattattg caatgtggaa ttgggaacgg aaaaattatt 5160
   ttattaaaga gtagttcaac aaacgggcca tattgttgta taagtgatga aatactgaat 5220
   ttaaaactta gtttatatgt ggtaaaatgt tttaatcaag tttaggagga attaattatg 5280
   aagtgtaatg aatgtaacag ggttcaatta aaagagggaa gcgtatcatt aaccctataa 5340
   actacgtctg ccctcattat tggagggtga aatgtgaata catcctattc acaatcgaat 5400
   ttacgacaca accaaatttt aatttggctt tgcattttat ctttttttag cgtattaaat 5460
   gaaatggttt tgaacgtctc attacctgat attgcaaatg attttaataa accacctgcg 5520
   agtacaaact gggtgaacac agcctttatg ttaacctttt ccattggaac agctgtatat 5580
   ggaaagctat ctgatcaatt aggcatcaaa aggttactcc tatttggaat tataataaat 5640
   tgtttcgggt cggtaattgg gtttgttggc cattctttct tttccttact tattatggct 5700
   cgttttattc aaggggctgg tgcagctgca tttccagcac tcgtaatggt tgtagttgcg 5760
   cgctatattc caaaggaaaa taggggtaaa gcatttggtc ttattggatc gatagtagcc 5820
   atgggagaag gagtcggtcc agcgattggt ggaatgatag cccattatat tcattggtcc 5880
   tatcttctac tcattcctat gataacaatt atcactgttc cgtttcttat gaaattatta 5940
   aagaaagaag taaggataaa aggtcatttt gatatcaaag gaattatact aatgtctgta 6000
   ggcattgtat tttttatgtt gtttacaaca tcatatagca tttcttttct tatcgttagc 6060
   gtgctgtcat tcctgatatt tgtaaaacat atcaggaaag taacagatcc ttttgttgat 6120
   cccggattag ggaaaaatat accttttatg attggagttc tttgtggggg aattatattt 6180
   ggaacagtag cagggtttgt ctctatggtt ccttatatga tgaaagatgt tcaccagcta 6240
   agtactgccg aaatcggaag tgtaattatt ttccctggaa caatgagtgt cattattttc 6300
   ggctacattg gtgggatact tgttgataga agaggtcctt tatacgtgtt aaacatcgga 6360
   gttacatttc tttctgttag ctttttaact gcttcctttc ttttagaaac aacatcatgg 6420
   ttcatgacaa ttataatcgt atttgtttta ggtgggcttt cgttcaccaa aacagttata 6480
   tcaacaattg tttcaagtag cttgaaacag caggaagctg gtgctggaat gagtttgctt 6540
   aactttacca gctttttatc agagggaaca ggtattgcaa ttgtaggtgg tttattatcc 6600
   atacccttac ttgatcaaag gttgttacct atggaagttg atcagtcaac ttatctgtat 6660
   agtaatttgt tattactttt ttcaggaatc attgtcatta gttggctggt taccttgaat 6720
   gtatataaac attctcaaag ggatttctaa atcgttaagg gatcaacttt gggagagagt 6780
   tcaaaattga tccttttttt ataacagttc gaagcggccg caattcttga agacgaaagg 6840
   gcctcgtgat acgcctattt ttataggtta atgtcatgat aataatggtt tcttagacgt 6900
   caggtggcac ttttcgggga aatgtgcgcg gaacccctat ttgtttattt ttctaaatac 6960
   attcaaatat gtatccgctc atgagacaat aaccctgata aatgcttcaa taatattgaa 7020
   aaaggaagag tatgagtatt caacatttcc gtgtcgccct tattcccttt tttgcggcat 7080
   tttgccttcc tgtttttgct cacccagaaa cgctggtgaa agtaaaagat gctgaagatc 7140
   agttgggtgc acgagtgggt tacatcgaac tggatctcaa cagcggtaag atccttgaga 7200
   gttttcgccc cgaagaacgt tttccaatga tgagcacttt taaagttctg ctatgtggcg 7260
   cggtattatc ccgtattgac gccgggcaag agcaactcgg tcgccgcata cactattctc 7320
   agaatgactt ggttgagtac tcaccagtca cagaaaagca tcttacggat ggcatgacag 7380
   taagagaatt atgcagtgct gccataacca tgagtgataa cactgcggcc aacttacttc 7440
   tgacaacgat cggaggaccg aaggagctaa ccgctttttt gcacaacatg ggggatcatg 7500
   taactcgcct tgatcgttgg gaaccggagc tgaatgaagc cataccaaac gacgagcgtg 7560
   acaccacgat gcctgcagca atggcaacaa cgttgcgcaa actattaact ggcgaactac 7620
   ttactctagc ttcccggcaa caattaatag actggatgga ggcggataaa gttgcaggac 7680
   cacttctgcg ctcggccctt ccggctggct ggtttattgc tgataaatct ggagccggtg 7740
   agcgtgggtc tcgcggtatc attgcagcac tggggccaga tggtaagccc tcccgtatcg 7800
   tagttatcta cacgacgggg agtcaggcaa ctatggatga acgaaataga cagatcgctg 7860
   agataggtgc ctcactgatt aagcattggt aactgtcaga ccaagtttac tcatatatac 7920
   tttagattga tttaaaactt catttttaat ttaaaaggat ctaggtgaag atcctttttg 7980
   ataatctcat gaccaaaatc ccttaacgtg agttttcgtt ccactgagcg tcagaccccg 8040
   tagaaaagat caaaggatct tcttgagatc ctttttttct gcgcgtaatc tgctgcttgc 8100
   aaacaaaaaa accaccgcta ccagcggtgg tttgtttgcc ggatcaagag ctaccaactc 8160
   tttttccgaa ggtaactggc ttcagcagag cgcagatacc aaatactgtc cttctagtgt 8220
   agccgtagtt aggccaccac ttcaagaact ctgtagcacc gcctacatac ctcgctctgc 8280
   taatcctgtt accagtggct gctgccagtg gcgataagtc gtgtcttacc gggttggact 8340
   caagacgata gttaccggat aaggcgcagc ggtcgggctg aacggggggt tcgtgcacac 8400
   agcccagctt ggagcgaacg acctacaccg aactgagata cctacagcgt gagctatgag 8460
   aaagcgccac gcttcccgaa gggagaaagg cggacaggta tccggtaagc ggcagggtcg 8520
   gaacaggaga gcgcacgagg gagcttccag ggggaaacgc ctggtatctt tatagtcctg 8580
   tcgggtttcg ccacctctga cttgagcgtc gatttttgtg atgctcgtca ggggggcgga 8640
   gcctatggaa aaacgccagc aacgcggcct ttttacggtt cctggccttt tgctggcctt 8700
   ttgctcacat gttctttcct gcgttatccc ctgattctgt ggataaccgt attaccgcct 8760
   ttgagtgagc tgataccgct cgccgcagcc gaacgaccga gcgcagcgag tcagtgagcg 8820
   aggaagcgga agagcgcctg atgcggtatt ttctccttac gcatctgtgc ggtatttcac 8880
   accgcatatg gtgcactctc agtacaatct gctctgatgc cgcatagtta agccagtata 8940
   cactccgcta tcgctacgtg actgggtcat ggctgcgccc cgacacccgc caacacccgc 9000
   tgacgcgccc tgacgggctt gtctgctccc ggcatccgct tacagacaag ctgtgaccgt 9060
   ctccgggagc tgcatgtgtc agaggttttc accgtcatca ccgaaacgcg cgaggcagct 9120
   gcggtaaagc tcatcagcgt ggtcgtgaag cgattcacag atgtctgcct gttcatccgc 9180
   gtccagctcg ttgagtttct ccagaagcgt taatgtctgg cttctgataa agcgggccat 9240
   gttaagggcg gttttttcct gtttggtcac ttgatgcctc cgtgtaaggg ggaatttctg 9300
   ttcatggggg taatgatacc gatgaaacga gagaggatgc tcacgatacg ggttactgat 9360
   gatgaacatg cccggttact ggaacgttgt gagggtaaac aactggcggt atggatgcgg 9420
   cgggaccaga gaaaaatcac tcagggtcaa tgccagcgct tcgttaatac agatgtaggt 9480
   gttccacagg gtagccagca gcatcctgcg atgcagatcc ggaacataat ggtgcagggc 9540
   gctgacttcc gcgtttccag actttacgaa acacggaaac cgaagaccat tcatgttgtt 9600
   gctcaggtcg cagacgtttt gcagcagcag tcgcttcacg ttcgctcgcg tatcggtgat 9660
   tcattctgct aaccagtaag gcaaccccgc cagcctagcc gggtcctcaa cgacaggagc 9720
   acgatcatgc gcacccgtgg ccaggaccca acgctgcccg agatgcgccg cgtgcggctg 9780
   ctggagatgg cggacgcgat ggatatgttc tgccaagggt tggtttgcgc attcacagtt 9840
   ctccgcaaga attgattggc tccaattctt ggagtggtga atccgttagc gaggtgccgc 9900
   cggcttccat tcaggtcgag gtggcccggc tccatgcacc gcgacgcaac gcggggaggc 9960
   agacaaggta tagggcggcg cctacaatcc atgccaaccc gttccatgtg ctcgccgagg 10020
   cggcataaat cgccgtgacg atcagcggtc cagtgatcga agttaggctg gtaagagccg 10080
   cgagcgatcc ttgaagctgt ccctgatggt cgtcatctac ctgcctggac agcatggcct 10140
   gcaacgcggg catcccgatg ccgccggaag cgagaagaat cataatgggg aaggccatcc 10200
   agcctcgcgt cg 10212
<210> 82
   <211> 10426
   <212> DNA
   <213> Artificial Sequence
<220>
   <223> Description of Artificial Sequence: Recombinant pAN267 plasmid
<400> 82
   aacaaaattc tccagtcttc acatcggttt gaaaggagga agcggaagaa tgaagtaaga 60
   gggatttttg actccgaagt aagtcttcaa aaaatcaaat aaggagtgtc aagaatgttt 120
   gcaaaacgat tcaaaacctc tttactgccg ttattcgctg gatttttatt gctgtttcat 180
   ttggttctgg caggaccggc ggctgcgagt gctgaaacgg cgaacaaatc gaatgagctt 240
   acagcaccgt cgatcaaaag cggaaccatt cttcatgcat ggaattggtc gttcaatacg 300
   ttaaaacaca atatgaagga tattcatgat gcaggatata cagccattca gacatctccg 360
   attaaccaag taaaggaagg gaatcaagga gataaaagca tgtcgaactg gtactggctg 420
   tatcagccga catcgtatca aattggcaac cgttacttag gtactgaaca agaatttaaa 480
   gaaatgtgtg cagccgctga agaatatggc ataaaggtca ttgttgacgc ggtcatcaat 540
   cataccacca gtgattatgc cgcgatttcc aatgaggtta agagtattcc aaactggaca 600
   catggaaaca cacaaattaa aaactggtct gatcgaaata gtacataatg gatttcctta 660
   cgcgaaatac gggcagacat ggcctgcccg gttattatta tttttgacac cagaccaact 720
   ggtaatggta gcgaccggcg ctcaggatcg tctcggtacc aagagtttgt agaaacgcaa 780
   aaaggccatc cgtcaggatg gccttctgct taatttgatg cctggcagtt tatggcgggc 840
   gtcctgcccg ccaccctccg ggccgttgct tcgcaacgtt caaatccgct cccggcggat 900
   ttgtcctact caggagagcg ttcaccgaca aacaacagat aaaacgaaag gcccagtctt 960
   tcgactgagc ctttcgtttt atttgatgcc tggcagttcc ctactctcgc atggggagac 1020
   cccacactac catcggcgct acggcgtttc acttctgagt tcggcatggg gtcaggtggg 1080
   accaccgcgc tactgccgcc aggcaaattc tgttttatca gaccgcttct gcgttctgat 1140
   ttaatctgta tcaggctgaa aatcttctct catccgccaa aacaggatcc atcacgaagc 1200
   gtcgtatcga aaaaattaat tttgcgcaac ggagaccacc gcttccttct tcttgccttg 1260
   tttcacaaac ggcatcattt cacgaagctt tcttcccact acttcgattt gatgttcgtt 1320
   ctcgcttgca ttgatagcgt tgaaacgagg acggtttact tggttttcga cgatccactc 1380
   ttttgcgaat gtaccgtttt ggatatcttt taatacttct ttcatagatt cttttacttt 1440
   agcgtccaca acgcgagggc ctgatacgaa atctccccac tgtgctgtgt cagagattga 1500
   atatctcatt cctgcaagtc cttcttcgta cataaggtct acgattaatt tcagctcatg 1560
   aagacactcg aagtatgcaa gttcaggctg ataacctgct tcagttaagg tttcaaatcc 1620
   ggctttgaca agcgcgctta atccgccgca aagaactgct tgctcaccga acaaatctgt 1680
   ttctgtttct tctttaaatg tcgtttctaa tacgcccgct cttgcgccgc cgattccttt 1740
   agcataagcg agggctttgt ctcttgcttc tccagtcaca tcttgataga ttgcgaacaa 1800
   tgcaggtacg ccagctcctt gctcatatgt tcttcttacc aagtgtcccg ggcctttagg 1860
   ggccactaag aatacatcta catccgccgg aggaacaatt tgatggaaat gcacgttaaa 1920
   tccatgagcg aatactaatg attttcctgc tgtcaattca tctttgattt cagcttcgta 1980
   tactttttgc tgctgctcat ccggaagcag aaccatgatg atttcggctt gggctgccgc 2040
   ttcttttact gaaaatactt tatgtccgtc ttcttgggct tgagtgaaag attttccttg 2100
   tctaacaccg acgatcacgt ctactccgct ttctttaagg ttcagggcat gtgcgtggcc 2160
   ttgcgaaccg tacccgataa ccgctactgt ttttccagcc aatacgttct ctttgatatc 2220
   accgttataa tatactttta ccatttcaat ctctcccttg ttatgtttta tacaatagat 2280
   attgttttat tggatgacgc cttttgctgg ttcccctcgc aaaagccgtt gtacctgttc 2340
   tcgcgatttc tttaatgcca taaggtttta ataactcaat aagcgcttca attttgttag 2400
   attcacctgt cacctgaaca acgatgctgt ctctgctgac atcaacgaca gaggctctaa 2460
   acggttctat gattccatta atctctgttc ttgttgaagg tgcggagaca accttgatta 2520
   aggccagctc cctctggaca atcgattgat ttgtgatgtc tgtgactttc agcacatcaa 2580
   tctgtttgtt gagctgtttc gttaactgtt caacatcatt ttcaccttca acatgaacga 2640
   cgaaggtgat tctggaaacg ccggctgttt ctgtgtgtcc aactgtaatg ctttcaatgt 2700
   tgtaatgcct ttttgtgaat agaccggtga tccggtttaa caccccggag cggttcacca 2760
   cagtcaatgt gataattctt ttcaaggttt cacccccacc atttcatgca gccctttccc 2820
   cggagccacc atcgggaata ctttttcttc gctggcaacc cgcacgtcaa tgacaacagg 2880
   ttctcttgat gttaatgcct cttccagctt ttcctttgct tccgcttctg atgaaattct 2940
   gatgccttta atgccgtatg cttcggacaa tttgacgaag tcaggctgag aagcgaattt 3000
   agattctgaa taacgttctt catagaaaat ttcctgccac tgtctgacca ttccgagaca 3060
   agcgttattt aaaatcacta ccttgaccgg aagatttaat tcgcgaataa catcgagttc 3120
   ttgaagcgtc atttggaatc cgccgtctcc gacaaccgcg acaacagtag catctttttc 3180
   ggccagctgt gcgccgatcg ccgccggaag accgaatccc atcgttccaa gtccgcctga 3240
   cgtgacccat ttatctgctt tttggaacgg ataaaattgc gctgaccaca tttgatgctg 3300
   gcctacatcc gttgcgacaa tggcctctcc ttttgtaaat tgatgaatat attcaatcaa 3360
   tttctgaggt ttaaaacctt cttcttcatt atctacatac cagagcggat actcttcttt 3420
   ccattctgcg agctgttttt tccattcgct tgaatcgctt tgtttgccgt cttgtttgat 3480
   cagctcctgc aggacaattt tgctgtctcc gactacagga atctgtgttt tcatgatttt 3540
   tccgatttca gctggatcaa tatcgatgtg ggctatcttt gcgtttctgg caaagtgttt 3600
   caggtttcct gtgacacggt catcaaaacg ggcgccgata ctgattaata gatcacattc 3660
   atgaagggcc atattggctg tataagtacc gtgcattccc gccatcccta ggaaaagcgg 3720
   atggtcagcc gggaagcctc cgagccccaa aagggtgtgt gccacaggga tttgctgctg 3780
   ttcagcataa ttttttaatt cttctgacgc ttttccgtgc agtacgcccg cacccgccag 3840
   gatcaccggt tttttcgcac tgctcacggc ttccacaagc ttgcggatct gcaaataatt 3900
   cggctctgtt gtcggctggt atcccgggag attcatctca tgatcgtagc tgaattctcc 3960
   ttcaattgtt gctacatctt tcggaatatc aatcaataca ggtccgggtc ttccagttgt 4020
   tgcaatatgg aacgcttctt taatgatgcg cggcagatct tccggctggc gaacctggta 4080
   gctgtgtttt gttactggca tcgtaatccc taaaatgtct gcttcctgaa atgcatcgct 4140
   cccgattaca gaggttgcta cctgccctgt aaagacgact aacggcaatg aatcaatcat 4200
   ggcatcagca aggcctgtaa caaggtttgt cgctcccggc ctgacgtggc aatgacgaca 4260
   ccggtttccg gagacccttg cgtatccctc cgctgcatga attgctcctt gttcgtgacg 4320
   ggaaggatat gtaccaacct gaatgtatag cttatcgtaa atcggaagca cagccccgcc 4380
   cggataaccg aagatcattt ctactttctc tttttttaat gattcaatca gcattaatcg 4440
   tccgctcatc gtctgtgtac attcggcaga tgctgaatcc acctgtacat tagtccccat 4500
   tttatctcct cctctagagc gtcctgctgt tgttaagatt attataccac accttgtaga 4560
   taaagtcaac aactttttgc aaaatttttc aggaatttta gcagaggttg ttctggatgt 4620
   agaacaaaac atctttccgc tcttgtgctg ttaggatatc tttcttggaa gctaggtagg 4680
   cctcgagtta tggcagttgg ttaaaaggaa acaaaaagac cgttttcaca caaaacggtc 4740
   tttttcgatt tctttttaca gtcacagcca cttttgcaaa aaccggacag cttcatgcct 4800
   tataactgct gtttcggtcg acgaagcggc cgccgtttaa acgaattcct gcagctggcg 4860
   aatggcgatt ttcgttcgtg aatacatgtt ataataacta taactaataa cgtaacgtga 4920
   ctggcaagag atatttttaa aacaatgaat aggtttacac ttactttagt tttatggaaa 4980
   tgaaagatca tatcatatat aatctagaat aaaattaact aaaataatta ttatctagat 5040
   aaaaaattta gaagccaatg aaatctataa ataaactaaa ttaagtttat ttaattaaca 5100
   actatggata taaaataggt actaatcaaa atagtgagga ggatatattt gaatacatac 5160
   gaacaaatta ataaagtgaa aaaaatactt cggaaacatt taaaaaataa ccttattggt 5220
   acttacatgt ttggatcagg agttgagagt ggactaaaac caaatagtga tcttgacttt 5280
   ttagtcgtcg tatctgaacc attgacagat caaagtaaag aaatacttat acaaaaaatt 5340
   agacctattt caaaaaaaat aggagataaa agcaacttac gatatattga attaacaatt 5400
   attattcagc aagaaatggt accgtggaat catcctccca aacaagaatt tatttatgga 5460
   gaatggttac aagagcttta tgaacaagga tacattcctc agaaggaatt aaattcagat 5520
   ttaaccataa tgctttacca agcaaaacga aaaaataaaa gaatatacgg aaattatgac 5580
   ttagaggaat tactacctga tattccattt tctgatgtga gaagagccat tatggattcg 5640
   tcagaggaat taatagataa ttatcaggat gatgaaacca actctatatt aactttatgc 5700
   cgtatgattt taactatgga cacgggtaaa atcataccaa aagatattgc gggaaatgca 5760
   gtggctgaat cttctccatt agaacatagg gagagaattt tgttagcagt tcgtagttat 5820
   cttggagaga atattgaatg gactaatgaa aatgtaaatt taactataaa ctatttaaat 5880
   aacagattaa aaaaattata aaaaaattga aaaaatggtg gaaacacttt tttcaatttt 5940
   tttgttttat tatttaatat ttgggaaata ttcattctaa ttggtaatca gattttagaa 6000
   aacaataaac ccttgcatag ggggatcgat atccgtttag gctgggcggt gatagcttct 6060
   cgttcaggca gtacgcctct tttcttttcc agacctgagg gaggcggaaa tggtgtgagg 6120
   ttcccgggga aaagccaaat aggcgatcgc gggagtgctt tatttgaaga tcaggctatc 6180
   actgcggtca atagatttca caatgtgatg gctggacagc ctgaggaact ctcgaacccg 6240
   aatggaaaca accagatatt tatgaatcag cgcggctcac atggcgttgt gctggcaaat 6300
   gcaggttcat cctctgtctc tatcaatacg gcaacaaaat tgcctgatgg caggtatgac 6360
   aataaagctg gagcgggttc atttcaagtg aacgatggta aactgacagg cacgatcaat 6420
   gccaggtctg tagctgtgct ttatcctgat gatattgcaa aagcgcctca tgttttcctt 6480
   gagaattaca aaacaggtgt aacacattct ttcaatgatc aactgacgat taccttgcgt 6540
   gcagatgcga atacaacaaa agccgtttat caaatcaata atggaccaga cgacaggcgt 6600
   ttaaggatgg agatcaattc acaatcggaa aaggagatcc aatttggcaa aacatacacc 6660
   atcatgttaa aaggaacgaa cagtgatggt gtaacgagga ccgagaaata cagttttgtt 6720
   aaaagagatc cagcgtcggc caaaaccatc ggctatcaaa atccgaatca ttggagccag 6780
   gtaaatgctt atatctataa acatgatggg agccgagtaa ttgaattgac cggatcttgg 6840
   cctggaaaac caatgactaa aaatgcagac ggaatttaca cgctgacgct gcctgcggac 6900
   acggatacaa ccaacgcaaa agtgattttt aataatggca gcgcccaagt gcccggtcag 6960
   aatcagcctg gctttgatta cgtgctaaat ggtttatata atgactcggg cttaagcggt 7020
   tctcttcccc attgagggca aggctagacg ggacttaccg aaagaaacca tcaatgatgg 7080
   tttctttttt gttcataaat cagacaaaac ttttctcttg caaaagtttg tgaagtgttg 7140
   cacaatataa atgtgaaata cttcacaaac aaaaagacat caaagagaaa cataccctgc 7200
   aaggatgctg atattgtctg catttgcgcc ggagcaaacc aaaaacctgg tgagacacgc 7260
   cttgaattag tagaaaagaa cttgaagatt ttcaaaggca tcgttagtga agtcatggcg 7320
   agcggatttg acggcatttt cttagtcggg cggcacctcg ctaacggatt caccactcca 7380
   agaattggag ccaatcaatt cttgcggaga actgtgaatg cgcaaaccaa cccttggcag 7440
   aacatatcca tcgcgtccgc catctccagc agccgcacgc ggcgcatctc gggcagcgtt 7500
   gggtcctggc cacgggtgcg catgatcgtg ctcctgtcgt tgaggacccg gctaggctgg 7560
   cggggttgcc ttactggtta gcagaatgaa tcaccgatac gcgagcgaac gtgaagcgac 7620
   tgctgctgca aaacgtctgc gacctgagca acaacatgaa tggtcttcgg tttccgtgtt 7680
   tcgtaaagtc tggaaacgcg gaagtcagcg ccctgcacca ttatgttccg gatctgcatc 7740
   gcaggatgct gctggctacc ctgtggaaca cctacatctg tattaacgaa gcgctggcat 7800
   tgaccctgag tgatttttct ctggtcccgc cgcatccata ccgccagttg tttaccctca 7860
   caacgttcca gtaaccgggc atgttcatca tcagtaaccc gtatcgtgag catcctctct 7920
   cgtttcatcg gtatcattac ccccatgaac agaaatcccc cttacacgga ggcatcagtg 7980
   accaaacagg aaaaaaccgc ccttaacatg gcccgcttta tcagaagcca gacattaacg 8040
   cttctggaga aactcaacga gctggacgcg gatgaacagg cagacatctg tgaatcgctt 8100
   cacgaccacg ctgatgagct ttaccgcagc tgcctcgcgc gtttcggtga tgacggtgaa 8160
   aacctctgac acatgcagct cccggagacg gtcacagctt gtctgtaagc ggatgccggg 8220
   agcagacaag cccgtcaggg cgcgtcagcg ggtgttggcg ggtgtcgggg cgcagccatg 8280
   acccagtcac gtagcgatag cggagtgtat actggcttaa ctatgcggca tcagagcaga 8340
   ttgtactgag agtgcaccat atgcggtgtg aaataccgca cagatgcgta aggagaaaat 8400
   accgcatcag gcgctcttcc gcttcctcgc tcactgactc gctgcgctcg gtcgttcggc 8460
   tgcggcgagc ggtatcagct cactcaaagg cggtaatacg gttatccaca gaatcagggg 8520
   ataacgcagg aaagaacatg tgagcaaaag gccagcaaaa ggccaggaac cgtaaaaagg 8580
   ccgcgttgct ggcgtttttc cataggctcc gcccccctga cgagcatcac aaaaatcgac 8640
   gctcaagtca gaggtggcga aacccgacag gactataaag ataccaggcg tttccccctg 8700
   gaagctccct cgtgcgctct cctgttccga ccctgccgct taccggatac ctgtccgcct 8760
   ttctcccttc gggaagcgtg gcgctttctc atagctcacg ctgtaggtat ctcagttcgg 8820
   tgtaggtcgt tcgctccaag ctgggctgtg tgcacgaacc ccccgttcag cccgaccgct 8880
   gcgccttatc cggtaactat cgtcttgagt ccaacccggt aagacacgac ttatcgccac 8940
   tggcagcagc cactggtaac aggattagca gagcgaggta tgtaggcggt gctacagagt 9000
   tcttgaagtg gtggcctaac tacggctaca ctagaaggac agtatttggt atctgcgctc 9060
   tgctgaagcc agttaccttc ggaaaaagag ttggtagctc ttgatccggc aaacaaacca 9120
   ccgctggtag cggtggtttt tttgtttgca agcagcagat tacgcgcaga aaaaaaggat 9180
   ctcaagaaga tcctttgatc ttttctacgg ggtctgacgc tcagtggaac gaaaactcac 9240
   gttaagggat tttggtcatg agattatcaa aaaggatctt cacctagatc cttttaaatt 9300
   aaaaatgaag ttttaaatca atctaaagta tatatgagta aacttggtct gacagttacc 9360
   aatgcttaat cagtgaggca cctatctcag cgatctgtct atttcgttca tccatagttg 9420
   cctgactccc cgtcgtgtag ataactacga tacgggaggg cttaccatct ggccccagtg 9480
   ctgcaatgat accgcgagac ccacgctcac cggctccaga tttatcagca ataaaccagc 9540
   cagccggaag ggccgagcgc agaagtggtc ctgcaacttt atccgcctcc atccagtcta 9600
   ttaattgttg ccgggaagct agagtaagta gttcgccagt taatagtttg cgcaacgttg 9660
   ttgccattgc tgcaggcatc gtggtgtcac gctcgtcgtt tggtatggct tcattcagct 9720
   ccggttccca acgatcaagg cgagttacat gatcccccat gttgtgcaaa aaagcggtta 9780
   gctccttcgg tcctccgatc gttgtcagaa gtaagttggc cgcagtgtta tcactcatgg 9840
   ttatggcagc actgcataat tctcttactg tcatgccatc cgtaagatgc ttttctgtga 9900
   ctggtgagta ctcaaccaag tcattctgag aatagtgtat gcggcgaccg agttgctctt 9960
   gcccggcgtc aacacgggat aataccgcgc cacatagcag aactttaaaa gtgctcatca 10020
   ttggaaaacg ttcttcgggg cgaaaactct caaggatctt accgctgttg agatccagtt 10080
   cgatgtaacc cactcgtgca cccaactgat cttcagcatc ttttactttc accagcgttt 10140
   ctgggtgagc aaaaacagga aggcaaaatg ccgcaaaaaa gggaataagg gcgacacgga 10200
   aatgttgaat actcatactc ttcctttttc aatattattg aagcatttat cagggttatt 10260
   gtctcatgag cggatacata tttgaatgta tttagaaaaa taaacaaata ggggttccgc 10320
   gcacatttcc ccgaaaagtg ccacctgacg tctaagaaac cattattatc atgacattaa 10380
   cctataaaaa taggcgtatc acgaggccct ttcgtcttca agaatt                10426
<210> 83
   <211> 4191
   <212> DNA
   <213> Artificial Sequence
<220>
   <223> Description of Artificial Sequence: Recombinant pAN263 plasmid
<400> 83
   ccaatacgca aaccgcctct ccccgcgcgt tggccgattc attaatgcag cccggatccg 60
   aaagaagagg atatcccggt tttacagaag gcattggatg atccaaaggt gtccatcaga 120
   agacaggctg ttgtgtactt aggaatgatt gaaacacctg atgttcttcc tctattgtat 180
   aaagcacttg aggacaaagc tgtatcagtc agaagaacgg ccggagactg cctgtctgat 240
   atcggcgatc ctcaagccat tcctgctatg atcaagtcat taagcgactc cagcaagctt 300
   gttcgctggc gtgccgccat gttcctgtac gaagtcggcg atgaaagtgc aattgaagct 360
   ttgcgcgctg ccgaagatga ccccgaattt gaggtcagcc ttcaagtcaa aatggcgctt 420
   gaacgtattg agcatggaga agaagcaaaa ggttctgttt ggaaacaaat gacggaaagc 480
   agaaaaaaag gcgaataaag ataaaaaagg tgcagatcat gcaccttttt tatgtgaatt 540
   ggtcgaccga aacagcagtt ataaggcatg aagctgtccg gtttttgcaa aagtggctgt 600
   gactgtaaaa agaaatcgaa aaagaccgtt ttgtgtgaaa acggtctttt tgtttccttt 660
   taaccaactg ccataactcg aggcctacct agcttccaag aaagatatcc taacagcaca 720
   agagcggaaa gatgttttgt tctacatcca gaacaacctc tgctaaaatt cctgaaaaat 780
   tttgcaaaaa gttgttgact ttatctacaa ggtgtggtat aataatctta acaacagcag 840
   gacgctctag aggaggagac accatggcag aattacgcag taatatgatc acacaaggaa 900
   tcgatagagc tccgcaccgc agtttgcttc gtgcagcagg ggtaaaagaa gaggatttcg 960
   gcaagccgtt tattgcggtg tgtaattcat acattgatat cgttcccggt catgttcact 1020
   tgcaggagtt tgggaaaatc gtaaaagaag caatcagaga agcagggggc gttccgtttg 1080
   aatttaatac cattggggta gatgatggca tcgcaatggg gcatatcggt atgagatatt 1140
   cgctgccaag ccgtgaaatt atcgcagact ctgtggaaac ggttgtatcc gcacactggt 1200
   ttgacggaat ggtctgtatt ccgaactgcg acaaaatcac accgggaatg cttatggcgg 1260
   caatgcgcat caacattccg acgatttttg tcagcggcgg accgatggcg gcaggaagaa 1320
   caagttacgg gcgaaaaatc tccctttcct cagtattcga aggggtaggc gcctaccaag 1380
   cagggaaaat caacgaaaac gagcttcaag aactagagca gttcggatgc ccaacgtgcg 1440
   ggtcttgctc aggcatgttt acggcgaact caatgaactg tctgtcagaa gcacttggtc 1500
   ttgctttgcc gggtaatgga accattctgg caacatctcc ggaacgcaaa gagtttgtga 1560
   gaaaatcggc tgcgcaatta atggaaacga ttcgcaaaga tatcaaaccg cgtgatattg 1620
   ttacagtaaa agcgattgat aacgcgtttg cactcgatat ggcgctcgga ggttctacaa 1680
   ataccgttct tcataccctt gcccttgcaa acgaagccgg cgttgaatac tctttagaac 1740
   gcattaacga agtcgctgag cgcgtgccgc acttggctaa gctggcgcct gcatcggatg 1800
   tgtttattga agatcttcac gaagcgggcg gcgtttcagc ggctctgaat gagctttcga 1860
   agaaagaagg agcgcttcat ttagatgcgc tgactgttac aggaaaaact cttggagaaa 1920
   ccattgccgg acatgaagta aaggattatg acgtcattca cccgctggat caaccattca 1980
   ctgaaaaggg aggccttgct gttttattcg gtaatctagc tccggacggc gctatcatta 2040
   aaacaggcgg cgtacagaat gggattacaa gacacgaagg gccggctgtc gtattcgatt 2100
   ctcaggacga ggcgcttgac ggcattatca accgaaaagt aaaagaaggc gacgttgtca 2160
   tcatcagata cgaagggcca aaaggcggac ctggcatgcc ggaaatgctg gcgccaacat 2220
   cccaaatcgt tggaatggga ctcgggccaa aagtggcatt gattacggac ggacgttttt 2280
   ccggagcctc ccgtggcctc tcaatcggcc acgtatcacc tgaggccgct gagggcgggc 2340
   cgcttgcctt tgttgaaaac ggagaccata ttatcgttga tattgaaaaa cgcatcttgg 2400
   atgtacaagt gccagaagaa gagtgggaaa aacgaaaagc gaactggaaa ggttttgaac 2460
   cgaaagtgaa aaccggctac ctggcacgtt attctaaact tgtgacaagt gccaacaccg 2520
   gcggtattat gaaaatctag acccctggcg taatagcgaa gaggcccgca ccgatcgccc 2580
   ttcccaacag ttgcgcagcc tgaatggcga atgagcttgc gccgtcccgt caagtcagcg 2640
   taatgctctg ccagtgttac aaccaattaa ccaattctga ttagaaaaac tcatcgagca 2700
   tcaaatgaaa ctgcaattta ttcatatcag gattatcaat accatatttt tgaaaaagcc 2760
   gtttctgtaa tgaaggagaa aactcaccga ggcagttcca taggatggca agatcctggt 2820
   atcggtctgc gattccgact cgtccaacat caatacaacc tattaatttc ccctcgtcaa 2880
   aaataaggtt atcaagtgag aaatcaccat gagtgacgac tgaatccggt gagaatggca 2940
   aaaggttatg catttctttc cagacttgtt caacaggcca gccattacgc tcgtcatcaa 3000
   aatcactcgc atcaaccaaa ccgttattca ttcgtgattg cgcctgagcg agacgaaata 3060
   cgcgatcgct gttaaaagga caattacaaa caggaatcga atgcaaccgg cgcaggaaca 3120
   ctgccagcgc atcaacaata ttttcacctg aatcaggata ttcttctaat acctggaatg 3180
   ctgttttccc agggatcgca gtggtgagta accatgcatc atcaggagta cggataaaat 3240
   gcttgatggt cggaagaggc ataaattccg tcagccagtt tagtctgacc atctcatctg 3300
   taacatcatt ggcaacgcta cctttgccat gtttcagaaa caactctggc gcatcgggct 3360
   tcccatacaa tcaatagatt gtcgcacctg attgcccgac attatcgcga gcccatttat 3420
   acccatataa atcagcatcc atgttggaat ttaatcgcgg cctcgacgag caagacgttt 3480
   cccgttgaat atggctcata acaccccttg tattactgtt tatgtaagca gacagtttta 3540
   ttgttcatga tgatatattt ttatcttgtg caatgtaaca tcagagattt tgagacactc 3600
   gacaagatga tcttcttgag atcgttttgg tctgcgcgta atctcttgct ctgaaaacga 3660
   aaaaaccgcc ttgcagggcg gtttttcgaa ggttctctga gctaccaact ctttgaaccg 3720
   aggtaactgg cttggaggag cgcagtcacc aaaacttgtc ctttcagttt agccttaacc 3780
   ggcgcatgac ttcaagacta actcctctaa atcaattacc agtggctgct gccagtggtg 3840
   cttttgcatg tctttccggg ttggactcaa gacgatagtt accggataag gcgcagcggt 3900
   cggactgaac ggggggttcg tgcatacagt ccagcttgga gcgaactgcc tacccggaac 3960
   tgagtgtcag gcgtggaatg agacaaacgc ggccataaca gcggaatgac accggtaaac 4020
   cgaaaggcag gaacaggaga gcgcacgagg gagccgccag gggaaacgcc tggtatcttt 4080
   atagtcctgt cgggtttcgc caccactgat ttgagcgtca gatttcgtga tgcttgtcag 4140
   gggggcggag cctatggaaa aacggctttg ccgcggccct ctcacttccc t 4191
<210> 84
   <211> 702
   <212> DNA
   <213> Bacillus subtilis
<220>
   <221> CDS
   <222> (1)..(699)
<400> 84
   ttg tta ctg gtt atc gat gtg ggg aac acc aat act gta ctt ggt gta      48
   Met Leu Leu Val Ile Asp Val Gly Asn Thr Asn Thr Val Leu Gly Val
   1               5                   10                  15
   tat cat gat gga aaa tta gaa tat cac tgg cgt ata gaa aca agc agg      96
   Tyr His Asp Gly Lys Leu Glu Tyr His Trp Arg Ile Glu Thr Ser Arg
               20                  25                  30
   cat aaa aca gaa gat gag ttt ggg atg att ttg cgc tcc tta ttt gat      144
   His Lys Thr Glu Asp Glu Phe Gly Met Ile Leu Arg Ser Leu Phe Asp
           35                  40                  45
   cac tcc ggg ctt atg ttt gaa cag ata gat ggc att att att tcg tca      192
   His Ser Gly Leu Met Phe Glu Gln Ile Asp Gly Ile Ile Ile Ser Ser
       50                  55                  60
   gta gtg ccg cca atc atg ttt gcg tta gaa aga atg tgc aca aaa tac      240
   Val Val Pro Pro Ile Met Phe Ala Leu Glu Arg Met Cys Thr Lys Tyr
   65                  70                  75                  80
   ttt cat atc gag cct caa att gtt ggt cca ggt atg aaa acc ggt tta      288
   Phe His Ile Glu Pro Gln Ile Val Gly Pro Gly Met Lys Thr Gly Leu
                   85                  90                  95
   aat ata aaa tat gac aat ccg aaa gaa gta ggg gca gac aga atc gta      336
   Asn Ile Lys Tyr Asp Asn Pro Lys Glu Val Gly Ala Asp Arg Ile Val
               100                 105                 110
   aat gct gtc gct gcg ata cac ttg tac ggc aat cca tta att gtt gtc      384
   Asn Ala Val Ala Ala Ile His Leu Tyr Gly Asn Pro Leu Ile Val Val
           115                 120                 125
   gat ttc gga acc gcc aca acg tac tgc tat att gat gaa aac aaa caa      432
   Asp Phe Gly Thr Ala Thr Thr Tyr Cys Tyr Ile Asp Glu Asn Lys Gln
       130                 135                 140
   tac atg ggc ggg gcg att gcc cct ggg att aca att tcg aca gag gcg      480
   Tyr Met Gly Gly Ala Ile Ala Pro Gly Ile Thr Ile Ser Thr Glu Ala
   145                 150                 155                 160
   ctt tac tcg cgt gca gca aag ctt cct cgt atc gaa atc acc cgg ccc      528
   Leu Tyr Ser Arg Ala Ala Lys Leu Pro Arg Ile Glu Ile Thr Arg Pro
                   165                 170                 175
   gac aat att atc gga aaa aac act gtt agc gcg atg caa tct gga att      576
   Asp Asn Ile Ile Gly Lys Asn Thr Val Ser Ala Met Gln Ser Gly Ile
               180                 185                 190
   tta ttt ggc tat gtc ggc caa gtg gaa gga atc gtt aag cga atg aaa      624
   Leu Phe Gly Tyr Val Gly Gln Val Glu Gly Ile Val Lys Arg Met Lys
           195                 200                 205
   tgg cag gca aaa cag gac cca agg tca ttg cga cag gag gcc tgg cgc      672
   Trp Gln Ala Lys Gln Asp Pro Arg Ser Leu Arg Gln Glu Ala Trp Arg
       210                 215                 220
   cgc tca ttg cga acg aat cag att gta tag                              702
   Arg Ser Leu Arg Thr Asn Gln Ile Val
   225                 230
<210> 85
   <211> 233
   <212> PRT
   <213> Bacillus subtilis
<400> 85
   Met Leu Leu Val Ile Asp Val Gly Asn Thr Asn Thr Val Leu Gly Val
   1               5                   10                  15
   Tyr His Asp Gly Lys Leu Glu Tyr His Trp Arg Ile Glu Thr Ser Arg
               20                  25                  30
   His Lys Thr Glu Asp Glu Phe Gly Met Ile Leu Arg Ser Leu Phe Asp
           35                  40                  45
   His Ser Gly Leu Met Phe Glu Gln Ile Asp Gly Ile Ile Ile Ser Ser
       50                  55                  60
   Val Val Pro Pro Ile Met Phe Ala Leu Glu Arg Met Cys Thr Lys Tyr
   65                  70                  75                  80
   Phe His Ile Glu Pro Gln Ile Val Gly Pro Gly Met Lys Thr Gly Leu
                   85                  90                  95
   Asn Ile Lys Tyr Asp Asn Pro Lys Glu Val Gly Ala Asp Arg Ile Val
               100                 105                 110
   Asn Ala Val Ala Ala Ile His Leu Tyr Gly Asn Pro Leu Ile Val Val
           115                 120                 125
   Asp Phe Gly Thr Ala Thr Thr Tyr Cys Tyr Ile Asp Glu Asn Lys Gln
       130                 135                 140
   Tyr Met Gly Gly Ala Ile Ala Pro Gly Ile Thr Ile Ser Thr Glu Ala
   145                 150                 155                 160
   Leu Tyr Ser Arg Ala Ala Lys Leu Pro Arg Ile Glu Ile Thr Arg Pro
                   165                 170                 175
   Asp Asn Ile Ile Gly Lys Asn Thr Val Ser Ala Met Gln Ser Gly Ile
               180                 185                 190
   Leu Phe Gly Tyr Val Gly Gln Val Glu Gly Ile Val Lys Arg Met Lys
           195                 200                 205
   Trp Gln Ala Lys Gln Asp Pro Arg Ser Leu Arg Gln Glu Ala Trp Arg
       210                 215                 220
   Arg Ser Leu Arg Thr Asn Gln Ile Val
   225                 230
<210> 86
   <211> 1623
   <212> DNA
   <213> Bacillus subtilis
<220>
   <221> CDS
   <222> (1)..(1620)
<400> 86
   atg tat ttg gca ttc cag gtg caa aaa ttg atg cgg tat ttg acg ctt      48
   Met Tyr Leu Ala Phe Gln Val Gln Lys Leu Met Arg Tyr Leu Thr Leu
   1               5                   10                  15
   tac aag ata aag gac ctg aaa tta tcg ttg ccc ggc acg aac aaa acg      96
   Tyr Lys Ile Lys Asp Leu Lys Leu Ser Leu Pro Gly Thr Asn Lys Thr
               20                  25                  30
   cag caa ttc atg gcc caa gca gtc ggc cgt tta act gga aaa ccg gga     144
   Gln Gln Phe Met Ala Gln Ala Val Gly Arg Leu Thr Gly Lys Pro Gly
           35                  40                  45
   gtc gtg tta gtc aca tca gga ccg ggt gcc tct aac ttg gca aca ggc     192
   Val Val Leu Val Thr Ser Gly Pro Gly Ala Ser Asn Leu Ala Thr Gly
       50                  55                  60
   ctg ctg aca gcg aac act gaa gga gac cct gtc gtt gcg ctt gct gga     240
   Leu Leu Thr Ala Asn Thr Glu Gly Asp Pro Val Val Ala Leu Ala Gly
   65                  70                  75                  80
   aac gtg atc cgt gca tat cgt tta aaa cgg aca cat caa tct ttg gat     288
   Asn Val Ile Arg Ala Tyr Arg Leu Lys Arg Thr His Gln Ser Leu Asp
                   85                  90                  95
   aat gcg gcg cta ttc cag ccg att aca aaa tac agt gta gaa gtt caa     336
   Asn Ala Ala Leu Phe Gln Pro Ile Thr Lys Tyr Ser Val Glu Val Gln
               100                 105                 110
   gat gta aaa aat ata ccg gaa gct gtt aca aat gca ttt agg ata gcg     384
   Asp Val Lys Asn Ile Pro Glu Ala Val Thr Asn Ala Phe Arg Ile Ala
           115                 120                 125
   tca gca ggg cag gct ggg gcc gct ttt gtg agc ttt ccg caa gat gtt     432
   Ser Ala Gly Gln Ala Gly Ala Ala Phe Val Ser Phe Pro Gln Asp Val
       130                 135                 140
   gtg aat gaa gtc aca aat acg aaa aac gtg cgt gct gtt gca gcg cca     480
   Val Asn Glu Val Thr Asn Thr Lys Asn Val Arg Ala Val Ala Ala Pro
   145                 150                 155                 160
   aaa ctc ggt cct gca gca gat gat gca atc agt gcg gcc ata gca aaa 528
   Lys Leu Gly Pro Ala Ala Asp Asp Ala Ile Ser Ala Ala Ile Ala Lys
                   165                 170                 175
   atc caa aca gca aaa ctt cct gtc gtt ttg gtc ggc atg aaa ggc gga 576
   Ile Gln Thr Ala Lys Leu Pro Val Val Leu Val Gly Met Lys Gly Gly
               180                 185                 190
   aga ccg gaa gca att aaa gcg gtt cgc aag ctt ttg aaa aag gtt cag 624
   Arg Pro Glu Ala Ile Lys Ala Val Arg Lys Leu Leu Lys Lys Val Gln
           195                 200                 205
   ctt cca ttt gtt gaa aca tat caa gct gcc ggt acc ctt tct aga gat 672
   Leu Pro Phe Val Glu Thr Tyr Gln Ala Ala Gly Thr Leu Ser Arg Asp
       210                 215                 220
   tta gag gat caa tat ttt ggc cgt atc ggt ttg ttc cgc aac cag cct 720
   Leu Glu Asp Gln Tyr Phe Gly Arg Ile Gly Leu Phe Arg Asn Gln Pro
   225                 230                 235                 240
   ggc gat tta ctg cta gag cag gca gat gtt gtt ctg acg atc ggc tat 768
   Gly Asp Leu Leu Leu Glu Gln Ala Asp Val Val Leu Thr Ile Gly Tyr
                   245                 250                 255
   gac ccg att gaa tat gat ccg aaa ttc tgg aat atc aat gga gac cgg 816
   Asp Pro Ile Glu Tyr Asp Pro Lys Phe Trp Asn Ile Asn Gly Asp Arg
               260                 265                 270
   aca att atc cat tta gac gag att atc gct gac att gat cat gct tac 864
   Thr Ile Ile His Leu Asp Glu Ile Ile Ala Asp Ile Asp His Ala Tyr
           275                 280                 285
   cag cct gat ctt gaa ttg atc ggt gac att ccg tcc acg atc aat cat 912
   Gln Pro Asp Leu Glu Leu Ile Gly Asp Ile Pro Ser Thr Ile Asn His
       290                 295                 300
   atc gaa cac gat gct gtg aaa gtg gaa ttt gca gag cgt gag cag aaa 960
   Ile Glu His Asp Ala Val Lys Val Glu Phe Ala Glu Arg Glu Gln Lys
   305                 310                 315                 320
   atc ctt tct gat tta aaa caa tat atg cat gaa ggt gag cag gtg cct 1008
   Ile Leu Ser Asp Leu Lys Gln Tyr Met His Glu Gly Glu Gln Val Pro
                   325                 330                 335
   gca gat tgg aaa tca gac aga gcg cac cct ctt gaa atc gtt aaa gag 1056
   Ala Asp Trp Lys Ser Asp Arg Ala His Pro Leu Glu Ile Val Lys Glu
               340                 345                 350
   ttg cgt aat gca gtc gat gat cat gtt aca gta act tgc gat atc ggt 1104
   Leu Arg Asn Ala Val Asp Asp His Val Thr Val Thr Cys Asp Ile Gly
           355                 360                 365
   tcg cac tcc att tgg atg tca cgt tat ttc cgc agc tac gag ccg tta 1152
   Ser His Ser Ile Trp Met Ser Arg Tyr Phe Arg Ser Tyr Glu Pro Leu
       370                 375                 380
   aca tta atg atc agt aac ggt atg caa aca ctc ggc gtt gcg ctt cct 1200
   Thr Leu Met Ile Ser Asn Gly Met Gln Thr Leu Gly Val Ala Leu Pro
   385                 390                 395                 400
   tgg gca atc ggc gct tca ttg gtg aaa ccg gga gaa aaa gtg gtt tct 1248
   Trp Ala Ile Gly Ala Ser Leu Val Lys Pro Gly Glu Lys Val Val Ser
                   405                 410                 415
   gtc tct ggt gac ggc ggt ttc tta ttc tca gca atg gaa tta gag aca 1296
   Val Ser Gly Asp Gly Gly Phe Leu Phe Ser Ala Met Glu Leu Glu Thr
                   420                     425                     430
   gca gtt cga cta aaa gca cca att gta cac att gta tgg aac gac agc     1344
   Ala Val Arg Leu Lys Ala Pro Ile Val His Ile Val Trp Asn Asp Ser
           435                     440                     445
   aca tat gac atg gtg cat ttc cag caa ttg aaa aaa tat aac cgt aca     1392
   Thr Tyr Asp Met Val His Phe Gln Gln Leu Lys Lys Tyr Asn Arg Thr
       450                     455                     460
   tct gcg gtc gat ttc gga aat atc gat atc gtg aaa tat gcg gaa agc     1440
   Ser Ala Val Asp Phe Gly Asn Ile Asp Ile Val Lys Tyr Ala Glu Ser
   465                     470                     475                     480
   ttc gga gca act gcg ttg cgc gta gaa tca cca gac cag ctg gca gat     1488
   Phe Gly Ala Thr Ala Leu Arg Val Glu Ser Pro Asp Gln Leu Ala Asp
                   485                     490                     495
   gtt ctg cgt caa ggc atg aac gct gaa ggt cct gtc atc atc gat gtc     1536
   Val Leu Arg Gln Gly Met Asn Ala Glu Gly Pro Val Ile Ile Asp Val
               500                     505                     510
   ccg gtt gac tac agt gat aac att aat tta gca agt gac aag ctt ccg     1584
   Pro Val Asp Tyr Ser Asp Asn Ile Asn Leu Ala Ser Asp Lys Leu Pro
           515                     520                     525
   aaa gaa ttc ggg gaa ctc atg aaa acg aaa gct ctc tag                 1623
   Lys Glu Phe Gly Glu Leu Met Lys Thr Lys Ala Leu
       530                     535                     540
<210> 87
   <211> 540
   <212> PRT
   <213> Bacillus subtilis
<400> 87
   Met Tyr Leu Ala Phe Gln Val Gln Lys Leu Met Arg Tyr Leu Thr Leu
   1               5                   10                  15
   Tyr Lys Ile Lys Asp Leu Lys Leu Ser Leu Pro Gly Thr Asn Lys Thr
               20                  25                  30
   Gln Gln Phe Met Ala Gln Ala Val Gly Arg Leu Thr Gly Lys Pro Gly
           35                  40                  45
   Val Val Leu Val Thr Ser Gly Pro Gly Ala Ser Asn Leu Ala Thr Gly
       50                  55                  60
   Leu Leu Thr Ala Asn Thr Glu Gly Asp Pro Val Val Ala Leu Ala Gly
   65                  70                  75                  80
   Asn Val Ile Arg Ala Tyr Arg Leu Lys Arg Thr His Gln Ser Leu Asp
                   85                  90                  95
   Asn Ala Ala Leu Phe Gln Pro Ile Thr Lys Tyr Ser Val Glu Val Gln
               100                 105                 110
   Asp Val Lys Asn Ile Pro Glu Ala Val Thr Asn Ala Phe Arg Ile Ala
           115                 120                 125
   Ser Ala Gly Gln Ala Gly Ala Ala Phe Val Ser Phe Pro Gln Asp Val
       130                 135                 140
   Val Asn Glu Val Thr Asn Thr Lys Asn Val Arg Ala Val Ala Ala Pro
   145                 150                 155                 160
   Lys Leu Gly Pro Ala Ala Asp Asp Ala Ile Ser Ala Ala Ile Ala Lys
   165 170 175
   Ile Gln Thr Ala Lys Leu Pro Val Val Leu Val Gly Met Lys Gly Gly
   180 185 190
   Arg Pro Glu Ala Ile Lys Ala Val Arg Lys Leu Leu Lys Lys Val Gln
   195 200 205
   Leu Pro Phe Val Glu Thr Tyr Gln Ala Ala Gly Thr Leu Ser Arg Asp
   210 215 220
   Leu Glu Asp Gln Tyr Phe Gly Arg Ile Gly Leu Phe Arg Asn Gln Pro
   225 230 235 240
   Gly Asp Leu Leu Leu Glu Gln Ala Asp Val Val Leu Thr Ile Gly Tyr
   245 250 255
   Asp Pro Ile Glu Tyr Asp Pro Lys Phe Trp Asn Ile Asn Gly Asp Arg
   260 265 270
   Thr Ile Ile His Leu Asp Glu Ile Ile Ala Asp Ile Asp His Ala Tyr
   275 280 285
   Gln Pro Asp Leu Glu Leu Ile Gly Asp Ile Pro Ser Thr Ile Asn His
   290 295 300
   Ile Glu His Asp Ala Val Lys Val Glu Phe Ala Glu Arg Glu Gln Lys
   305 310 315 320
   Ile Leu Ser Asp Leu Lys Gln Tyr Met His Glu Gly Glu Gln Val Pro
   325 330 335
   Ala Asp Trp Lys Ser Asp Arg Ala His Pro Leu Glu Ile Val Lys Glu
   340 345 350
   Leu Arg Asn Ala Val Asp Asp His Val Thr Val Thr Cys Asp Ile Gly
   355 360 365
   Ser His Ser Ile Trp Met Ser Arg Tyr Phe Arg Ser Tyr Glu Pro Leu
   370 375 380
   Thr Leu Met Ile Ser Asn Gly Met Gln Thr Leu Gly Val Ala Leu Pro
   385 390 395 400
   Trp Ala Ile Gly Ala Ser Leu Val Lys Pro Gly Glu Lys Val Val Ser
   405 410 415
   Val Ser Gly Asp Gly Gly Phe Leu Phe Ser Ala Met Glu Leu Glu Thr
   420 425 430
   Ala Val Arg Leu Lys Ala Pro Ile Val His Ile Val Trp Asn Asp Ser
   435 440 445
   Thr Tyr Asp Met Val His Phe Gln Gln Leu Lys Lys Tyr Asn Arg Thr
   450 455 460
   Ser Ala Val Asp Phe Gly Asn Ile Asp Ile Val Lys Tyr Ala Glu Ser
   465 470 475 480
   Phe Gly Ala Thr Ala Leu Arg Val Glu Ser Pro Asp Gln Leu Ala Asp
   485 490 495
   Val Leu Arg Gln Gly Met Asn Ala Glu Gly Pro Val Ile Ile Asp Val
   500 505 510
   Pro Val Asp Tyr Ser Asp Asn Ile Asn Leu Ala Ser Asp Lys Leu Pro
   515 520 525
   Lys Glu Phe Gly Glu Leu Met Lys Thr Lys Ala Leu
   530 535 540
<210> 88
   <211> 23
   <212> DNA
   <213> Artificial Sequence
<220>
   <223> Description of Artificial Sequence: ribosome binding site
<220>
   <221> misc_feature
   <222> 14-20
   <223> n = a, c, g, or t
<400> 88
   agaaaggagg tgannnnnnn atg 23
<210> 89
   <211> 7
   <212> PRT
   <213> Artificial Sequence
<220>
   <223> Description of Artificial Sequence: PanC C terminus
<400> 89
   Ile Arg Glu Met Glu Arg Ile
   1 5
<210> 90
   <211> 5
   <212> PRT
   <213> Artificial Sequence
<220>
   <223> Description of Artificial Sequence: PanC C terminus
<400> 90
   Ile Arg Glu Arg Arg
   1 5
<210> 91
   <211> 7
   <212> PRT
   <213> Artificial Sequence
<220>
   <223> Description of Artificial Sequence: PanC C terminus
<400> 91
   Ile Arg Arg Lys Glu Val Asn
   1 5
<210> 92
   <211> 6688
   <212> DNA
   <213> Artificial Sequence
<220>
   <223> Description of Artificial Sequence: Recombinant pAN336 plasmid
<400> 92
   tgcgccgcta cagggcgcgt ccattcgcca ttcaggctgc gcaactgttg ggaagggcga 60
   tcggtgcggg cctcttcgct attacgccag ctggcgaaag ggggatgtgc tgcaaggcga 120
   ttaagttggg taacgccagg gttttcccag tcacgacgtt gtaaaacgac ggccagtgaa 180
   ttgtaatacg actcactata gggcgaattg ggcccgacgt cgcatgcacc aggcttctca 240
   ggcgctgact tagaaaacct cttgaatgaa gctgcgcttg tagcggctcg tcaaaacaag 300
   aaaaaaatcg atgcgcgtga tattgacgaa gcgacggacc gtgtaattgc cggacccgct 360
   aagaagagcc gcgttatctc caagaaagaa cgcaatatcg tggcttatca cgaaggcgga 420
   cacaccgtta tcggtctcgt tttagatgag gcagatatgg ttcataaagt aacgattgtt 480
   cctcggggcc aggctggcgg ttatgctgtt atgctgccaa gagaagaccg ttatttccaa 540
   acaaagccgg agctgcttga taaaattgtc ggcctcttgg gcggacgtgt tgctgaagag 600
   attatcttcg gtgaagtcag cacaggggcg cacaatgact tccagcgtgc gacgaatatt 660
   gcaagacgaa tggttacaga attcggtatg tcagaaaaac tgggaccgtt gcaatttgga 720
   cagtctcagg gcggtcaggt attcttaggc cgtgatttca acaacgaaca gaactacagt 780
   gatcaaatcg cttacgaaat tgatcaggaa attcagcgca tcatcaaaga atgttatgag 840
   cgtgcgaaac aaatcctgac tgaaaatcgt gacaagcttg aattgattgc ccaaacgctt 900
   ctgaaagttg aaacgcttga cgctgaacaa atcaaacacc ttatcgatca tggaacatta 960
   cctgagcgta atttctcaga tgatgaaaag aacgatgatg tgaaagtaaa cattctgaca 1020
   aaaacagaag aaaagaaaga cgatacgaaa gagtaattcg ctttctttct aaaaaaactg 1080
   ccggctgacg ctggcagttt ttttatgtaa atgattggct cagctgcggc ttttacaatc 1140
   atccaattct ggtatcgatt tgtttacaaa tgagccgctg atcgtgtatg gtattgtaga 1200
   atgtttgtaa aaagtaaagt agagaaacta ttcaaaagtg gtgatagagg ttgttactgg 1260
   ttatcgatgt ggggaacacc ctgcagctcg agtgaaatac cgcacagatg cgtaaggaga 1320
   aaataccgca tcaggcgata aacccagcga accatttgag gtgataggta agattatacc 1380
   gaggtatgaa aacgagaatt ggacctttac agaattactc tatgaagcgc catatttaaa 1440
   aagctaccaa gacgaagagg atgaagagga tgaggaggca gattgccttg aatatattga 1500
   caatactgat aagataatat atcttttata tagaagatat cgccgtatgt aaggatttca 1560
   gggggcaagg cataggcagc gcgcttatca atatatctat agaatgggca aagcataaaa 1620
   acttgcatgg actaatgctt gaaacccagg acaataacct tatagcttgt aaattctatc 1680
   ataattgtgg tttcaaaatc ggctccgtcg atactatgtt atacgccaac tttcaaaaca 1740
   actttgaaaa agctgttttc tggtatttaa ggttttagaa tgcaaggaac agtgaattgg 1800
   agttcgtctt gttataatta gcttcttggg gtatctttaa atactgtaga aaagaggaag 1860
   gaaataataa atggctaaaa tgagaatatc accggaattg aaaaaactga tcgaaaaata 1920
   ccgctgcgta aaagatacgg aaggaatgtc tcctgctaag gtatataagc tggtgggaga 1980
   aaatgaaaac ctatatttaa aaatgacgga cagccggtat aaagggacca cctatgatgt 2040
   ggaacgggaa aaggacatga tgctatggct ggaaggaaag ctgcctgttc caaaggtcct 2100
   gcactttgaa cggcatgatg gctggagcaa tctgctcatg agtgaggccg atggcgtcct 2160
   ttgctcggaa gagtatgaag atgaacaaag ccctgaaaag attatcgagc tgtatgcgga 2220
   gtgcatcagg ctctttcact ccatcgacat atcggattgt ccctatacga atagcttaga 2280
   cagccgctta gccgaattgg attacttact gaataacgat ctggccgatg tggattgcga 2340
   aaactgggaa gaagacactc catttaaaga tccgcgcgag ctgtatgatt ttttaaagac 2400
   ggaaaagccc gaagaggaac ttgtcttttc ccacggcgac ctgggagaca gcaacatctt 2460
   tgtgaaagat ggcaaagtaa gtggctttat tgatcttggg agaagcggca gggcggacaa 2520
   gtggtatgac attgccttct gcgtccggtc gatcagggag gatatcgggg aagaacagta 2580
   tgtcgagcta ttttttgact tactggggat caagcctgat tgggagaaaa taaaatatta 2640
   tattttactg gatgaattgt tttagtacct agatttagat gtctaaaaag ctttaactac 2700
   aagcttttta gacatctaat cttttctgaa gtacatccgc aactgtccat actctgatgt 2760
   tttatatctt ttctaaaagt tcgctagata ggggtcccga gcgcctacga ggaatttgta 2820
   tcgccattcg ccattcaggc tgcgcaactg ttgggaaggg cgatcggtgc ggtcgactgg 2880
   caggcaaaac aggacccaag gtcattgcga caggaggcct ggcgccgctc attgcgaacg 2940
   aatcagattg tatagacatc gttgatccat tcttaaccct aaaagggctg gaattgattt 3000
   atgaaagaaa ccgcgtagga agtgtatagg aggtttagta atggattatt tagtaaaagc 3060
   acttgcgtat gacggaaaag ttcgggctta tgcagcgaga acgactgata tggtaaatga 3120
   ggggcagaga cgccatggta cgtggccgac agcatccgct gcactaggcc gtacaatgac 3180
   agcttcactt atgctcggcg ctatgctgaa gggcgatgat aagctgaccg tgaaaatcga 3240
   gggcggaggt ccgatcggag ctattgtagc tgatgccaat gccaaaggag aagtcagagc 3300
   ctatgtctct aacccgcaag ttcattttga tttaaatgaa caaggtaagc ttgatgtcag 3360
   acgtgcggtt ggaacaaacg gaacgttaag tgtcgtaaaa gatttaggtt tgcgcgagtt 3420
   cttcacagga caagtagaaa tcgtttcagg agaattagga gatgatttta cttactatct 3480
   tgtgtcatct gagcaggttc cttcatcagt gggcgtaggt gtgctcgtaa atcctgacaa 3540
   taccattctt gcggcagggg gctttattat tcagctgatg ccgggaacag atgatgaaac 3600
   aatcacaaaa attgaacagc gtctatctca agtagagccg atttctaagc tcatccaaaa 3660
   agggctgaca ccagaagaaa ttttagaaga agtcctaggc gagaaacctg agattttgga 3720
   aacgatgcct gtcagattcc attgcccttg ttcaaaagaa cggttcgaaa cagccatttt 3780
   aggactaggc aaaaaagaaa ttcaagatat gatagaagaa gatggacaag ccgaagcagt 3840
   atgccatttt tgtaatgaaa agtacttatt tacaaaagaa gagctggaag ggcttcgtga 3900
   ccaaactacc cgctaagctc tttagcgggt ttttaatttg agaaaagggg ctgaaagcag 3960
   gtttgaaatc aagaacaatc tggacgcgtt ggatgcatag cttgagtatt ctatagtgtc 4020
   acctaaatag cttggcgtaa tcatggtcat agctgtttcc tgtgtgaaat tgttatccgc 4080
   tcacaattcc acacaacata cgagccggaa gcataaagtg taaagcctgg ggtgcctaat 4140
   gagtgagcta actcacatta attgcgttgc gctcactgcc cgctttccag tcgggaaacc 4200
   tgtcgtgcca gctgcattaa tgaatcggcc aacgcgcggg gagaggcggt ttgcgtattg 4260
   ggcgctcttc cgcttcctcg ctcactgact cgctgcgctc ggtcgttcgg ctgcggcgag 4320
   cggtatcagc tcactcaaag gcggtaatac ggttatccac agaatcaggg gataacgcag 4380
   gaaagaacat gtgagcaaaa ggccagcaaa aggccaggaa ccgtaaaaag gccgcgttgc 4440
   tggcgttttt cgataggctc cgcccccctg acgagcatca caaaaatcga cgctcaagtc 4500
   agaggtggcg aaacccgaca ggactataaa gataccaggc gtttccccct ggaagctccc 4560
   tcgtgcgctc tcctgttccg accctgccgc ttaccggata cctgtccgcc tttctccctt 4620
   cgggaagcgt ggcgctttct catagctcac gctgtaggta tctcagttcg gtgtaggtcg 4680
   ttcgctccaa gctgggctgt gtgcacgaac cccccgttca gcccgaccgc tgcgccttat 4740
   ccggtaacta tcgtcttgag tccaacccgg taagacacga cttatcgcca ctggcagcag 4800
   ccactggtaa caggattagc agagcgaggt atgtaggcgg tgctacagag ttcttgaagt 4860
   ggtggcctaa ctacggctac actagaagga cagtatttgg tatctgcgct ctgctgaagc 4920
   cagttacctt cggaaaaaga gttggtagct cttgatccgg caaacaaacc accgctggta 4980
   gcggtggttt ttttgtttgc aagcagcaga ttacgcgcag aaaaaaagga tctcaagaag 5040
   atcctttgat cttttctacg gggtctgacg ctcagtggaa cgaaaactca cgttaaggga 5100
   ttttggtcat gagattatca aaaaggatct tcacctagat ccttttaaat taaaaatgaa 5160
   gttttaaatc aatctaaagt atatatgagt aaacttggtc tgacagttac caatgcttaa 5220
   tcagtgaggc acctatctca gcgatctgtc tatttcgttc atccatagtt gcctgactcc 5280
   ccgtcgtgta gataactacg atacgggagg gcttaccatc tggccccagt gctgcaatga 5340
   taccgcgaga cccacgctca ccggctccag atttatcagc aataaaccag ccagccggaa 5400
   gggccgagcg cagaagtggt cctgcaactt tatccgcctc catccagtct attaattgtt 5460
   gccgggaagc tagagtaagt agttcgccag ttaatagttt gcgcaacgtt gttggcattg 5520
   ctacaggcat cgtggtgtca cgctcgtcgt ttggtatggc ttcattcagc tccggttccc 5580
   aacgatcaag gcgagttaca tgatccccca tgttgtgcaa aaaagcggtt agctccttcg 5640
   gtcctccgat cgttgtcaga agtaagttgg ccgcagtgtt atcactcatg gttatggcag 5700
   cactgcataa ttctcttact gtcatgccat ccgtaagatg cttttctgtg actggtgagt 5760
   actcaaccaa gtcattctga gaataccgcg cccggcgacc gagttgctct tgcccggcgt 5820
   caatacggga taatagtgta tgacatagca gaactttaaa agtgctcatc attggaaaac 5880
   gttcttcggg gcgaaaactc tcaaggatct taccgctgtt gagatccagt tcgatgtaac 5940
   ccactcgtgc acccaactga tcttcagcat cttttacttt caccagcgtt tctgggtgag 6000
   caaaaacagg aaggcaaaat gccgcaaaaa agggaataag ggcgacacgg aaatgttgaa 6060
   tactcatact cttccttttt caatattatt gaagcattta tcagggttat tgtctcatga 6120
   gcggatacat atttgaatgt atttagaaaa ataaacaaat aggggttccg cgcacatttc 6180
   cccgaaaagt gccacctgta tgcggtgtga aataccgcac agatgcgtaa ggagaaaata 6240
   ccgcatcagg cgaaattgta aacgttaata ttttgttaaa attcgcgtta aatatttgtt 6300
   aaatcagctc attttttaac caataggccg aaatcggcaa aatcccttat aaatcaaaag 6360
   aatagaccga gatagggttg agtgttgttc cagtttggaa caagagtcca ctattaaaga 6420
   acgtggactc caacgtcaaa gggcgaaaaa ccgtctatca gggcgatggc ccactacgtg 6480
   aaccatcacc caaatcaagt tttttgcggt cgaggtgccg taaagctcta aatcggaacc 6540
   ctaaagggag cccccgattt agagcttgac ggggaaagcc ggcgaacgtg gcgagaaagg 6600
   aagggaagaa agcgaaagga gcgggcgcta gggcgctggc aagtgtagcg gtcacgctgc 6660
   gcgtaaccac cacacccgcc gcgcttaa                                   6688
<210> 93
   <211> 8503
   <212> DNA
   <213> Artificial Sequence
<220>
   <223> Description of Artificial Sequence: Recombinant pAN004 plasmid
<400> 93
<210> 94
   <211> 7381
   <212> DNA
   <213> Artificial Sequence
<220>
   <223> Description of Artificial Sequence:recombinant pAN006 plasmid
<400> 94
   ttgcggccgc ttcgaaagct gtaatataaa aaccttcttc aactaacggg gcaggttagt 60
   gacattagaa aaccgactgt aaaaagtaca gtcggcatta tctcatatta taaaagccag 120
   tcattaggcc tatctgacaa ttcctgaata gagttcataa acaatcctgc atgataacca 180
   tcacaaacag aatgatgtac ctgtaaagat agcggtaaat atattgaatt acctttatta 240
   atgaattttc ctgctgtaat aatgggtaga aggtaattac tattattatt gatatttaag 300
   ttaaacccag taaatgaagt ccatggaata atagaaagag aaaaagcatt ttcaggtata 360
   ggtgttttgg gaaacaattt ccccgaacca ttatatttct ctacatcaga aaggtataaa 420
   tcataaaact ctttgaagtc attctttaca ggagtccaaa taccagagaa tgttttagat 480
   acaccatcaa aaattgtata aagtggctct aacttatccc aataacctaa ctctccgtcg 540
   ctattgtaac cagttctaaa agctgtattt gagtttatca cccttgtcac taagaaaata 600
   aatgcagggt aaaatttata tccttcttgt tttatgtttc ggtataaaac actaatatca 660
   atttctgtgg ttatactaaa agtcgtttgt tggttcaaat aatgattaaa tatctctttt 720
   ctcttccaat tgtctaaatc aattttatta aagttcattt gatatgcctc ctaaattttt 780
   atctaaagtg aatttaggag gcttacttgt ctgctttctt cattagaatc aatccttttt 840
   taaaagtcaa tattactgta acataaatat atattttaaa aatatcccac tttatccaat 900
   tttcgtttgt tgaactaatg ggtgctttag ttgaagaata aagaccacat taaaaaatgt 960
   ggtcttttgt gtttttttaa aggatttgag cgtagcgaaa aatccttttc tttcttatct 1020
   tgataataag ggtaactatt gaattcggta ccaagagttt gtagaaacgc aaaaaggcca 1080
   tccgtcagga tggccttctg cttaatttga tgcctggcag tttatggcgg gcgtcctgcc 1140
   cgccaccctc cgggccgttg cttcgcaacg ttcaaatccg ctcccggcgg atttgtccta 1200
   ctcaggagag cgttcaccga caaacaacag ataaaacgaa aggcccagtc tttcgactga 1260
   gcctttcgtt ttatttgatg cctggcagtt ccctactctc gcatggggag accccacact 1320
   accatcggcg ctacggcgtt tcacttctga gttcggcatg gggtcaggtg ggaccaccgc 1380
   gctactgccg ccaggcaaat tctgttttat cagaccgctt ctgcgttctg atttaatctg 1440
   tatcaggctg aaaatcttct ctcatccgcc aaaacaggat cctacggaaa tggagcggca 1500
   aaaccgtttt actctcaaaa tcttaaaaga aaacccccga taaagggggc ttttcttcta 1560
   caaaattgta cgggctggtt cgttccccag catttgttca attttgtttt gatcattcag 1620
   aacagccact ttcggctcat ggcttgccgc ttcttgatca gacatcattt tgtaggaaat 1680
   aataatgacc ttatctcctt cctgcacaag gcgtgcggct gcaccgttta agcatatgac 1740
   gccgcttccc cgtttaccag gaataatata cgtttcaaga cgtgctccat tattattatt 1800
   cacaatttgt actttttcat taggaagcat tcccacagca tcaatgagat cttcatcaat 1860
   tgtaatgctt cccacatagt tcaggtttgc ttccgtaaca gttgccctgt gaagtttgcc 1920
   gctcatcatt gttcgataca tattatattc tctccatttc tcgaatatca ataatgatat 1980
   tatctattaa acgcgctttt gaaaaagcaa ctgcaacagc gagaatcatc tttccagcaa 2040
   tttcattcac aggctcgagt tccggatagg aataaagctc tacatagtct atggttccgc 2100
   tagtcgtttc aatgatatct tttgcagctt ttatcaccgc ttcaggatct ctttcaccgg 2160
   cttggacaag ttccgcactt gtttgaaggg cccgatacag cttaggcgct tcttttcttt 2220
   cctcagctgt taagtataca ttgcgagagc ttttggctaa gccgtcttcc tctctgaccg 2280
   tatcgacagg aaccaattca atatccatga agaagtcgct gattaaccca tcaacaacag 2340
   ctacctgctg cgcatctttt aaaccgaaat aggcacgagt cggcttgact agattgaaaa 2400
   gcttcgtcag tacgatcgcg accccgtcaa aatgtccttc tcttgagcgc ccgcataaca 2460
   cgtctgtgcg tctttctaca tgaatcgtga cattcttttc accgggatac atatcatgag 2520
   catctggcgt aaaaagaata tcgactccgg cgttttctgc aagagctgca tcccgctcaa 2580
   tatcgcgcgg atatgcttca aaatcttcat tagggccgaa ttgtgcagga ttcacaaaaa 2640
   tactcataat aacggcgtcg ttttcttgtc ttgctttgtc tgctaaggtt aaatgcccct 2700
   catgcagaaa ccccatcgtc ggaacaaatc cgattgactt gccctctgaa tggtattgtt 2760
   ttatggcttc tttcagctgt gaaatatcag taatctgtct catcttattt tcccccgtac 2820
   aagccgtcaa gcactgtctg gttcatttga aaggaatgct tttgttcagg gaaagcacga 2880
   tgtcttacat cctgaacata tccgctgatt gctgtttcga tggtttcatc aatgcgcgta 2940
   tattgcttta caaatttagg tgttctctca acaccgtggc cgataatatc atgataaacg 3000
   agaacttgtc cgtccgcttt cacaccagcc ccgattccaa tgaccggtat gcttagcgtc 3060
   tcggcaattt tggctgtgag ttctgccggc acacattcca gcacaagcat catagctcct 3120
   gcttcttcgc attttatact gtcttctatt aattttttgg cgctttgttc gtctttgccc 3180
   tgtactttat agccgcccag tacgccgact gactgcggtg tcaaacctaa gtgactgact 3240
   actggaatgc ctccaagcgt caatgcgcga atggattcaa acacgccttc tccgccctca 3300
   agcttcagtg cgtcagctcc gctttcctga acgatagccg ctgcattttt cagcgtatct 3360
   tccttagaca ggtgataaga cataaacggc atatctgtca caataaaggt attcggcgca 3420
   ccccttttaa cggcttttgt atgatggatc atgtccgcaa ctgtcacacc gacagttgaa 3480
   tcaaggccga ggacgaccat tccaagtgaa tcaccgacta aaatcatgtc aactcccgct 3540
   tgttcagcaa gtttagctgc cggataatca taagcggtca gcatgacaat cggttcttca 3600
   gactccttca tttttagaaa atccagtttt gttttcatgt tttctcctcc tctagagcgt 3660
   cctgctgttg ttaagattat tataccacac cttgtagata aagtcaacaa ctttttgcaa 3720
   aatttttcag gaattttagc agaggttgtt ctggatgtag aacaaaacat ctttccgctc 3780
   ttgtgctgtt aggatatctt tcttggaagc taggtaggcc tcgagttatg gcagttggtt 3840
   aaaaggaaac aaaaagaccg ttttcacaca aaacggtctt tttcgatttc tttttacagt 3900
   cacagccact tttgcaaaaa ccggacagct tcatgcctta taactgctgt ttcggtcgac 3960
   ctgcaggcat gcaagcttcg cgaagcggcc gccgacgcga ggctggatgg ccttccccat 4020
   tatgattctt ctcgcttccg gcggcatcgg gatgcccgcg ttgcaggcca tgctgtccag 4080
   gcaggtagat gacgaccatc agggacagct tcaaggatcg ctcgcggctc ttaccagcct 4140
   aacttcgatc actggaccgc tgatcgtcac ggcgatttat gccgcctcgg cgagcacatg 4200
   gaacgggttg gcatggattg taggcgccgc cctatacctt gtctgcctcc ccgcgttgcg 4260
   tcgcggtgca tggagccggg ccacctcgac ctgaatggaa gccggcggca cctcgctaac 4320
   ggattcacca ctccaagaat tggagccaat caattcttgc ggagaactgt gaatgcgcaa 4380
   accaaccctt ggcagaacat atccatcgcg tccgccatct ccagcagccg cacgcggcgc 4440
   atctcgggca gcgttgggtc ctggccacgg gtgcgcatga tcgtgctcct gtcgttgagg 4500
   acccggctag gctggcgggg ttgccttact ggttagcaga atgaatcacc gatacgcgag 4560
   cgaacgtgaa gcgactgctg ctgcaaaacg tctgcgacct gagcaacaac atgaatggtc 4620
   ttcggtttcc gtgtttcgta aagtctggaa acgcggaagt cagcgccctg caccattatg 4680
   ttccggatct gcatcgcagg atgctgctgg ctaccctgtg gaacacctac atctgtatta 4740
   acgaagcgct ggcattgacc ctgagtgatt tttctctggt cccgccgcat ccataccgcc 4800
   agttgtttac cctcacaacg ttccagtaac cgggcatgtt catcatcagt aacccgtatc 4860
   gtgagcatcc tctctcgttt catcggtatc attaccccca tgaacagaaa ttccccctta 4920
   cacggaggca tcaagtgacc aaacaggaaa aaaccgccct taacatggcc cgctttatca 4980
   gaagccagac attaacgctt ctggagaaac tcaacgagct ggacgcggat gaacaggcag 5040
   acatctgtga atcgcttcac gaccacgctg atgagcttta ccgcagctgc ctcgcgcgtt 5100
   tcggtgatga cggtgaaaac ctctgacaca tgcagctccc ggagacggtc acagcttgtc 5160
   tgtaagcgga tgccgggagc agacaagccc gtcagggcgc gtcagcgggt gttggcgggt 5220
   gtcggggcgc agccatgacc cagtcacgta gcgatagcgg agtgtatact ggcttaacta 5280
   tgcggcatca gagcagattg tactgagagt gcaccatatg cggtgtgaaa taccgcacag 5340
   atgcgtaagg agaaaatacc gcatcaggcg ctcttccgct tcctcgctca ctgactcgct 5400
   gcgctcggtc gttcggctgc ggcgagcggt atcagctcac tcaaaggcgg taatacggtt 5460
   atccacagaa tcaggggata acgcaggaaa gaacatgtga gcaaaaggcc agcaaaaggc 5520
   caggaaccgt aaaaaggccg cgttgctggc gtttttccat aggctccgcc cccctgacga 5580
   gcatcacaaa aatcgacgct caagtcagag gtggcgaaac ccgacaggac tataaagata 5640
   ccaggcgttt ccccctggaa gctccctcgt gcgctctcct gttccgaccc tgccgcttac 5700
   cggatacctg tccgcctttc tcccttcggg aagcgtggcg ctttctcata gctcacgctg 5760
   taggtatctc agttcggtgt aggtcgttcg ctccaagctg ggctgtgtgc acgaaccccc 5820
   cgttcagccc gaccgctgcg ccttatccgg taactatcgt cttgagtcca acccggtaag 5880
   acacgactta tcgccactgg cagcagccac tggtaacagg attagcagag cgaggtatgt 5940
   aggcggtgct acagagttct tgaagtggtg gcctaactac ggctacacta gaaggacagt 6000
   atttggtatc tgcgctctgc tgaagccagt taccttcgga aaaagagttg gtagctcttg 6060
   atccggcaaa caaaccaccg ctggtagcgg tggttttttt gtttgcaagc agcagattac 6120
   gcgcagaaaa aaaggatctc aagaagatcc tttgatcttt tctacggggt ctgacgctca 6180
   gtggaacgaa aactcacgtt aagggatttt ggtcatgaga ttatcaaaaa ggatcttcac 6240
   ctagatcctt ttaaattaaa aatgaagttt taaatcaatc taaagtatat atgagtaaac 6300
   ttggtctgac agttaccaat gcttaatcag tgaggcacct atctcagcga tctgtctatt 6360
   tcgttcatcc atagttgcct gactccccgt cgtgtagata actacgatac gggagggctt 6420
   accatctggc cccagtgctg caatgatacc gcgagaccca cgctcaccgg ctccagattt 6480
   atcagcaata aaccagccag ccggaagggc cgagcgcaga agtggtcctg caactttatc 6540
   cgcctccatc cagtctatta attgttgccg ggaagctaga gtaagtagtt cgccagttaa 6600
   tagtttgcgc aacgttgttg ccattgctgc aggcatcgtg gtgtcacgct cgtcgtttgg 6660
   tatggcttca ttcagctccg gttcccaacg atcaaggcga gttacatgat cccccatgtt 6720
   gtgcaaaaaa gcggttagct ccttcggtcc tccgatcgtt gtcagaagta agttggccgc 6780
   agtgttatca ctcatggtta tggcagcact gcataattct cttactgtca tgccatccgt 6840
   aagatgcttt tctgtgactg gtgagtactc aaccaagtca ttctgagaat agtgtatgcg 6900
   gcgaccgagt tgctcttgcc cggcgtcaat acgggataat accgcgccac atagcagaac 6960
   tttaaaagtg ctcatcattg gaaaacgttc ttcggggcga aaactctcaa ggatcttacc 7020
   gctgttgaga tccagttcga tgtaacccac tcgtgcaccc aactgatctt cagcatcttt 7080
   tactttcacc agcgtttctg ggtgagcaaa aacaggaagg caaaatgccg caaaaaaggg 7140
   aataagggcg acacggaaat gttgaatact catactcttc ctttttcaat attattgaag 7200
   catttatcag ggttattgtc tcatgagcgg atacatattt gaatgtattt agaaaaataa 7260
   acaaataggg gttccgcgca catttccccg aaaagtgcca cctgacgtct aagaaaccat 7320
   tattatcatg acattaacct ataaaaatag gcgtatcacg aggccctttc gtcttcaaga 7380
   a 7381
<210> 95
   <211> 36
   <212> DNA
   <213> Artificial Sequence
<220>
   <223> Description of artificial sequence: synthetic oligonucleotide
<400> 95
   ctagattaga aaggaggatt taaatatgta tcgtac 36
<210> 96
   <211> 32
   <212> DNA
   <213> Artificial Sequence
<220>
   <223> Description of artificial sequence: synthetic oligonucleotide
<400> 96
   taatctttcc tcctaaattt atacatagca tg 32
<210> 97
   <211> 36
   <212> DNA
   <213> Artificial Sequence
<220>
   <223> Description of artificial sequence: synthetic oligonucleotide
<400> 97
   ctagattaga aaggaggttt aattaatgta tcgtac 36
<210> 98
   <211> 32
   <212> DNA
   <213> Artificial Sequence
<220>

## Claims

1. A method of producing a panto-compound comprising culturing a manipulated microorganism which overexpresses
(i) at least Bacillus pantothenate synthetase; and further
(ii) ketopantoate hydroxymethyltransferase, and aspartate-alpha-decarboxylase
at a level greater than that expressed prior to manipulation of said microorganism or in a comparable microorganism which has not been manipulated, under conditions such that the panto-compound is produced, wherein the method is without supplementation of beta-alanine.

2. The method of claim 1, wherein the microorganism further overexpresses ketopantoate reductase.

## Patentansprüche

1. Verfahren zur Herstellung einer Panto-Verbindung, umfassend Kultivieren eines manipulierten Mikroorganismus, der
(i) wenigstens Bacillus-Pantothenat-Synthetase; und ferner
(ii) Ketopantoat-Hydroxymethyltransferase und Aspartat-alpha-Decarboxylase
auf einem höheren Niveau als dem vor Manipulation des Mikroorganismus oder in einem vergleichbaren Mikroorganismus, der nicht manipuliert wurde, exprimierten überexprimiert, unter solchen Bedingungen, dass die Panto-Verbindung produziert wird, wobei bei dem Verfahren keine Beta-Alanin-Supplementierung erfolgt.

2. Verfahren nach Anspruch 1, wobei der Mikroorganismus ferner Ketopantoat-Reduktase überexprimiert.

## Revendications

1. Méthode de production d'un panto-composé, comprenant la culture d'un microorganisme manipulé qui surexprime
(i) au moins une pantothénate synthétase de *Bacillus ;* et en outre
(ii) une cétopantoate hydroxyméthyltransférase, et une aspartate-alpha-décarboxylase,
à un taux supérieur à celui exprimé préalablement à la manipulation dudit microorganisme ou dans un microorganisme comparable n'ayant pas été manipulé, dans des conditions telles que le panto-composé soit produit, où la méthode est effectuée sans complémentation par de la bêta-alanine.

2. Méthode selon la revendication 1, dans laquelle le microorganisme surexprime en outre la cétopantoate réductase.
